(19) Europäisches Patentamt
European Patent Office
Office européen des brevets

(11) Numéro de publication: **0 169 775**
**B1**

# (12) FASCICULE DE BREVET EUROPEEN

(45) Date de publication du fascicule du brevet: 24.05.89

(51) Int. Cl.⁴: C 07 H 21/00, A 61 K 31/70

(21) Numéro de dépôt: 85401434.7

(22) Date de dépôt: 12.07.85

(54) Nouveaux oligonucléotides, leur procédé de préparation et leurs applications comme médiateurs dans le développement des effets des interférons.

(30) Priorité: 19.07.84 FR 8411469

(43) Date de publication de la demande: 29.01.86 Bulletin 86/5

(45) Mention de la délivrance du brevet: 24.05.89 Bulletin 89/21

(84) Etats contractants désignés: AT BE CH DE GB IT LI LU NL SE

(56) Documents cités:
THE JOURNAL OF BIOLOGICAL CHEMISTRY, vol. 256, no. 7, 10 avril 1981, pages 3253-3257, US; C. BAGLIONI et al.: "Analogs of (2'-5') oligo (A). Endonuclease activation and inhibition of protein synthesis in intact cells"
TETRAHEDRON LETTERS, vol. 22, no. 47, 1981, pages 4699-4702, Pergamon Press Ltd., GB; G. GOSSELIN et al.: "Synthèse du trimère de la bêta-D-xylofurannosyl-9 adénine à liaisons internucléotidiques 2'-5'"
EUR. J. BIOCHEM., vol. 132, 1983, pages 77-84, FEBS; M.C. HAUGH et al.: "Analogues and analogue inhibitors of ppp (A2'p)nA - Their stability and biological activity"
JOURNAL OF MEDICINAL CHEMISTRY, vol. 26, no. 11, novembre 1983, pages 1674-1678, American Chemical Society, US; P.F. TORRENCE: "Structure-activity relationships for and potentiation of the antimitogenic activity of 2-5A core derived from 2-5A, a mediator of interferon action"
BIOCHEMISTRY, vol. 22, 1983, pages 2127-2135, American Chemical Society, US; B.G. HUGHES et al.: "2', 5'-oligoadenylates and related 2',5'-oligonucleotide analogues. 2. Effect on cellular proliferation, protein synthesis, and endoribonuclease activity"

(73) Titulaire: Etablissement Public dit: CENTRE NATIONAL DE LA RECHERCHE SCIENTIFIQUE (CNRS), 15, Quai Anatole France, F-75007 Paris (FR)

(72) Inventeur: Lebleu, Bernard Résidence le Jardin aux Fontaines, B2 140 Rue Pioch de Boutonnet, F-34000 Montpellier (FR)
Inventeur: Bayard, Bernard, 11, rue des Marronniers, F-34170 Castelnau Le Lez (FR)

(74) Mandataire: Gutmann, Ernest et al, S.C. Ernest Gutmann - Yves Plasseraud 67, boulevard Haussmann, F-75008 Paris (FR)

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



ACTORUM AG

## Description

L'invention a pour objet de nouveaux oligonucléotides, leur procédé de préparation et leur application biologique, comme médiateurs dans le développement de l'action des interférons, notamment dans le développement d'une partie au moins de l'action antivirale des interférons.

On sait que les interférons constituent une famille de protéines caractérisées notamment par leurs propriétés antivirales.

On a observé que l'effet antiviral des interférons est médiatisé par la synthèse de protéines particulières. Des essais spécifiques ont permis d'identifier la fonction de deux d'entre elles, qui sont toutes les deux des enzymes (Baglioni C., 1979, Interferon induced enzymatic activities and their role in the antiviral state, Cell 17, 255-264).

L'une d'entre elles est une oligonucléotide polymérase (2-5A synthétase). Cette oligonucléotide de polymérase catalyse, après activation par les ARN bicaténaires et à partir d'ATP, la synthèse d'une famille d'oligonucléotides.

Ces oligonucléotides sont des chaînes courtes d'adénosines reliées par des liaisons phosphodiester 2'→5' (Kerr I.M. et Brown R.E., 1978, pppA2'-p5'A2'p5'A: An inhibitor of protein synthesis synthesized with an enzyme fraction from interferon treated cells, PNAS 75, 256-260) dont la formule générale peut être représentée par pppA(2'p5'A)n. Ces oligonucléotides peuvent être désignés par «oligonucléotides 2'-5'», notamment «oligonadénylates 2'-5'» ou par $(2'-5')(A)_n$. L'un de ces oligoadénylates peut être représenté par la formule suivante:

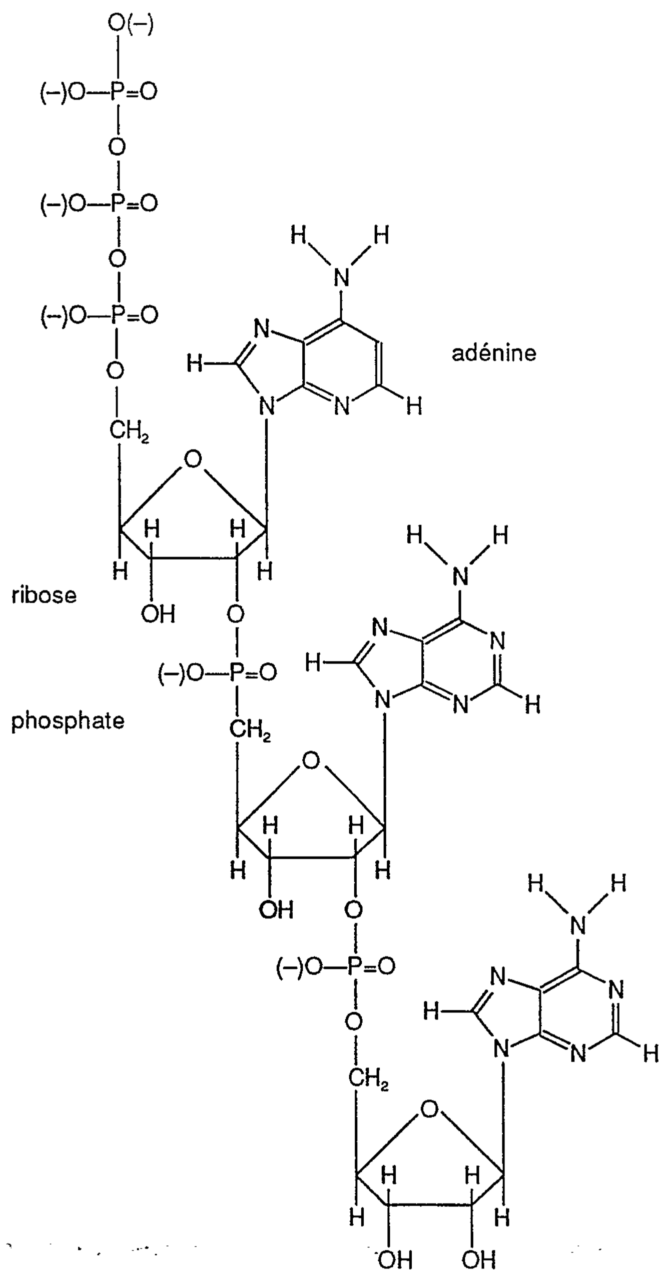

Il est composé de chaînes courtes contenant plusieurs groupes adénosine (adénine + ribose), liés entre eux par des liaisons phosphodiester, comme représenté, et dans laquelle la position en 5′ du noyau d'adénine de l'adénosine terminal est lié à un nombre variable de groupes phosphate (jusqu'à 3 sur l'oligoadénylate 2′→5′ représenté).

Lorsque l'oligoadénylate 2′→5′ est totalement déphosphorylé, c'est-à-dire lorsque la position en 5′ du noyau d'adénine de l'adénosine terminal est libre du susdit nombre variable de groupes phosphate, le composé résultant est désigné par «noyau (2′→5′)A3", qui est une abréviation pour «noyau riboadénylyl (2′→5′) riboadénylyl (2′→5′) riboadénosine».

Les noyaux 2′-5′ correspondant aux oligoadénylates (2′-5′) déphosphorylés sont également appelés «cores».

Dans la suite de la description, on désignera également par «oligoadénylate 2′-5′ non modifiés» les oligoadénylates 2′-5′ induits dans les cellules traitées par de l'interféron.

Il est convenu que l'expression «oligoadénylate 2′→5′» mentionnée ci-dessus et utilisée ci-après désignera également, par commodité de langage, le noyau $(2'-5')(A)_n$ partiellement ou entièrement déphosphorylé.

La découverte de ces oligoadénylates 2′→5′ a révélé une nouvelle classe d'oligonucléotides biologiquement actifs, que l'on suppose présenter un rôle important comme médiateurs de l'action de l'interféron, notamment dans l'activation de l'endoribonucléase L, qui est présente aussi bien dans les cellules traitées par l'interféron que dans celles non traitées, et dans l'inhibition de la synthèse des protéines. Mais les liaisons phosphodiester 2′→5′ de ces adénylates sont rapidement clivées par une enzyme désignée par 2′-phosphodiestérase (cf. la référence de Baglioni mentionnée ci-dessus).

L'endoribonucléase L ainsi que la 2′-phosphodiestérase sont présentes à des taux sensiblement égaux dans les cellules traitées ainsi que dans les cellules non traitées par l'interféron.

Lorsque la cellule est traitée par l'interféron, la concentration d'oligonucléotide 2′-5′ polymérase augmente. L'infection par certains virus de cellules ainsi traitées entraîne la production au site de réplication virale d'ARN bicaténaires activant l'oligonucléotide 2′-5′ polymérase. Il en résulte une augmentation, transitoire et éventuellement localisée au site de replication du virus, de la concentration en oligoadénylate 2′→5′ (Nilsen T.W. et Baglioni C., 1984, Interferon 5, J. Gresser Ed., Academic Press, New York). Ces oligonucléotides activent eux-mêmes en s'y liant spécifiquement l'endoribonucléase L qui dégrade les ARN messagers viraux.

Lorsque l'interféron est enlevé du milieu de culture, l'activité de l'oligonucléotide 2′→5′ polymérase décroît et la cellule perd son état antiviral.

La synthèse des protéines induites par l'interféron est transitoire et, par conséquent, les cellules maintenues dans les cultures de tissu ne maintiennent pas un niveau élevé de ces protéines.

Par ailleurs, les oligoadénylates 2′→5′ induits dans les cellules traitées par l'interféron présentent l'inconvénient d'avoir une stabilité métabolique faible. En effet, les oligoadénylates 2′→5′ non modifiés sont, d'une part, rapidement hydrolysés par une phosphodiestérase spécifique dégradant la molécule de manière processive à partir de son ribose 2′ terminal, d'autre part, sont dégradés sous l'action d'une phosphatase du côté du premier ribose relié au nombre variable de groupes phosphate (Lebleu B. et Content J., 1982, Interferon 3, J. Gresser Ed., Academic Press, New York).

Des recherches ont été entreprises pour trouver des composés analogues aux oligoadénylates 2′→5′ non modifiés et présentant une activité accrue, en comparaison avec l'activité d'oligoadénylates 2′→5′ induits dans les cellules traitées par l'interféron. Parmi ces composés analogues, certains comprennent par exemple un groupe méthylène entre le phosphate en β et le phosphate en γ des groupes phosphate de l'extrémité 5′ et sont désignés par $pCH_2ppA_3$-: (Baglioni C. et coll., 1981. Analogs of (2′-5′)oligo(A). Endonuclease activation and inhibition of protein synthesis in intact cells, The Journal of Biological Chemistry. vol. 256, n° 7, p. 3253-3257).

Différentes recherches ont été effectuées pour synthétiser (par voie enzymatique et/ou chimique) des analogues modifiés des oligoadénylates 2′→5′ induits dans les cellules traitées par l'interféron, qui seraient résistants aux actions de dégradation, sans perdre leur activité biologique.

Parmi ces recherches, on peut citer la synthèse enzymatique à l'aide de la 2-5A synthétase de «cordycépine 2→5A» à partir de 2′déoxyadénosine triphosphate (Doetsch et coll., 1981).

La cordycépine a été considérée comme inhibant la synthèse des protéines en système acellulaire et le composé déphosphorylé correspondant («core» ou «noyau») a été considéré comme bloquant la transformation blastique de lymphocytes humains.

Ces résultats ont cependant été infirmés (Chapekar M.S. et coll., 1983, Biochem, Res. Comm., 115, 137-143) et il semble que les effets observés aient été provoqués par l'accumulation de produits toxiques de dégradation de la cordycépine.

On peut également citer, parmi les recherches effectuées, la synthèse chimique d'un analogue du noyau des oligoadénylates 2′→5′, en série xylose (Imbach J.L. et coll., 1981, Tetrahedron Letters, vol. 22, n° 47, p. 4699-4702), dénommé «xylo 2′-5′A». Cet analogue s'est révélé présenter une stabilité plus importante vis-à-vis des phosphodiestérases que le noyau des oligoadénylates 2′-5′ non modifiés, une activité intéressante vis-à-vis de virus à ADN, tel que l'Herpès, mais pas vis-à-vis de virus à ARN (Eppstein D., et coll., 1983, Nature, 302. 723-724).

On peut aussi mentionner la synthèse chimique et la modification à son extrémité 2′ terminale un oligoadénylate 2′-5′A en un composé appelé «tailed 2′-5′A» dans lequel une chaîne d'hexylamine a été associée à un noyau morpholine, lui-même condensé par un groupe phosphate sur le groupe OH en 2′ du ribose terminal. Ce dérivé est très stable vis-à-vis des phoshodiestérases et active l'endoribonucléase L en système acellulaire (Imai J. et coll., 1982, J. Biochem. Chem., 257, 12739-12741), mais son activité antivirale n'a pas été établie.

Parmi ces recherches, on peut également mentionner la synthèse chimique de dérivés modifiés d'oligoadénylates 2′-5′ tels que les dérivés du 2′-5′A triphosphatés (représenté par la formule pppA2′p5′A2′p5A) dans lesquels les atomes de phosphore en bêta et gamme du groupe triphosphate en 5′ sont séparés par un groupe méthylène.

Une autre modification pour obtenir des oligoadénylates 2′-5′A modifiés concerne le remplacement d'un groupe hydroxyle en 3′ par un groupe $OCH_3$, soit dans l'adénosine terminal, soit dans tous les adénosines (J.A.J. den Hartog et coll., 1981, J. Org. Chem., 46, 2242-2251).

Mais il s'est révélé que ces deux derniers groupes de composés étaient faiblement actifs, voire inactifs et ne présentaient pas une stabilité métabolique satisfaisante (cf. la référence mentionnée ci-dessus et Baglioni et coll., 1981, J. Biol. Chem., 256, 2353-2357).

D'autres analogues, tels que des 5′S-méthylthiophosphorothioates ont été synthétisés, tels que celui désigné par $CH_3Sp(A2'p)_2dA$ dans lequel dA représente le groupe 3′-déoxyadénosine. Certains de ces analogues se sont révélés stables. Mais a priori, les différences apparentes des propriétés de ces analogues ne semblent pas permettre d'envisager leur utilisation sur des cellules humaines à des fins thérapeutiques (Haugh M.C., Cayley P.J. et coll., 1983, Europ. J. Biochem, 132, 77-84).

Des recherches ont également porté sur l'incidence de la modification du ou des groupes phosphate portés par le carbone en 5′ des oligoadénylates 2′-5′, tel que le noyau de l'oligoadénylate (2′-5′) diphosphaté substitué par le groupe phényl et représenté par Phpp5′A2′p5′A, vis-à-vis de l'activité antimitogénique (cf. Torrence et coll. 1983, J. Medicinal Chemistry 26, n° 12, 1674-1678). Les composés préparés dans le cadre de ces recherches se sont révélés présenter une activité antimitogénique, mais on a trouvé que certains d'entre eux n'activent pas l'endoribonucléase L dans un système cellulaire in vitro, ce qui empêche d'établir une corrélation entre l'activité antimitogénique et l'action antivirale.

D'autres oligonucléotides analogues des $(2'-5')(A)_n$ ont été synthétisés par voie enzymatique en remplaçant l'adénosine, notamment par la 8-azaadénosine, la toyocamycine, la sangivamycine, la formycine, la 8-bromoadénosine, la tubercidine et la guanosine. Il s'est révélé que la plupart de ces composés étaient dégradés dans des extraits cellulaires. Seuls des tests d'inhibition de la synthèse des protéines et de la prolifération cellulaire ont été effectués dans des cellules intactes, mais l'activité antivirale n'a pas été établie (B.G. Hugues et R.K. Robins, 1983, Biochemistry, 22, n° 9, 2127-2135).

Aucun des analogues des $(2'-5')(A)_n$ synthétisés à ce jour n'a présenté des propriétés de stabilité – tant vis-à-vis des phosphodiestérases que des phosphatases –, et d'activité biologique suffisantes pour qu'on puisse envisager de les utiliser dans le traitement thérapeutique des affections virales.

La Société Demanderesse a trouvé de nouveaux oligonucléotides ayant une structure différente de celle des oligoadénylates 2′-5′A non modifiés et de ses analogues connus, présentant une activité antivirale semblable à celle de l'interféron, qui sont résistants vis-à-vis de la dégradation par la 2′-phosphodiestérase et par les phosphatases permettant d'envisager leur utilisation dans le traitement d'affections virales, dans la mesure où ils sont associés à des vecteurs appropriés leur permettant de traverser la membrane de la cellule.

L'un des aspects de l'invention est de proposer de nouveaux oligonucléotides qui peuvent être reconnus par l'endoribonucléase L, c'est-à-dire qui peuvent former avec l'endoribonucléase L un complexe actif.

Un autre aspect de l'invention est de proposer de nouveaux oligonucléotides qui ont une résistance accrue vis-à-vis de la dégradation par la 2′-phosphodiestérase.

Un autre aspect de l'invention est de fournir de nouveaux oligonucléotides qui ont une résistance accrue vis-à-vis des phosphatases.

Un autre aspect de l'invention est de fournir de nouveaux oligonucléotides biologiquement actifs, qui présentent notamment une activité antivirale efficace, dans la mesure où ils sont associés à des vecteurs appropriés leur permettant de traverser la membrane cellulaire.

Un des autres aspects de l'invention est de fournir de nouveaux oligonucléotides susceptibles de servir à la préparation de composés biologiquement actifs qui présentent notamment une activité antivirale efficace.

Ces différents aspects sont obtenus par de nouveaux oligonucléotides comprenant une chaîne contenant n unités nucléosidiques, identiques ou différentes, n étant égal ou supérieur à 2, ces unités nucléosidiques étant reliées par des liaisons 2′-5′, qui comprennent un groupe de liaisons contenant au moins un atome de phosphore et dans lesquels:

– la «première unité» nucléosidique de la susdite chaîne est reliée par l'intermédiaire de son carbone en 5′ à un nombre variable de groupes phosphate, et l'un des atomes d'oxygène d'au moins l'un des groupes phosphate, lequel atome d'oxygène relié uniquement au phosphore des groupes phosphate et n'intervenant pas dans la liaison entre deux groupes phosphate, est remplacé par un atome de soufre, de sélénium ou un groupe NH, et/ou l'une au moins des liaisons

entre deux groupes phosphate adjacents comporte un groupe NH ou un atome de soufre; et éventuellement

- la «dernière unité» nucléosidique de la susdite chaîne est reliée, par l'intermédiaire de son atome de carbone en 2':

- soit à un groupe phosphoglycéryl,

- soit à un groupe phosphate, lequel est relié au carbone en 5' d'un «groupe nucléosidique modifié», dans lequel la liaison directe entre le carbone en 2' et 3' a été supprimée et les carbones en 2' et 3' sont respectivement porteurs de groupes aldéhyde ou de groupes alcool, éventuellement estérifiés.

Une unité nucléosidique désigne un composé constitué par un pentose lié à une base purique ou pyrimidique, dans laquelle le pentose peut être sous la forme pyrane ou furane.

Dans la suite de la description, les formules représenteront les pentoses généralement sous la forme furane.

Les unités nucléosidiques sont selon l'invention avantageusement constituées par des adénosines, l'adénosine désigne le composé constitué par du ribose lié à de l'adénine et peut être représenté par la formule:

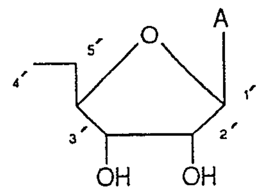

dans laquelle le ribose est sous la forme furane, mais peut être également sous la forme pyrane et dans laquelle A représente l'adénine.

Dans le cadre de l'invention, l'adénine désigne la molécule représentée par la formule suivante:

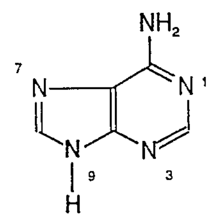

Les unités nucléosidiques selon l'invention peuvent également être constituées par des dérivés d'adénosines, les dérivés d'adénosine désignant le composé constitué par du ribose, lié à un dérivé de l'adénine. Parmi ces dérivés de l'adénine, on peut citer ceux de formule suivante:

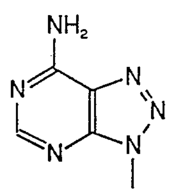

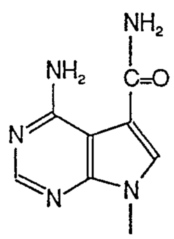

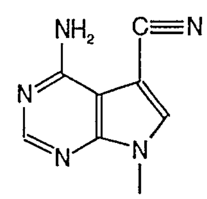

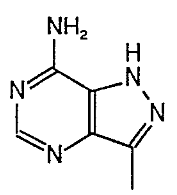

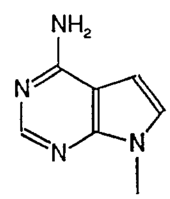

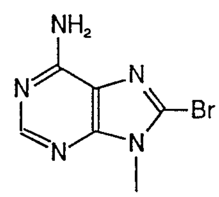

Les dérivés d'adénosine correspondant seront respectivement désignés par 8-azaadénosine, sangivamycine, toyocamycine, formycine, tubercidine, 8-bromoadénosine.

Le nombre d'unités nucléosidiques constituant les oligonucléotides de l'invention n'est pas limité dans les valeurs supérieurs, sous réserve que les oligonucléotides obtenus puissent être associés à un véhicule physiologiquement acceptable.

Ce nombre peut rapidement être limité dans la mesure où l'augmentation de ce nombre et la synthèse correspondante plus difficiles ne seraient pas supportées par une augmentation suffisante de l'activité.

Le nombre d'unités nucléosidiques devrait être choisi de façon à ce que le poids moléculaire soit de préférence compris de 1500 à 5000 daltons.

Dans une classe préférée d'oligonucléotides selon l'invention, la valeur de n n'est pas supérieure à 10, et est de préférence de 7 ou 8.

Les oligonucléotides dans lesquels la valeur de n est 3 ou 4 sont particulièrement préférés.

Dans une classe préférée d'oligonucléotides selon l'invention, la première unité nucléosidique est liée à un ou plusieurs groupes phosphate.

De préférence, le nombre de ces groupes phosphate est de 1 à 3.

Dans une classe préférée de composés de l'invention, la première unité nucléosidique est liée aux groupes phosphate suivants:

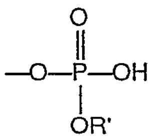 , 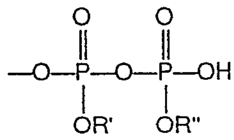 ,

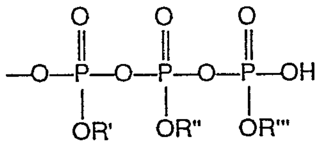 ,

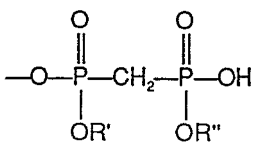

dans lesquelles R′, R″, R‴ représentent, indépendamment les uns des autres:

- un atome d'hydrogène,
- un radical alcoyle ayant de 1 à 4 atomes de carbone, en particulier un méthyle,
- un radical éthyle substitué en bêta par un groupe cyano, aryle ou arylsulfonyle,
- un radical trihalogénoéthyle.

Dans une classe préférée de composés selon l'invention, la liaison 2′→5′ reliant deux unités nucléosidiques et comprenant au moins un atome de phosphore est une liaison phosphodiester, une liaison phosphotriester, une liaison alcoylphosphonate.

La liaison 2′→5′ phosphodiester qui relie deux unités nucléosidiques adjacents dans les oligonucléotides selon l'invention peut être représentée comme suit:

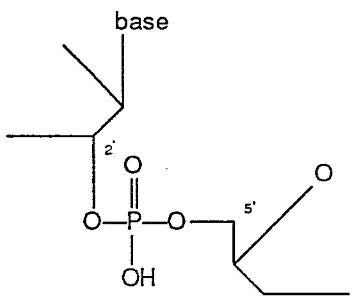

La liaison 2′→5′ phosphotriester qui relie deux unités nucléosidiques adjacents dans les oligonucléotides de l'invention peut être représentée comme suit:

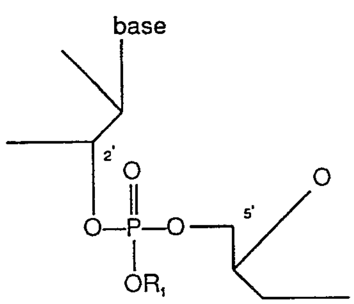

dans laquelle $R_1$ représente un radical alcoyle ayant de 1 à 4 atomes de carbone;

- un radical alcoyle ayant de 1 à 4 atomes de carbone, en particulier un méthyle,
- un radical éthyle substitué en bêta par un groupe cyano, aryle ou arylsulfonyle,
- un radical trihalogénoéthyle.

La liaison 2′→5′ phosphonate qui relie deux unités nucléosidiques adjacents dans les oligonucléotides selon l'invention peut être représentée comme suit:

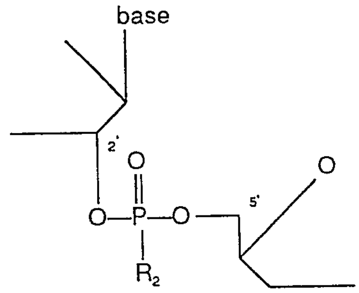

dans laquelle $R_2$ peut représenter un alcoyle ayant de 1 à 4 atomes de carbone, en particulier le méthyle.

Par convention, dans un oligonucléotide selon l'invention contenant n unités nucléosidiques, on désignera, ci-après, l'unité nucléosidique de rang n, lorsque le dernier élément de la chaîne est un groupe phosphoglycéryle, par l'expression «dernière unité nucléosidique».

Dans ce cas, l'oligonucléotide selon l'invention pourra être désigné par $(2'-5')(A)_nPGro$.

Lorsque le dernier élément de l'oligonucléotide selon l'invention est:

- soit un groupe nucléosidique de formule:

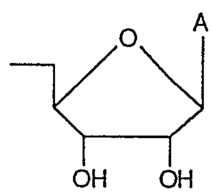

A ayant les significations ci-dessus indiquées;

- soit un «groupe nucléosidique modifié» comme défini ci-après;

on désignera, par convention dans les définitions l'unité nucléosidique de rang n − 1 par l'expression «dernière unité nucléosidique».

Par «groupe nucléosidique modifié», on définit un groupe nucléosidique dans lequel:

- la liaison directe entre le carbone 2′ et le carbone 3′ reliant directement la liaison a été supprimée et peut être représenté par la formule suivante:

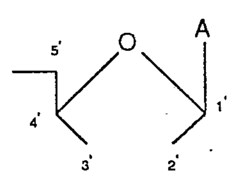

dans laquelle le pentose est sous la forme furane, mais peut également être sous la forme pyrane et dans laquelle A représente l'adénine ou un dérivé de l'adénine comme défini ci-dessus;
- les carbones en 2' et 3' sont porteurs de fonctions aldéhyde ou de fonctions alcool, éventuellement estérifiées; dans le cas où les carbones en 2' et 3' portent des fonctions alcool, on pourra désigner les oligonucléotides selon l'invention par (2'-5')$(A)_n$Ox Red.

Les oligonucléotides selon l'invention peuvent être représentés par la formule (I) suivante:

(I)

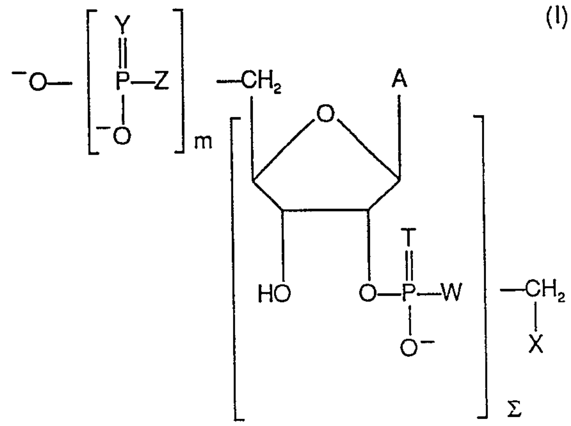

dans laquelle:
- Y et T sont identiques ou différents et représentent O, S, Se, NH;
- Z et W sont identiques ou différents et représentent O, S, NH;
- l'un au moins des éléments Y et Z de l'un au moins des groupes phosphate étant différent de l'oxygène;
- X est choisi dans le groupe constitué par:

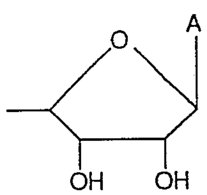

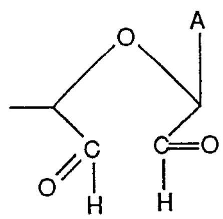

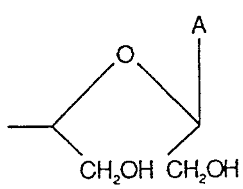

—$CHOHCH_2OH$

les fonctions alcools de ces radicaux étant éventuellement estérifiées par des acides carboxyliques $R_3COOH$, $R_3$ représentant un radical alcoyle de 1 à 5 atomes de carbone, ou un radical phényle;
- Σ est un nombre entier égal à:
- n lorsque X représente -$CHOHCH_2OH$,
- n - 1 lorsque X représente:

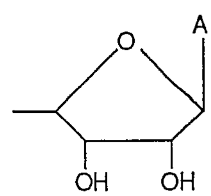

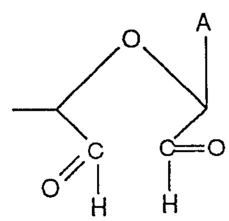

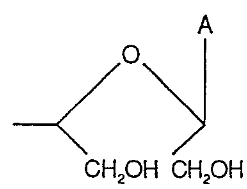

n étant un nombre entier supérieur ou égal à 2;
- m est un nombre entier supérieur ou égal à 1;
- A est une base choisie parmi l'adénine ou ses dérivés, notamment ceux de formule:

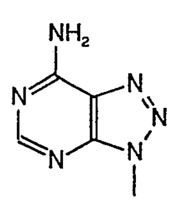

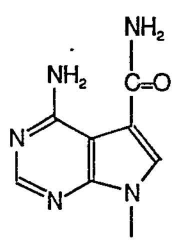

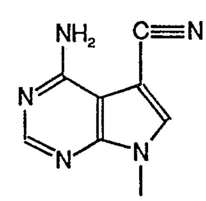

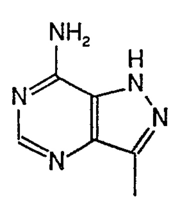

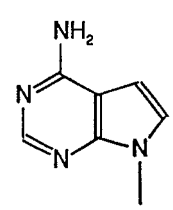

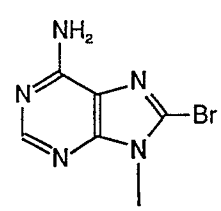

Dans une classe préférée d'oligonucléotides selon l'invention, le nombre m varie de préférence de 1 à 3.

Parmi les groupes reliés à la première unité nucléosidique, et de formule:

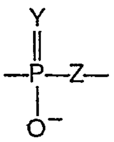

l'un au moins est tel que Y représente Se, S ou NH et/ou Z représente S ou NH, et les autres groupes:

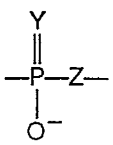

représentant des groupes phosphate.

Dans une classe préférée d'oligonucléotides selon l'invention, Y représente S, et Z représente O.

Une classe préférée d'oligonucléotides selon l'invention est celle dans laquelle X est soit un groupe nucléosidique modifié pouvant être représenté par:

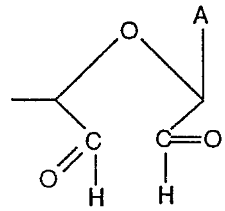

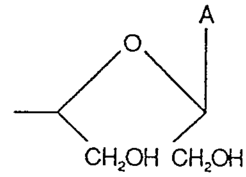

soit le groupe $-CHOHCH_2OH$.

Les fonctions alcool des radicaux:

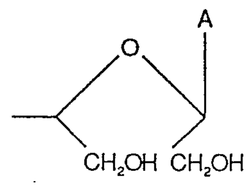

$-CHOHCH_2OH$

sont éventuellement estérifiées par un acide carboxylique $R_3COOH$, dans lequel $R_3$ représente un radical alcoyle de 1 à 5 atomes de carbone ou un radical phényle.

Une classe préférée d'oligonucléotides selon l'invention est constituée par ceux de formule (II) suivante:

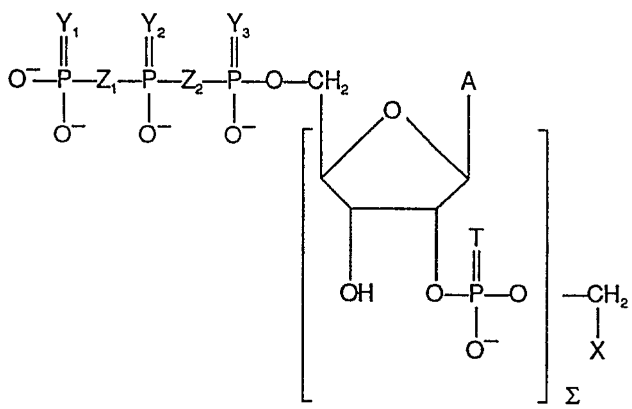

(II)

dans laquelle:

- $Y_1$, $Y_2$, $Y_3$, T sont identiques ou différents et représentent O, S, Se, NH;
- $Z_1$ et $Z_2$ sont identiques ou différents et représentent O, S, NH;

l'un au moins des éléments $Y_1$, $Y_2$, $Y_3$, $Z_1$, $Z_2$ étant différent de l'oxygène;

- X est choisi dans le groupe constitué par:

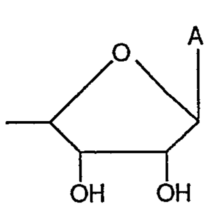

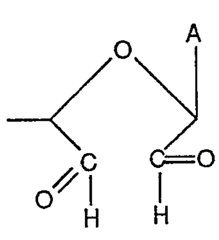

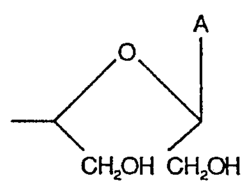

$-CHOHCH_2OH$

les fonctions alcools de ces radicaux étant éventuellement estérifiées par des acides carboxyliques $R_3COOH$, $R_3$ représentant un radical alcoyle de 1 à 5 atomes de carbone ou un radical phényle;

- Σ représente un nombre entier égal à n, lorsque X représente $-CHOHCH_2OH$, et Σ représente un nombre entier égal à n - 1 lorsque X est différent de $-CHOHCH_2OH$, n étant un nombre entier supérieur ou égal à 2;
- A est une base choisie parmi l'adénine ou ses dérivés, notamment ceux de formule:

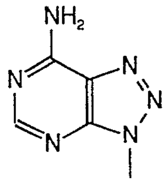
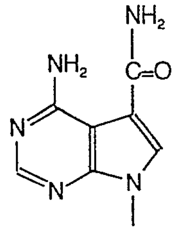
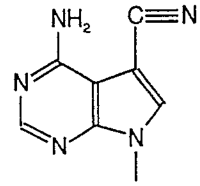
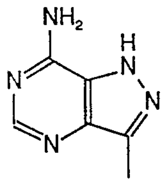
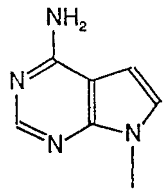
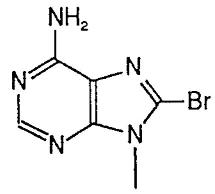

Le phosphore du groupe:

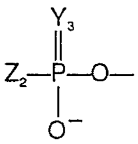

sera appelé phosphore alpha.

Le phosphore du groupe:

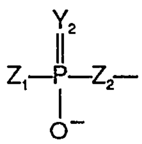

sera appelé phosphore bêta.

Le phosphore du groupe:

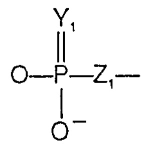

sera appelé phosphore gamma.

A l'intérieur de la classe d'oligonucléotides de formule (II), une classe préférée d'oligonucléotides selon l'invention est constituée par les oligonucléotides de formule (III):

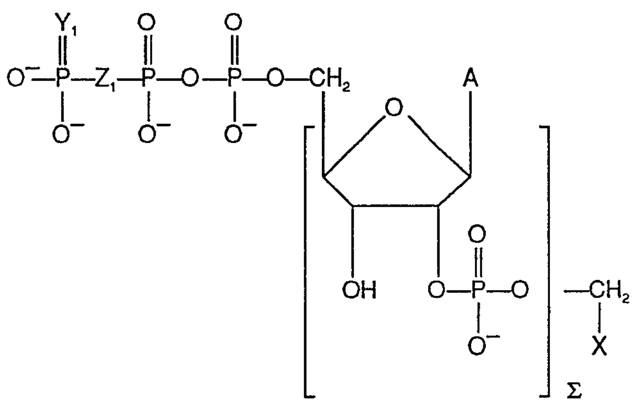

(III)

dans laquelle:

- $Y_1$ représente S, Se ou NH;
- $Z_1$ représente O, NH ou S;
- Σ, X et A ayant les significations indiquées ci-dessus.

A l'intérieur de cette classe d'oligonucléotides, une classe préférée d'oligonucléotides est constituée par ceux dans lesquels:

- X représente:
- soit:

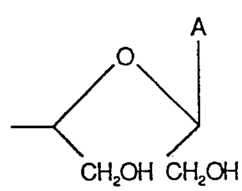

A représentant l'adénine;
- soit:
$-CHOHCH_2OH$.

Une autre classe préférée d'oligonucléotides selon l'invention est constituée par ceux de formule (IV) suivante:

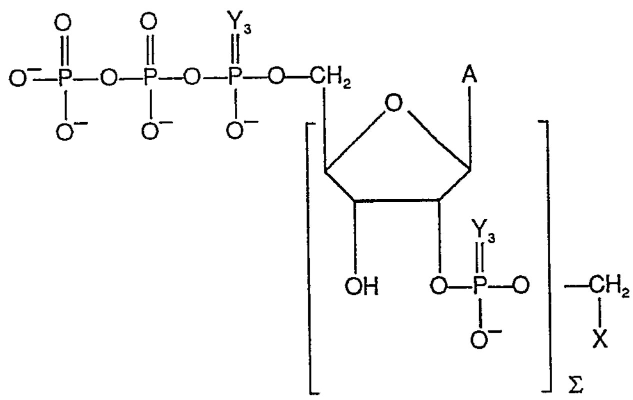

(IV)

dans laquelle:
- $Y_3$ représente S, Se ou NH,
- Σ, X et A ayant les significations ci-dessus indiquées.

A l'intérieur de cette classe d'oligonucléotides, une classe préférée d'oligonucléotide selon l'invention est constituée par ceux dans lesquels:
- X représente:
- soit:

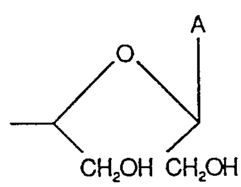

A représentant l'adénine;
- soit:
$-CHOHCH_2OH$.

Une autre classe d'oligonucléotides préférée selon l'invention est constituée par celle de formule (V) suivante:

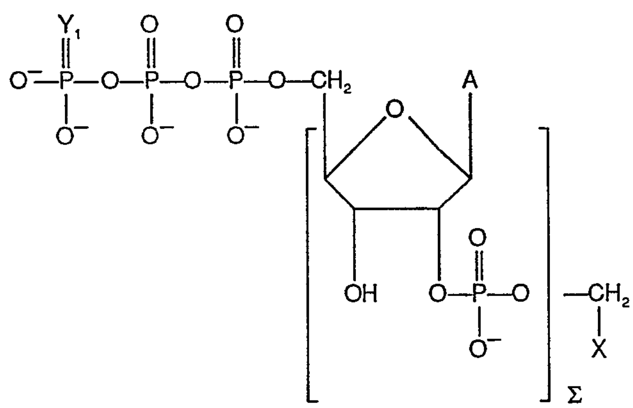

(V)

dans laquelle:
- $Y_1$ représente Se, S ou NH;
- Σ, X et A ayant les significations indiquées ci-dessus.

A l'intérieur de cette classe, une classe préférée d'oligonucléotides selon l'invention est constituée par ceux dans lesquels:
- X représente:
- soit:

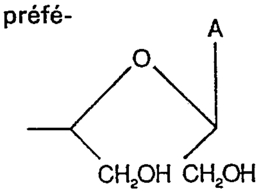

A représentant l'adénine;
- soit:
$-CHOHCH_2OH$.

Une autre classe préférée d'oligonucléotides selon l'invention est constituée par ceux de formule (VI) suivante:

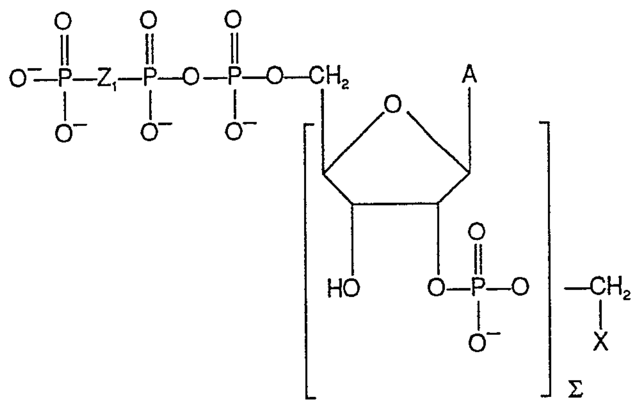

(VI)

dans laquelle:
- $Z_1$ représente S ou NH;
- Σ, X et A ayant les significations ci-dessus indiquées.

A l'intérieur de cette classe, une classe préférée d'oligonucléotides est constituée par ceux dans lesquels:
- X représente:
- soit:

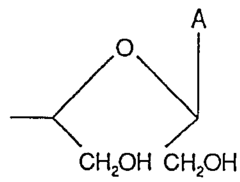

A représentant l'adénine;
- soit:
$-CHOHCH_2OH$.

Une autre classe préférée d'oligonucléotides selon l'invention est constituée par ceux de formule (VII) suivante:

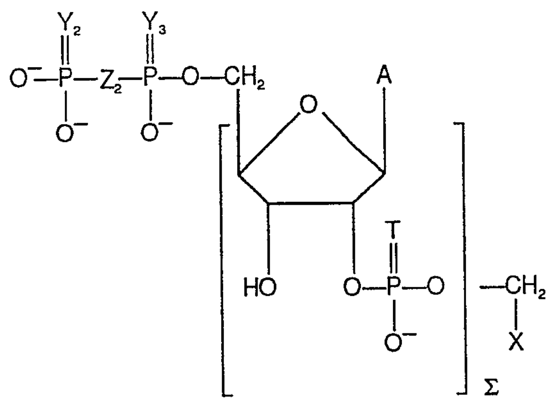

(VII)

dans laquelle:
- $Y_2$, $Y_3$, T sont identiques ou différents et représentent O, S, Se, NH;
- $Z_2$ représente O, S, NH;

l'un au moins des éléments $Y_2$, $Y_3$, $Z_2$ étant différent de l'oxygène;
- X est choisi dans le groupe constitué par:

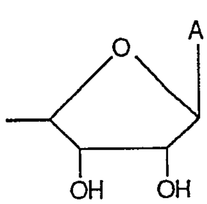

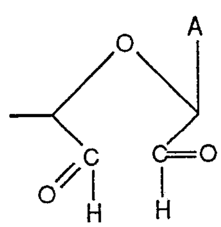

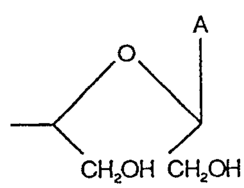

$—CHOHCH_2OH$

- Σ est un nombre entier égal à n, lorsque X représente $-CHOHCH_2OH$ et est un nombre entier égal à n-1, lorsque X est différent de $-CHOHCH_2OH$, n étant un nombre entier variant de 2 à 10;
- A est une base choisie parmi l'adénine ou ses dérivés, notamment ceux de formule:

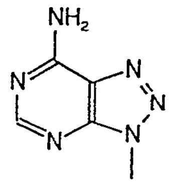
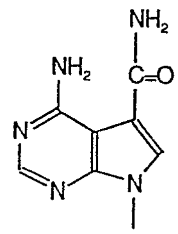
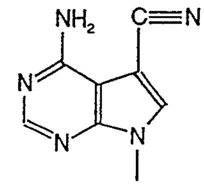
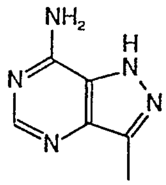
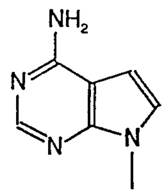
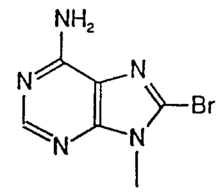

A l'intérieur de cette classe de nucléotides, une classe préférée est constituée par ceux dans lesquels T représente l'oxygène.

Une autre classe préférée d'oligonucléotides selon l'invention est constituée par ceux de formule (VIII) suivante:

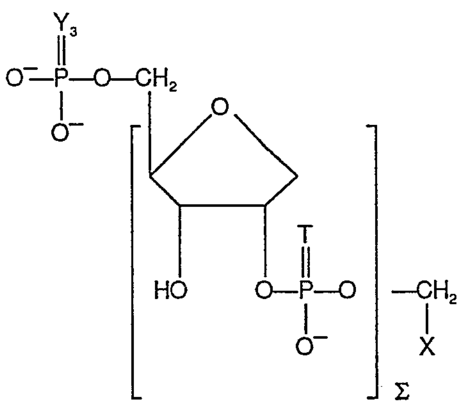

(VIII)

dans laquelle:

$Y_3$ représente S, Se, NH;

- T représente O, S, Se, NH;

- X est choisi dans le groupe constitué par:

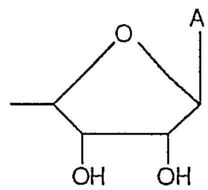
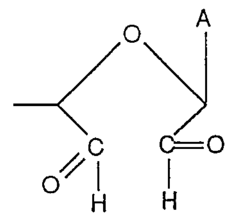
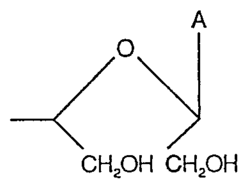

—$CHOHCH_2OH$

- Σ représente un nombre entier égal à n, lorsque X représente -$CHOHCH_2OH$ et un nombre entier égal à n-1 lorsque X est différent de -$CHOHCH_2OH$, n étant un nombre entier variant de 2 à 10;

- A est une base choisie parmi l'adénine ou ses dérivés, notamment ceux de formule:

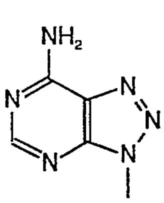
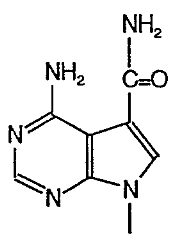
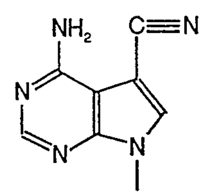
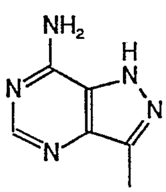
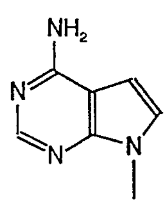
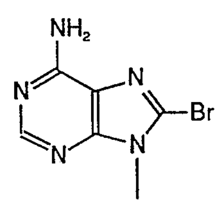

A l'intérieur de cette classe de nucléotides, une classe préférée est constituée par ceux dans lesquels T représente l'oxygène.

On donne ci-après les formules IX à XIV d'oligonucléotides non revendiqués, mais qui peuvent être utilisés pour la synthèse d'oligonucléotides préférés de l'invention, ainsi que les formules XV à XVIII d'oligonucléotides préférés de l'invention.

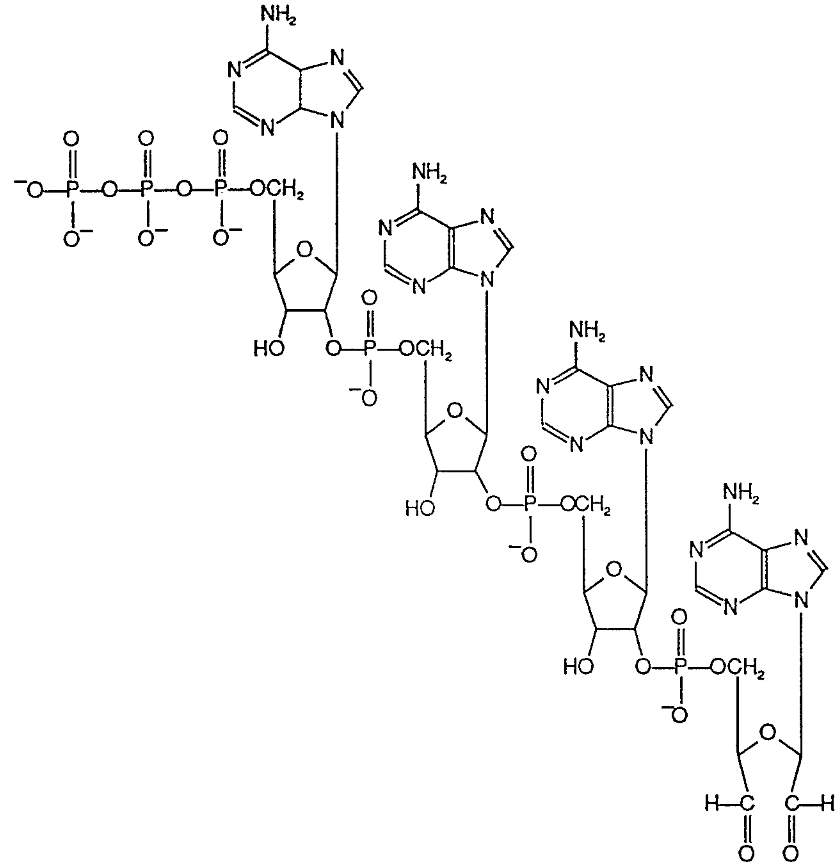

(IX)

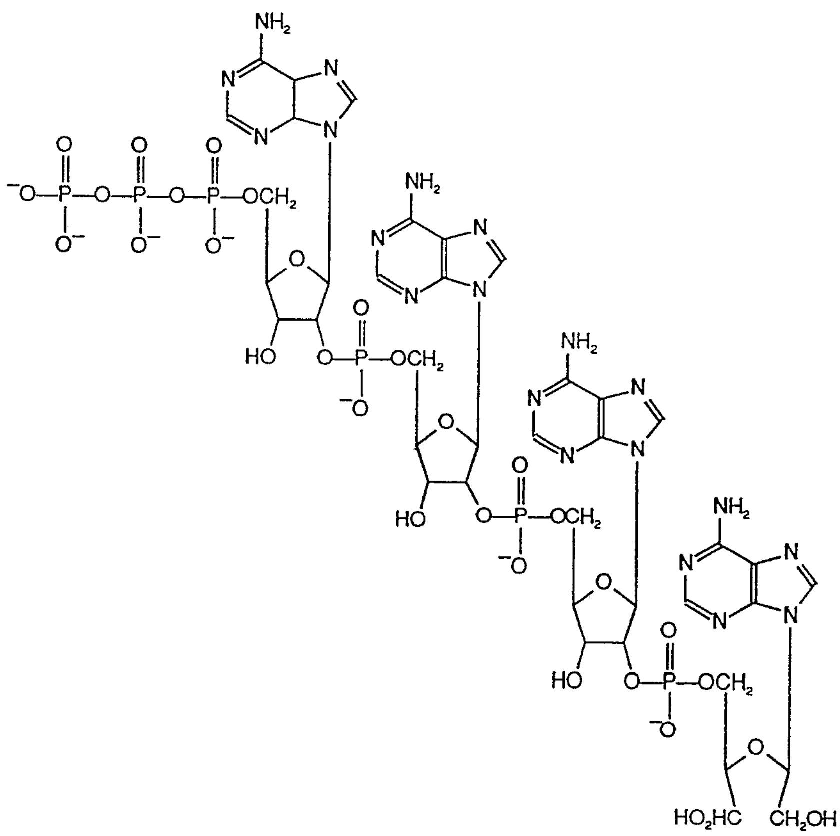

(X)

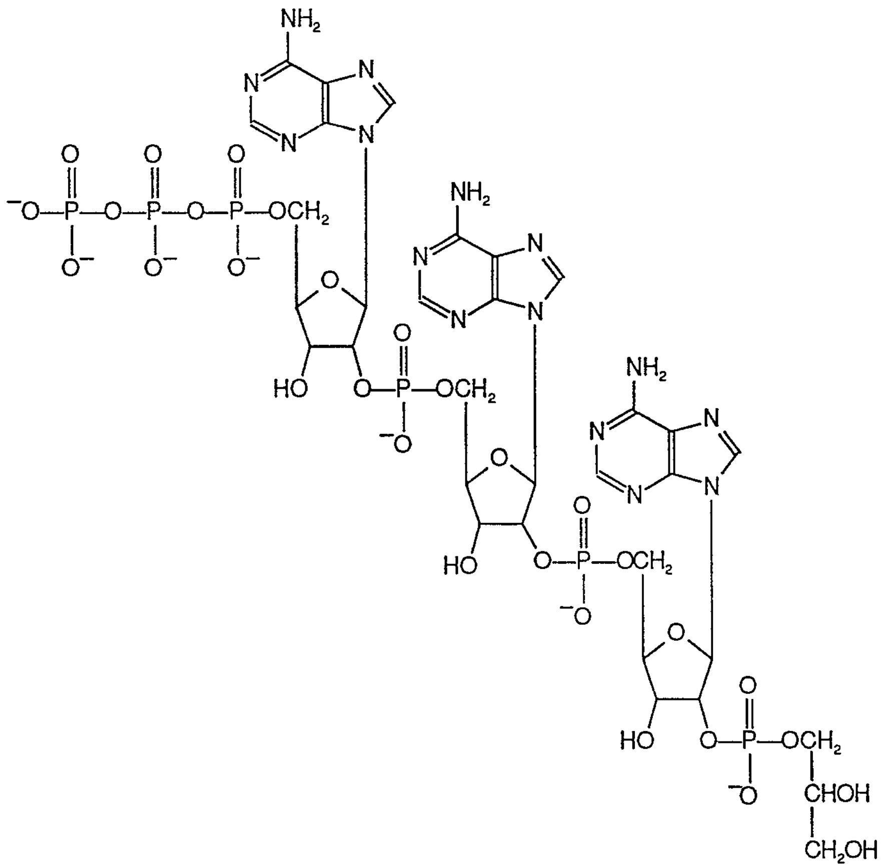

(XI)

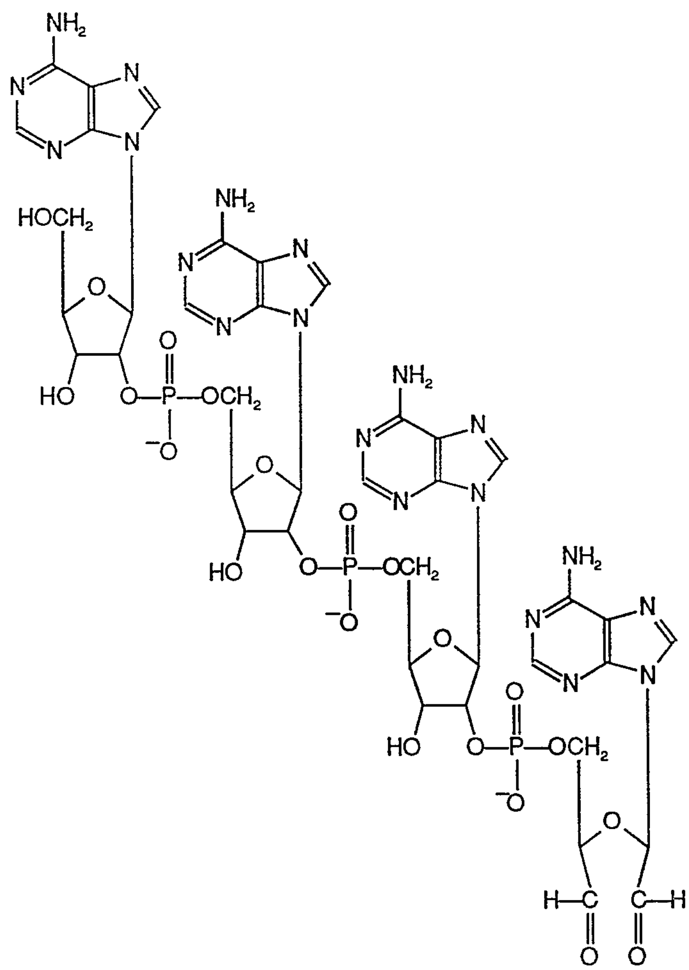

(XII)

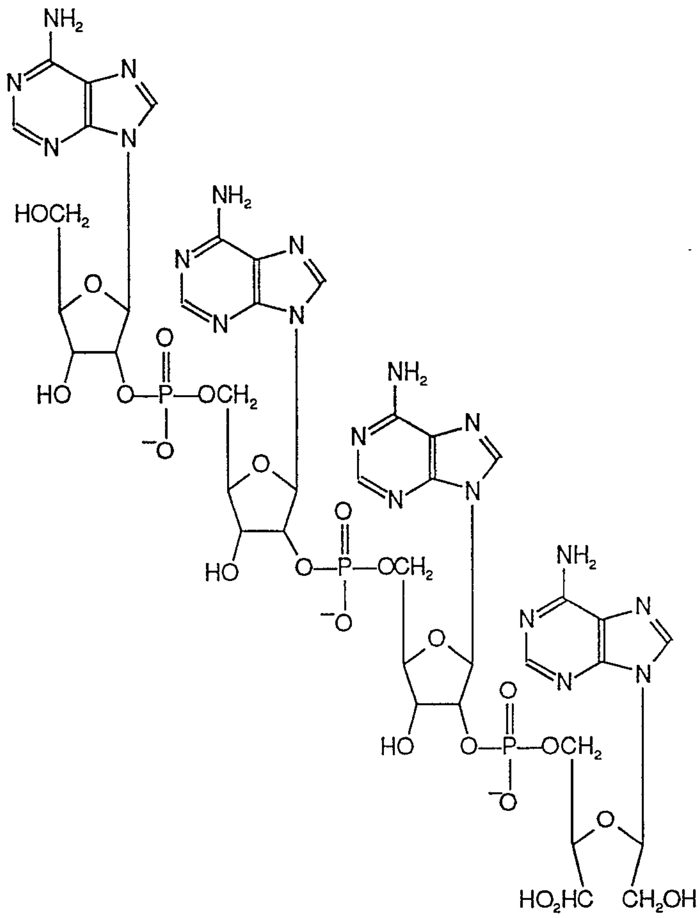

(XIII)

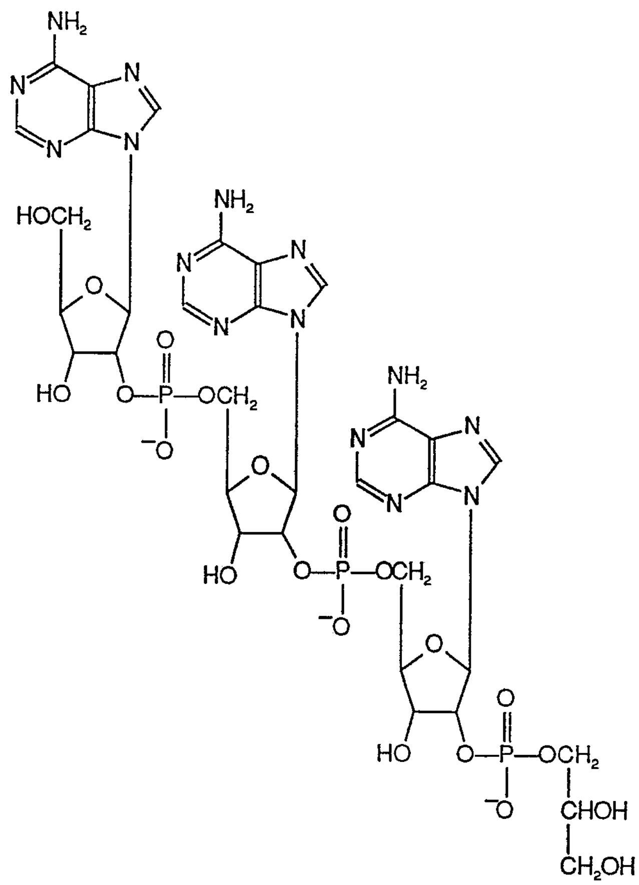

(XIV)

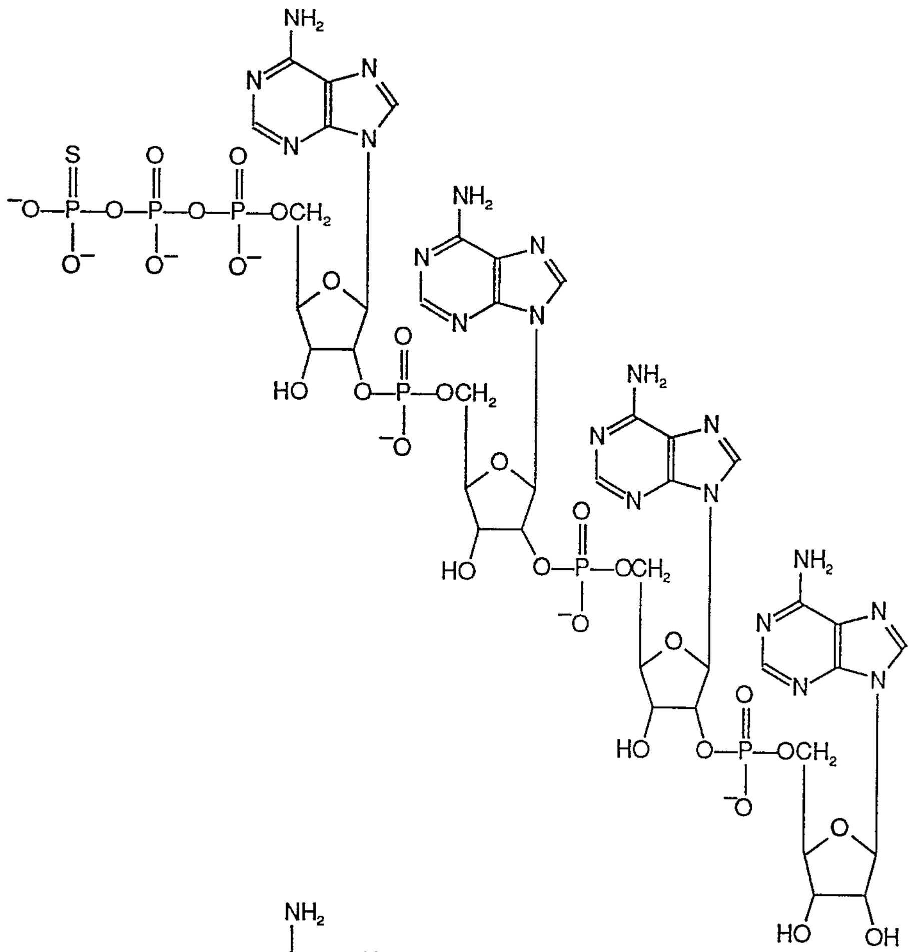

(XV)

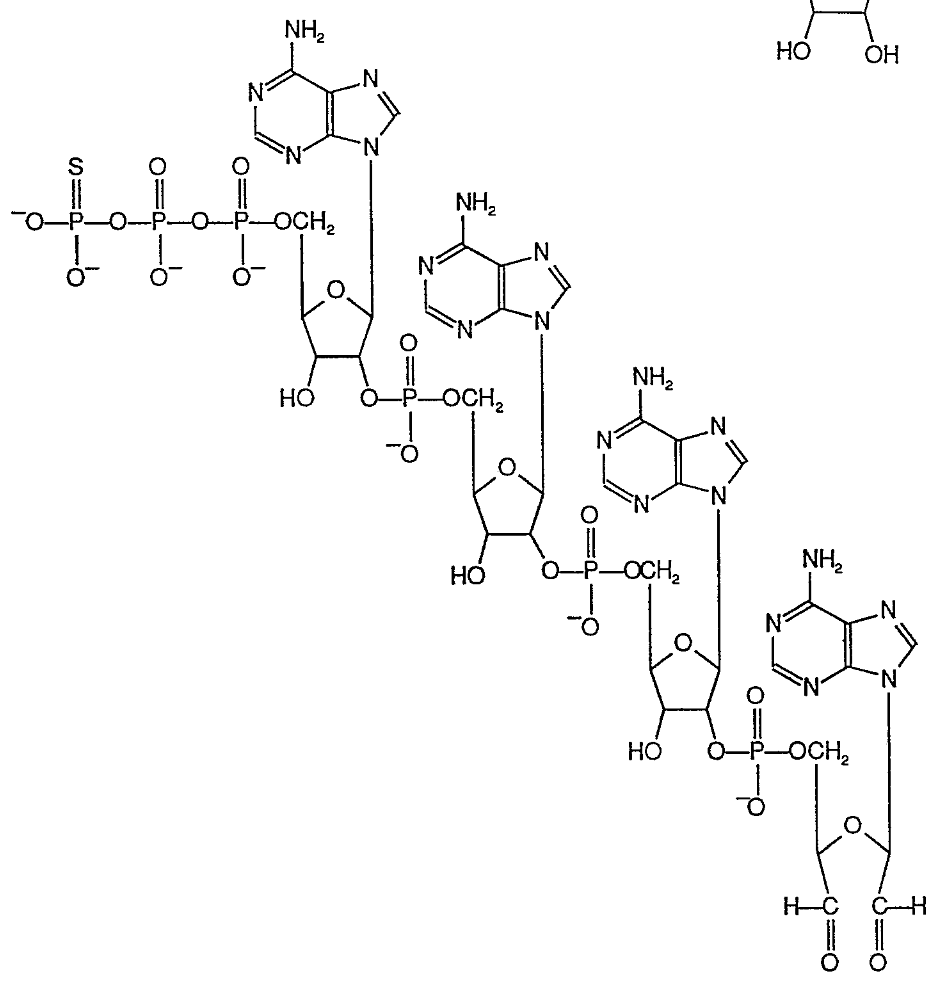

(XVI)

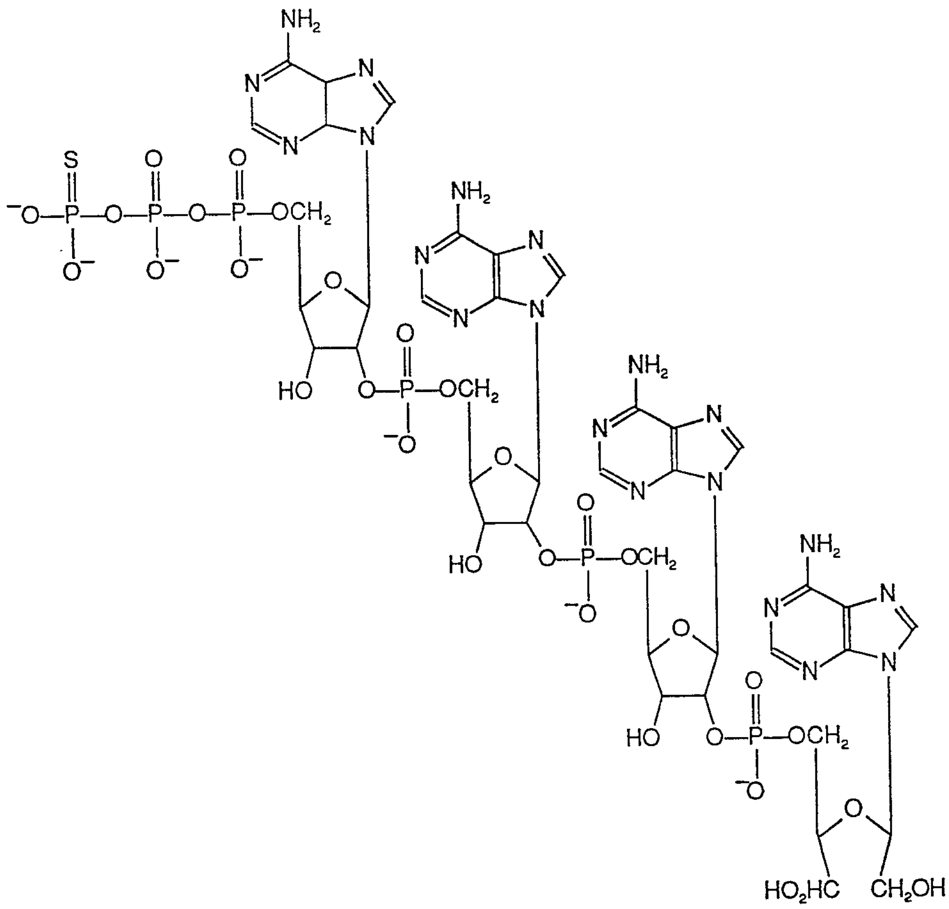

(XVII)

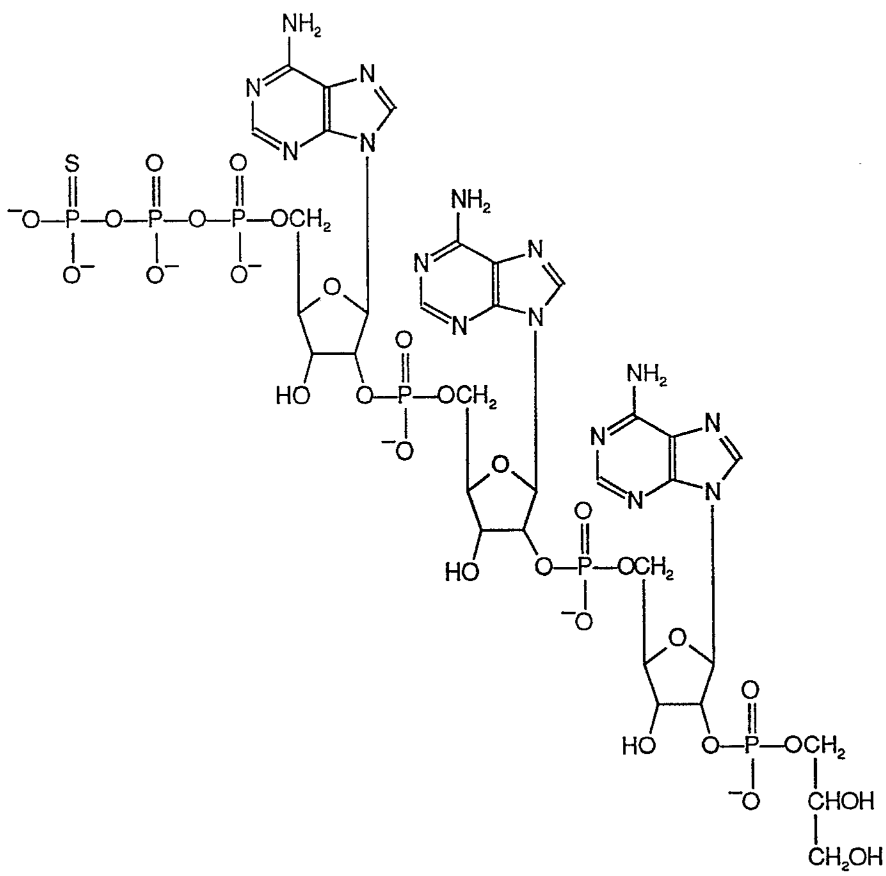

(XVIII)

L'invention concerne également les sels qui peuvent être obtenus par réaction des susdits oligonucléotides avec les bases appropriées, en particulier les sels d'ammoniums quaternaire, tels que le sel de triéthylammonium, et les sels minéraux, tels que le sel de sodium.

L'invention concerne également un procédé de préparation des oligonucléotides.

Pour préparer les oligonucléotides selon l'invention, on peut avoir recours soit à une synthèse chimique totale, soit à une synthèse enzymatique suivie de modifications chimiques.

En ce qui concerne la synthèse chimique, on peut se reporter au protocole décrit dans Methods of Enzymology, 79, 1981, 233-234.

En ce qui concerne la synthèse enzymatique des oligonucléotides de formule (I) selon l'invention, elle comprend:

- la polymérisation de composés de formule (XIX) suivante:

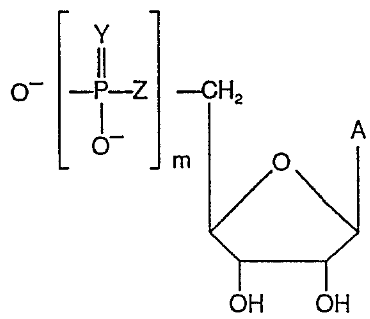

(XIX)

dans laquelle:

- Y représente O, S, Se ou NH;
- Z représente O, S ou NH;

l'un au moins des éléments Y et Z de l'un au moins des groupes phosphate étant différent de l'oxygène;

- m est supérieur ou égal à 1;
- A a les significations indiquées ci-dessus;

pour obtenir un composé de formule (Ibis) suivante:

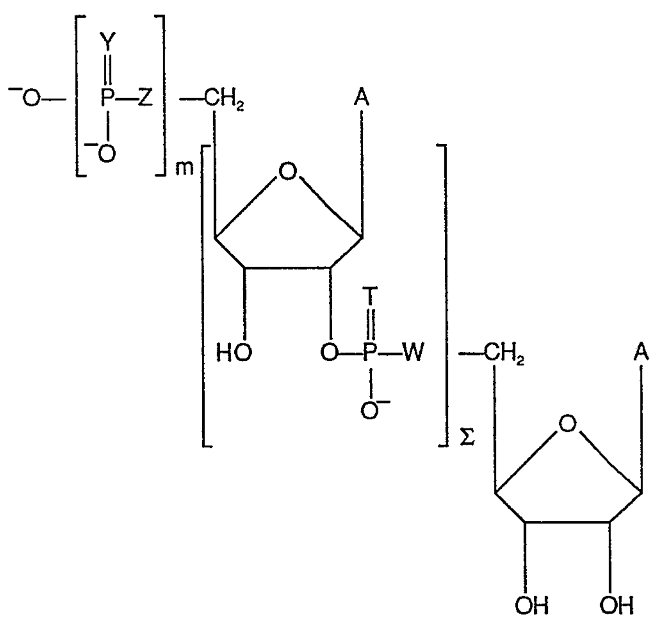

(I bis)

dans laquelle:

- Y et T, identiques ou différents, représentent O, S, Se, NH;
- Z et W, identiques ou différents, représentent O, S, NH;

l'un au moins des éléments Y et Z de l'un au moins des groupes phosphate étant différent de l'oxygène;

- Σ est un nombre entier égal à n-1, n étant supérieur ou égal à 2;
- m est un nombre entier supérieur ou égal à 1;
- A a les significations indiquées ci-dessus;
- et si nécessaire les étapes chimiques suivantes à savoir:
- l'oxydation éventuelle du groupe glycol pour introduire des fonctions aldéhyde sur les carbones en 2' et 3' de la dernière unité nucléosidique et obtenir le composé de formule (Iter):

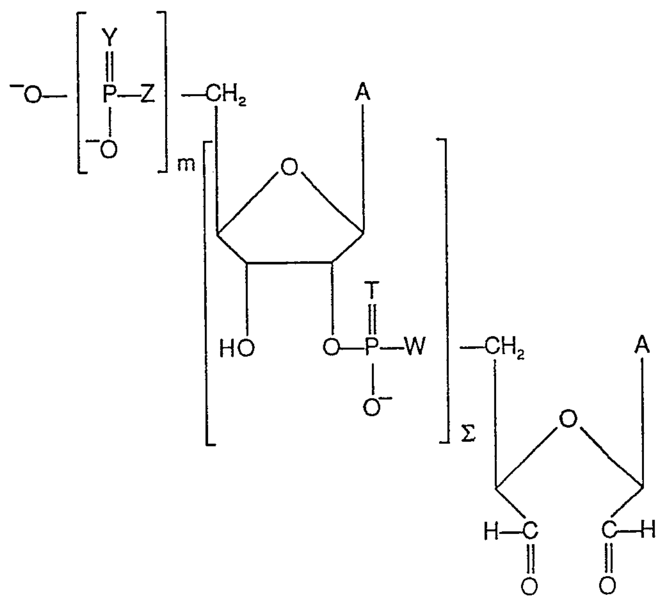

(I ter)

- la réduction éventuelle des deux fonctions aldéhyde en fonctions alcool pour obtenir le com- formule (Iquater):

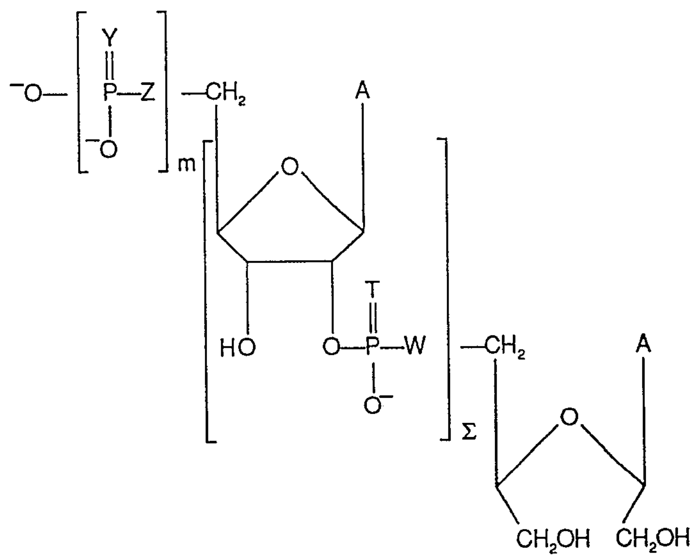

(I quater)

- l'hydrolyse éventuelle, dans des conditions évitant la béta-élimination, pour obtenir le composé de formule (Iquinquiès) suivante:

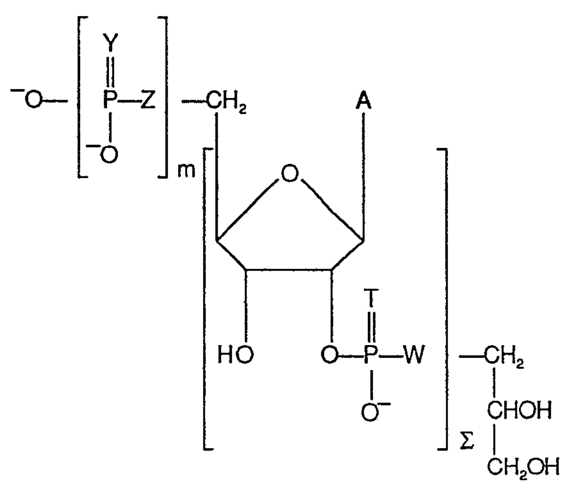

(I quinquies)

Un mode de réalisation préféré d'oligonucléotides selon l'invention de formule (XX) suivante:

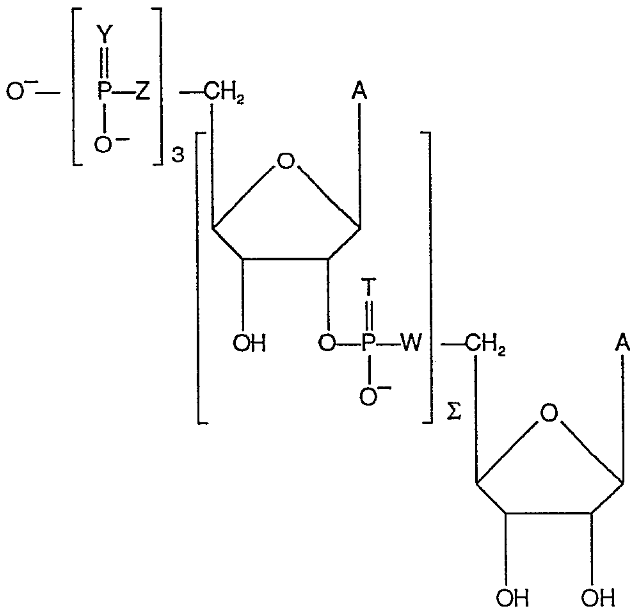

(XX)

dans laquelle:
- Y et T, identiques ou différents, représentent O, S, Se, NH;
- Z et W, identiques ou différents, représentent O, S, NH;
- Y et Z de l'un au moins des groupes phosphate ne représentent pas simultanément l'oxygène;
- Σ est un nombre entier variant de 1 à 9;
- A est une base choisie parmi l'adénine ou ses dérivés, notamment ceux de formule:

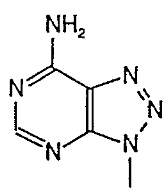
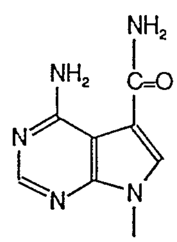
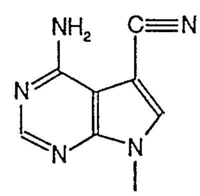
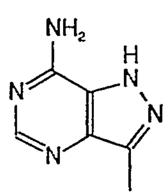
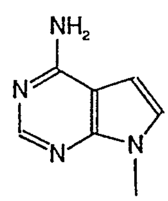
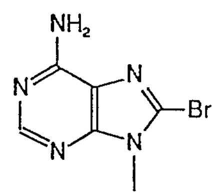

comprend:
1) la polymérisation d'un composé de formule (XIXbis) suivante:

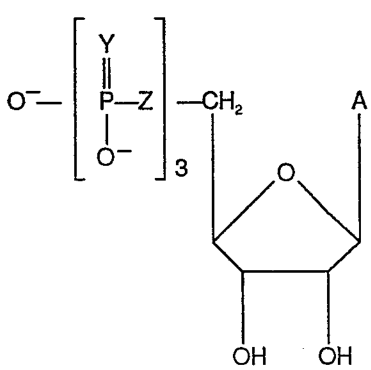

(XIX bis)

dans laquelle:
- Y représente O, S, Se ou NH;
- Z représente O, S ou NH;
- Y et Z de l'un au moins des groupes phosphate ne représentent pas simultanément l'oxygène;
- A a les significations indiquées ci-dessus;

2) l'oxydation éventuelle du groupe glycol terminal, notamment par l'ion periodate pour transformer le glycol en deux fonctions aldéhyde et obtenir un composé de formule (XXI) suivante:

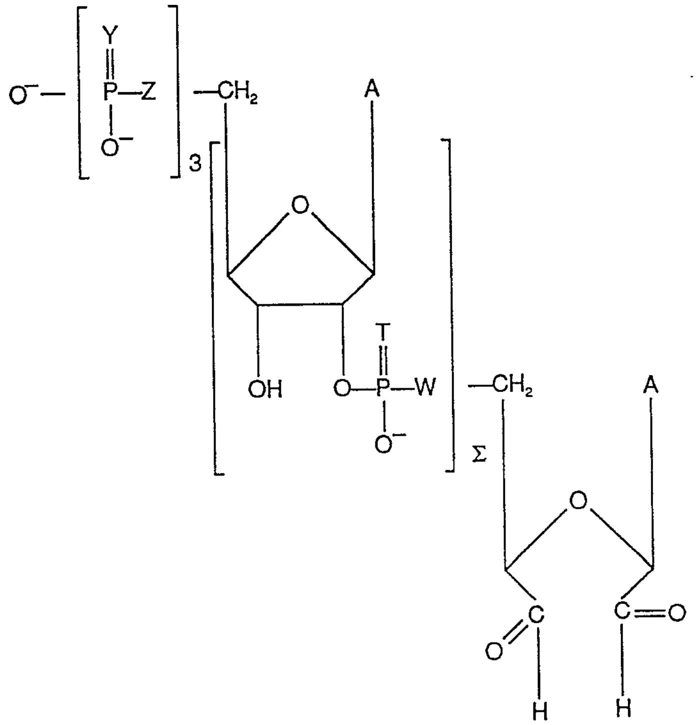

(XXI)

dans laquelle Y, Z, T, w, Σ et A ont les significations indiquées ci-dessus;

3) la réduction éventuelle des fonctions aldéhyde, notamment par le borohydrure de sodium pour transformer les deux susdites fonctions aldéhyde en fonctions alcool et obtenir le composé de formule (XXII) suivante:

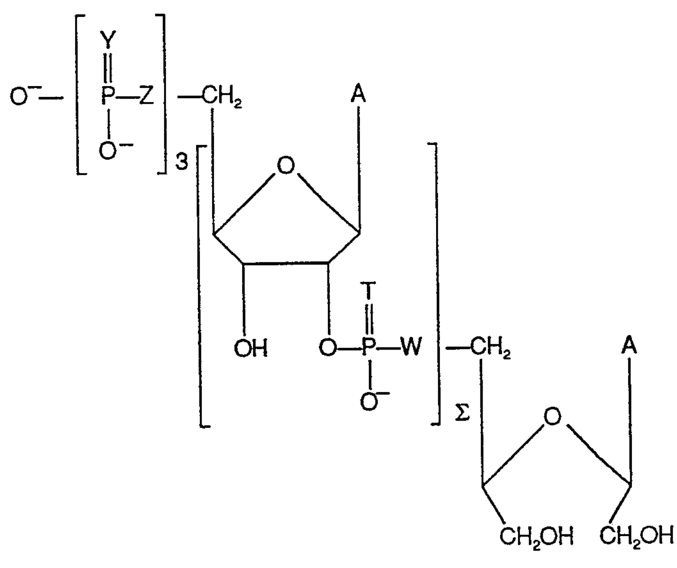

(XXII)

4) l'hydrolyse éventuelle, notamment l'hydrolyse acide ménagée, pour éliminer le noyau de ribose et obtenir le composé de formule (XXIII) suivante:

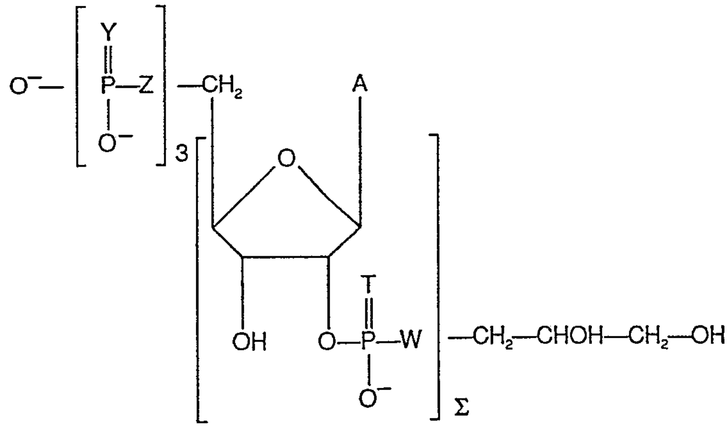

(XXIII)

Parmi les composés de formule (XIX) sont disponibles dans le commerce, ceux de formule (XIXbis) suivante:

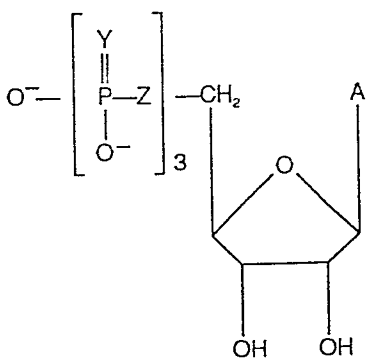

(XIX bis)

dans lesquels Y représente le soufre et Z représente l'oxygène, ainsi que ceux dans lesquels Y représente l'oxygène et Z représente le groupe NH, A représentant l'adénine ou un de ses dérivés comme définis ci-dessus.

L'oxydation du groupe glycol de la dernière unité nucléosidique pour supprimer la liaison directe entre le carbone en 2′ et le carbone en 3′ et introduire deux fonctions aldéhyde peut être effectuée par l'acide périodique dans des conditions de pH rigoureusement contrôlées pour éviter une bêta élimination.

L'expression «conditions de pH rigoureusement contrôlées» signifie le maintien du milieu réactionnel à pH 4,0 à 0-4°C et à l'obscurité.

La réduction des fonctions aldéhyde en fonctions alcool peut être effectuée par le borohydrure de sodium. L'hydrolyse pour transformer:

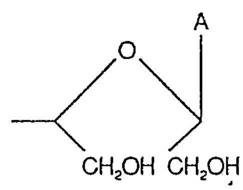

en groupe $-CHOHCH_2OH$ est de préférence une hydrolyse acide ménagée, effectuée selon des méthodes classiques.

Un procédé d'obtention selon l'invention des oligonucléotides de formule (V) suivante:

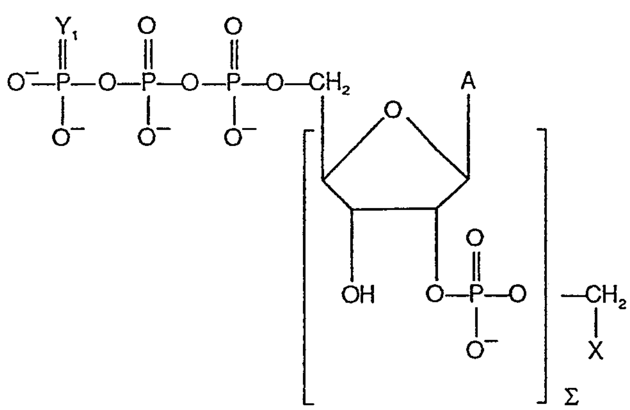

(V)

dans laquelle:
- $Y_1$ représente NH, Se, S;
- X représente:

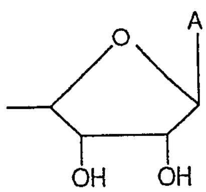

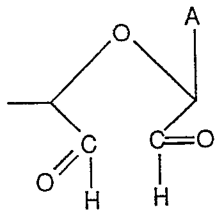

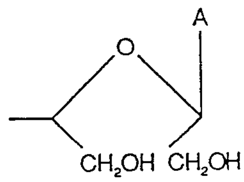

—$CHOHCH_2OH$

- Σ est un nombre entier égal à n, lorsque X représente $-CHOHCH_2OH$, n-1 lorsque X est différent de $-CHOHCH_2OH$, n variant de 2 à 10;

est caractérisé en ce que l'on polymérise le composé de formule (XXIV) suivante:

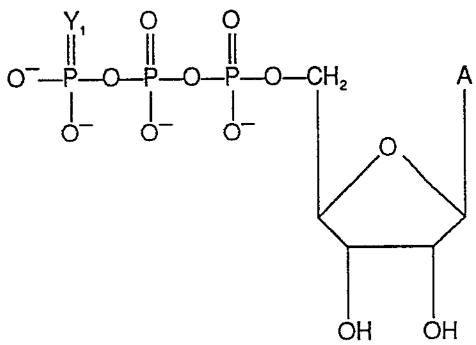

(XXIV)

et que l'on effectue les étapes 2), 3) et 4) comme indiqué ci-dessus.

A l'intérieur de la classe de procédés qui vient d'être définie, un procédé d'obtention selon l'invention des oligonucléotides de formule (Vbis) suivante:

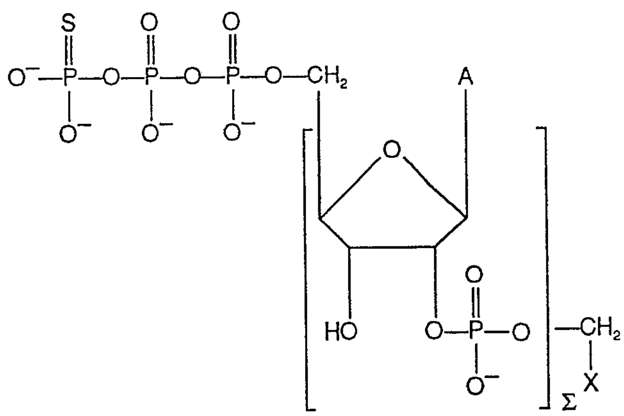

(V bis)

dans laquelle:

- E, X ont les significations précédemment indiquées;
- A a la signification précédemment indiquée, et de préférence représente l'adénine;

est caractérisé en ce que l'on polymérise un composé de formule (XXIVbis) suivante:

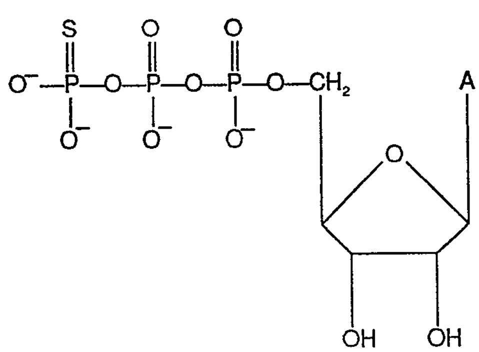

(XXIV bis)

et que l'on effectue les étapes 2), 3) et 4) comme indiqué ci-dessus.

Un procédé d'obtention selon l'invention des oligonucléotides de formule (VI) suivante:

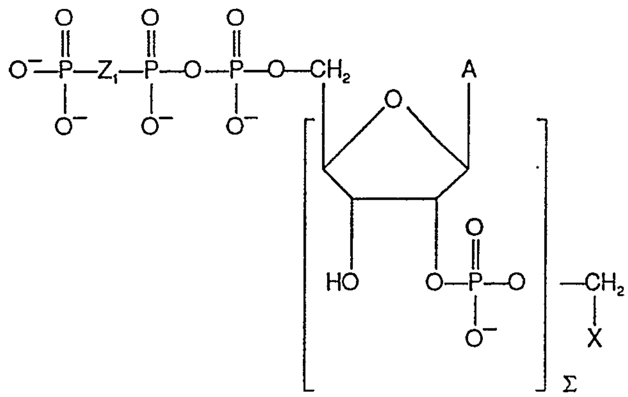

(VI)

dans laquelle:

- $Z_1$ représente NH ou S;
- X est choisi dans le groupe constitué par:

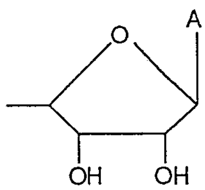

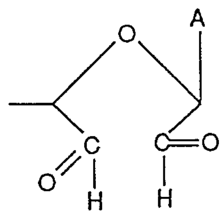

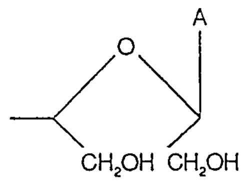

$—CHOHCH_2OH$

- Σ est un nombre entier égal à 1, lorsque X représente $-CHOHCH_2OH$ et égal à n-1 lorsque X est différent de $CHOHCH_2OH$, n variant de 2 à 10;

est caractérisé en ce qu'on polymérise le composé de formule (XXV) suivante:

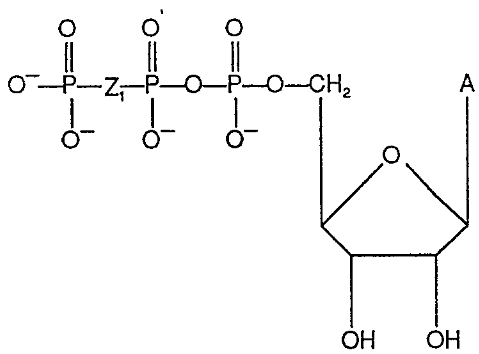

(XXV)

dans laquelle $Z_1$ représente S ou NH et A a la signification indiquée ci-dessus;
et que l'on effectue les étapes 2), 3) et 4) comme indiqué ci-dessus.

Un procédé d'obtention d'oligonucléotides selon l'invention de formule (IV) suivante:

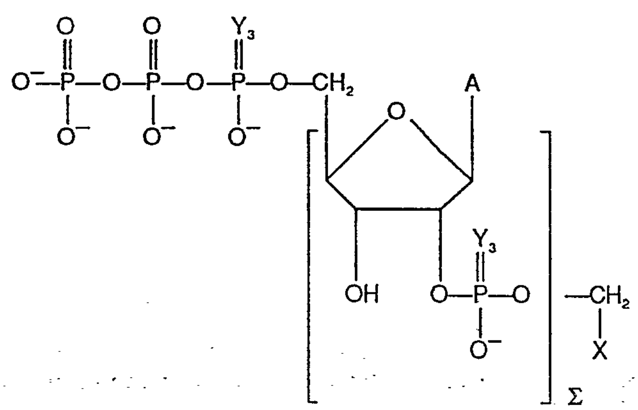

(IV)

dans laquelle:
- $Y_3$ représente NH, S ou Se;
- Σ, X et A ont les significations indiquées ci-dessus;

est caractérisé en ce qu'on polymérise le composé de formule (XXVI) suivante:

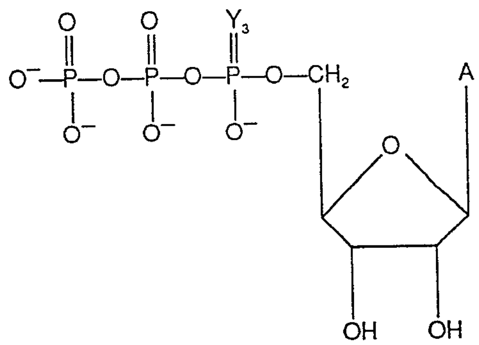

(XXVI)

dans laquelle: - $Y_3$ représente S, Se, NH;
- A a les significations indiquées ci-dessus;

et que l'on effectue les étapes 2), 3) et 4), comme indiqué ci-dessus.

Exemple 1

Cet exemple concerne la préparation d'oligonucléotides (2'-5')$(A)_n$ synthétisés par voie enzymatique et modifiés chimiquement. Ces composés sont non revendiqués, mais peuvent être utilisés dans la préparation d'oligonucléotides selon l'invention et représentés par les formules suivantes:

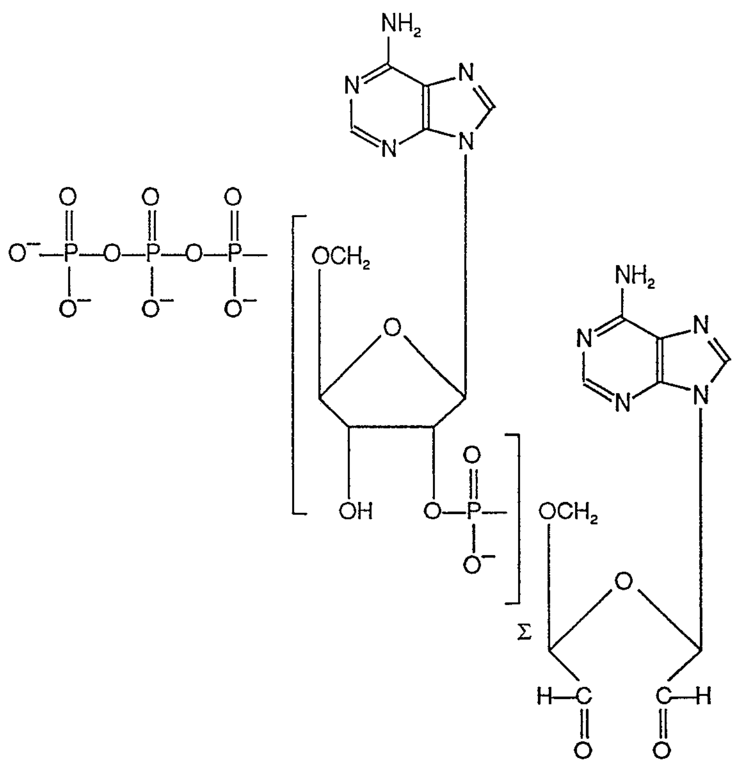

(XXVII)

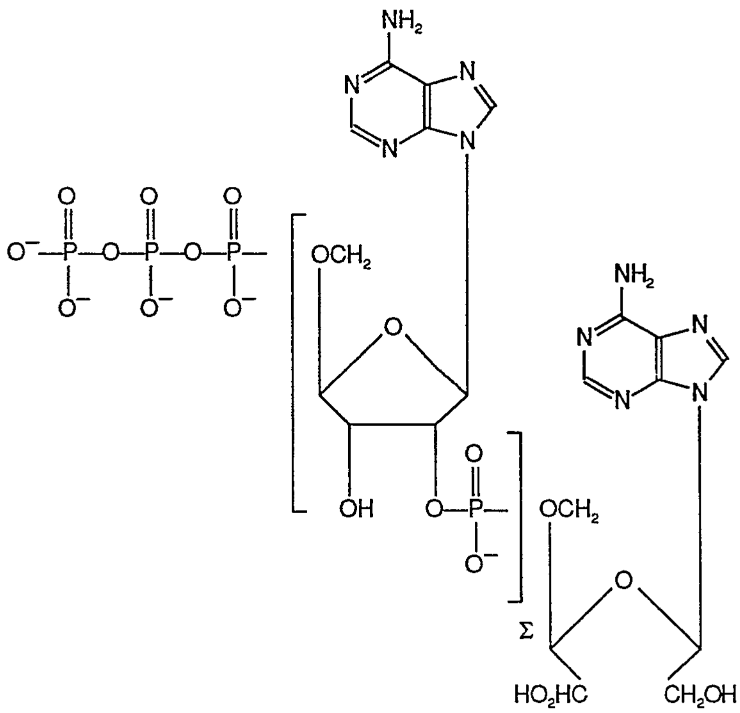

(XXVIII)

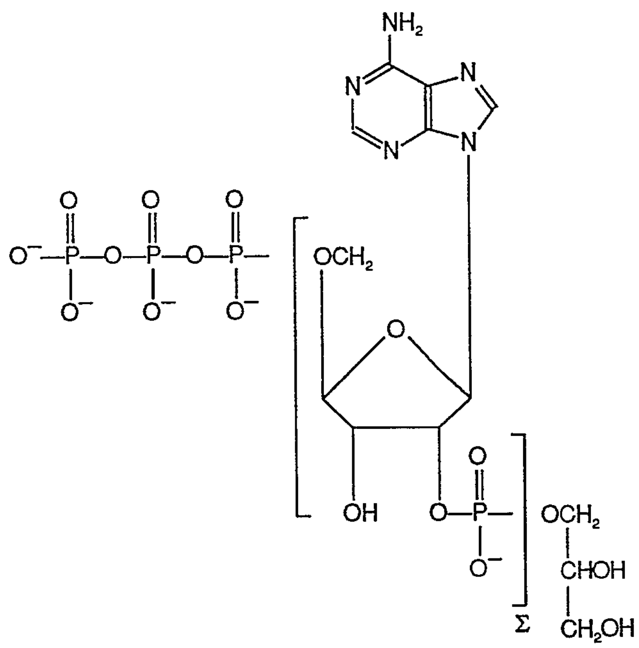

(XXIX)

et de leurs dérivés déphosphorylés correspondants.

Matériaux et méthodes

Matériaux

Les milieux proviennent d'Eurobio (Paris) et les sérums des Laboratoires Flow.

On utilise par exemple de l'interféron de leucocytes humains (Hu IFN α) purifié à une activité spécifique de $2 \times 10^6$ UI/mg de protéines.

Le polyacide riboinosinique-polyacide ribocytidylique [ci-après désigné par l'abréviation poly(rI).poly(rC)] est obtenu par exemple de PL Biochemicals. La phosphatase alcaline bactérienne de type III-R provient de Sigma et est conservée à 4 °C.

Le [$\gamma^{32}P$] ATP (activité spécifique 2000 Ci/mM) et le (2'-5')$(A)_n$-pCp [$^{32}P$] (activité spécifique 3000 Ci/mM) proviennent d'Amersham.

Le boro[$^3H$]hydrure de sodium (activité spécifique 30 Ci/mol) est fourni par le Commissariat à l'Energie Atomique.

Le diéthylaminoéthyl-trisacryl (ci-après désigné par DEAE trisacryl) provient de l'Industrie Biologique Française.

Cellules et virus

On maintient des cellules HeLa en monocouches dans un milieu commercialisé sous la désignation RPMI 1640, notamment par les Laboratoires Eurobio, Paris, complété par du sérum de veau fœtal à 10% (v/v), 50 UI/ml de pénicilline et 50 μg/ml de streptomycine. On fait croître des cellules L929 dans un milieu minimal essentiel additionné de 5% (v/v) de sérum de cheval donneur, de 3 g/l de bouillon de phosphate bactotryptose, 3,4 g/l de glucose et d'antibiotiques comme mentionné ci-dessus. On utilise la souche Indiana du virus de stomatite vésiculaire (VSV) et on la fait pousser dans des cellules L929.

Synthèse enzymatique d'oligoadénylates $(2'-5')(A)_n$

Les oligoadénylates $(2'-5')(A)_n$ sont synthétisés par voie enzymatique selon la méthode décrite par Minks et coll. (J. Biol. Chem., 1979, 254, 5058-5064).

On peut résumer la préparation des composés $(2'-5')(A)_n$ comme suit.

Des extraits cytoplasmiques sont préparés à partir de cellules HeLa traitées avec 200 unités par ml d'interféron de leucocytes humains pendant 48 heures. L'extrait est incubé avec 5 mM d'ATP et 20 μg/ml de poly(rI).poly(rC) pendant 2 heures, porté à ébullition pendant 3 mn à 100°C et centrifugé à 10000 × g pendant 10 mn. Des oligomères $[\gamma\text{-}^{32}P]$ $(2'-5')(A)_n$ sont synthétisés en incubant des extraits cellulaires avec du $[\gamma\text{-}^{32}P]$ ATP dans les mêmes conditions.

Fractionnement des oligoadénylates $(2'-5')(A)_n$

On synthétise approximativement 4000 unités de densité optique à 260 nm de $(2'-5')(A)_n$, c'est-à-dire 100 μmoles, n allant de 2 à 15 (qui seront fractionnées par la suite) dans des extraits de cellules HeLa traitées par l'interféron à 37°C pendant 2 heures comme décrit ci-dessus. Les protéines sont précipitées par incubation du mélange à 100°C pendant 5 mn et centrifugation à 15000 × g pendant 10 mn. Le surnageant est dilué 3 fois avec de l'eau et ajusté à pH 8,5 avec du KOH 0,1 M avant d'être chargé sur une colonne (2,5 × 64 cm) de DEAE trisacryl M, équilibré avec un tampon à pH 8,5 de bicarbonate de triéthylammonium 0,25 M. On lave la colonne avec 1500 ml de ce tampon et les oligoadénylates $(2'-5')(A)_n$ sont élués avec un gradient linéaire (1500 ml/1500 ml) de tampon à pH 8,5 de bicarbonate de triéthylammonium 0,125-0,45 M. Les oligomères en pics individuels sont identifiés par chromatographie liquide haute performance (HPLC). Les fractions sont concentrées sous vide sous pression réduite et coévaporées avec de l'eau plusieurs fois, afin d'éliminer le tampon de bicarbonate de triéthylammonium. Des quantités en mg de chacun des oligomères peuvent être obtenues sous forme purifiée et contrôlées par HPLC.

Synthèse et purification des noyaux ou «cores» de $(2'-5')(A)_n$

Les oligoadénylates déphosphorylés que l'on désigne également par «cores» ou noyaux (400 unités de $A_{260}$) obtenus par digestion enzymatique de $(2'-5')(A)_n$ de phosphatase alcaline sont fractionnés par chromatographie à échange d'ions sur une colonne (1,5 × 25 cm) de DEAE-trisacryl M. Chaque oligoadénylate déphosphorylé est obtenu sous forme pure par une élution de la colonne avec un gradient linéaire (300 ml/300 ml) de tampon à pH 8,5 de bicarbonate de triéthylammonium (60-100 mM).

Modification chimique d'oligoadénylates $(2'-5')(A)_n$

On effectue l'oxydation, à l'aide de periodate, du $(2'-5')(A)_n$ pendant 15 heures dans des conditions ménagées, afin d'éviter la bêta-élimination. De façon classique, on ajoute 100 μl de métapériodate de sodium 16 mM dans du tampon d'acétate de sodium 0,2 M à pH 4,0 à 100 μl de $(2'-5')(A)_n$ 1 mM dans l'eau distillée à 4°C. On agite le mélange à 4°C dans l'obscurité pendant 15 heures. L'excès de périodate est détruit immédiatement après la phase d'oxydation avec 10 μl d'éthylène glycol et le dérivé dialdéhyde en 2',3', conforme à l'invention, correspondant au $(2'-5')(A)_n$ est réduit à 4°C pendant 5 heures par 100 μl de borohydrure de sodium 0,1 M dans un tampon de borate 0,1 M à pH 9,0. Dans certains cas, le dialdéhyde en 2',3' est réduit avec du boro$[^3H]$-hydrure de sodium. On acidifie ensuite le mélange avec de l'acide acétique 0,1 M et on dessale sur une colonne de Séphadex G-15. Le dérivé O-phosphoglycérylé conforme à l'invention de $(2'-5')(A)_n$ est obtenu par une hydrolyse acide ménagée de la liaison ribose-oxygène avec de l'acide sulfurique 0,005 M à 80°C pendant 30 minutes. On désignera ci-après par $(2'-5')(A)_n$PGro, les 2'-5' oligoadénylates selon l'invention qui comportent un groupe terminal O-phosphoglycéryle.

Le diagramme ci-après résume, à titre d'exemple, les principales modifications chimiques effectuées à partir de l'oligoadénylate $(2'-5')(A)_4$ non modifié pour obtenir les oligoadénylates $(2'-5')(A)_4$ non revendiqués mais susceptibles d'être utilisés dans la préparation d'oligonucléotides selon l'invention.

Composé de départ: oligoadénylate $(2'-5')(A)_4$:

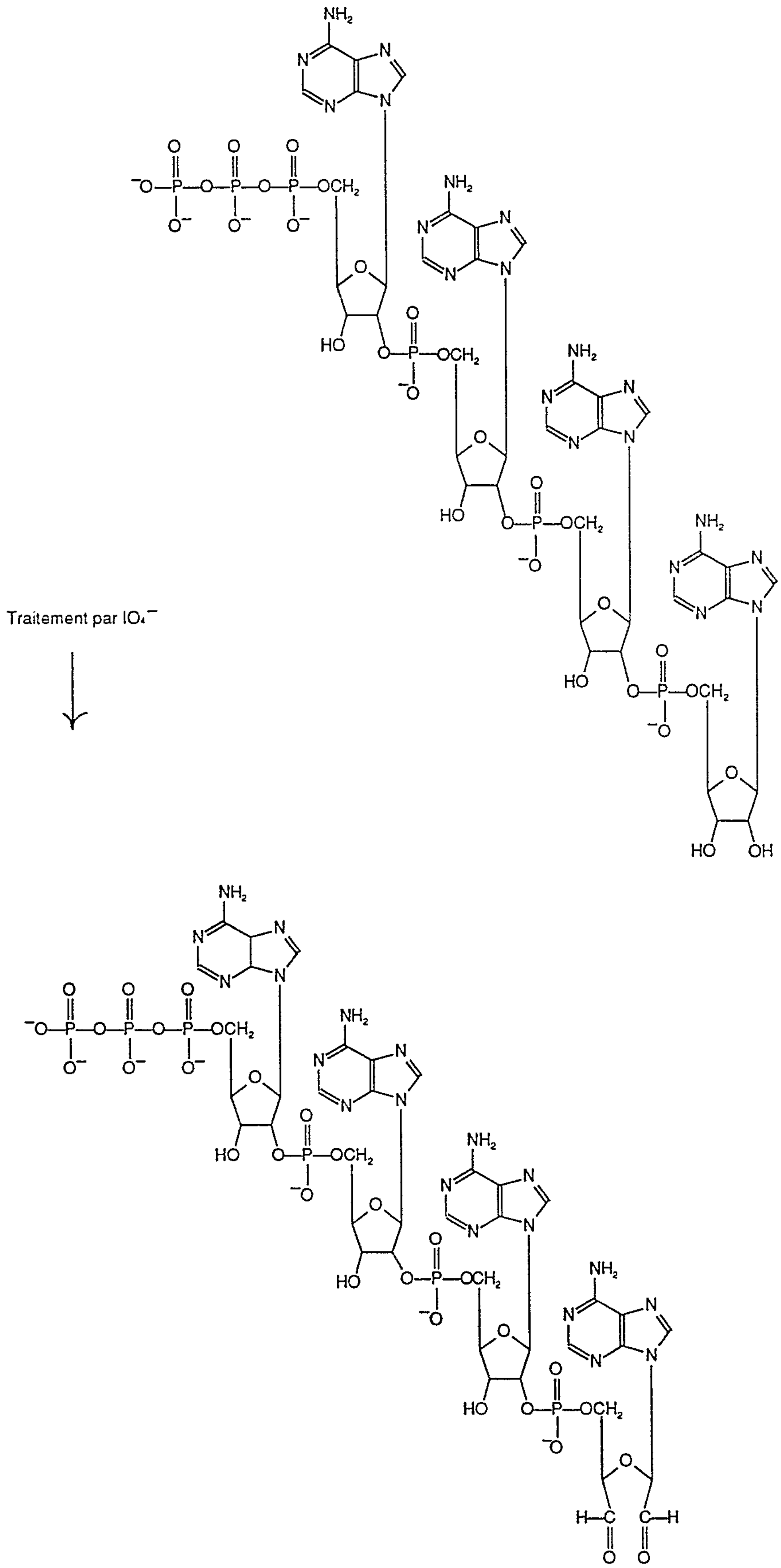


(IX)

L'oxydation, par exemple par l'ion periodate, du groupe alpha-glycol de la molécule (2'-5')(A)$_4$ introduit deux fonctions aldéhyde en 2' et 3', ce qui conduit au composé (IX).

Composé de formule (IX):

Traitement par $BH_4Na$

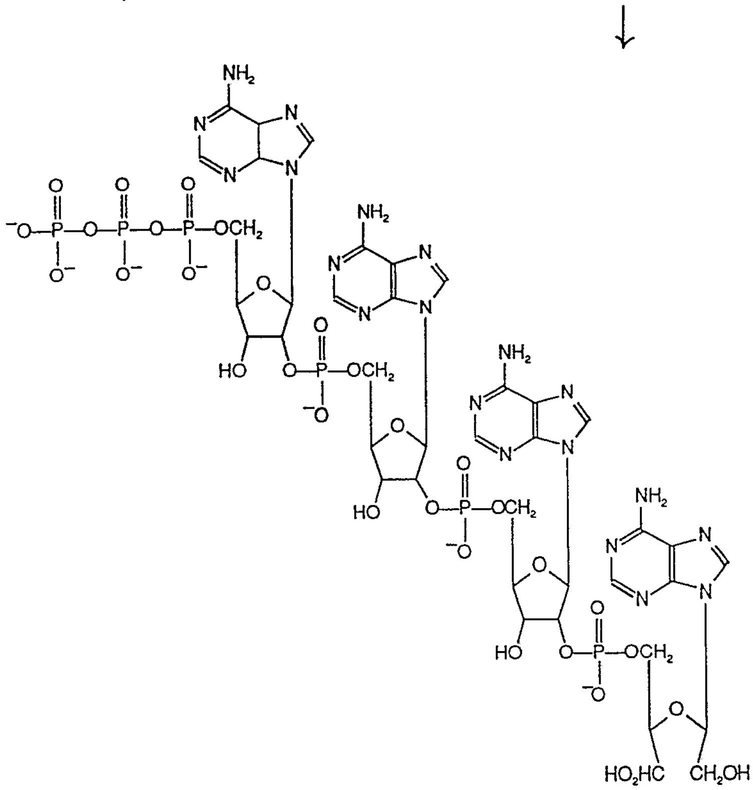

Les deux fonctions aldéhyde sont réduites par exemple par du borohydrure de sodium en deux fonctions alcool, ce qui conduit au composé (X).

Composé de formule (X):

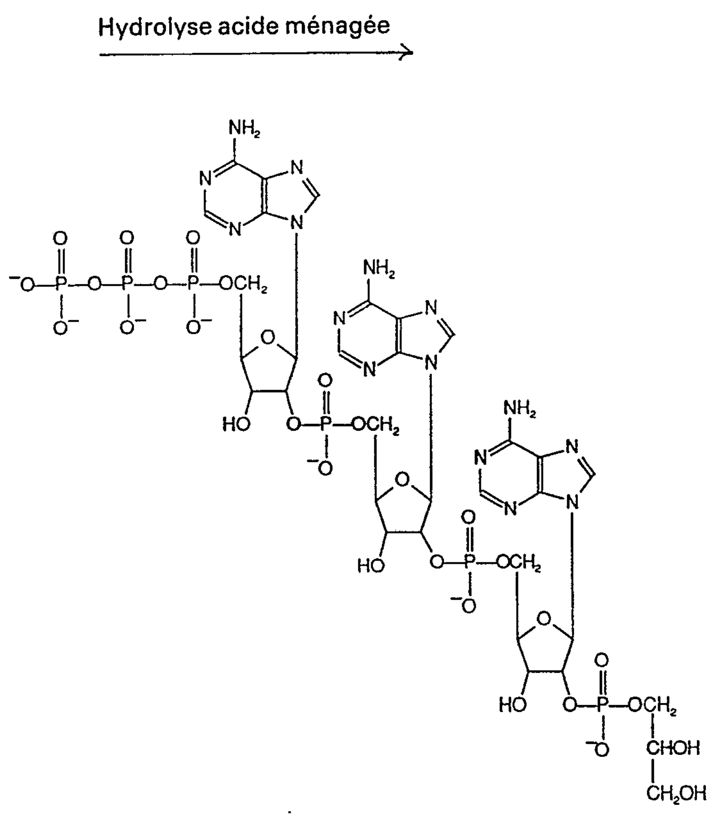


(XI)

L'hydrolyse acide ménagée avec par exemple de l'acide sulfurique dilué donne un (2'–5') oligoadénylate comportant un groupe terminal 2'-O-phosphoglycéryle. Les noyaux déphosphorylés correspondant aux composés de formules (IX), (X), (XI) s'obtiennent par traitement de ceux-ci, par la phosphatase alcaline bactérienne.

On obtient ainsi respectivement les composés de formules suivantes non revendiqués mais qui peuvent être utilisés dans la préparation d'oligonucléotides selon l'invention:

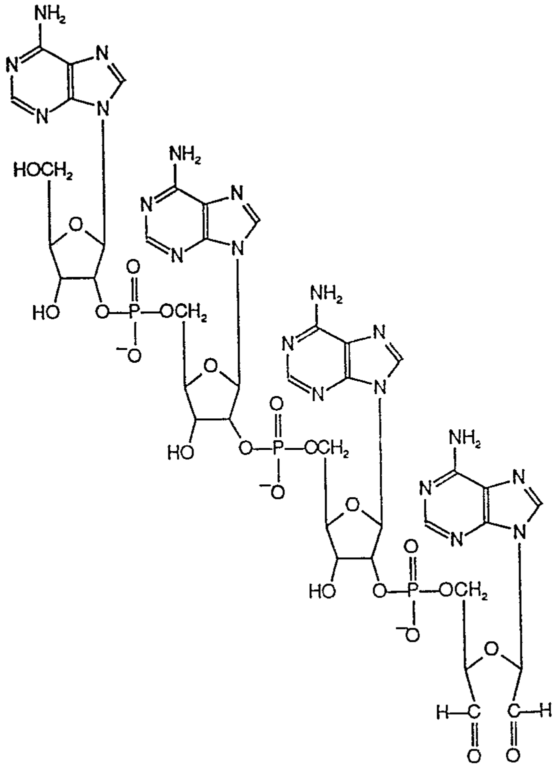

(XII)

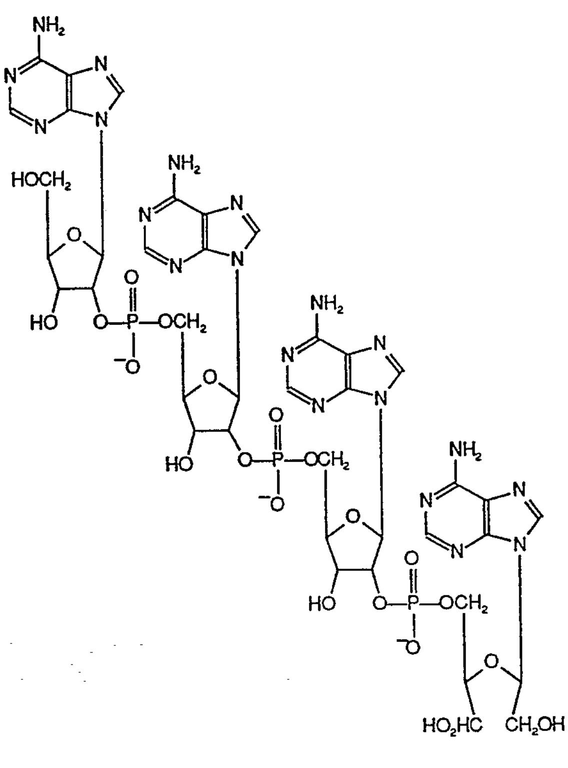

(XIII)

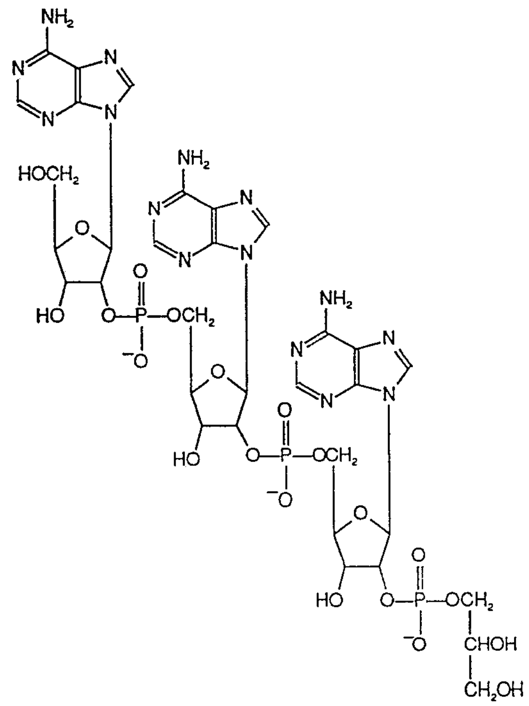

(XIV)

Analyse par chromatographie liquide hautes performances des $(2'\text{-}5')(A)_n$ phosphorylés et non phosphorylés et de leurs dérivés O-phosphoglycérylé

Les oligoadénylates $(2'\text{-}5')(A)_n$, les $(2'\text{-}5')(A)_n$-PGRo (dérivé 2'-O-phosphoglycéryle de $(2'\text{-}5')(A)_n$) et les oligoadénylates déphosphorylés correspondants (noyaux) sont isolés et caractérisés sur une colonne commercialisée sous le nom μBondapak $C_{18}$ dans un tampon phosphate d'aluminium (Brown R.E. et coll., 1981, Methods Enzymol. 78B, 208-216 et Knight M. et coll., 1980, Nature, 288, 189-192). On équilibre la colonne avec un tampon de phosphate d'ammonium 50 mM à pH 7,0 pour la séparation des $(2'\text{-}5')(A)_n$ phosphorylés ou avec un tampon phosphate d'ammonium 4 mM à pH 6,5 pour la séparation des noyaux déphosphorylés et on élue pendant 25 minutes avec 25 ml d'un gradient linéaire 0-50% de méthanol/eau (1:1 v/v). Toutes les séparations sont effectuées avec un chromatographe HPLC commercialisé sous le nom Varian 5000.

Analyse par électrophorèse haut voltage d'oligoadénylates $(2'\text{-}5')(A)_n$ et leurs produits de dégradation

Les oligoadénylates individuels $(2'\text{-}5')(A)_n$ et leurs produits de dégradation tels que des phosphates inorganiques (Pi), ATP et AmP sont séparés par électrophorèse sur papier commercialisé sous le nom Whatman DE81 dans de l'acide formique à 8,7% (v/v) à pH 1,8 pendant 0,5 à 6 heures à 60 V/cm dans un appareil d'électrophorèse haut voltage commercialisé sous le nom Gilson. On repère les composants radio-actifs et on les quantifie par une autoradiographie avec une pellicule commercialisée sous le nom Kodak X-Omat AR.

Activité de la phosphodiestérase dans les extraits cellulaires HeLa

La stabilité des $(2'\text{-}5')(A)_n$ et des noyaux analogues conformes à l'invention est déterminée dans les extraits cellulaires HeLa en mesurant la disparition des oligonucléotides. Le $(2'\text{-}5')(A)_5$ (5 μl) est incubé à la concentration finale de 0,02 mM avec 5 μl d'extraits cellulaires HeLa (22 mg de protéines par ml) en présence d'acide 4-(2-hydroxyéthyl)-1-pipérazine éthane sulfonique (Hepes) 20 mM à pH 7,6 d'acétate de magnésium 2,5 mM, de chlorure d'ammonium 33 mM, de dithiothreitol et de fluorure de phénylméthylsulfonyl 1 mM (tampon 1). La réaction est arrêtée par chauffage à 100 °C pendant 2 minutes et on centrifuge à 10000 Xg pendant 10 minutes. Le $(2'\text{-}5')(A)_3$ (1 nM) est additionné au surnageant comme référence interne et les produits résiduels sont quantifiés par HPLC comme décrit ci-dessus.

Activité de la phosphatase dans les extraits cellulaires HeLa

Les oligoadénylates $(2'\text{-}5')(A)_n$ et leurs analogues conformes à l'invention sont incubés dans des extraits cellulaires HeLa (5 μl) pendant des durées de temps différentes dans des essais de 20 μl, dans le tampon 1 défini ci-dessus (cf. l'essai à la phosphodiestérase) ou dans le même tampon complété avec de l'ATP 1 mM, du GTP guanosine triphosphate) 0,1 mM, du CTP (cytosine triphosphate) 0,6 mM, de la phosphate créatine 100 mM, de la phosphokinase créatine à raison de ce 160 μg/ml et tous les 20 aminoacides à raison de 500 μM chacun (tampon 2). On arrête la réaction en chauffant à 100 °C pendant 20 minutes, les protéines précipitées sont éliminées par centrifugation à 10000 Xg pendant 10 minutes. L'activité de la phosphatase est déterminée en mesurant la

disparition de [$\gamma$-$^{32}$P](2′-5′)$(A)_n$ et l'apparition concomitante de $^{32}P_i$, libérée après électrophorèse haut voltage, ou en mesurant l'accumulation de noyaux d'oligoadénylates, par chromatographie liquide hautes performances.

Micro-injection cellulaire

Des cellules d'HeLa sont cultivées sur des petits morceaux de verre (2 $mm^2$) à des densités qui permettent à environ 200 cellules de s'attacher à chacun des fragments de verre comme décrit dans Huez G. et coll., 1981, Proc. Natl Acad. Sci., 78, 908-911. On effectue des micro-injections selon la méthode originellement décrite par Graessmann (1983, Methods Enzymol., 101, 482-492). Un volume moyen de 0,5 nl (approximativement 1/10ème du volume cellulaire) est injecté dans le cytoplasme de chacune des cellules avec des micro-pipettes en verre de diamètre 0,5-1 µm. Les injections sont contrôlées sous un microscope à contraste de phase commercialisé par Leitz-Diavert avec un grossissement de 320.

Essai de l'activité antivirale

Les cellules sont infectées à des temps indiqués, généralement une heure après la micro-injection, avec du virus de stomatite vésiculaire (VSV) à une multiplicité de 10 pendant une heure à 37°C dans un milieu RPMI 1640 additionné de sérum de veau fœtal 5% (v/v). Les virus non adsorbés sont soigneusement éliminés par trois lavages avec du RPMI contenant du sérum de veau fœtal à 10% (v/v).

On détermine le titre de virus produit 18 heures plus tard selon les méthodes connues (Stewart W.E., 1970, J. Virol., 6, 795-799). Pour résumer $10^6$ cellules L929 sont mises dans des boîtes de Petri destinées à la culture de tissus (2 cm de diamètre). 24 heures après l'incubation, on étend 0,05 ml des suspensions de virus diluée (facteur de dilution 50) soigneusement sur la monocouche des cellules. Une heure plus tard, la suspension virale est éliminée par succion et on étend 2 ml d'agarose fondu à 1,6% (v/v) dans un milieu essentiel minimum complété par du sérum de veau fœtal à 2% (v/v) sur la monocouche des cellules. Les plaques sont incubées pendant 18 heures dans un incubateur à $CO_2$. Les plaques sont ensuite révélées par une solution à 1% (v/v) de rouge neutre dans une solution saline tamponnée isotonique de phosphate.

Résultats

Synthèse et modification chimique de (2′-5′)$(A)_n$

Des quantités en mg de (2′-5′)$(A)_n$ sont synthétisées enzymatiquement dans des extraits de cellules HeLa traités par l'interféron et fractionnés en une étape par une chromatographie à échange d'ions sur du DEAE trisacryl comme décrit précédemment. On effectue ensuite les modifications chimiques comme indiqué ci-dessus, pour obtenir les (2′-5′)$(A)_n$ modifiés, conformes à l'invention.

Stabilité du (2′-5′)$(A)_n$ et de ses analogues

Afin de tester la stabilité des composés selon l'invention vis-à-vis de la 2-phosphodiestérase, on a incubé les noyaux de (2′-5′)$(A)_n$ et leurs dérivés O-phosphoglycéryl, pendant 8 heures dans des extraits préparés soit à partir de cellules HeLa non traitées, soit à partir de cellules traitées par l'interféron, et leur disparition est suivie d'une chromatographie liquide haute performance. Pour les extraits cellulaires HeLa traités par l'interféron et selon les résultats publiés (Minks M.A., 1979, J. Biol. Chem., 254, 5058-5064; Williams B.R.G. et coll., 1978, Eur. J. Biochem., 92, 455-462; Schmidt A. et coll, 1979, Proc. Natl Acad. Sci. USA, 76, 4788-4792; Verhaegen-Lewalle M., 1982, Eur. J. Biochem., 126, 639-643), les noyaux non modifiés sont rapidement dégradés. Au contraire, le noyau (2′-5′)$(A)_n$-PGro ou le noyau du dérivé (2′-5′)$(A)_n$ porteur des deux fonctions alcool selon l'invention sont stables dans les mêmes conditions. Des résultats semblables ont été obtenus dans des extraits préparés à partir de cellules non traitées avec l'interféron.

La figure 1 est relative à la stabilité du noyau de (2′-5′)$(A)_5$ non modifié comparée à celle du noyau (2′-5′)$(A)_4$PGro.

En abscisses, on a représenté le temps, exprimé en heures, et en ordonnées le pourcentage de noyaux dégradés.

Les noyaux déphosphorylés de (2′-5′)$(A)_5$ (représentés par des triangles sur la figure 1) et leurs dérivés 2′-O-phosphoglycéryle correspondants (noyau (2′-5′)$(A)_4$PGro) (représentés par des cercles sur la figure 1) sont incubés avec des extraits cellulaires HeLa complétés (droite en trait plein) ou non (droite en trait pointillé) par de l'ATP 1 mM et un système régénérateur d'ATP.

Les noyaux sont introduits à une concentration initiale de 0,02 mM. On arrête les incubations aux temps indiqués par ébullition et les protéines dénaturées sont éliminées par centrifugation. Les noyaux résiduels du surnageant sont analysés par HPLC comme indiqué ci-dessus.

Activité antivirale des oligoadénylates (2′-5′)$(A)_n$ et de leurs dérivés phosphoglycéryle

Pour tester l'activité biologique des composés chargés tels que les (2′-5′)$(A)_n$ et de leurs analogues conformes à l'invention dans les cellules intactes, on a recours à la micro-injection avec des micropipettes, étant donné qu'elle permet d'introduire des quantités déterminées de composés dans le cytoplasme, et sans perturber, de façon significative, le métabolisme cellulaire (Graessmann M. 1983, Methods Enzymol., 101, 482-492).

Le tableau 1 ci-après est relatif à l'activité antivirale de (2′-5′)$(A)_5$ non modifié et de ses dérivés 2′-O-phosphoglycéryle selon l'invention.

Tableau 1

Activité antivirale des dérives 2'-0-Phosphoglyceryle des $(2'-5')(A)_n$ de l'invention $[(2'-5')(A)_n$-PGro] comparée a l'activité des oligoadenylates $(2'-5')(A)_n$ non modifies

| Essai n° | Composé testé | Concentration (µm) | Titre en virus (pfu/ 200 cellules) | % de témoin |
|---|---|---|---|---|
| 1 | – | – | $1,6 \times 10^3$ | 100,0 |
| | $(2'-5')(A)_5$ | 10 | $1,5 \times 10^3$ | 93,7 |
| | $(2'-5')(A)_4$PGro | 10 | $2,5 \times 10^1$ | 1,5 |
| 2 | – | – | $1,6 \times 10^4$ | 100,0 |
| | $(2'-5')(A)_4$PGro | 10 | $1,7 \times 10^2$ | 1,1 |
| | $(2'-5')(A)_4$PGro | 1,0 | $2,5 \times 10^3$ | 15,6 |
| | $(2'-5')(A)_4$PGro | 0,1 | $2,1 \times 10^4$ | 131 |

Les essais dont les résultats ont été rassemblés dans le tableau 1 ont été effectués comme suit.

Des cellules Hela qui croissent sur des morceaux de verre sont micro-injectées avec 0,5 nl à chacun des $(2'-5')(A)_5$ ou des produits relatifs aux concentrations indiquées.

Une heure après, les cellules sont infestées avec du virus de stomative vésiculaire (multiplicité de l'infection = 10) et le rendement du virus est déterminé 18 heures après, par essai de plaques dans des cellules L929.

Etant donné que 0,5 nl représente approximativement 1/10ème du volume cellulaire, on peut estimer que les concentrations intracytoplasmiques finales des oligomères soit environ un dixième des valeurs dans le tableau 1.

Comme le montre le tableau 1, le $(2'-5')(A)_5$ non modifié n'affecte pas la production de virus de stomative vésiculaire lorsqu'il est microinjecté dans les cellules HeLa à une concentration intracytoplasmique d'environ 1 µM.

Au contraire, le $(2'-5')(A)_4$PGro réduit la croissance du virus d'environ 100 fois à la même concentration et est toujours actif à une concentration finale de 100 nM.

Conclusion

Les modifications chimiques apportées sur les oligoadénylates 2'-5', conformément à l'invention, ont conduit à des dérivés qui sont toujours actifs vis-à-vis de l'endoribonucléase et qui sont stables vis-à-vis de la dégradation de la phosphodiestérase dans les extraits acellulaires, et présentent une activité biologique antivirale accrue dans les cellules.

Exemple 2

Synthèse du γS-$(2'-5')(A)_n$Ox Red

On rappelle que l'on désigne par γS-$(2'-5')(A)_n$Ox Red les composés qui peuvent être représentés par la formule suivante:

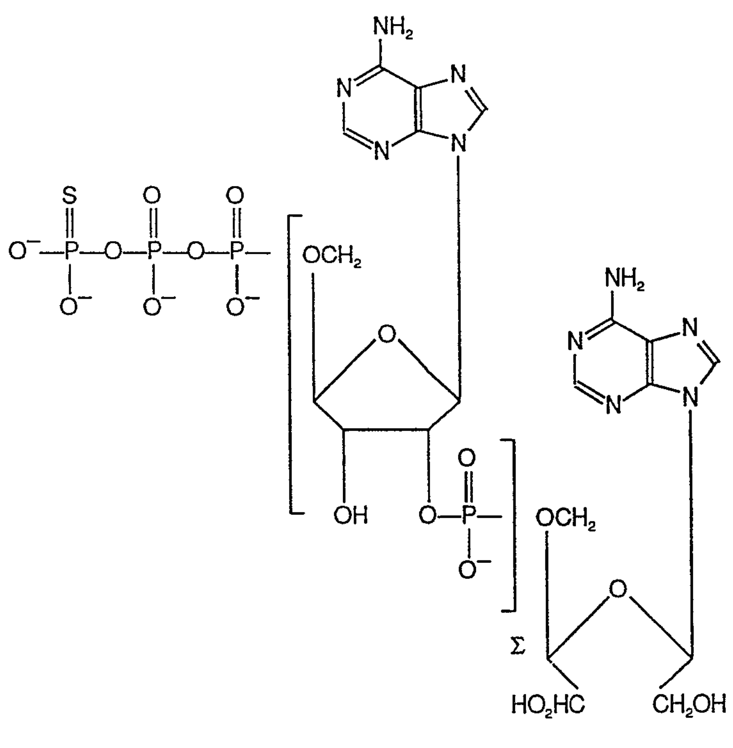

(XXX)

dans laquelle Σ est un nombre entier égal ou supérieur à 1.

Le produit de départ est l'adénosine 5'-O-(3-thiotriphosphate) (ci-après nommé γS ATP) de formule (XXXI):

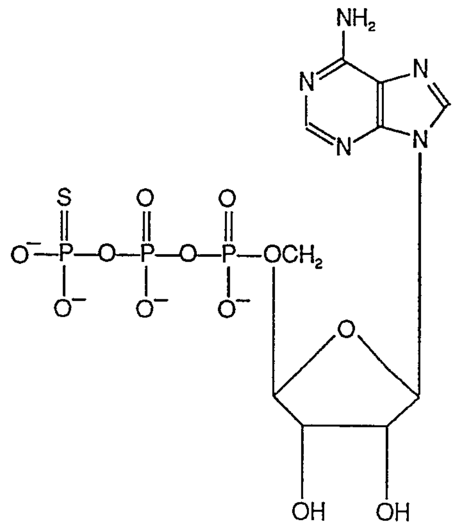

(XXXI)

que l'on polymérise par exemple par voie enzymatique à l'aide d'une préparation partiellement purifiée de 2–5A synthétase, pour obtenir le composé de formule (XXXII), ci-après désignés par γS-(2'–5')$(A)_n$:

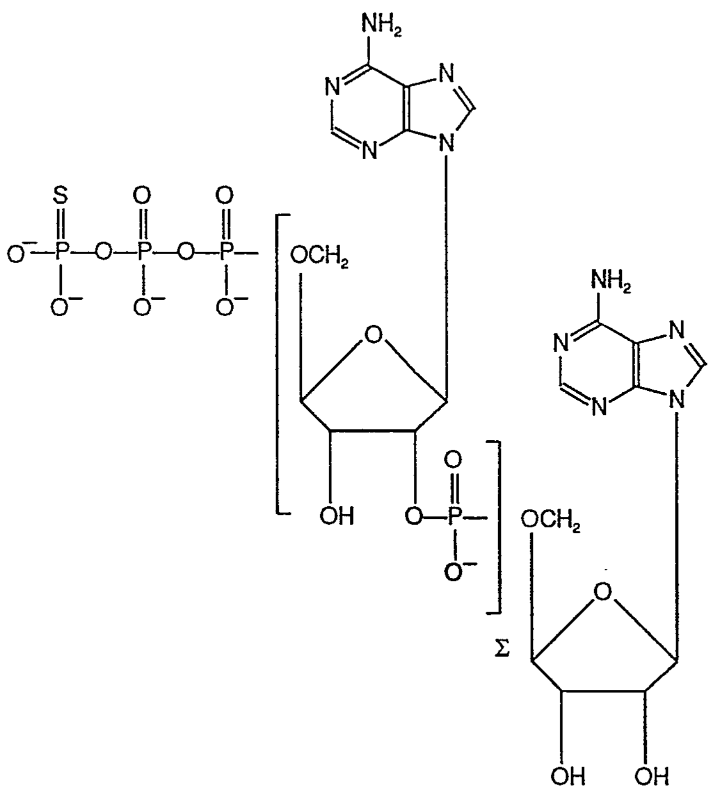

(XXXII)

dans laquelle Σ est un nombre entier égal ou supérieur à 1.

On purifie ensuite, par exemple, par chromatographie d'échange d'ions sur DEAE-trisacryl. On effectue une oxydation, par exemple par l'ion periodate, du glycol terminal, pour obtenir le composé de formule (XXXIII):

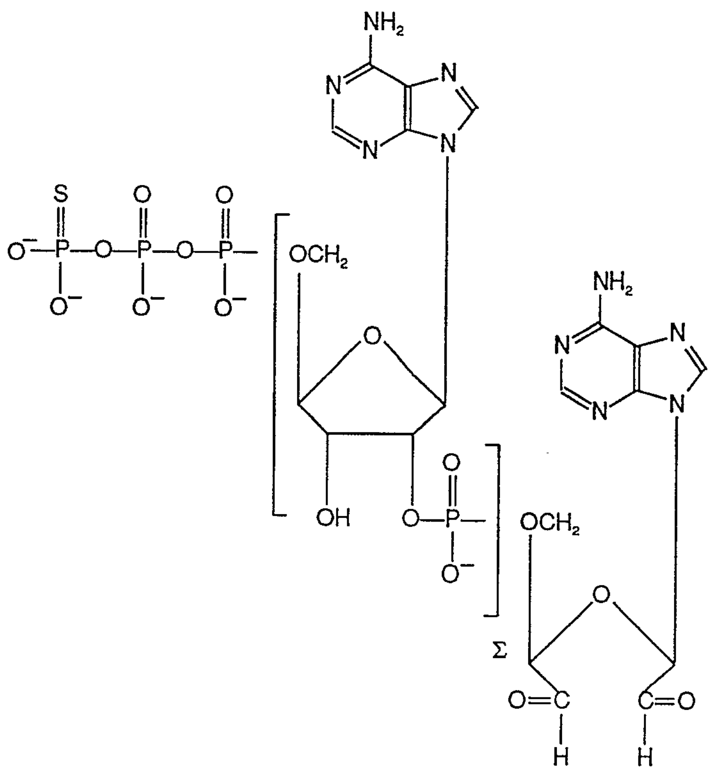

(XXXIII)

dans laquelle Σ est un nombre entier égal ou supérieur à 1.

Puis on réduit les groupes aldéhyde, par exemple, par le borohydrure de sodium pour obtenir le γS-(2′-5′)(A)$_n$Ox Red.

On purifie ensuite, par exemple, par filtration moléculaire et on analyse sur chromatographie HPLC.

Stabilité métabolique

Les oligoadénylates γS(2′-5′)(A)$_n$ conformes à l'invention est comportant un atome de soufre sur le phosphore en gamma du groupe triphosphate relié au carbone en 2′ à la première unité oligonucléosidique et qui peuvent être obtenus comme indiqué ci-dessus et en particulier le S(2′-5′)(A)$_n$Ox Red ont une stabilité métabolique en système acellulaire supérieure à celle des dérivés protégés uniquement à leurs extrémités 3′OH.

Les figures 2 et 3 ci-après représentent les pourcentages de dégradation dans un extrait de cellules HeLa, respectivement du (2′-5′)(A)$_4$ non modifié, du S-(2′-5′)(A)$_4$Ox Red selon l'invention et du (2′-5′)(A)$_4$Ox Red selon l'invention.

La figure 2 est relative à un milieu contenant de l'ATP (1 mM) (très proches des conditions in vivo).

La figure 3 est relative à un milieu sans ATP.

On a représenté sur chacune des figures 2 et 3 en abscisses le temps (en heures) et en ordonnées le pourcentage de produits non dégradés.

Dans les figures 2 et 3, la courbe repérée par des triangles est relative au γS(2′-5′)(A)$_4$Ox Red, la courbe repérée par des points est relative au composé (2′-5′)(A)$_4$Ox Red et la courbe repérée par des carrés est relative au composé (2′-5′)(A)$_4$ non modifié.

Activité biologique

a) Liaison à l'endoribonucléase

Les différents analogues selon l'invention se lient à l'endoribonucléase avec une affinité quasi identique à celle classiquement utilisée dans ce domaine de «radiobinding» initialement décrit par Knight et coll., 1980.

b) Activité antivirale

Les différents composés ont été micro-injectés à l'aide de micropipettes dans le cytoplasme de cellules HeLa. Comme le montrent les résultats indiqués dans le tableau 2 ci-après, le γS-(2′-5′)(A)$_n$Ox Red présente une activité antivirale nettement supérieure à celle du composé (2′-5′)(A)$_n$ non modifié.

Tableau 2

Activité antivirale d'analogues du (2′-5′)(A)$_n$

| Dérivé | Concentration | Titre en virus |
|---|---|---|
| 1 - | - | 4,0 × 10$^5$ |
| (2′-5′)(A)$_n$ | 10 μM | 1,2 × 10$^5$ (N.S.) |

Tableau 2 (suite)

Activité antivirale d'analogues du (2'-5')$(A)_n$

| Dérivé | Concentration | Titre en virus |
|---|---|---|
| 2 - | - | $3,3 \times 10^5$ |
| (2'-5')$(A)_n$ Ox. Red | 100 µM | $2,6 \times 10^2$ |
| (2'-5')$(A)_n$ Ox. Red | 10 nm | $5,2 \times 10^3$ |
| 3 - | - | $3,8 \times 10^5$ |
| γS(2'-5')$(A)_n$ | 10 µM | $3,3 \times 10^5$ (N.S.) |
| γS(2'-5')$(A)_n$ | 1 µM | $2,4 \times 10^5$ (N.S.) |
| 4 - | - | $2,3 \times 10^4$ |
| γS ATP | 10 µM | $1,3 \times 10^4$ (N.S.) |
| 5 - | - | $3,8 \times 10^5$ |
| γS(2'-5')$(A)_n$ Ox. Red | 1 µM | < 10 |
| γS(2'-5')$(A)_n$ Ox. Red | 10 nM | <10 |
| γS(2'-5')$(A)_n$ Ox. Red | 1 nm | < 10 |
| γS(2'-5')$(A)_n$ Ox. Red | 10 pM | $1,1 \times 10^2$ |
| γS(2'-5')$(A)_n$ Ox. Red | 1 pm | $2,5 \times 10^3$ |
| noyau | | |
| γS(2'-5')$(A)_n$ Ox. Red (1) | 10 µM | $6,2 \times 10^4$ |

N.S.: Différence par rapport au témoin non significative
< 10: Correspond à des cellules totalement protégées
(1): Le même produit déphosphorylé par la phosphatase alcaline

Les essais, pour déterminer l'activité antivirale, sont effectués comme suit.

Des cellules HeLa (200 par point expérimental environ) attachées à un support de verre sont microinjectées chacune par $5 \times 10^{-10}$ ml de (2'-5')$(A)_n$ ou d'un analogue de (2'-5')$(A)_n$ aux concentrations indiquées.

Les cellules sont infectées une heure plus tard par le virus de la stomatite vésiculaire (multiplicité d'infection = 10) et la multiplication virale est déterminée 18 heures plus tard par un essai en plages de lyse sur des fibroblastes de souris L929. Comme $5 \times 10^{-10}$ ml représentent environ 1/10ème du volume cellulaire, les concentrations intracellulaires finales en 2–5 A sont environ 1/10ème des valeurs indiquées dans ce tableau.

L'invention concerne également les sels que les oligonucléotides ci-dessus peuvent former avec les bases en particulier les bases minérales ou organiques. Parmi les sels de base minérale, on préfère les sels de sodium et de potassium. Parmi les sels organiques, on préfère les sels d'amine, d'alcoylamine et d'arylamine, en particulier ceux d'amines secondaires, tels que la diéthylamine, la pipérazine, ou d'amines tertiaires, tels que la méthylamine, la pyridine, la méthylpipérazine etc. Parmi tous ceux-ci, les sels physiologiquement acceptables sont préférés. Les sels peuvent être lyophilisés. Les composés selon l'invention ont des propriétés biologiquement intéressantes, en particulier des propriétés du type de l'interféron, et plus particulièrement une activité antivirale.

Les composés selon l'invention sont capables d'inhiber la synthèse de l'ADN, en particulier la replication dans les cellules et/ou de dégrader l'ARN viral, en empêchant ainsi la synthèse des protéines, plus particulièrement des protéines virales dans les cellules infectées par le virus à des concentrations nanomolaires.

Les composés selon l'invention sont stables et résistent à la dégradation par les phosphodiestérases et le temps de résistance vis-à-vis des phosphatases est accru.

Une classe préférée de composés selon l'invention résistent à la dégradation par les phosphatases.

Les oligonucléotides selon l'invention sont donc des substituts appropriés de l'interféron et de ses applications connues. Ils peuvent être préparés reproductiblement sous forme hautement purifiée, comme réactifs biologiques, en particulier comme référence de comparaison dans les essais qualitatifs et quantitatifs, dans les cultures cellulaires, des composés de l'interféron ou d'autres substances semblables à l'interféron.

L'invention concerne également les sels pharmaceutiquement acceptables des oligonucléotides définis ci-dessus en particulier ceux appropriés pour l'administration in vivo.

L'invention concerne également les compositions pharmaceutiques associant les susdits oligonucléotides, de préférence sous la forme de sels pharmaceutiquement acceptables, avec un véhicule pharmaceutique.

L'invention fournit ainsi des compositions pharmaceutiques ayant une activité semblable à celle de l'interféron en utilisant un composé chimique déterminé sous forme de haute pureté, ne présentant pas de toxicité, étant stable et facile à manipuler.

La composition selon l'invention peut être sous forme de préparation administrables par voie orale ou rectale, en utilisant des solides ou des liquides appropriés pour un tel type d'administration ou sous forme de préparations injectables

stériles contenant au moins l'un quelconque des nucléotides en association avec des véhicules liquides appropriés stériles, de préférence isotoniques.

D'autres formes appropriées de préparations consistant en pommades dans lesquelles les oligonucléotides de l'invention sont associés avec des véhicules en pommade.

L'une quelconque des techniques de préparation classiquement utilisées pour associer l'interféron avec les supports pharmaceutiques peut être utilisée pour préparer les compositions pharmaceutiques selon l'invention.

Les oligonucléotides selon l'invention peuvent être associés à d'autres vecteurs appropriés, tels que les liposomes.

Les compositions de l'invention ont des propriétés antivirales et sont en particulier susceptibles d'inhiber les maladies virales susceptibles d'être suivies de troubles tumoraux, par exemple les maladies induites par les virus de l'hépatite B ou les différentes formes de virus de l'herpès.

Plus généralement, les compositions de l'invention sont utiles pour le traitement et la prévention de maladies virales, et pour les traitements antitumoraux vis-à-vis des tumeurs susceptibles d'être également délimitées par les traitements à l'interféron.

On notera que les doses auxquelles les compositions sont utilisées sont déterminées selon la nature de la maladie qui afflige le patient et les conditions particulières de santé.

Les dosages appropriés sont déterminés par le médecin, comme la pratique l'exige dans ces domaines d'application.

**Revendications pour l'Etat contractant: AT**

1. Procédé de préparation d'oligonucléotides de formule (I)

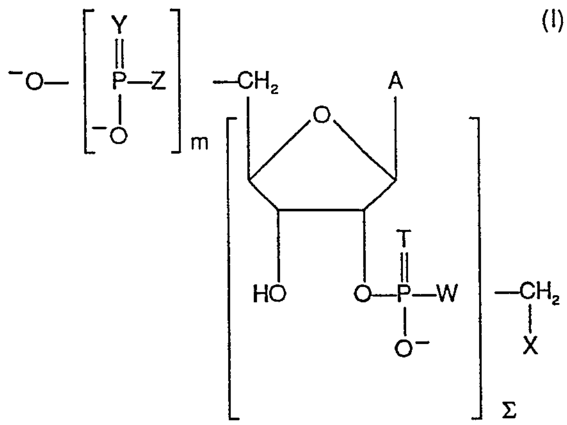
(I)

dans laquelle:
- Y et T sont identiques ou différents et représentent O, S, Se ou NH;
- Z et W sont identiques ou différents et représentent O, S ou NH;
- l'un au moins des éléments Y ou Z de l'un au moins des groupes phosphate étant différent de l'oxygène;
- m est supérieur ou égal a 1;
- X est choisi dans le groupe constitué par:

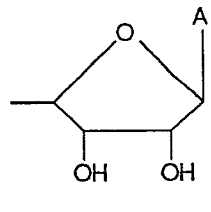

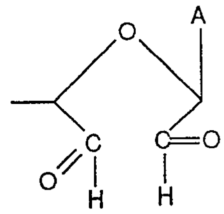

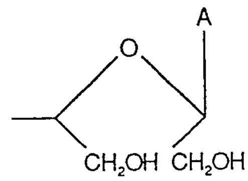

$—CHOHCH_2OH$

Les fonctions alcools de ces radicaux étant éventuellement estérifiées par des acides carboxyliques $R_3COOH$, $R_3$ représentant un radical alcoyle de 1 à 5 atomes de carbone ou un groupe phényle;
- Σ est un nombre entier égal à:
- n lorsque X représente $-CHOHCH_2OH$,
- n-1 lorsque X représente:

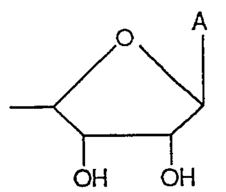

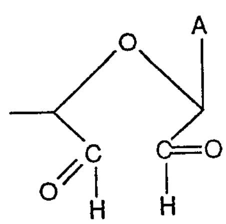

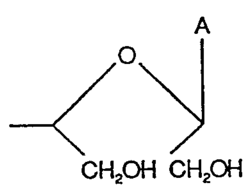

n étant un nombre entier supérieur ou égal à 2;
- m est un nombre entier supérieur ou égal à 1;
- A est une base choisie parmi l'adénine ou ses dérivés, notamment ceux de formule:

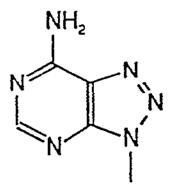

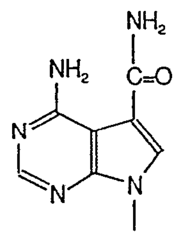

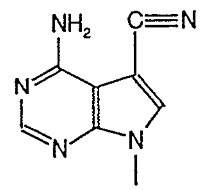

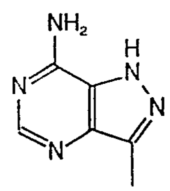

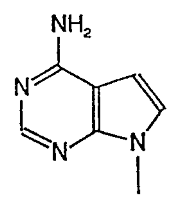

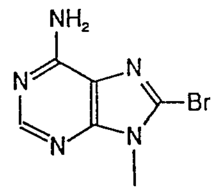

caractérisé en ce qu'il comprend:

1) la polymérisation de composés de formule (XIX) suivante:

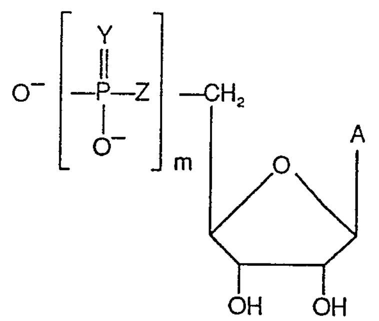

(XIX)

pour obtenir un composé de formule (Ibis) suivante:

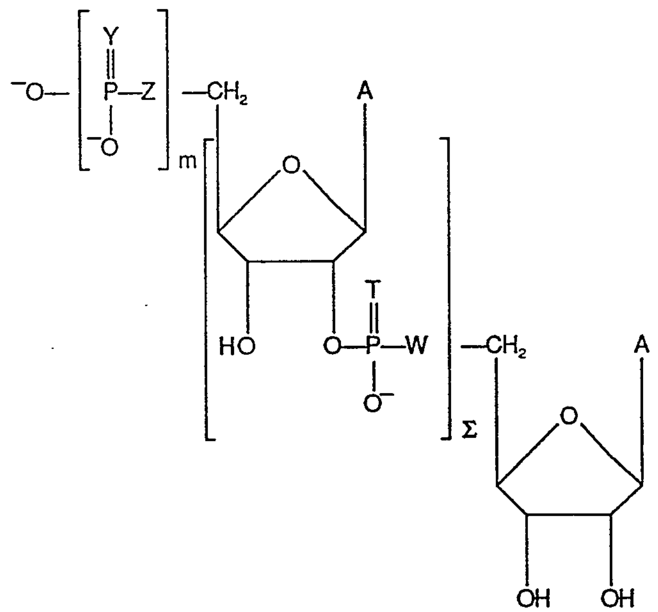

(I bis)

dans laquelle Y, T, Z, W, m et A ont les significations indiquées ci-dessus,

Σ est un nombre entier égal à n−1, n étant supérieur ou égal à 2,

– et si nécessaire les étapes chimiques suivantes à savoir:

2) l'oxydation éventuelle du groupe glycol pour introduire des fonctions aldéhyde sur les carbones en 2′ et 3′ de la dernière unité nucléosidique et obtenir le composé de formule (Iter):

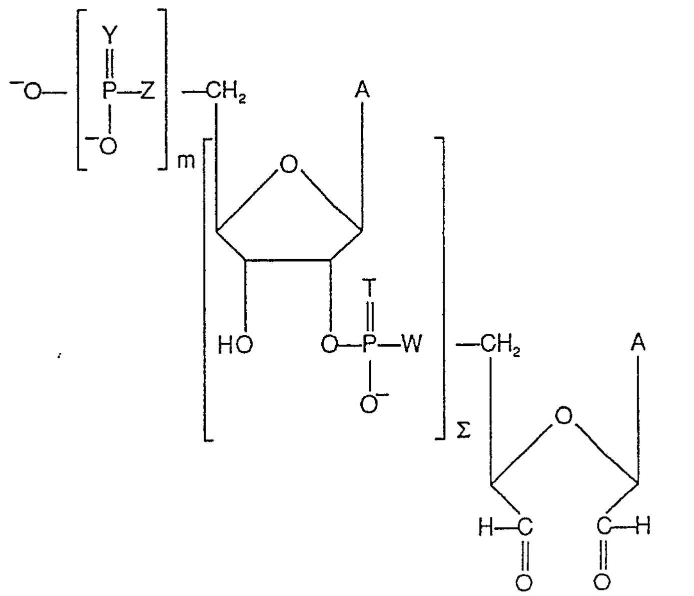

(I ter)

3) la réduction éventuelle des deux fonctions aldéhyde en fonctions alcool pour obtenir le composé de formule (Iquater):

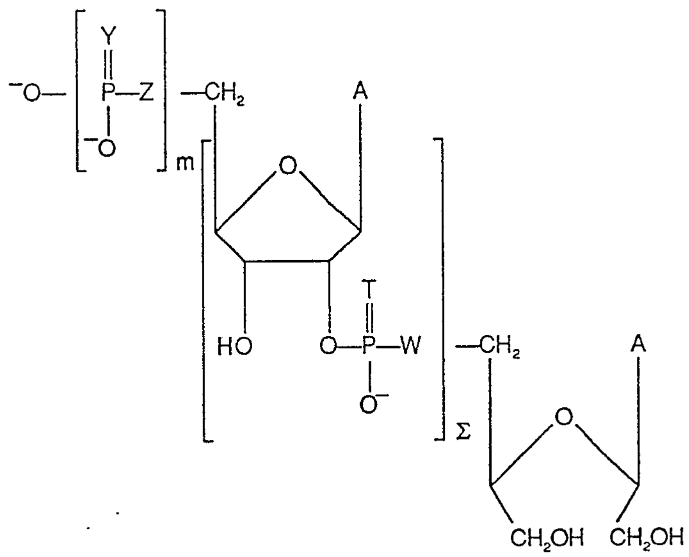

(I quater)

4) l'hydrolyse éventuelle, dans des conditions évitant la bêta-élimination, pour obtenir le composé de formule (Iquinquiès) suivante:

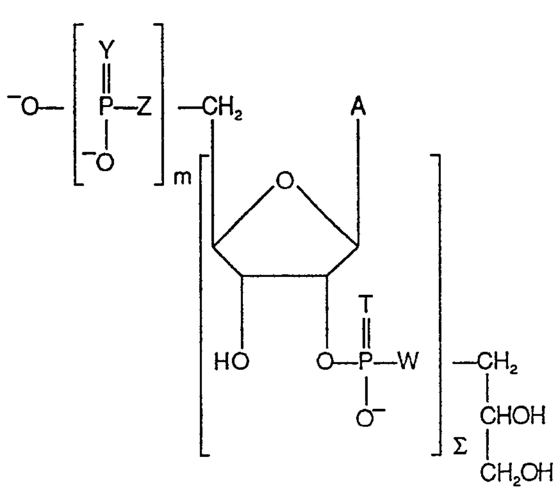

(I quinquies)

2. Procédé selon la revendication 1 de préparation d'oligonucléotides de formule (XX):

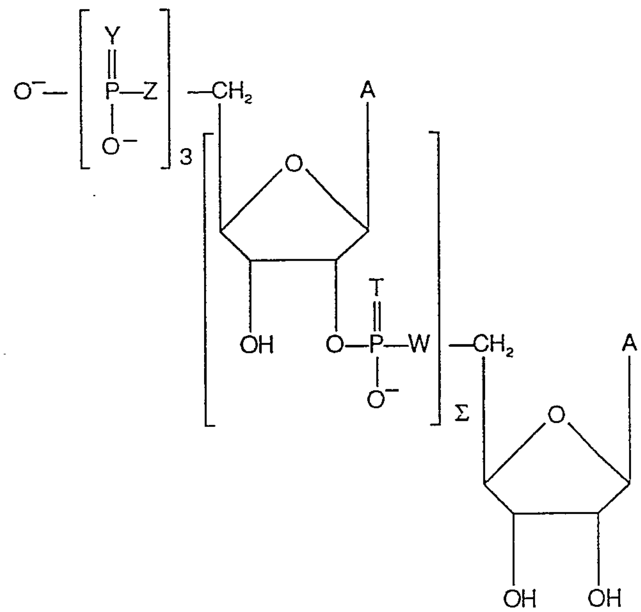

(XX)

dans laquelle:
- Y et T, identiques ou différents, représentent O, S, Se, NH;
- Z et W, identiques ou différents, représentent O, S, NH;
- les éléments Y et Z de l'un au moins des groupes phosphate ne représentent pas simultanément l'oxygène;
- Σ est un nombre entier variant de 1 à 9;
- A est une base choisie parmi l'adénine ou ses dérivés, notamment ceux de formule:

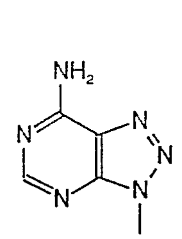

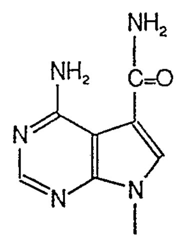

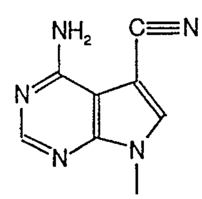

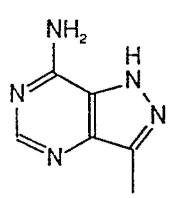

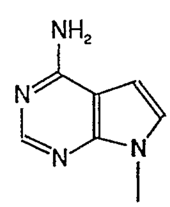

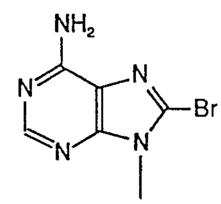

caractérisé en ce qu'il comprend:

1) la polymérisation d'un composé de formule (XIXbis) suivante:

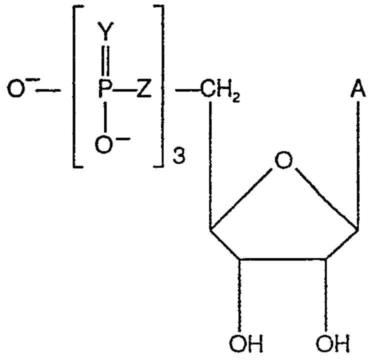

(XIX bis)

dans laquelle:

- Y représente O, S, Se ou NH;

- Z représente O, S ou NH;

- les éléments Y et Z de l'un au moins des groupes phosphate ne représentent pas simultanément l'oxygène;
- A a les significations indiquées à la revendication 1;

2) l'oxydation éventuelle du groupe glycol terminal, notamment par l'ion periodate pour transformer le glycol en deux fonctions aldéhyde et obtenir un composé de formule (XXI) suivante:

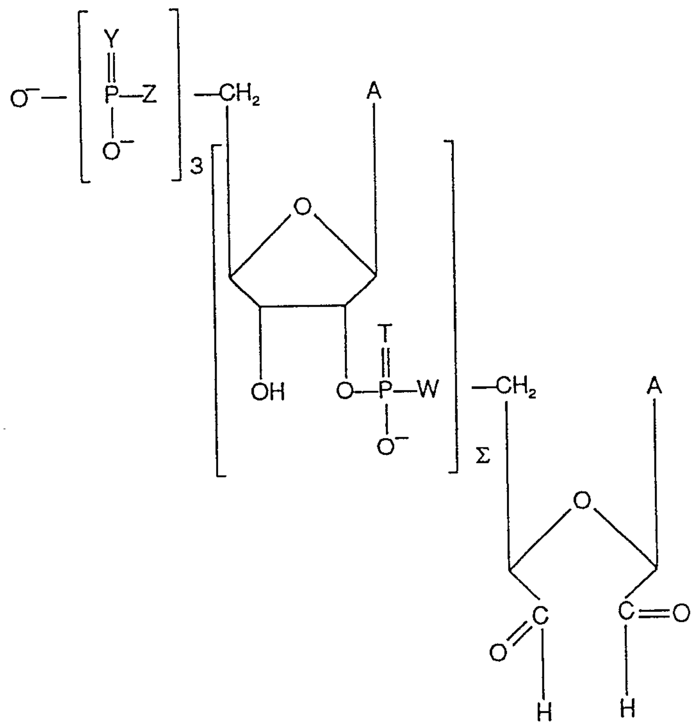

(XXI)

dans laquelle Y, Z, T, W, Σ et A ont les significations indiquées ci-dessus;

3) la réduction éventuelle des fonctions aldéhyde, notamment par le borohydrure de sodium pour transformer les deux susdites fonctions aldéhyde en fonctions alcool et obtenir le composé de formule (XXII) suivante:

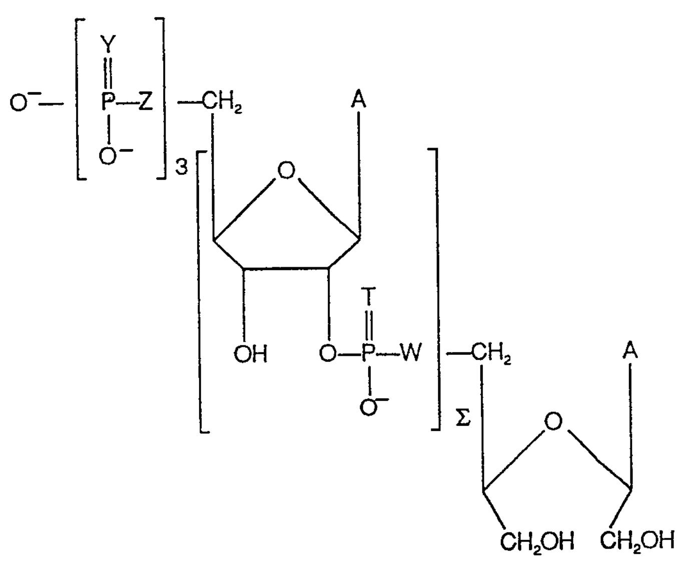

(XXII)

4) l'hydrolyse éventuelle, notamment l'hydrolyse acide ménagée, pour éliminer le noyau de ribose et obtenir le composé de formule (XXIII) suivante:

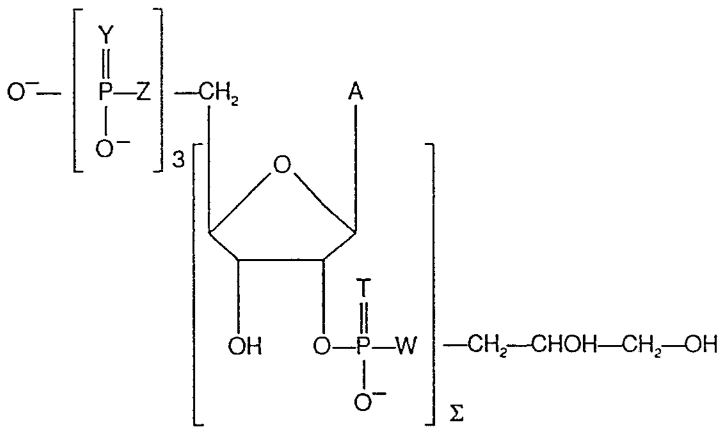

(XXIII)

3. Procédé selon la revendication 1 de préparation d'oligonucléotides répondant à la formule générale (II):

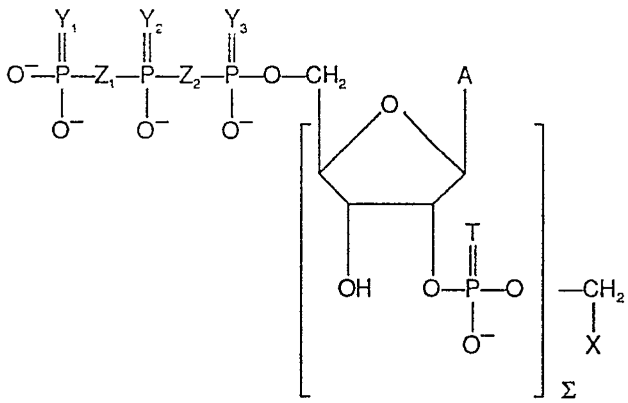

(II)

dans laquelle:
- $Y_1$, $Y_2$, $Y_3$, T sont identiques ou différents et représentent O, S, Se, NH;
- $Z_1$ et $Z_2$ sont identiques ou différents et représentent O, S, NH;
l'un au moins des éléments $Y_1$, $Y_2$, $Y_3$, $Z_1$, $Z_2$ étant différent de l'oxygène;
- X est choisi dans le groupe constitué par:

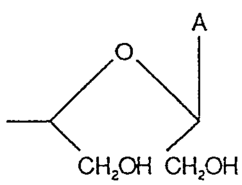

$-CHOHCH_2OH$

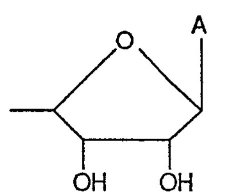

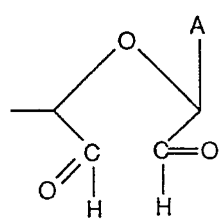

les fonctions alcools de ces radicaux étant éventuellement estérifiées par des acides carboxyliques $R_3COOH$, $R_3$ représentant un radical alcoyle de 1 à 5 atomes de carbone ou un groupe phényle;
- $\Sigma$ représente un nombre entier égal à n, lorsque X représente $-CHOHCH_2OH$, et $\Sigma$ représente un nombre entier égal à n-1 lorsque X est différent de $-CHOHCH_2OH$, n étant un nombre entier supérieur ou égal à 2;
- A est une base choisie parmi l'adénine ou ses dérivés, notamment ceux de formule:

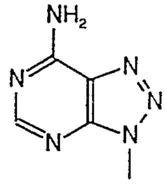

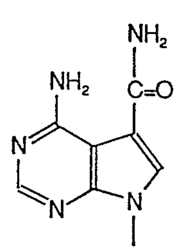

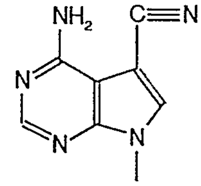

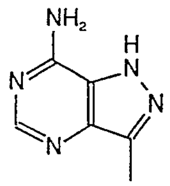

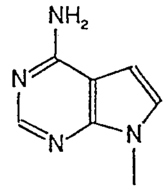

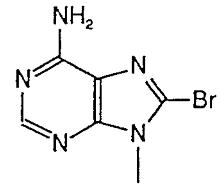

4. Procédé selon la revendication 1 de réparation d'oligonucléotides de formule (V):

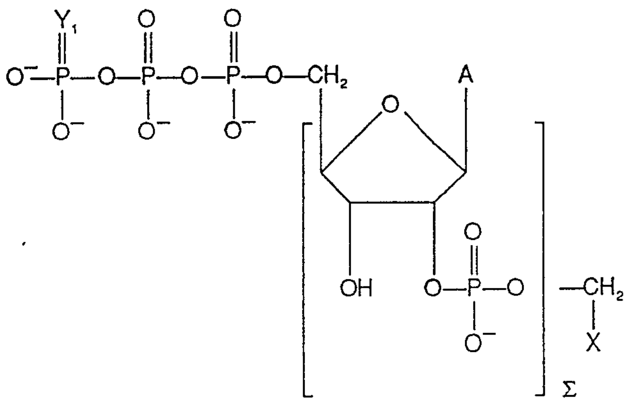

(V)

dans laquelle:
- $Y_1$ représente NH, Se, S; - X représente:

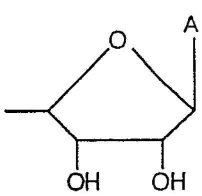

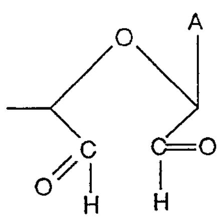

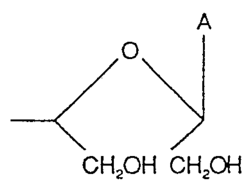

$-CHOHCH_2OH$

- Σ est un nombre entier égal à n, lorque X représente $-CHOHCH_2OH$, n-1 lorsque X est différent de $-CHOHCH_2OH$, n variant de 2 à 10;

caractérisé en ce que l'on polymérise le composé de formule (XXIV) suivante:

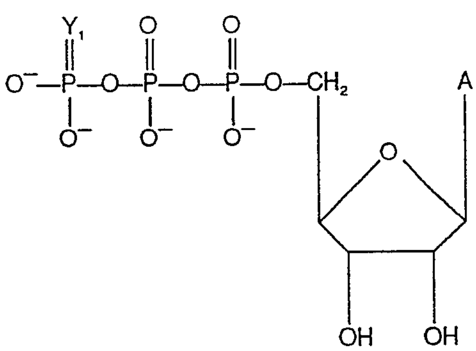

(XXIV)

et que l'on effectue les étapes 2°, 3° et 4° comme indiqué à la revendication 1.

5. Procédé selon la revendication 1 de préparation d'oligonucléotides de formule (VI):

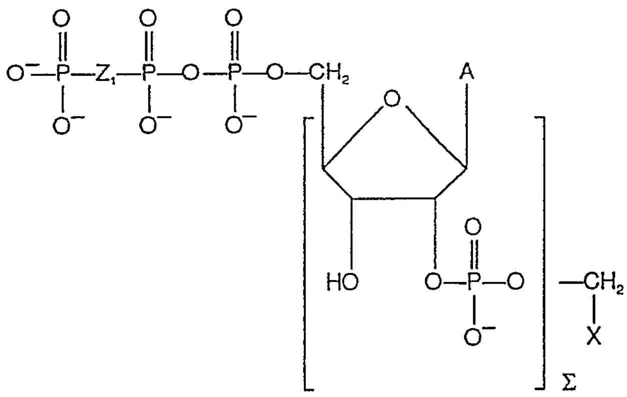

(VI)

dans laquelle:

- $Z_1$ représente S ou NH; - X est choisi dans le groupe constitué par:

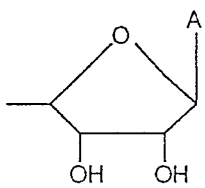

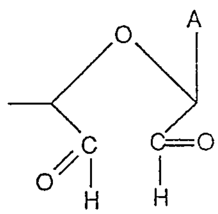

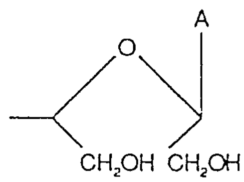

$—CHOHCH_2OH$

- Σ est un nombre entier égal à 1, lorsque X représente $-CHOHCH_2OH$ et égal à n−1 lorsque X est différent de $-CHOHCH_2OH$, n variant de 2 à 10;

caractérisé en ce qu'on polymérise le composé de formule (XXV) suivante:

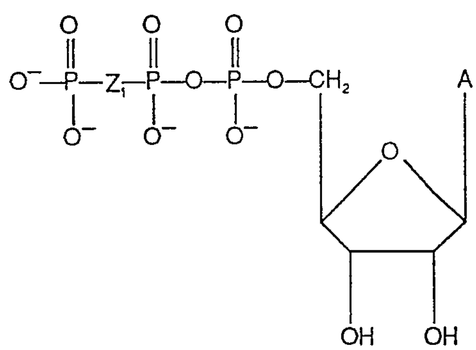

(XXV)

dans laquelle $Z_1$ représente S ou NH et A a la signification indiqué à la revendication 1;

et que l'on effectue les étapes 2°, 3° et 4° comme indiqué à la revendication 1.

6. Procédé selon la revendication 1 de préparation d'oligonucléotides de formule (IV):

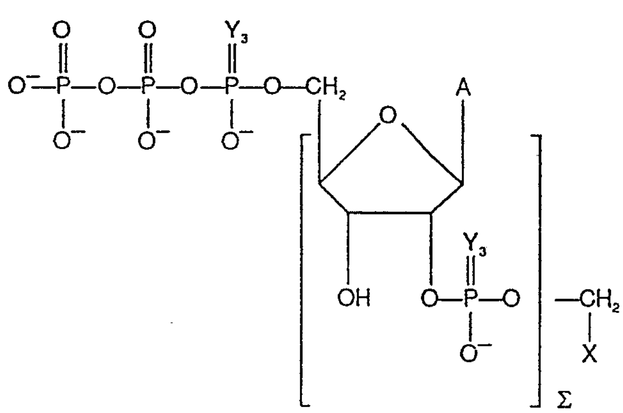

(IV)

dans laquelle:
- $Y_3$ représente NH, S ou Se;
- Σ, X et A ont les significations indiquées ci-dessus;

est caractérisé en ce qu'on polymérise le composé de formule (XXVI) suivante:

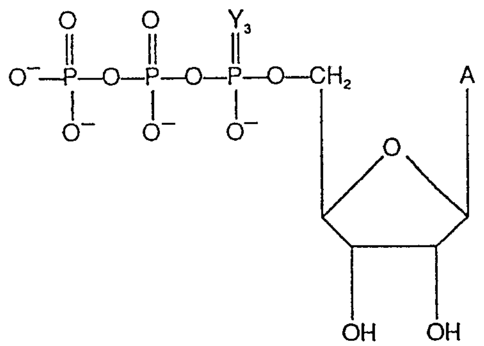

(XXVI)

dans laquelle:
- $Y_3$ représente S, Se, NH;
- A a les significations indiquées ci-dessus;

et que l'on effectue les étapes 2°, 3° et 4°, comme indiqué à la revendication 2.

7. Procédé selon la revendication 1 de préparation d'oligonucléotides de formule (Vbis) suivante:

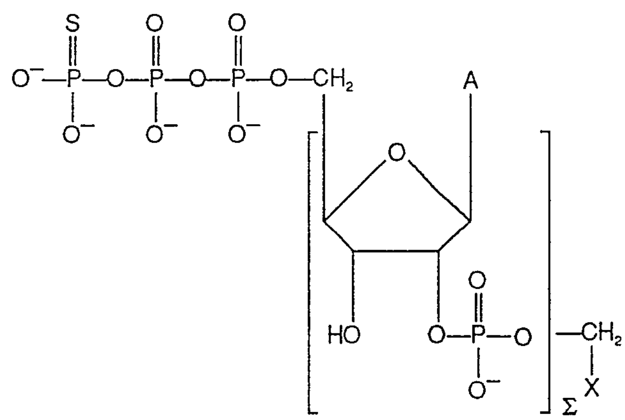

(V bis)

dans laquelle: - X représente:

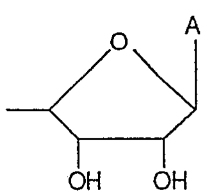

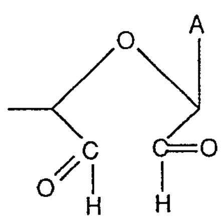

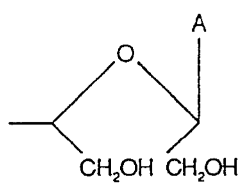

$-CHOHCH_2OH$

- Σ est un nombre entier égal à n lorsque X représente $-CHOHCH_2OH$, n-1 lorsque X est différent de $-CHOHCH_2OH$,
- A a les significations indiquées à la revendication 1, de préférence l'adénine;

caractérisé en ce qu'on polymère le composé de formule (XXIVbis) suivante:

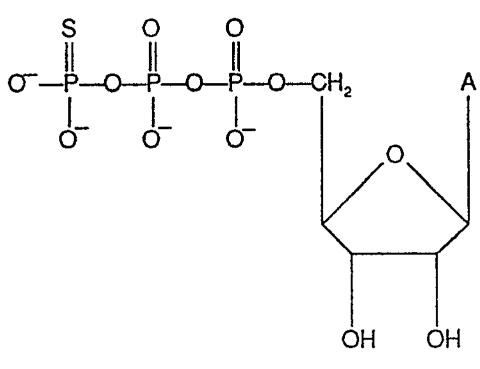

(XXIV bis)

et que l'on effectue les étapes 2°, 3° et 4° comme indiqué à la revendication 1.

8. Procédé de préparation d'oligonucléotides selon la revendication 1 de formule (VII):

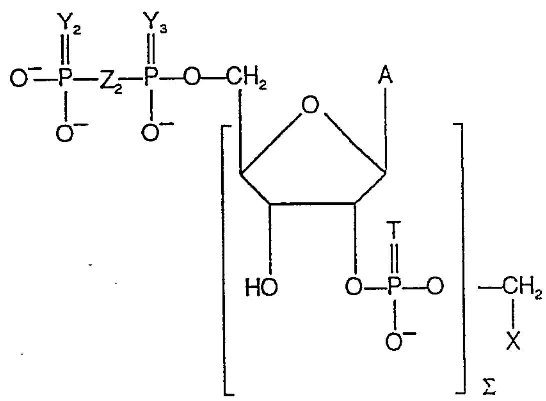

(VII)

dans laquelle:
- $Y_2$, $Y_3$, T sont identiques ou différents et représentent O, S, Se, NH;
- $Z_2$ représente O, S, NH;
l'un au moins des éléments $Y_2$, $Y_3$, $Z_2$ étant différent de l'oxygène;
- X est choisi dans le groupe constitué par:

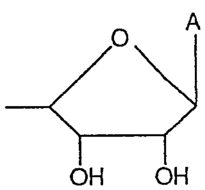
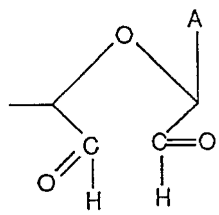
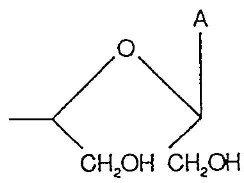
$-CHOHCH_2OH$

- Σ est un nombre entier égal à n, lorsque X représente $-CHOHCH_2OH$ et est un nombre entier égal à n−1, lorsque X est différent de $-CHOHCH_2OH$, n étant un nombre entier variant de 2 à 10;
- A est une base choisie parmi l'adénine ou ses dérivés, notamment ceux de formule:

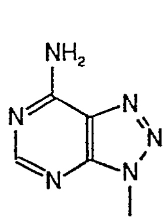
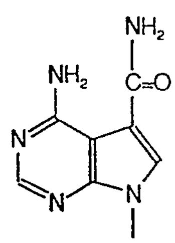
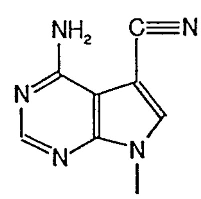
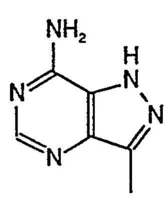
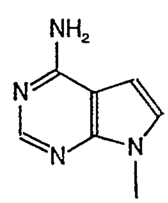
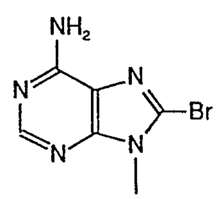

9. Procédé de préparation d'oligonucléotides selon la revendication 1 de formule (VIII):

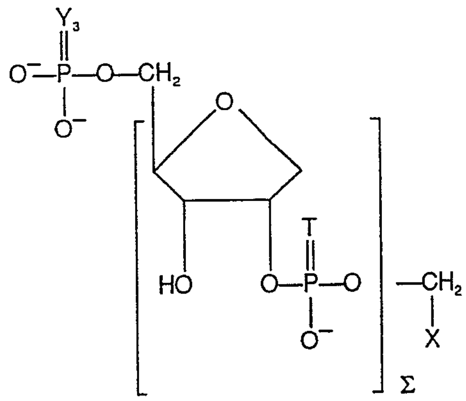

(VIII)

dans laquelle:
- $Y_3$ représente S, Se, NH;
- T représente O, S, Se, NH;
- X est choisi dans le groupe constitué par:

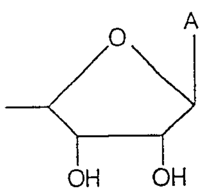

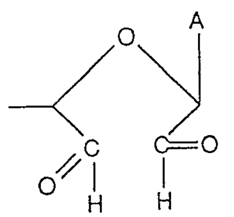

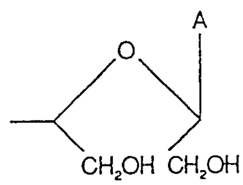

$-CHOHCH_2OH$

- Σ représente un nombre entier égal à n, lorsque X représente $-CHOHCH_2OH$ et un nombre entier égal à n−1 lorsque X est différent de $-CHOH-CH_2OH$, n étant un nombre entier variant de 2 à 10;
- A est une base choisie parmi l'adénine ou ses dérivés, notamment ceux de formule:

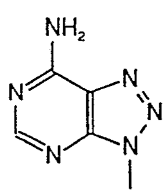

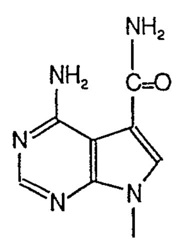

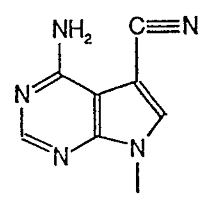

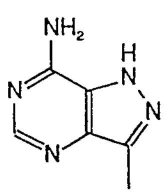

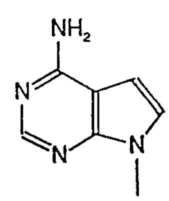

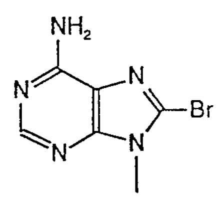

10. Procédé de préparation d'oligonucléotides selon l'une quelconque des revendications précédentes, dans lesquels T représente l'oxygène.

11. Procédé de préparation d'oligonucléotides selon la revendication 1, de formules:

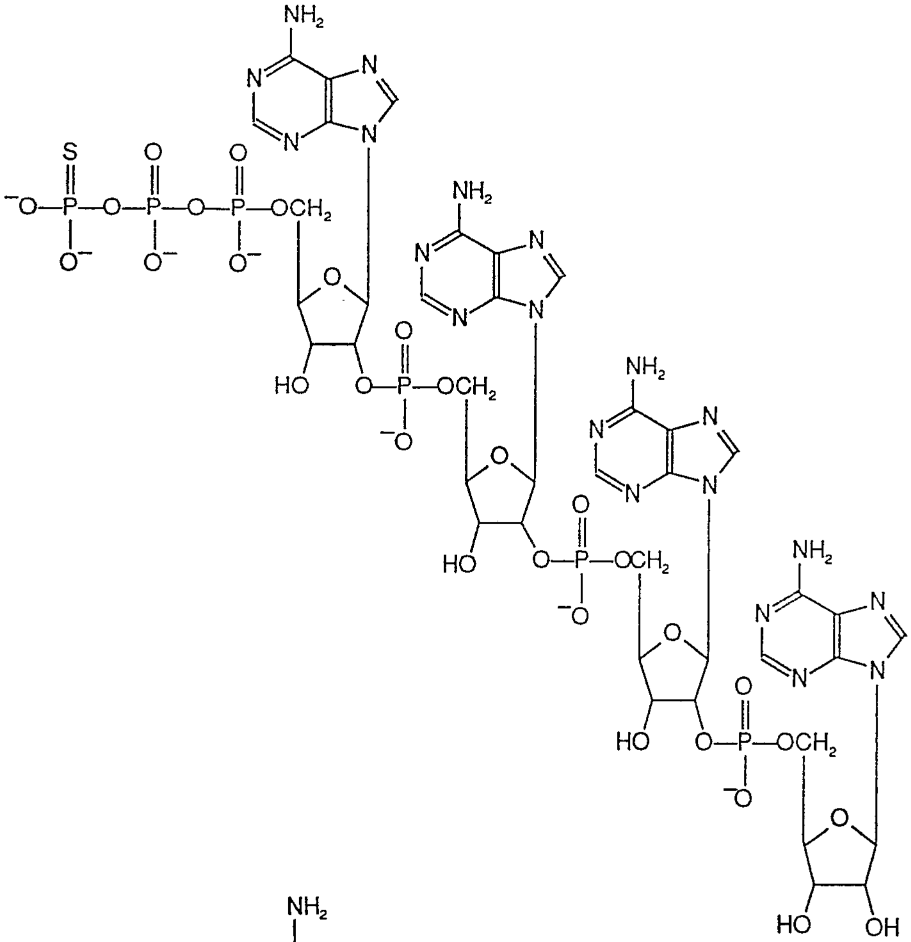

(XV)

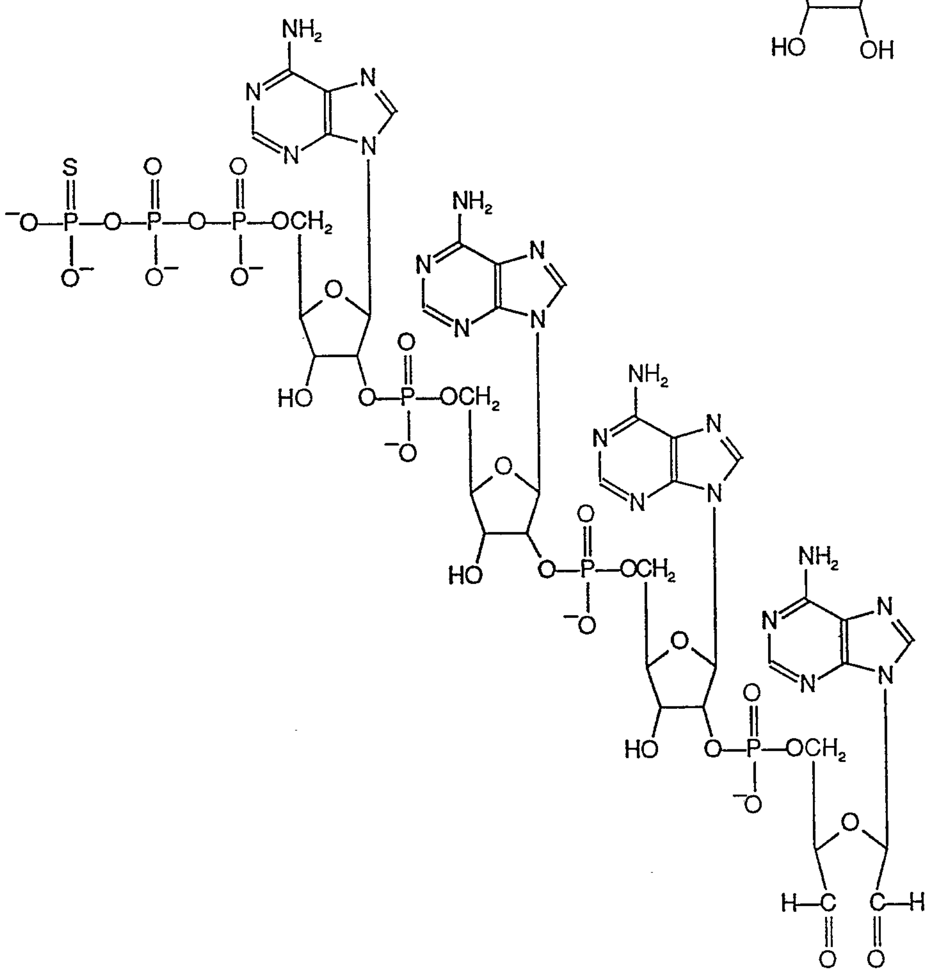

(XVI)

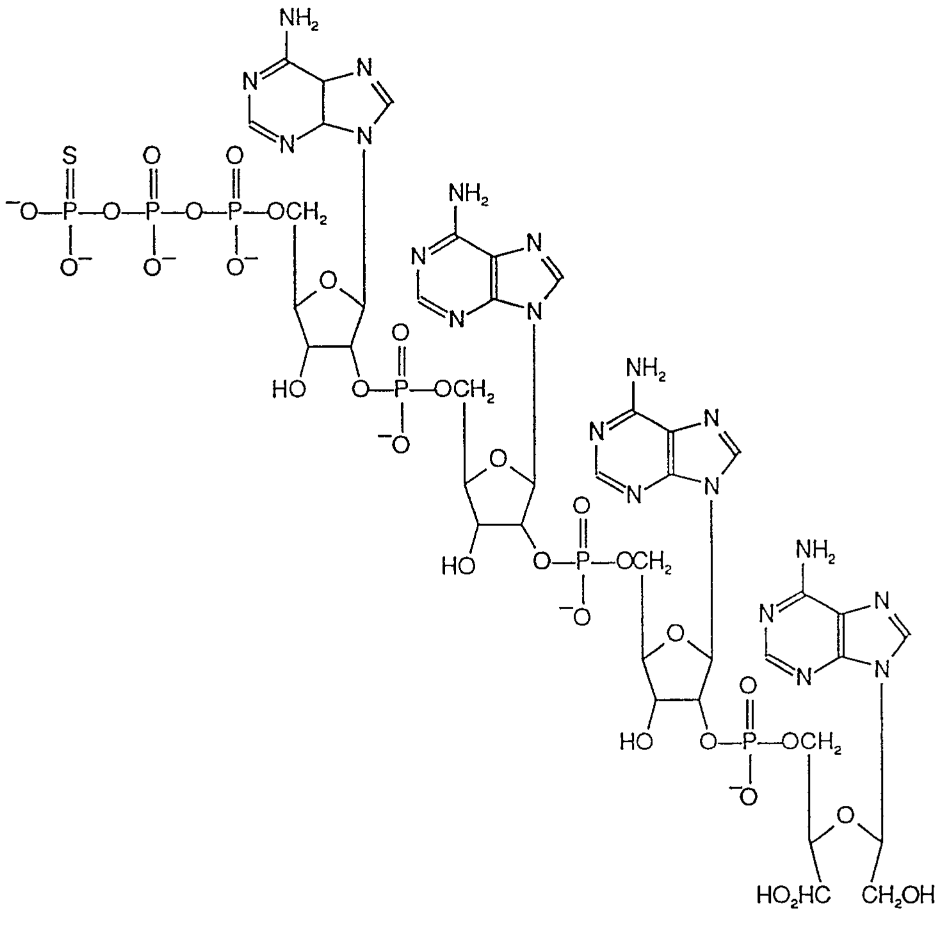

(XVII)

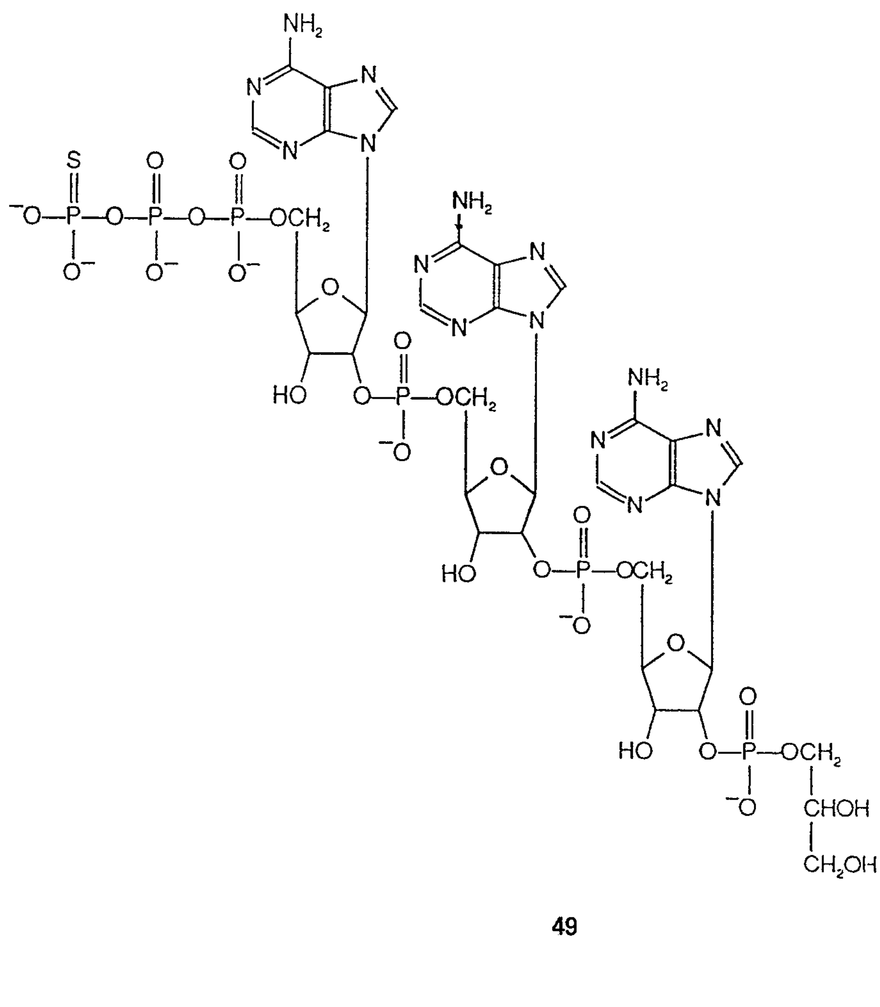

(XVIII)

**Revendications pour les Etats contractants: BE, CH, DE, GB, IT, LI, LU, NL, SE**

1. Oligonucléotides de formule générale (I):

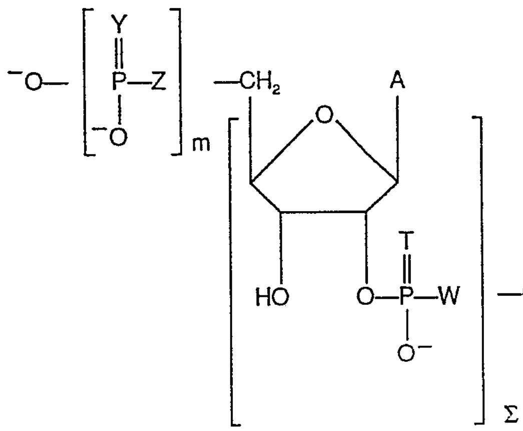

(I)

dans laquelle:
- Y et T sont identiques ou différents et représentent O, S, Se, NH;
- Z et W sont identiques ou différents et représentent O, S, NH;
- l'un au moins des éléments Y et Z de l'un au moins des groupes phosphate étant différent de l'oxygène;
- X est choisi dans le groupe constitué par:

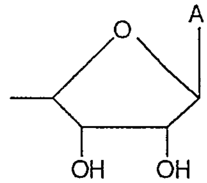 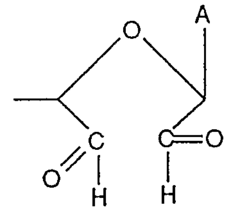

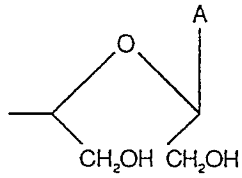 $—CHOHCH_2OH$

les fonctions alcools de ces radicaux étant éventuellement estérifiées par des acides carboxyliques $R_3COOH$, $R_3$ représentant un radical alcoyle de 1 à 5 atomes de carbone ou un groupe phényle;
- Σ est un nombre entier égal à:
- n lorsque X représente $-CHOHCH_2OH$,
- n–1 lorsque X représente:

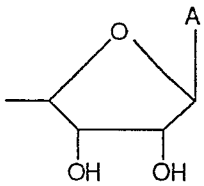 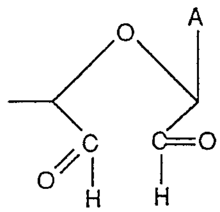 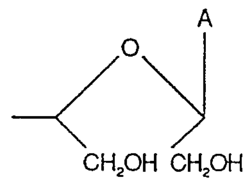

n étant un nombre entier supérieur ou égal à 2;
- m est un nombre entier supérieur ou égal à 1;
- A est une base choisie parmi l'adénine ou ses dérivés, notamment ceux de formule:

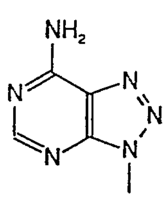 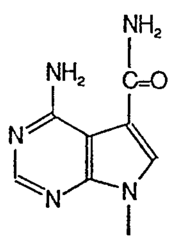 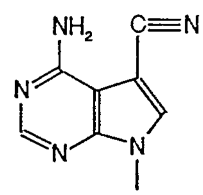

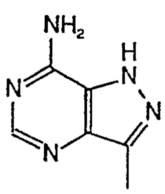 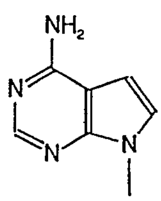 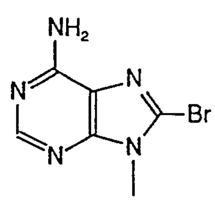

2. Oligonucléotides selon la revendication 1 répondant à la formule générale (II):

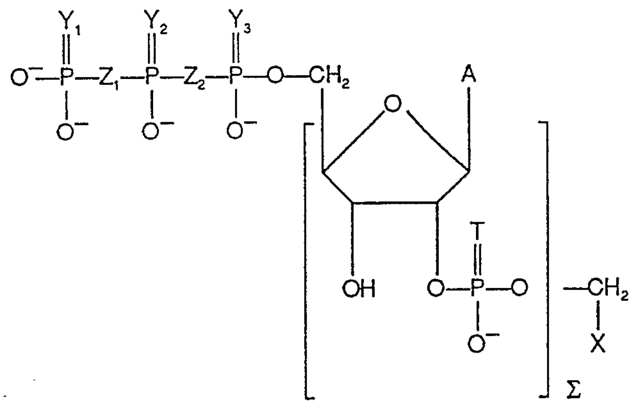

(II)

dans laquelle:
- $Y_1$, $Y_2$, $Y_3$, T sont identiques ou différents et représentent O, S, Se, NH;
- $Z_1$ et $Z_2$ sont identiques ou différents et représentent O, S, NH;
l'un au moins des éléments $Y_1$, $Y_2$, $Y_3$, $Z_1$, $Z_2$ étant différent de l'oxygène;
- X est choisi dans le groupe constitué par:

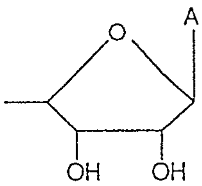

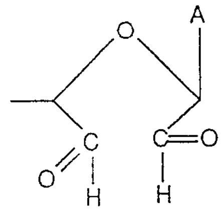

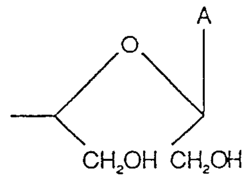

$-CHOHCH_2OH$

les fonctions alcools de ces radicaux étant éventuellement estérifiées par des acides carboxyliques $R_3COOH$, $R_3$ représentant un radical alcoyle de 1 à 5 atomes de carbone ou un groupe phényle;
- Σ représente un nombre entier égal à n, lorsque X représente $-CHOHCH_2OH$, et Σ représente un nombre entier égal à n−1 lorsque X est différent de $-CHOHCH_2OH$, n étant un nombre entier supérieur ou égal à 2;
- A est une base choisie parmi l'adénine ou ses dérivés, notamment ceux de formule:

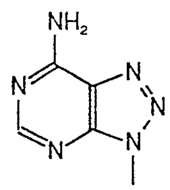

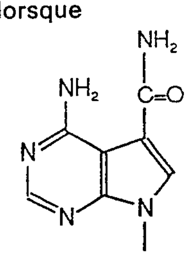

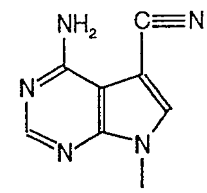

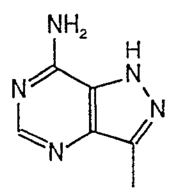

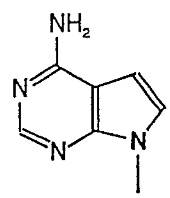

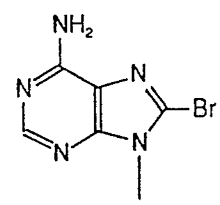

3. Oligonucléotides selon la revendication 1 de formule (VII):

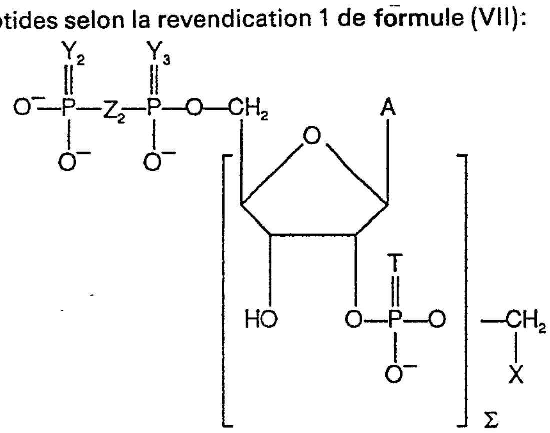

(VII)

dans laquelle:
- $Y_2$, $Y_3$, T sont identiques ou différents et représentent O, S, Se, NH;
- $Z_2$ représente O, S, NH;

l'un au moins des éléments $Y_2$, $Y_3$, $Z_2$ étant différent de l'oxygène;
- X est choisi dans le groupe constitué par:

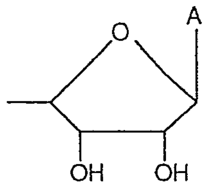
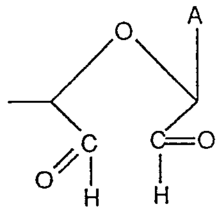
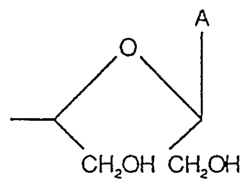
$-CHOHCH_2OH$

- Σ est un nombre entier égal à n, lorsque X représente $-CHOHCH_2OH$ et est un nombre entier égal à n−1, lorsque X est différent de $-CHOHCH_2OH$, n étant un nombre entier variant de 2 à 10;
- A est une base choisie parmi l'adénine ou ses dérivés, notamment ceux de formule:

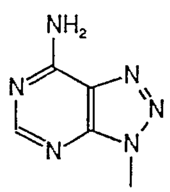
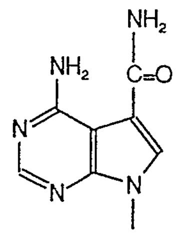
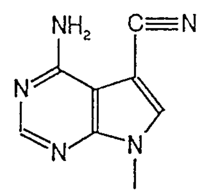
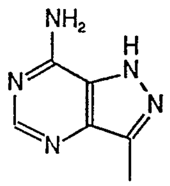
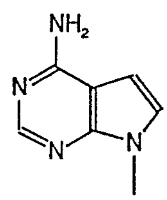
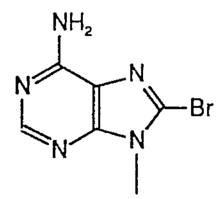

4. Oligonucléotides selon la revendication 1 de formule (VIII):

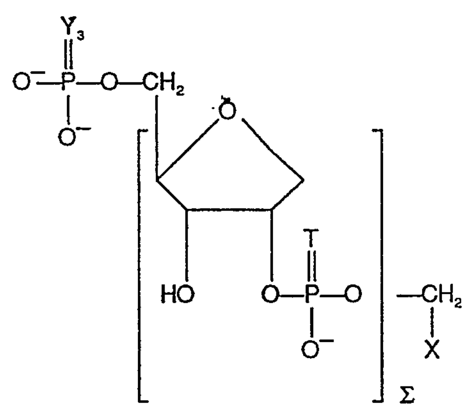
(VIII)

dans laquelle
- $Y_3$ représente S, Se, NH;
- T représente O, S, Se, NH;
- X est choisi dans le groupe constitué par:

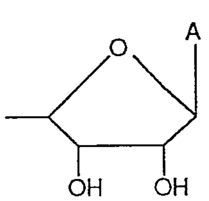
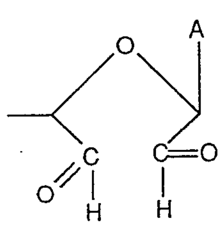
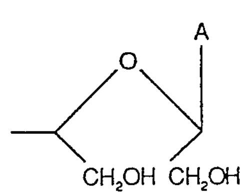
$-CHOHCH_2OH$

- Σ représente un nombre entier égal à n, lorsque X représente $-CHOHCH_2OH$ et un nombre entier égal à n-1 lorsque X est différent de $-CHOHCH_2OH$, n étant un nombre entier variant de 2 à 10;
- A est une base choisie parmi l'adénine ou ses dérivés, notamment ceux de formule:

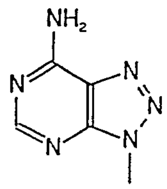
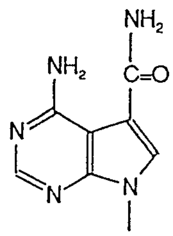
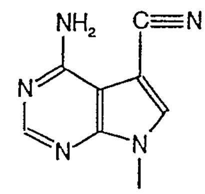
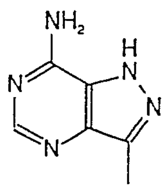
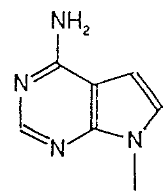
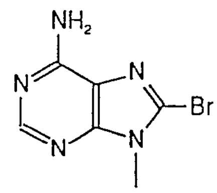

5. Oligonucléotides selon l'une quelconque des revendications précédentes, dans lesquels T représente l'oxygène.

6. Oligonucléotides selon la revendication 1, de formules:

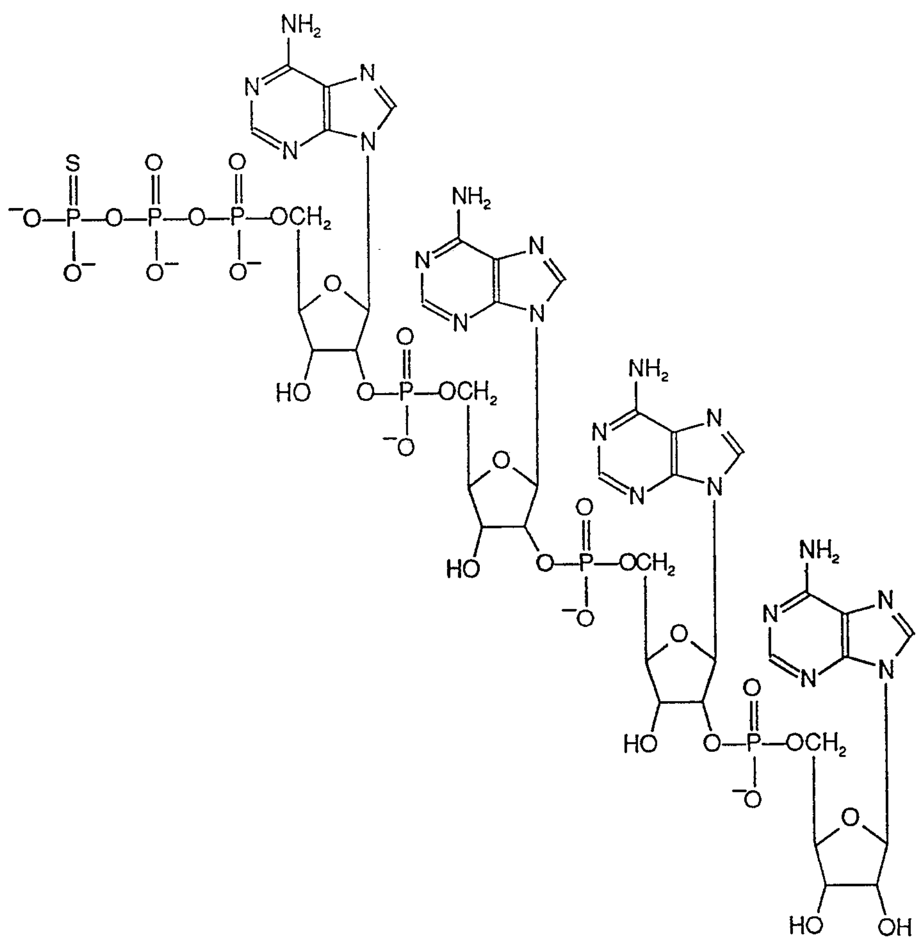
(XV)

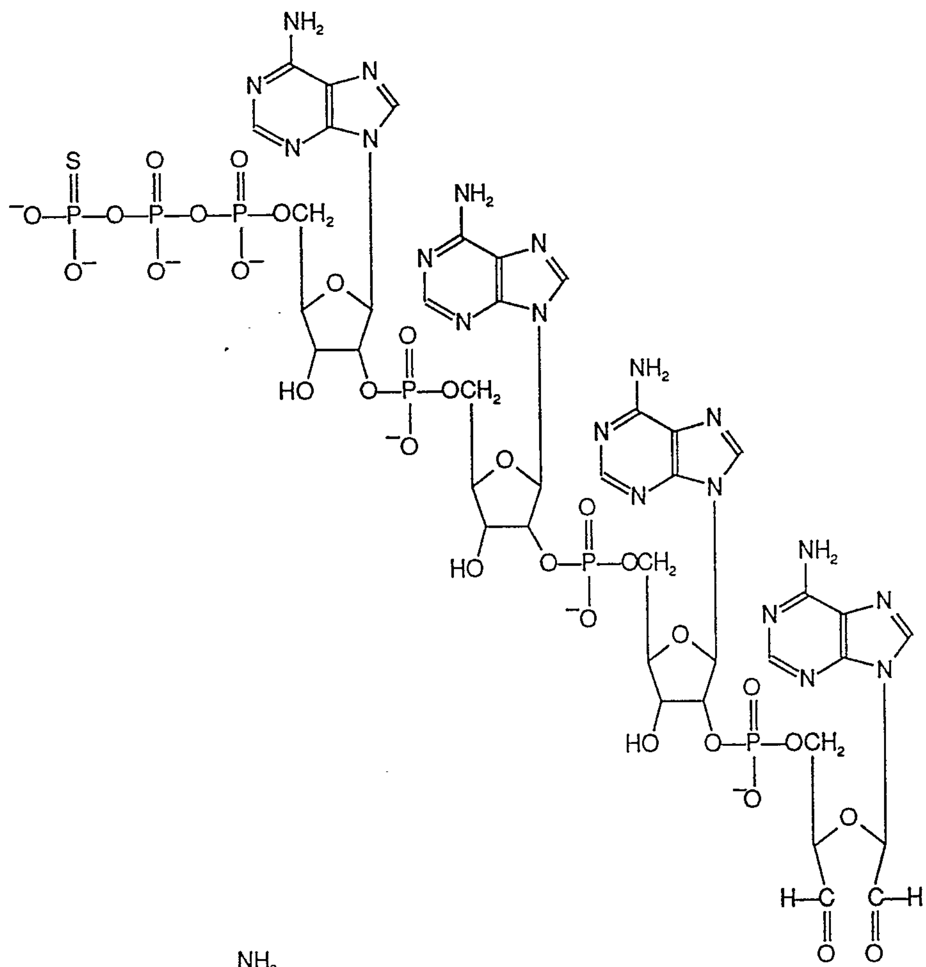

(XVI)

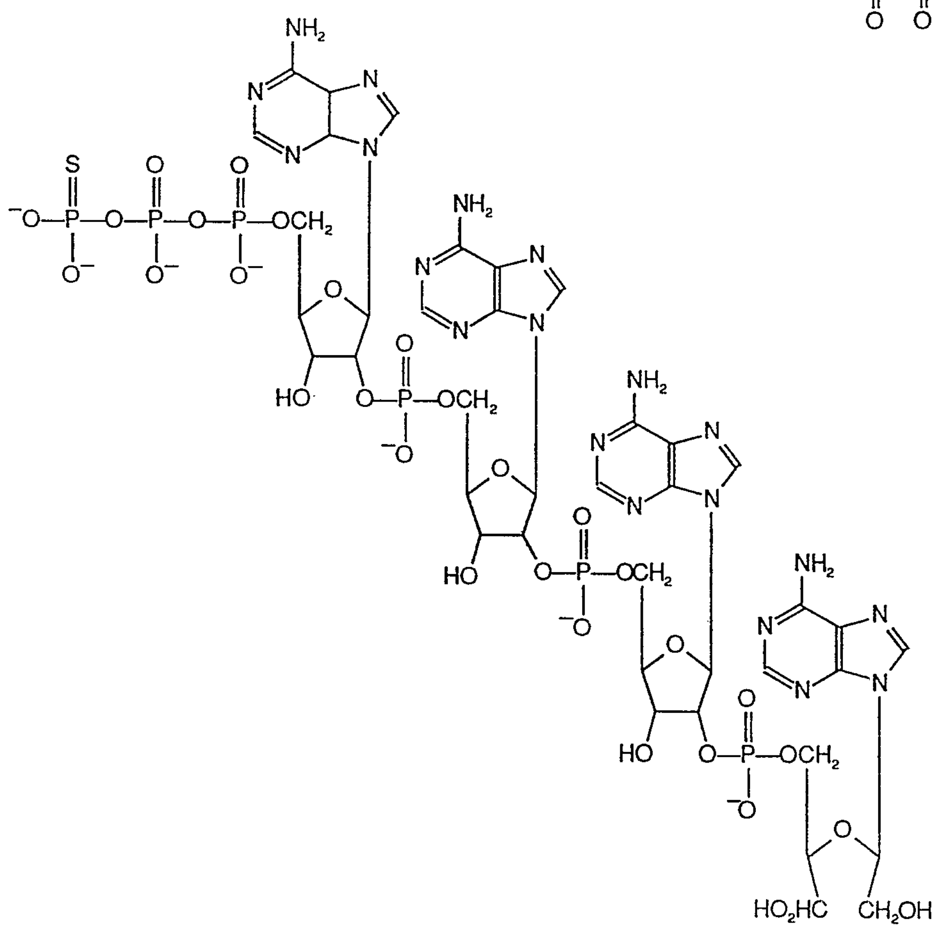

(XVII)

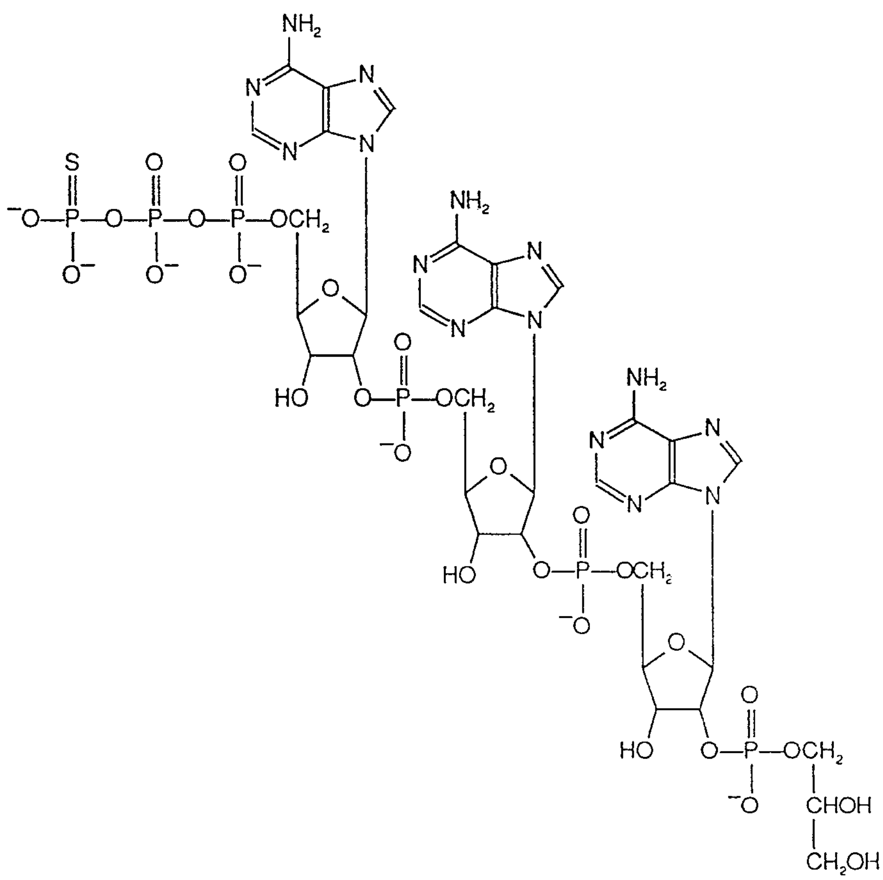

(XVIII)

7. Procédé de préparation d'oligonucléotides de formule (I) selon la revendication 1:

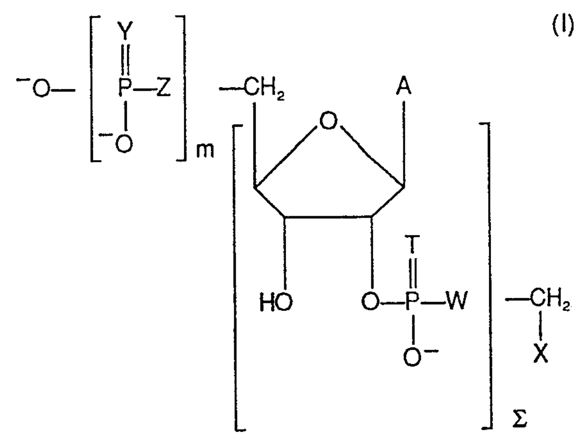

(I)

dans laquelle:
- Y et T sont identiques ou différents et représentent O, S, Se ou NH;
- Z et W sont identiques ou différents et représentent O, S ou NH;
l'un au moins des éléments Y ou Z de l'un au moins des groupes phosphate étant différent de l'oxygène;
- m est supérieur ou égal à 1;
- A et X ont les significations indiquées à la revendication 1, caractérisé en ce qu'il comprend:
- la polymérisation de composés de formule (XIX) suivante:

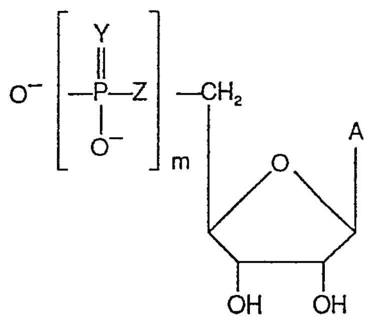

(XIX)

pour obtenir un composé de formule (Ibis) suivante:

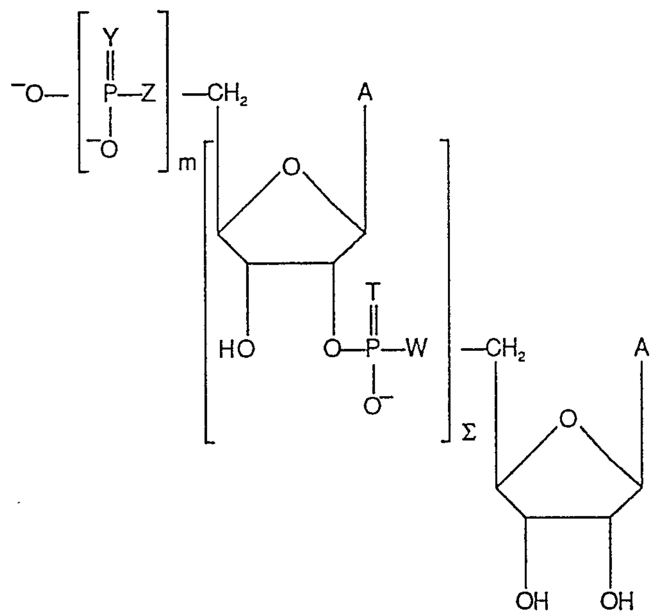

(I bis)

dans laquelle:
- Y et T, identiques ou différents, représentent O, S, Se ou NH;
- Z et W, identiques ou différents, représentent O, S ou NH;
l'un au moins des éléments Y et Z de l'un au moins des groupes phosphate étant différent de l'oxygène;
- Σ est un nombre entier égal à n−1, n étant supérieur ou égal à 2;
- m est un nombre entier supérieur ou égal à 1;
- A a les significations indiquées ci-dessus;
- et si nécessaire les étapes chimiques suivantes à savoir
- l'oxydation éventuelle du groupe glycol pour introduire des fonctions aldéhyde sur les carbones en 2′ et 3′ de la dernière unité nucléosidique et obtenir le composé de formule (Iter):

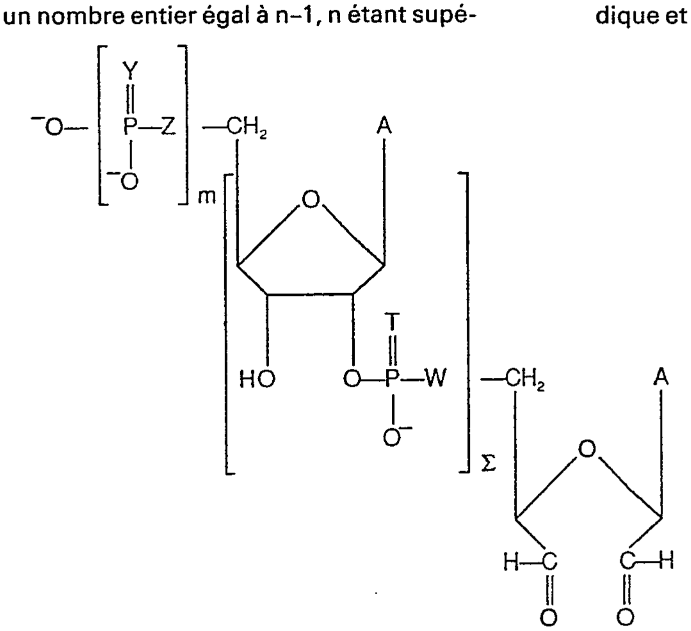

(I ter)

- la réduction éventuelle des deux fonctions aldéhyde en fonctions alcool pour obtenir le composé de formule (Iquater):

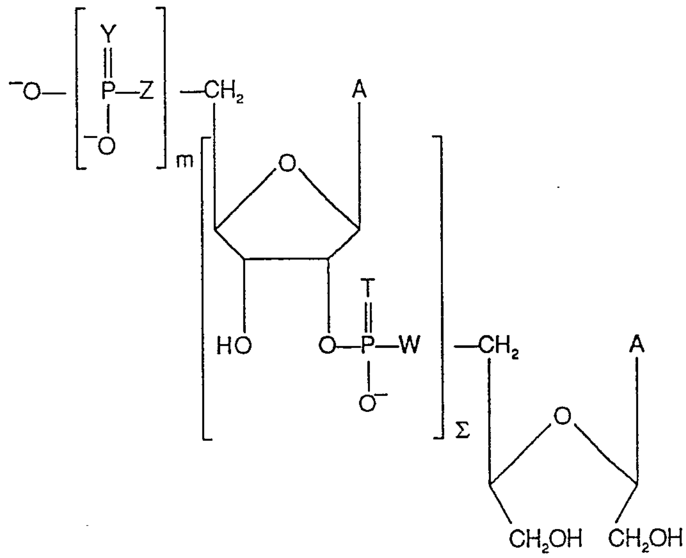

(I quater).

- l'hydrolyse éventuelle, dans des conditions évitant la bêta-élimination, pour obtenir le composé de formule (Iquinquiès) suivante:

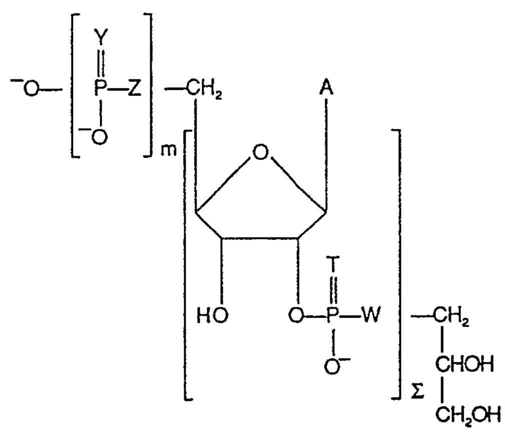

(I quinquies)

8. Procédé selon la revendication 7 de préparation d'oligonucléotides de formule (XX):

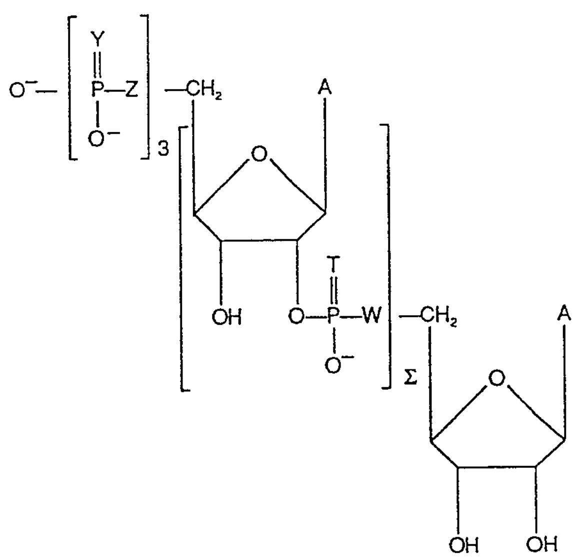

(XX)

dans laquelle:
- Y et T, identiques ou différents, représentent O, S, Se, NH;
- Z et W, identiques ou différents, représentent O, S, NH
- les éléments Y et Z de l'un au moins des groupes phosphate ne représentent pas simultanément l'oxygène;
- Σ est un nombre entier variant de 1 à 9;
- A est une base choisie parmi l'adénine ou ses dérivés, notamment ceux de formule:

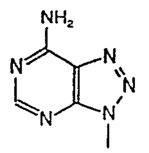
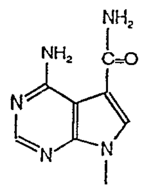
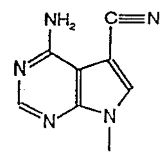
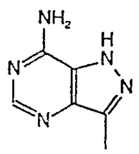
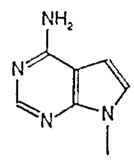
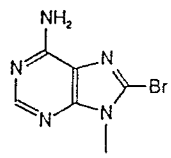

caractérisé en ce qu'il comprend:
1) la polymérisation d'un composé de formule (XIXbis) suivante:

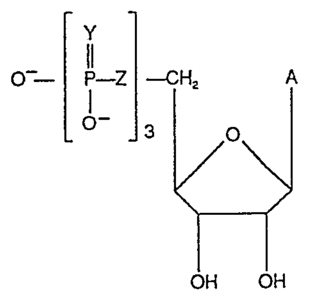
(XIX bis)

dans laquelle:
- Y représente O, S, Se ou NH;
- Z représente O, S ou NH;
- les éléments Y et Z de l'un au moins des groupes phosphate ne représentent pas simultanément l'oxygène;
- A a les significations indiquées à la revendication 1;

2) l'oxydation éventuelle du groupe glycol terminal, notamment par l'ion periodate pour transformer le glycol en deux fonctions aldéhyde et obtenir un composé de formule (XXI) suivante:

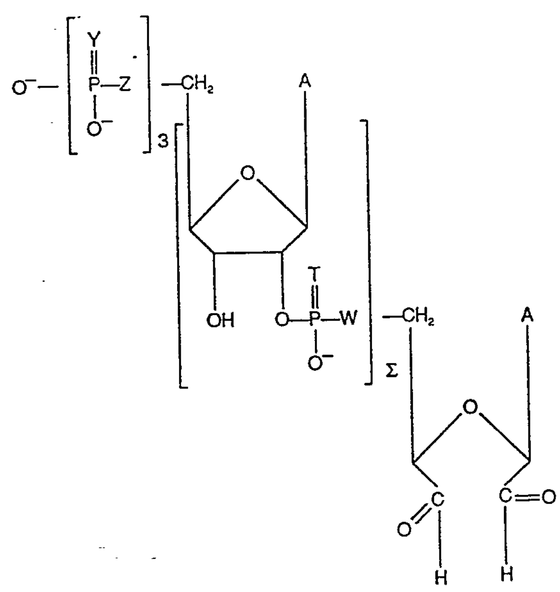
(XXI)

dans laquelle Y, Z, T, W, Σ et A ont les significations indiquées ci-dessus;

3) la réduction éventuelle des fonctions aldéhyde, notamment par le borohydrure de sodium pour transformer les deux susdites fonctions aldhéhyde en fonctions alcool et obtenir le composé de formule (XXII) suivante:

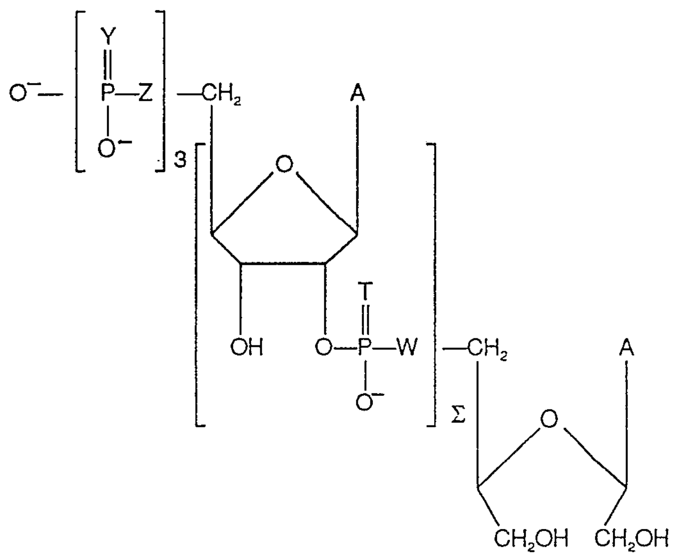

(XXII)

4) l'hydrolyse éventuelle, notamment l'hydrolyse acide ménagée, pour éliminer le noyau de ribose et obtenir le composé de formule (XXIII) suivante:

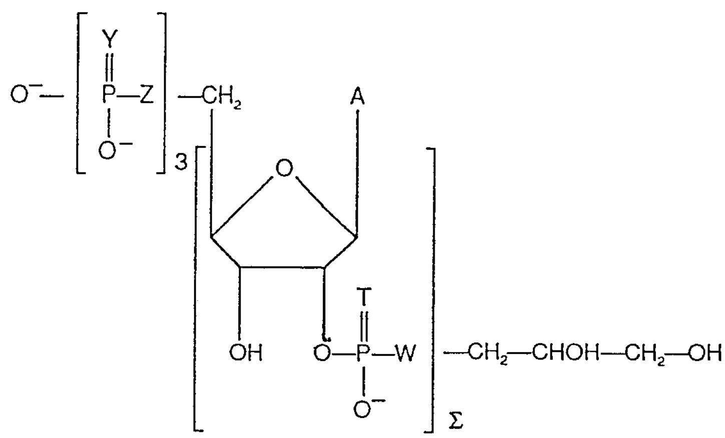

(XXIII)

9. Procédé selon la revendication 7 de préparation d'oligonucléotides de formule (V):

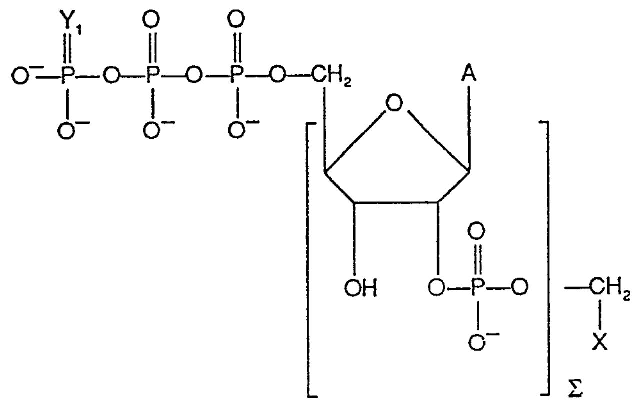

(V)

dans laquelle: – $Y_1$ représente NH, Se, S; – X représente:

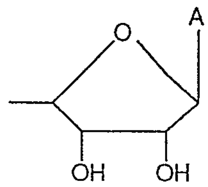
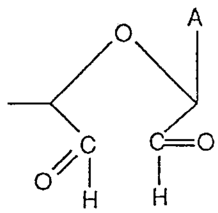
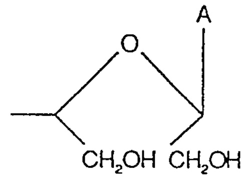
—$CHOHCH_2OH$

– Σ est un nombre entier égal à n, lorsque X représente -$CHOHCH_2OH$, n–1 lorsque X est différent de -$CHOHCH_2OH$, n variant de 2 à 10; est caractérisé en ce que l'on polymérise le composé de formule (XXIV) suivante:

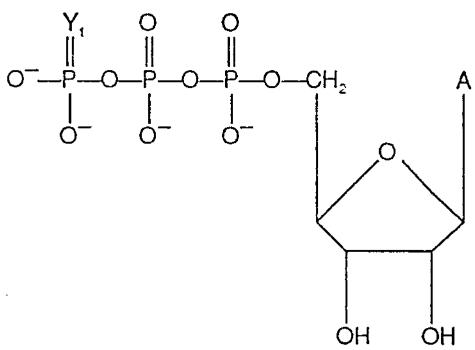

(XXIV)

et que l'on effectue les étapes 2°, 3° et 4° comme indiqué à la revendication 7.

10. Procédé selon la revendication 7 de préparation d'oligonucléotides de formule (VI):

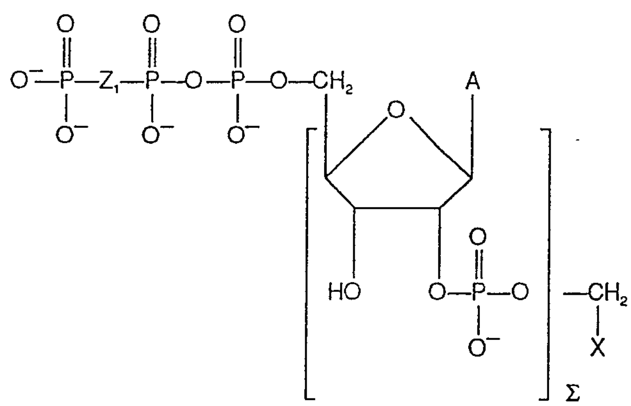

(VI)

dans laquelle:
– $Z_1$ représente S ou NH; – X est choisi dans le groupe constitué par:

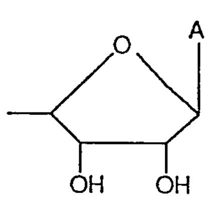
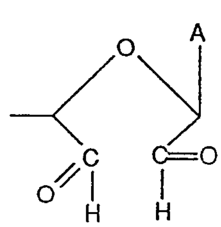
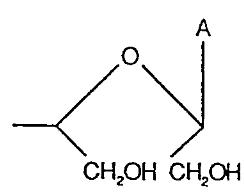
—$CHOHCH_2OH$

– Σ est un nombre entier égal à 1, lorsque X représente -$CHOHCH_2OH$ et égal à n–1 lorsque X est différent de -$CHOHCH_2OH$, n variant de 2 à 10;
caractérisé en ce qu'on polymérise le composé de formule (XXV) suivante:

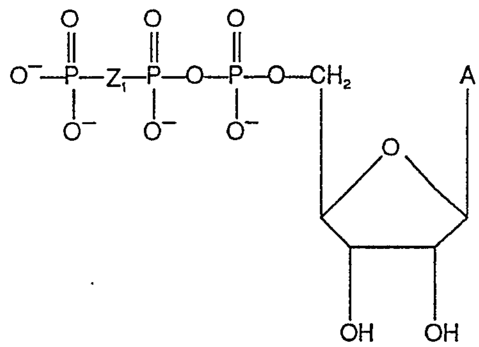

(XXV)

dans laquelle $Z_1$ représente S ou NH et A a la signification indiquée à la revendication 1; et que l'on effectue les étapes 2°, 3° et 4° comme indiqué à la revendication 7.

11. Procédé selon la revendication 7 de préparation d'oligonucléotides de formule (IV):

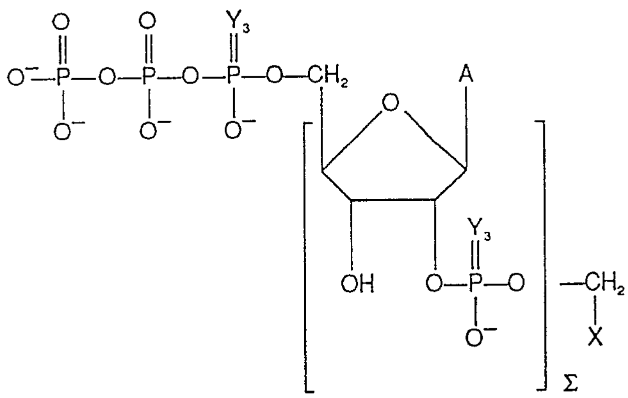

(IV)

dans laquelle:
- $Y_3$ représente NH, S ou Se;
- Σ, X et A ont les significations indiquées ci-dessus;

caractérisé en ce qu'on polymérise le composé de formule (XXVI) suivante:

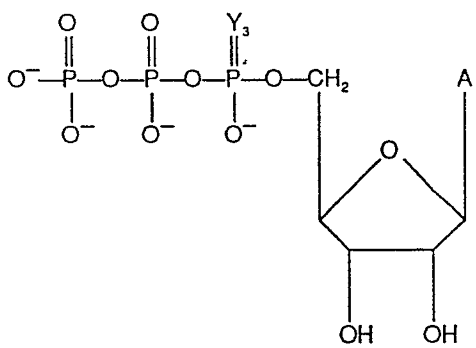

(XXVI)

dans laquelle:
- $Y_3$ représente S, Se, NH;
- A a les significations indiquées ci-dessus; et que l'on effectue les étapes 2°, 3° et 4°, comme indiqué à la revendication 7.

12. Procédé selon la revendication 7 de préparation d'oligonucléotides de formule (Vbis) suivante:

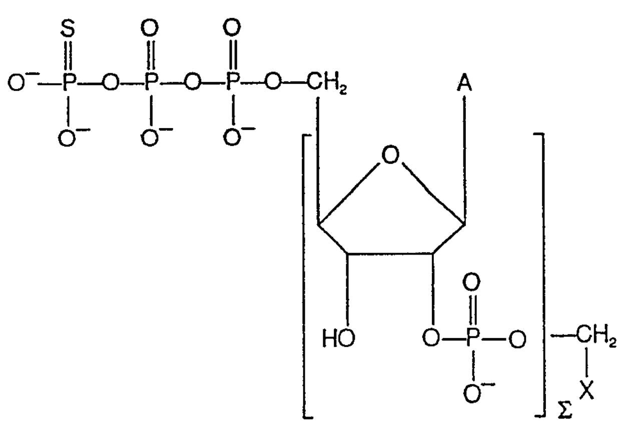

(V bis)

dans laquelle:
- X représente:

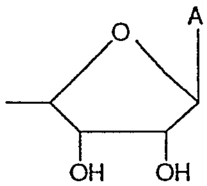
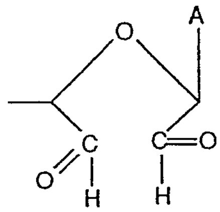
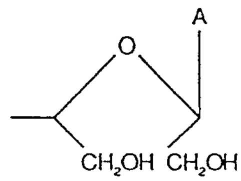
—$CHOHCH_2OH$

- Σ est un nombre entier égal à n lorsque X représente -$CHOHCH_2OH$, n-1 lorsque X est différent de -$CHOHCH_2OH$,
- A a les significations indiquées à la revendication 1, de préférence l'adénine;

caractérisé en ce qu'on polymère le composé de formule (XXIVbis) suivante:

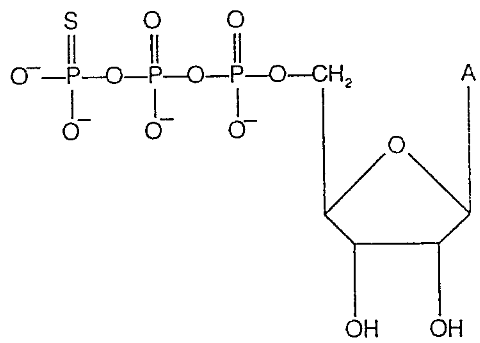
(XXIV bis)

et que l'on effectue les étapes 2°, 3° et 4° comme indiqué à la revendication 7.

**Patentansprüche für den Vertragsstaat: AT**

1. Verfahren zur Herstellung von Oligonukleotiden der Formel (I)

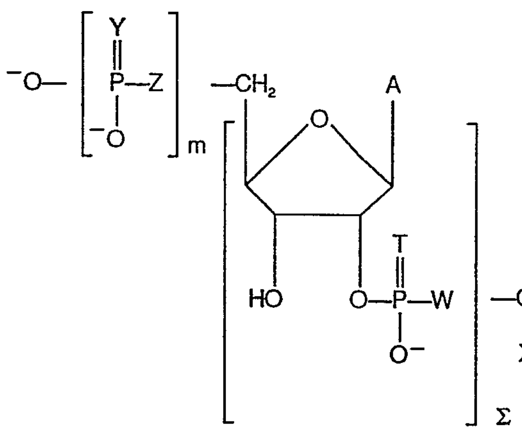
(I)

wobei Y und T gleich oder verschieden sind und O, S, Se oder NH darstellen;
Z und W gleich oder verschieden sind und O, S oder NH darstellen;
wenigstens eines der Elemente Y oder Z von wenigstens einer der Phosphatgruppen sich von Sauerstoff unterscheidet;
m höher als oder gleich 1 ist;
X ausgewählt ist aus der Gruppe

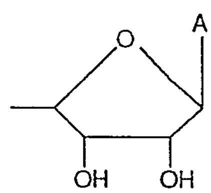
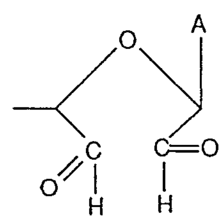
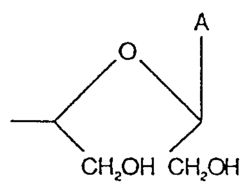
—$CHOHCH_2OH$

und die Alkoholgruppen dieser Reste gegebenenfalls durch Carbonsäure $R_3COOH$ verestert sind, wobei $R_3$ einen Alkylrest mit 1 bis 5 Kohlenstoffatomen oder eine Phenylgruppe darstellt;
Σ eine ganze Zahl gleich n ist, wenn X -$CHOHCH_2OH$ darstellt, eine ganze Zahl gleich n-1 ist, wenn X

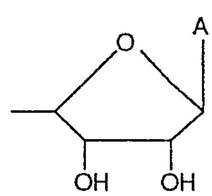
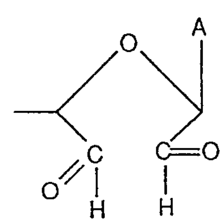

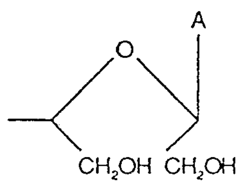

darstellt, wobei n eine ganze Zahl höher als oder gleich 2 ist;
m eine ganze Zahl höher als oder gleich 1 ist;

A eine Base ausgewählt aus Adenin oder dessen Derivaten, im besonderen solcher der Formel

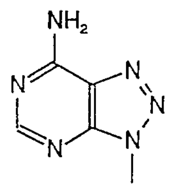
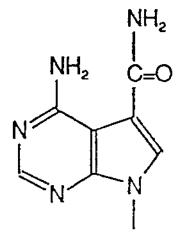
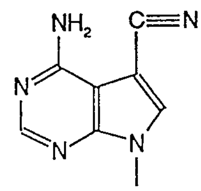
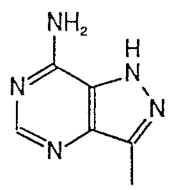
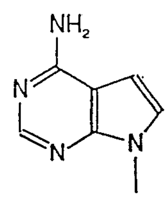
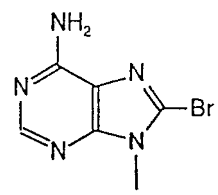

ist, dadurch gekennzeichnet, dass es
1) die Polymerisation der Verbindung der folgenden Formel (XIX)

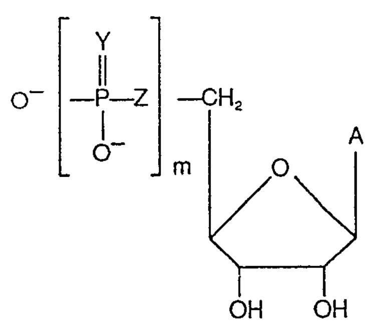

(XIX)

umfasst, um eine Verbindung der folgenden Formel (Ibis) zu erhalten

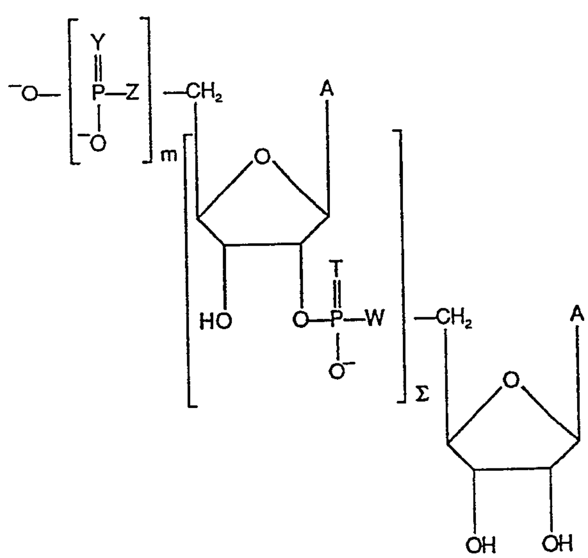

(I bis)

wobei Y, T, Z, W, m und A die oben genannten Bedeutungen haben,
Σ eine ganze Zahl gleich n−1 ist, wobei n höher als oder gleich 2 ist,
und wenn nötig, die folgenden chemischen Schritte durchläuft, und zwar:

2) gegebenenfalls die Oxidation der Glykolgruppe zur Einführung von Aldehydgruppen auf die Kohlenstoffatome in den Positionen 2′ und 3′ der letzten nukleosiden Einheit, wobei die Verbindung der Formel (Iter) erhalten wird,

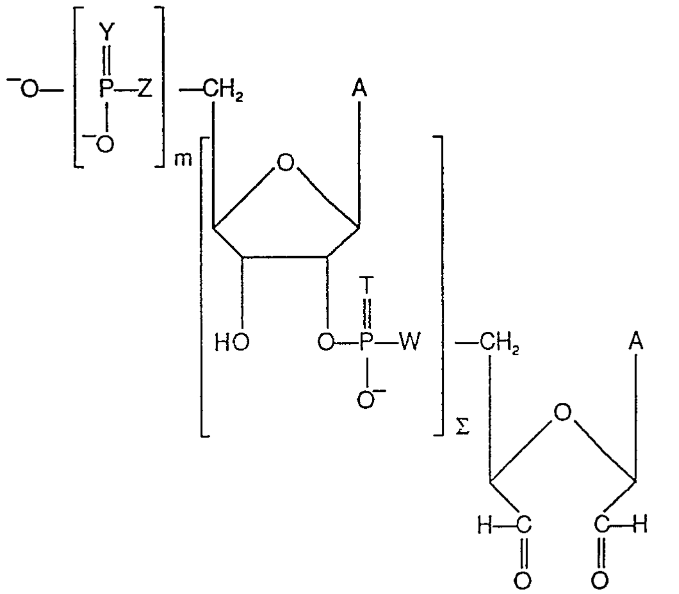

(I ter)

3) gegebenenfalls die Reduktion der beiden Aldehydgruppen in Alkoholgruppen, um die Verbindung der Formel (Iquater) zu erhalten,

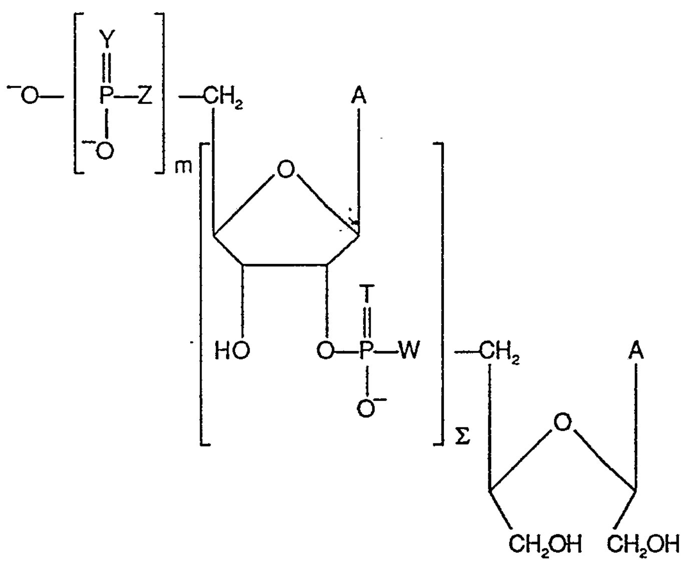

(I quater)

4) gegebenenfalls die Hydrolyse unter solchen Bedingungen, dass eine Beta-Elimination vermieden wird, um die Verbindung der folgenden Formel (Iquinquiès) zu erhalten,

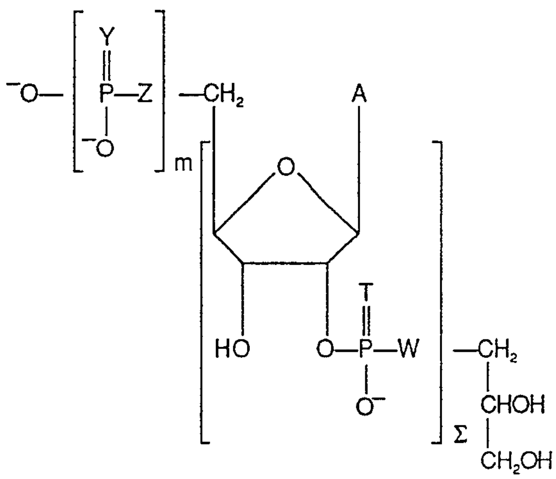

(I quinquies)

2. Verfahren nach Anspruch 1, zur Herstellung von Oligonukleotiden der Formel (XX)

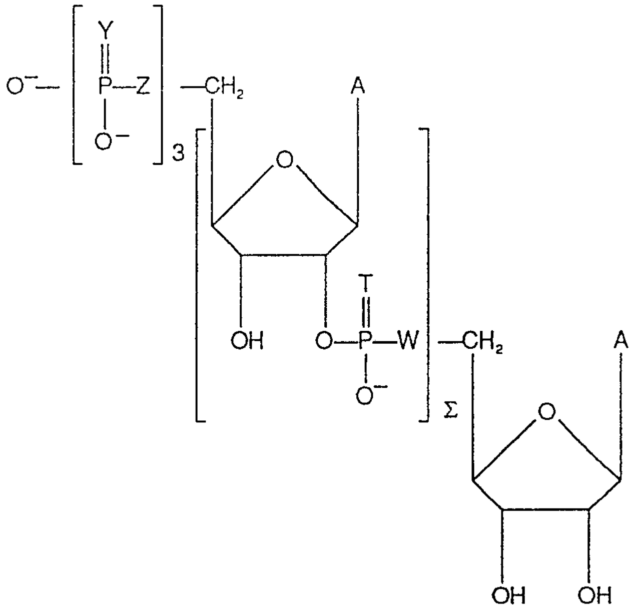

(XX)

wobei Y und T, gleich oder verschieden, O, S, Se, NH darstellen;
Z und W, gleich oder verschieden, O, S, NH darstellen;
die Elemente Y und Z von wenigstens einer der Phosphatgruppen nicht gleichzeitig Sauerstoff darstellt;
Σ eine ganze Zahl zwischen 1 und 9 ist;
A eine Base ausgewählt aus Adenin oder dessen Derivaten, im besonderen solcher der Formel

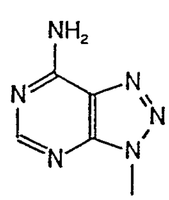
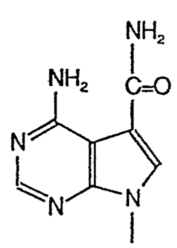
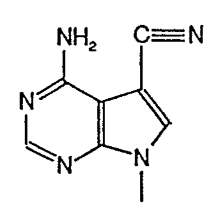
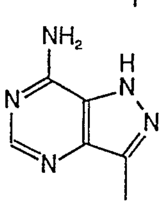
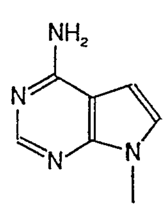
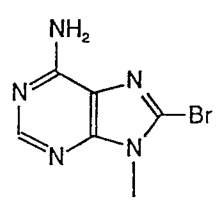

ist, dadurch gekennzeichnet, dass es
1) die Polymerisation einer Verbindung der folgenden Formel (XIXbis) umfasst,

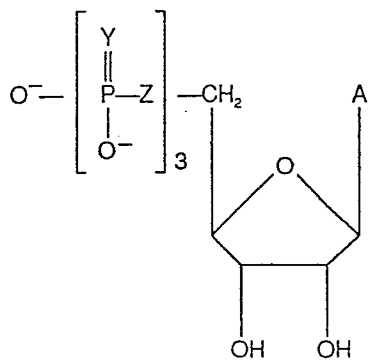

(XIX bis)

wobei Y O, S, Se oder NH darstellt;
Z O, S oder NH darstellt;
die Elemente Y und Z von wenigstens einer der Phosphatgruppen nicht gleichzeitig Sauerstoff darstellt;
A die in Anspruch 1 angegebenen Bedeutungen hat;
2) gegebenenfalls die Oxidation der Glykolendgruppe, im besonderen durch ein Perjodation, zur Umsetzung des Glykols in zwei Aldehydgruppen und zum Erhalt einer Verbindung der folgenden Formel (XXI) umfasst,

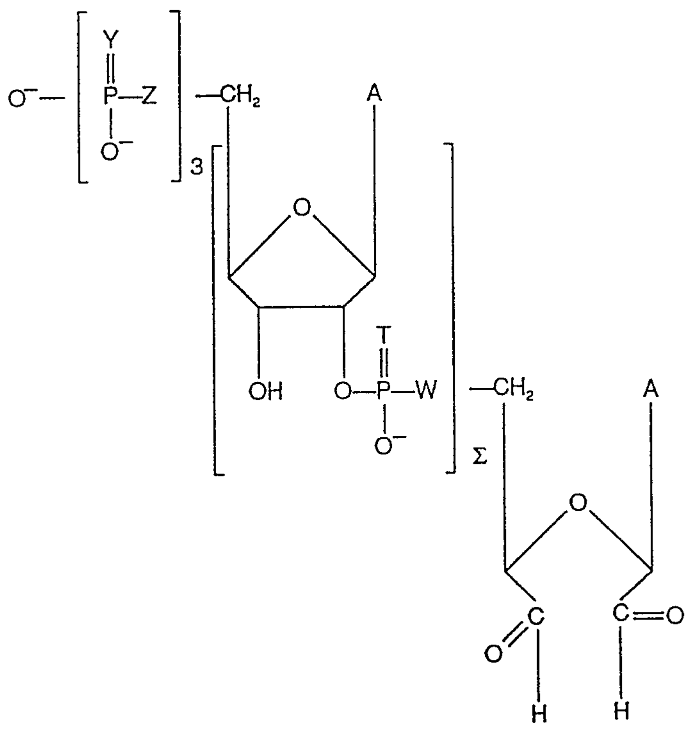

(XXI)

wobei Y, Z, T, W, Σ und A die oben genannten Bedeutungen haben;
3) gegebenenfalls die Reduktion der Aldehydgruppen, im besonderen durch das Natriumborhydrid, zur Umsetzung der beiden oben genannten Aldehydgruppen in Alkoholgruppen und zum Erhalt der Verbindung der folgenden Formel (XXII) umfasst,

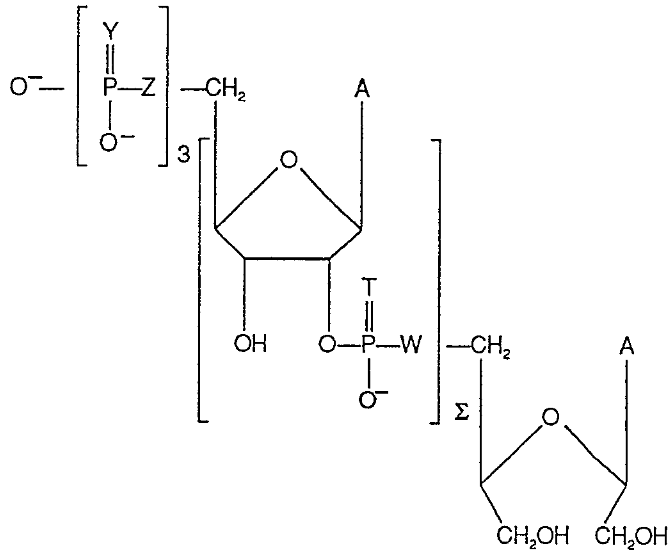

(XXII)

4) gegebenenfalls die Hydrolyse, im besonderen gesteuerte Hydrolyse, zur Freisetzung des Ribosekerns und zum Erhalt der Verbindung der folgenden Formel (XXIII) umfasst,

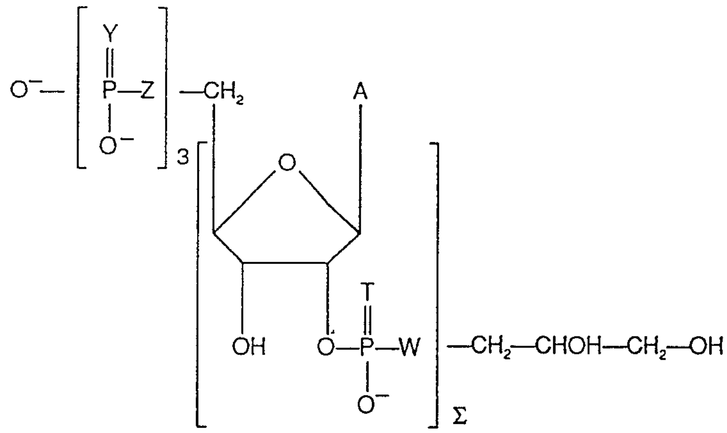

(XXIII)

3. Verfahren nach Anspruch 1, zur Herstellung von Oligonukleotiden entsprechend der allgemeinen Formel (II)

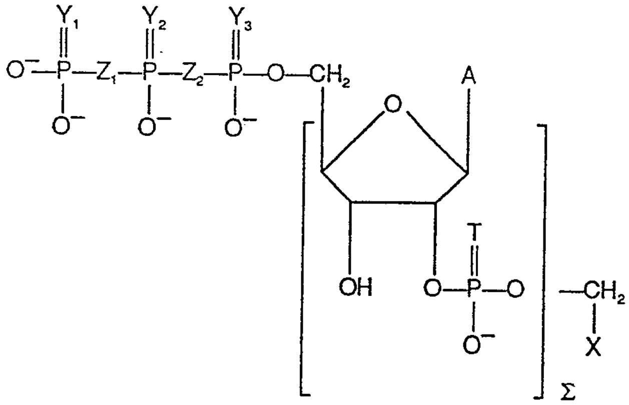

(II)

wobei $Y_1$, $Y_2$, $Y_3$, T gleich oder verschieden sind und O, S, Se, NH darstellen;
$Z_1$ und $Z_2$ gleich oder verschieden sind und O, S, NH darstellen;
wenigstens eines der Elemente $Y_1$, $Y_2$, $Y_3$, $Z_1$, $Z_2$ sich von Sauerstoff unterscheidet;
X ausgewählt ist aus der Gruppe

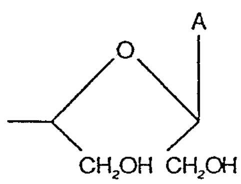

—$CHOHCH_2OH$

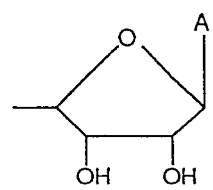

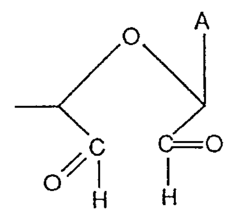

und die Alkoholgruppen dieser Reste gegebenenfalls durch die Carbonsäuren $R_3COOH$ verestert sind, wobei $R_3$ einen Alkylrest mit 1 bis 5 Kohlenstoffatomen oder eine Phenylgruppe darstellt;

$\Sigma$ eine ganze Zahl gleich n darstellt, wenn X $-CHOHCH_2OH$ darstellt, und $\Sigma$ eine ganze Zahl gleich n−1 darstellt, wenn X sich von $-CHOHCH_2OH$ unterscheidet, wobei n eine ganze Zahl höher als oder gleich 2 ist;

A eine Base ausgewählt aus Adenin oder dessen Derivaten, im besonderen solcher der Formel

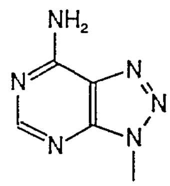

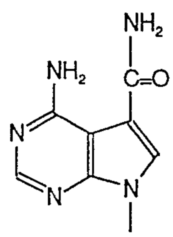

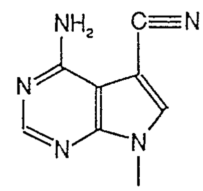

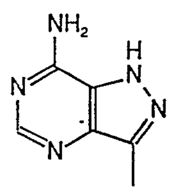

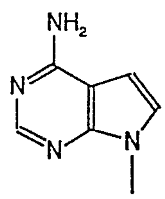

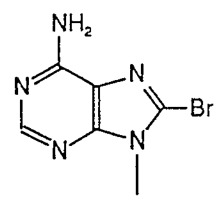

ist.

4. Verfahren nach Anspruch 1, zur Herstellung von Oligonukleotiden der Formel (V)

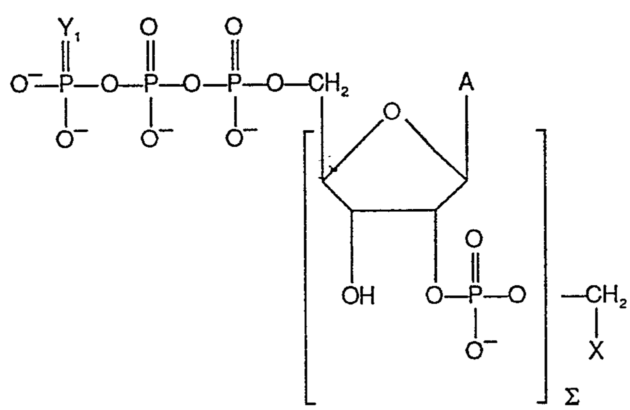

(V)

wobei $Y_1$ NH, Se, S darstellt;

und X

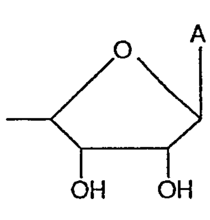

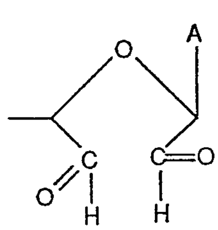

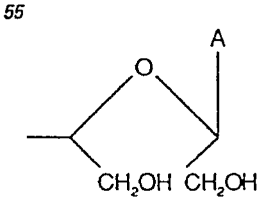

—$CHOHCH_2OH$

darstellt;

Σ eine ganze Zahl gleich n ist, wenn X $-CHOHCH_2OH$ darstellt, und n-1, wenn sich X von $-CHOHCH_2OH$ unterscheidet, wobei n zwischen 2 und 10 liegt;
dadurch gekennzeichnet, dass die Verbindung der folgenden Formel (XXIV) polymerisiert wird:

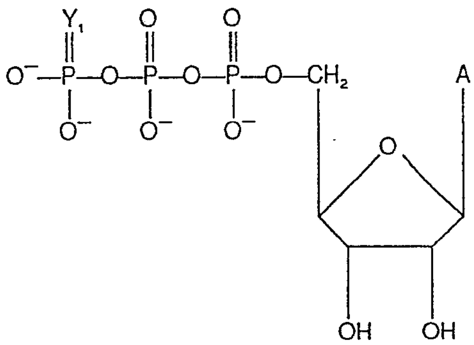

(XXIV)

und dass die Schritte 2), 3) und 4) gemäss Anspruch 1 durchgeführt werden.

5. Verfahren nach Anspruch 1, zur Herstellung von Oligonukleotiden der Formel (VI)

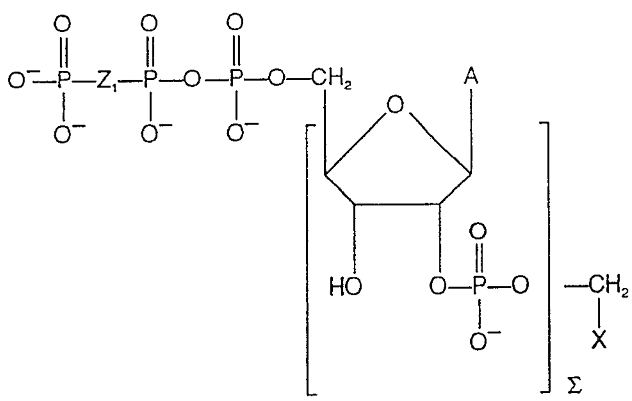

(VI)

wobei $Z_1$ S oder NH darstellt; X ausgewählt ist aus der Gruppe

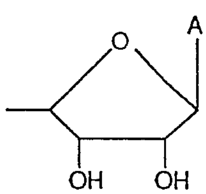

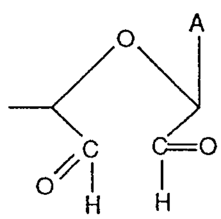

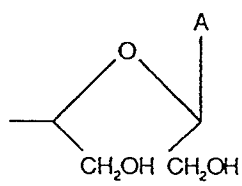

$-CHOHCH_2OH$

Σ eine ganze Zahl gleich 1 ist, wenn X $-CHOHCH_2OH$ darstellt, und gleich n-1 ist, wenn sich X von $-CHOHCH_2OH$ unterscheidet, wobei n zwischen 2 und 10 liegt,
dadurch gekennzeichent, dass die Verbindung der folgenden Formel (XXV) polymerisiert wird,

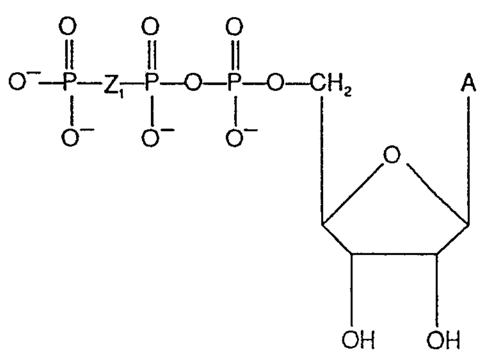

(XXV)

wobei $Z_1$ S oder NH darstellt und A die in Anspruch 1 angegebene Bedeutung hat;

und dass die Schritte 2), 3) und 4) gemäss Anspruch 1 durchgeführt werden.

6. Verfahren nach Anspruch 1, zur Herstellung von Oligonukleotiden der Formel (IV)

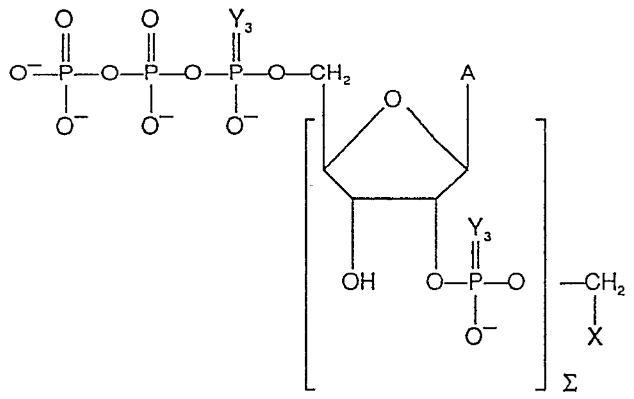

(IV)

wobei $Y_3$ NH, S oder Se darstellt;
Σ, X und A die oben angegebenen Bedeutungen haben, dadurch gekennzeichnet, dass die Verbindung der folgenden Formel (XXVI) polymerisiert wird,

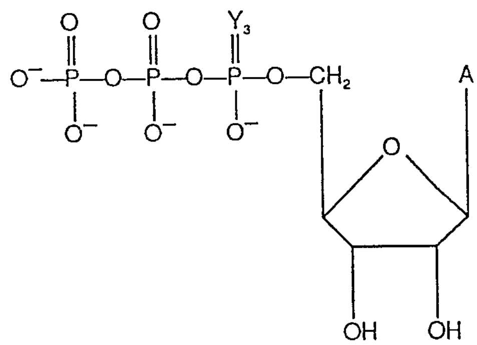

(XXVI)

wobei $Y_3$ S, Se, NH darstellt;
A die oben angegebenen Bedeutungen hat;
und dass die Schritte 2), 3) und 4) gemäss Anspruch 1 durchgeführt werden.

7. Verfahren nach Anspruch 1, zur Herstellung von Oligonukleotiden der folgenden Formel (Vbis)

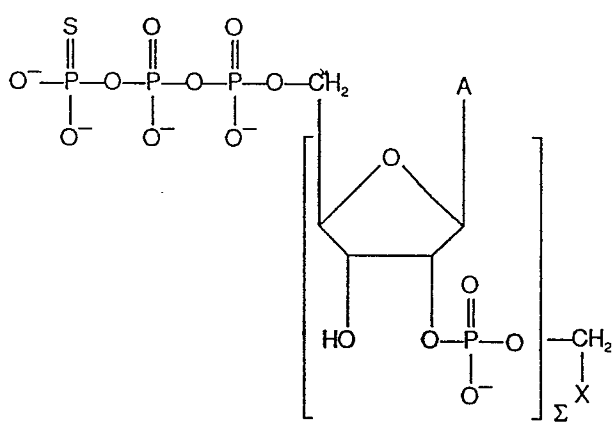

(V bis)

wobei X

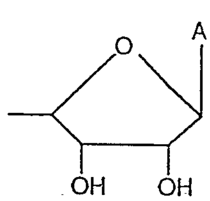

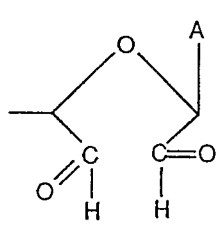

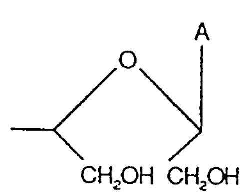

$-CHOHCH_2OH$

darstellt,
Σ eine ganze Zahl gleich n ist, wenn X $-CHOHCH_2OH$ darstellt, und n–1, wenn sich X von $-CHOHCH_2OH$ unterscheidet;

A die Bedeutungen wie in Anspruch 1 angegeben hat, vorzugsweise Adenin, dadurch gekennzeichnet, dass die Verbindung der folgenden Formel (XXIVbis) polymerisiert wird,

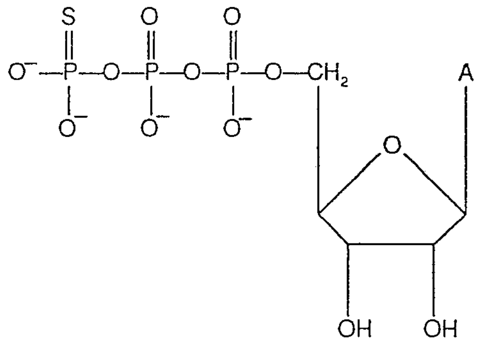

(XXIV bis)

und dass die Schritte 2), 3) und 4) gemäss Anspruch 1 durchgeführt werden.

8. Verfahren nach Anspruch 1, zur Herstellung von Oligonukleotiden der Formel (VII)

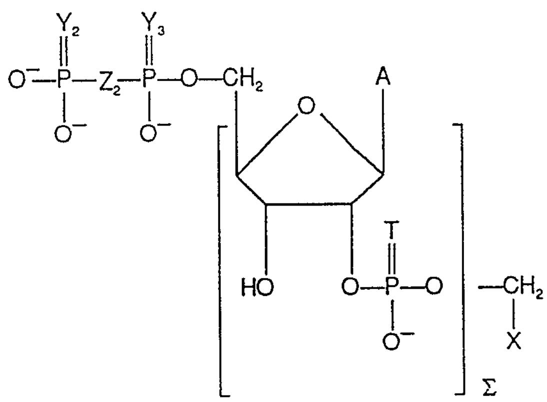

(VII)

wobei $Y_2$, $Y_3$, T gleich oder verschieden sind und O, S, Se, NH darstellen;
$Z_2$ O, S, NH darstellt;

wenigstens eines der Elemente $Y_2$, $Y_3$, $Z_2$ sich von Sauerstoff unterscheidet;
X ausgewählt ist aus der Gruppe

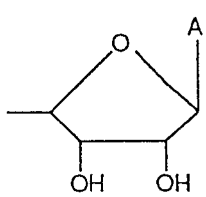

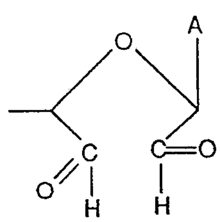

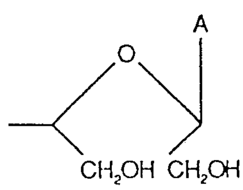

$-CHOHCH_2OH$

Σ eine ganze Zahl gleich n ist, wenn X $-CHOHCH_2OH$ darstellt, und eine ganze Zahl gleich n–1 ist, wenn sich X von $-CHOHCH_2OH$ unterscheidet, wobei n eine ganze Zahl zwischen 2 und 10 ist;
A eine Base ausgewählt aus Adenin oder dessen Derivaten, im besonderen solcher der Formel

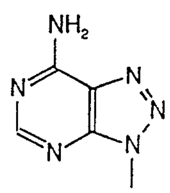

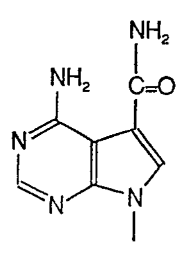

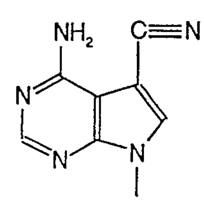

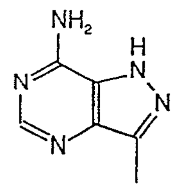
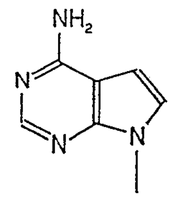
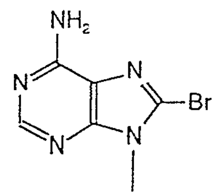

ist.

9. Verfahren nach Anspruch 1, zur Herstellung von Oligonukleotiden der Formel (VIII)

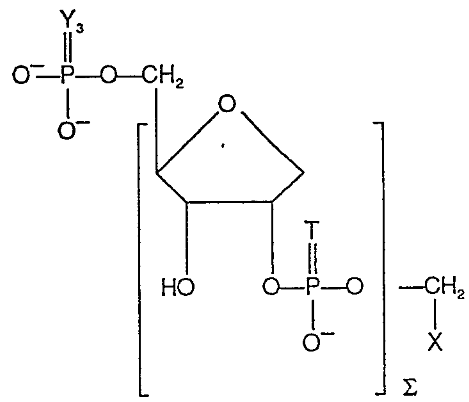

(VIII)

wobei $Y_3$ S, Se, NH darstellt; T O, S, Se NH darstellt; X ausgewählt ist aus der Gruppe

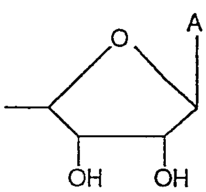
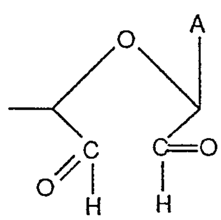
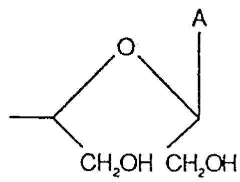
—CHOHCH$_2$OH

$\Sigma$ eine ganze Zahl gleich n darstellt, wenn X -CHOHCH$_2$OH darstellt, und eine ganze Zahl gleich n–1, wenn sich X von -CHOHCH$_2$OH unterscheidet, wobei n eine ganze Zahl zwischen 2 und 10 ist;

A eine Base ausgewählt aus Adenin oder dessen Derivaten, im besonderen solcher der Formel

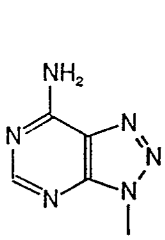
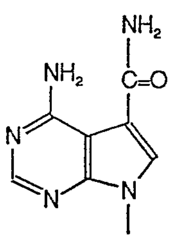
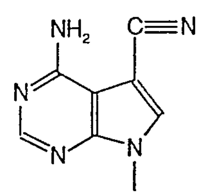
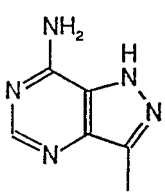
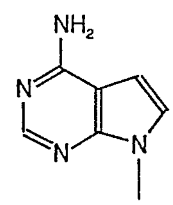
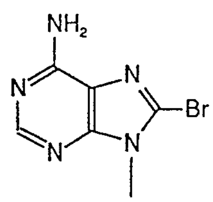

ist.

10. Verfahren zur Herstellung von Oligonukleotiden nach einem der vorstehenden Ansprüche, wobei T Sauerstoff darstellt.

11. Verfahren nach Anspruch 1, zur Herstellung von Oligonukleotiden der Formeln

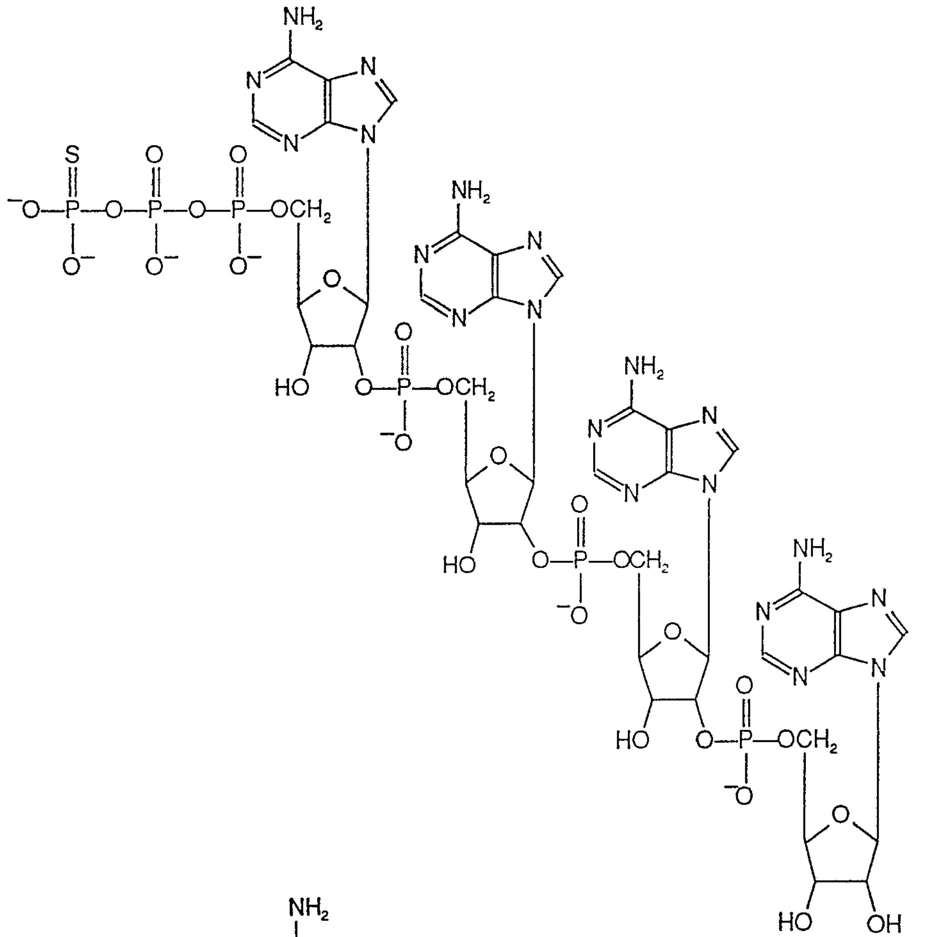

(XV)

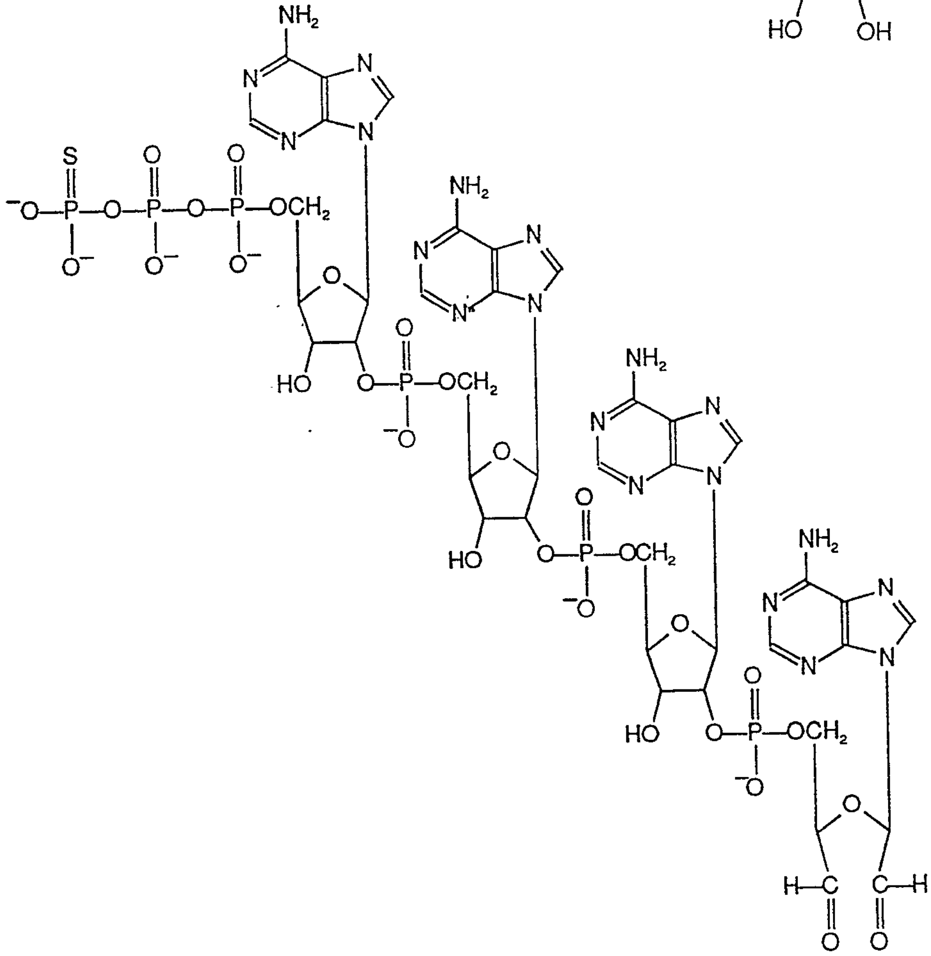

(XVI)

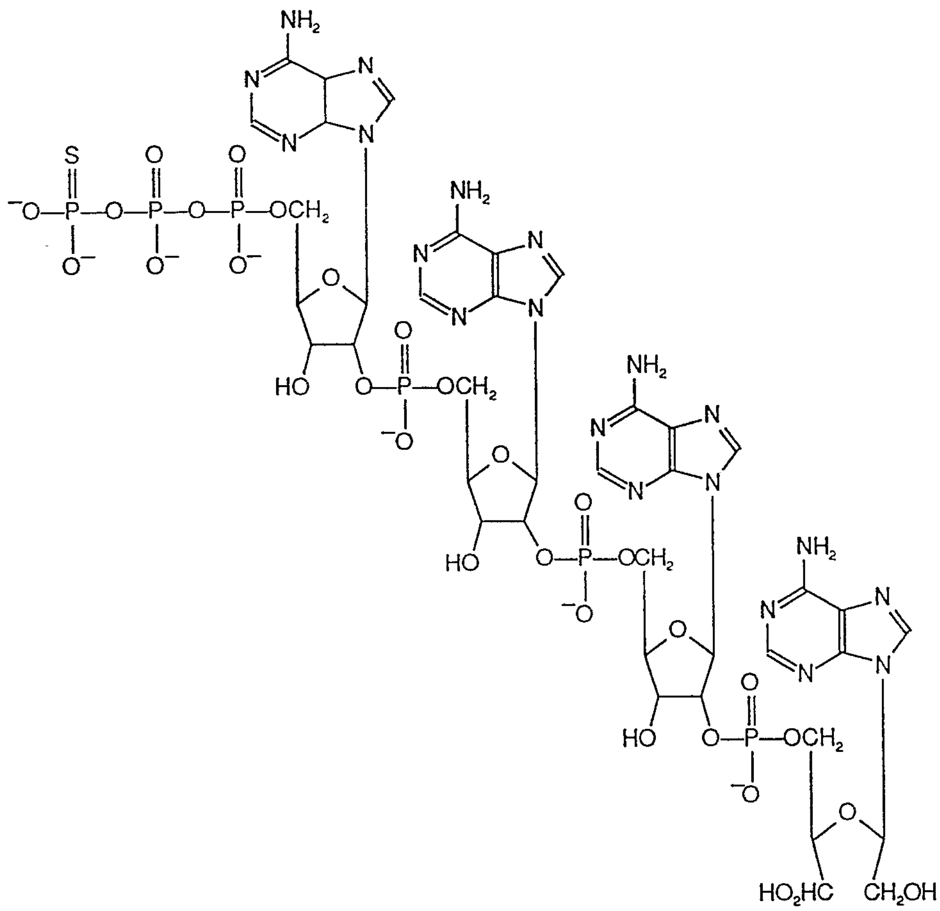

(XVII)

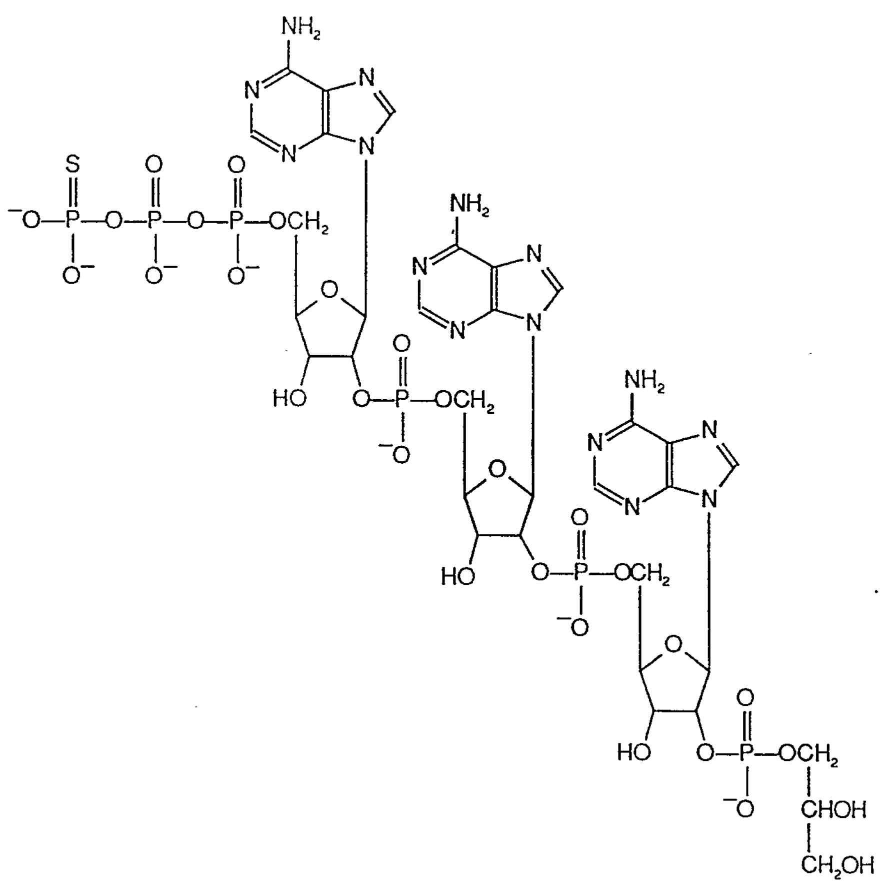

(XVIII)

**Patentansprüche für die Vertragsstaaten: BE, CH, DE, GB, IT, LI, LU, NL, SE**

1. Oligonukleotide der allgemeinen Formel (I):

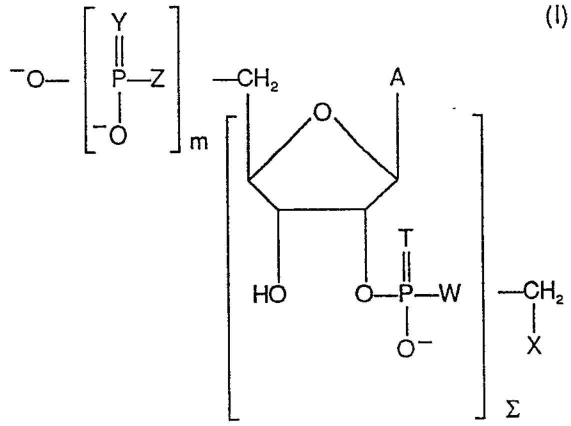

(I)

wobei Y und T gleich oder verschieden sind und O, S, Se, NH darstellen;
Z und W gleich oder verschieden sind und O, S, NH darstellen;
wenigstens eines der Elemente Y und Z von wengistens einer der Phosphatgruppen sich von Sauerstoff unterscheidet;
X ausgewählt ist aus der Gruppe

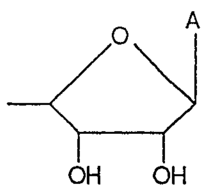
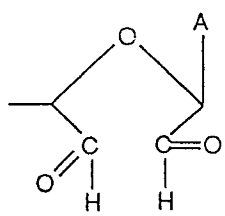
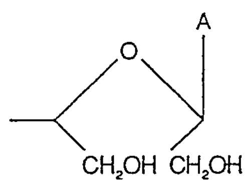

$—CHOHCH_2OH$

und die Alkoholgruppen dieser Reste gegebenenfalls durch Carbonsäure $R_3COOH$ verestert sind, wobei $R_3$ einen Alkylrest mit 1 bis 5 Kohlenstoffatomen oder eine Phenylgruppe darstellt;
Σeine ganze Zahl gleich n ist, wenn X $-CHOHCH_2OH$ darstellt, eine ganze Zahl gleich n–1 ist, wenn X

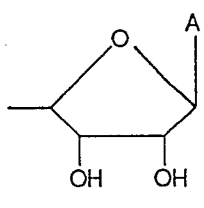
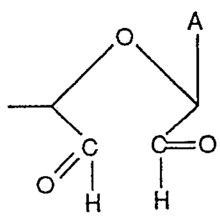
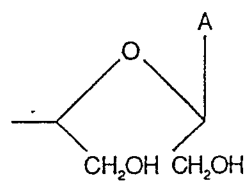

darstellt, wobei n eine ganze Zahl höher als oder gleich 2 ist;
m eine ganze Zahl höher als oder gleich 1 ist;
A eine Base ausgewählt aus Adenin oder dessen Derivaten, im besonderen solcher der Formel

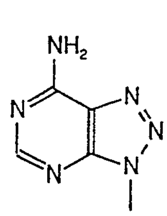
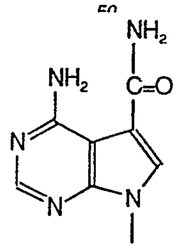
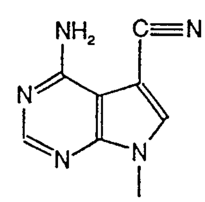
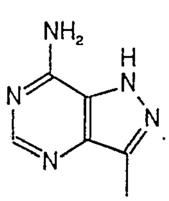
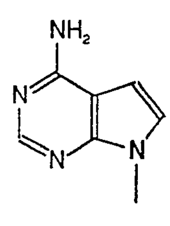
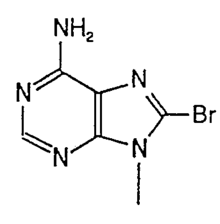

ist.

2. Oligonukleotide nach Anspruch 1, entsprechend der allgemeinen Formel (II)

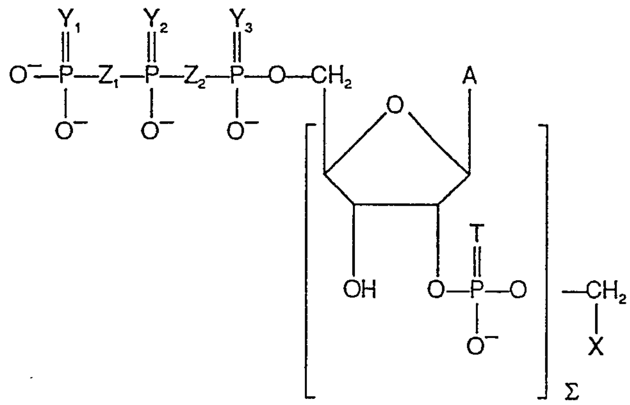

(II)

wobei $Y_1$, $Y_2$, $Y_3$, T gleich oder verschieden sind und O, S, Se, NH darstellen;
$Z_1$ und $Z_2$ gleich oder verschieden sind und O, S, NH darstellen;
wengistens eines der Elemente $Y_1$, $Y_2$, $Y_3$, $Z_1$, $Z_2$ sich von Sauerstoff unterscheidet;
X ausgewählt ist aus der Gruppe

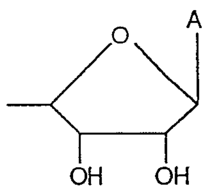 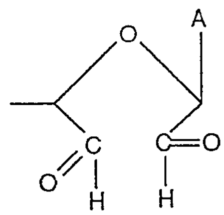 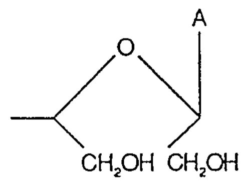 $—CHOHCH_2OH$

und die Alkoholgruppen dieser Reste gegebenenfalls durch die Carbonsäuren $R_3COOH$ verestert sind, wobei $R_3$ einen Alkylrest mit 1 bis 5 Kohlenstoffatomen oder eine Phenylgruppe darstellt;
Σ eine ganze Zahl gleich n darstellt, wenn X $-CHOHCH_2OH$ darstellt, und Σ eine ganze Zahl gleich n–1 darstellt, wenn X sich von $-CHOH-CH_2OH$ unterscheidet, wobei n eine ganze Zahl höher als oder gleich 2 ist;
A eine Base ausgewählt aus Adenin oder dessen Derivaten, im besonderen solcher der Formel

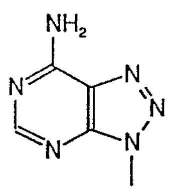 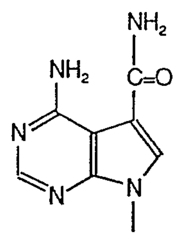 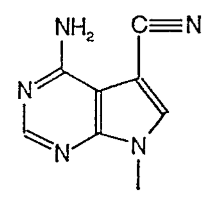

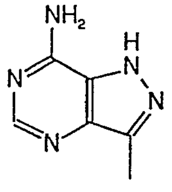 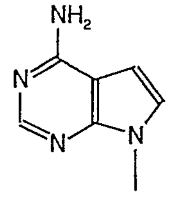 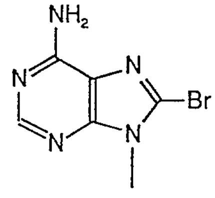

ist.

3. Oligonukleotide nach Anspruch 1 der Formel (VII)

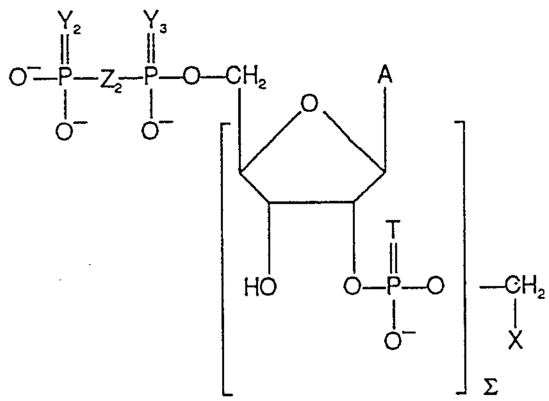

(VII)

wobei $Y_2$, $Y_3$, T gleich oder verschieden sind und O, S, Se, NH darstellen;
$Z_2$ O, S, NH darstellt;
wenigstens eines der Elemente $Y_2$, $Y_3$, $Z_2$ sich von Sauerstoff unterscheidet;
X ausgewählt ist aus der Gruppe

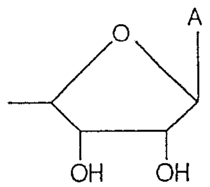

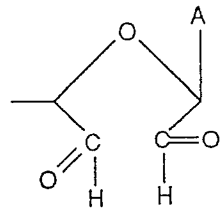

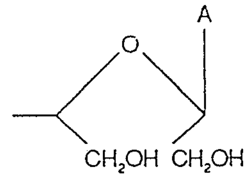

$-CHOHCH_2OH$

ist;

Σ eine ganze Zahl gleich n ist, wenn X $-CHOH-CH_2OH$ darstellt, und eine ganze Zahl gleich n-1 ist, wenn sich X von $-CHOHCH_2OH$ unterscheidet, wobei n eine ganze Zahl zwischen 2 und 10 ist;
A eine Base ausgewählt aus Adenin oder dessen Derivaten, im besonderen solcher der Formel

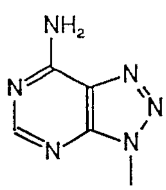

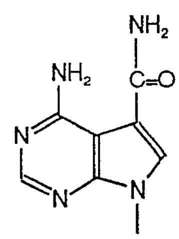

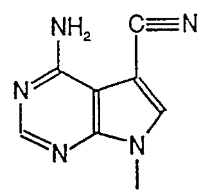

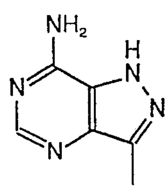

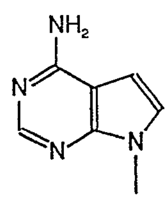

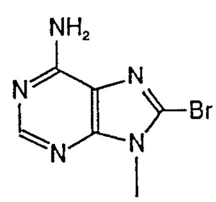

ist.

4. Oligonukleotide nach Anspruch 1 der allgemeinen Formel (VIII)

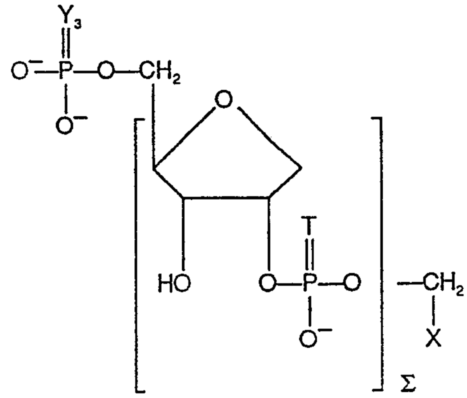

(VIII)

wobei $Y_3$ S, Se, NH darstellt; T O, S, Se, NH darstellt; X ausgewählt ist aus der Gruppe

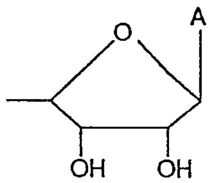
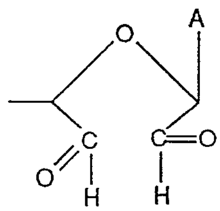
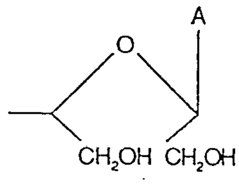
$-CHOHCH_2OH$

Σ eine ganze Zahl gleich n darstellt, wenn X $-CHOHCH_2OH$ darstellt, und eine ganze Zahl gleich n–1 ist, wenn sich X von $-CHOHCH_2OH$ unterschiedet, wobei n eine ganze Zahl zwischen 2 und 10 ist;

A eine Base ausgewählt aus Adenin oder dessen Derivaten, im besonderen solcher der Formel

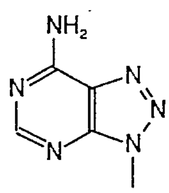
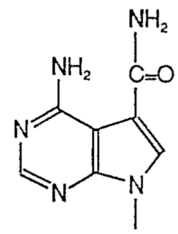
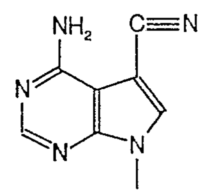
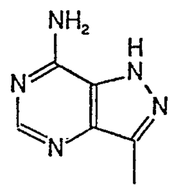
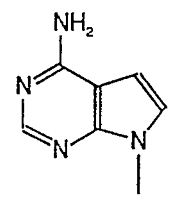
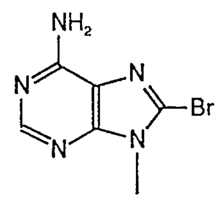

ist.

5. Oligonukleotide nach einem der vorstehenden Ansprüche, wobei T Sauerstoff darstellt.

6. Oligonukleotide nach Anspruch 1 der Formeln

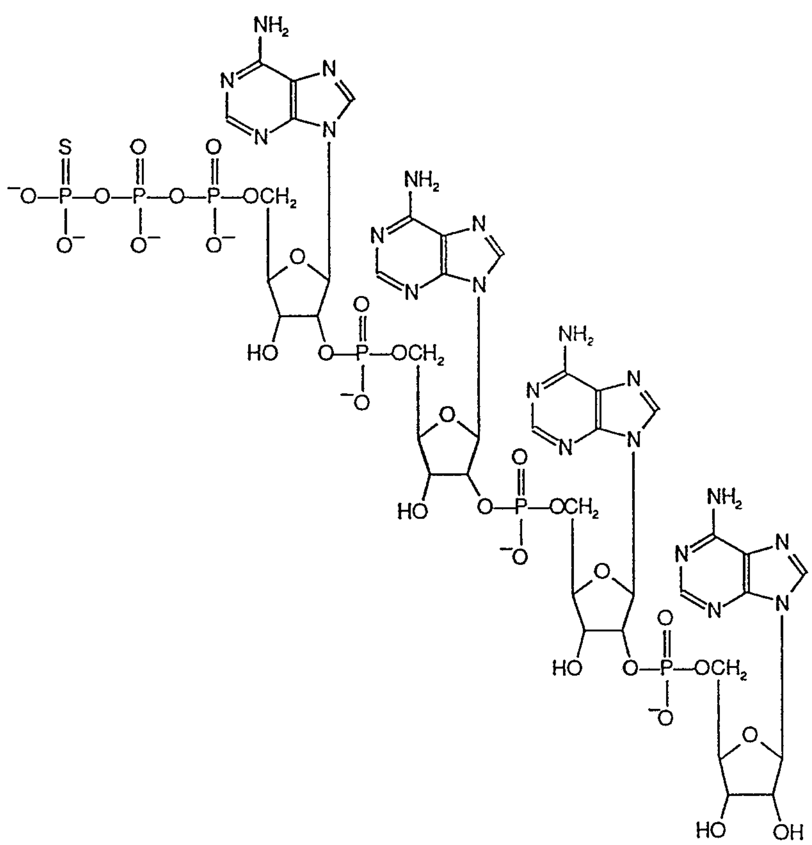

(XV)

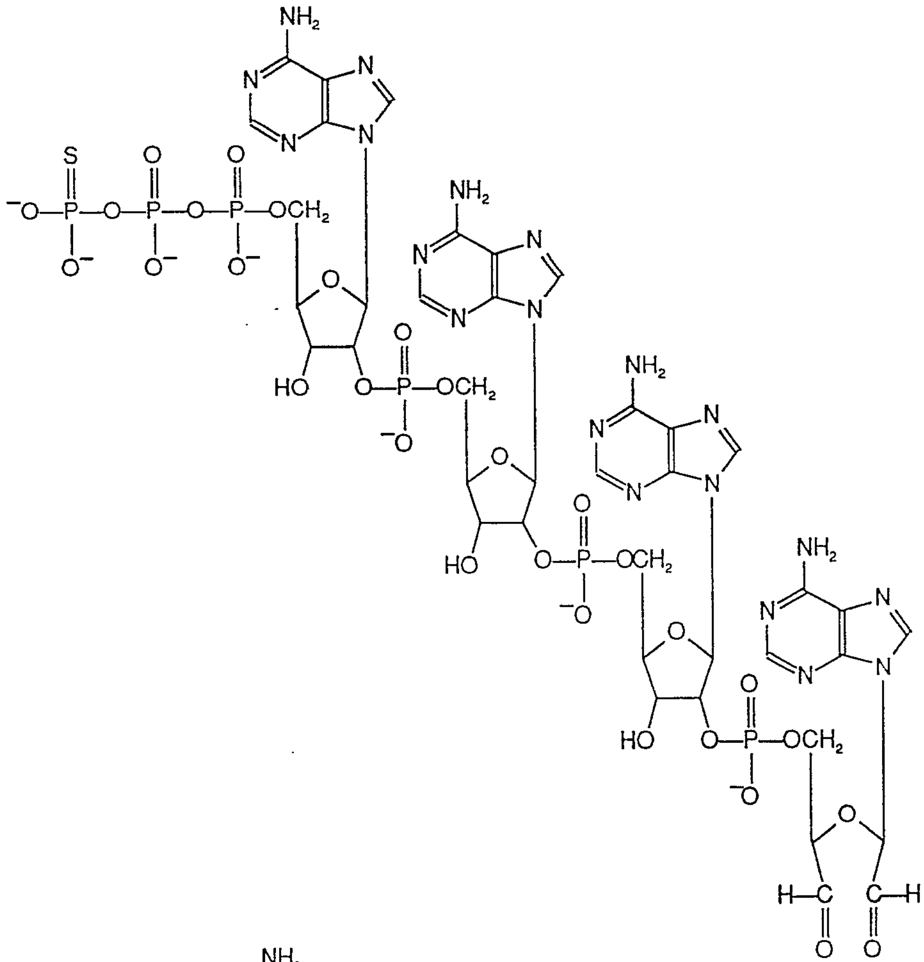

(XVI)

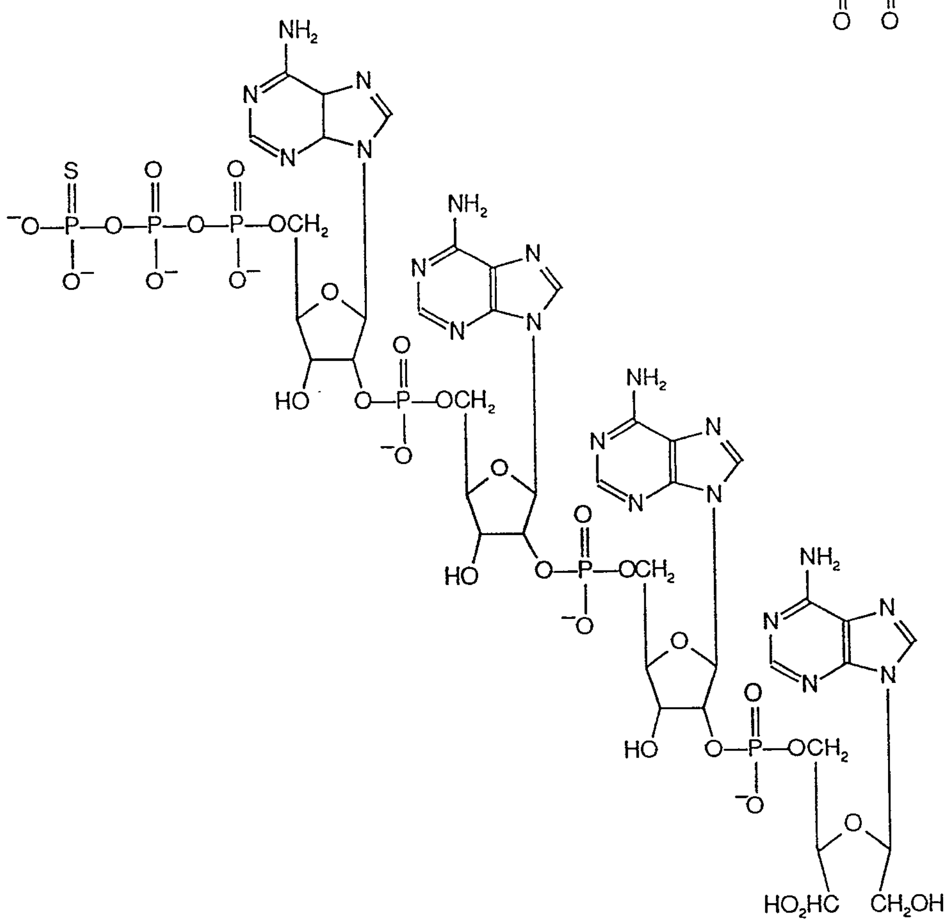

(XVII)

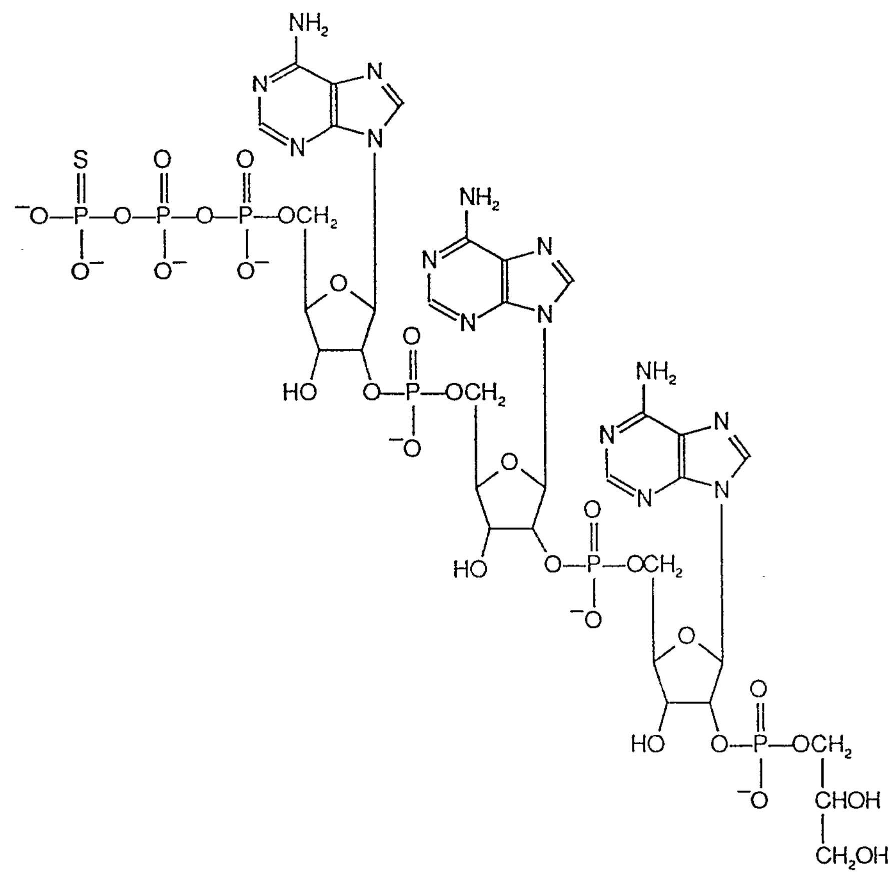

(XVIII)

7. Verfahren zur Herstellung von Oligonukleotiden der Formel (I) nach Anspruch 1

(I)

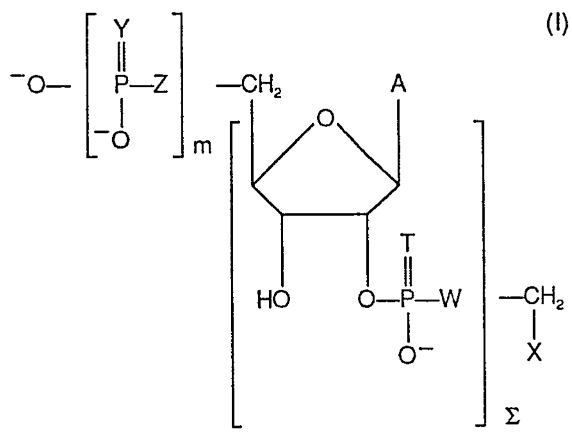

wobei Y und T gleich oder verschieden sind und O, S, Se oder NH darstellen;
Z und W gleich oder verschieden sind und O, S, oder NH darstellen;
wenigstens eines der Elemente Y oder Z von wenigstens einer der Phosphatgruppen sich von Sauerstoff unterscheidet;
m höher als oder gleich 1 ist;
A und X die in Anspruch 1 angegebene Bedeutung haben, dadurch gekennzeichnet, dass sie die Polymerisation der Verbindung der folgenden Formel (XIX)

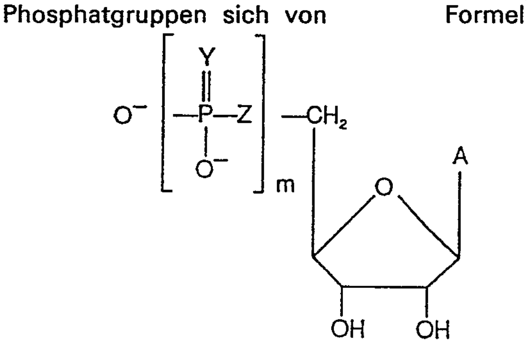

(XIX)

umfasst, um eine Verbindung der folgenden Formel (Ibis) zu erhalten

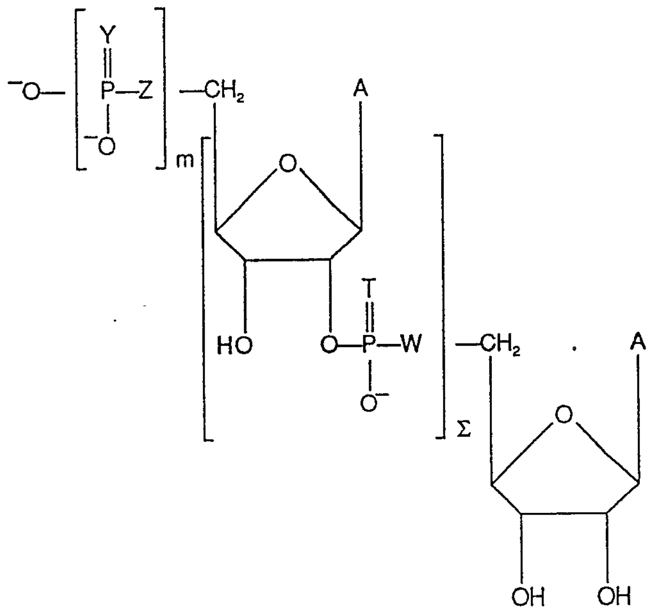

(I bis)

wobei Y und T, gleich oder verschieden, O, S, Se oder NH darstellen;
Z und W, gleich oder verschieden, O, S oder NH darstellen;
wenigstens eines der Elemente Y und Z von wenigstens einer der Phosphatgruppen sich von Sauerstoff unterscheidet;
Σ eine ganze Zahl gleich n−1 ist, wobei n höher als oder gleich 2 ist;
m eine ganze Zahl höher als oder gleich 1 ist;
A die oben angegebenen Bedeutungen hat;
und wenn nötig, die folgenden chemischen Schritte durchläuft, und zwar:
gegebenenfalls die Oxidation der Glykolgruppe zur Einführung von Aldehydgruppen auf die Kohlenstoffatome in den Positionen 2′ und 3′ der letzten nukleosiden Einheit, wobei die Verbindung der Formel (Iter) erhalten wird,

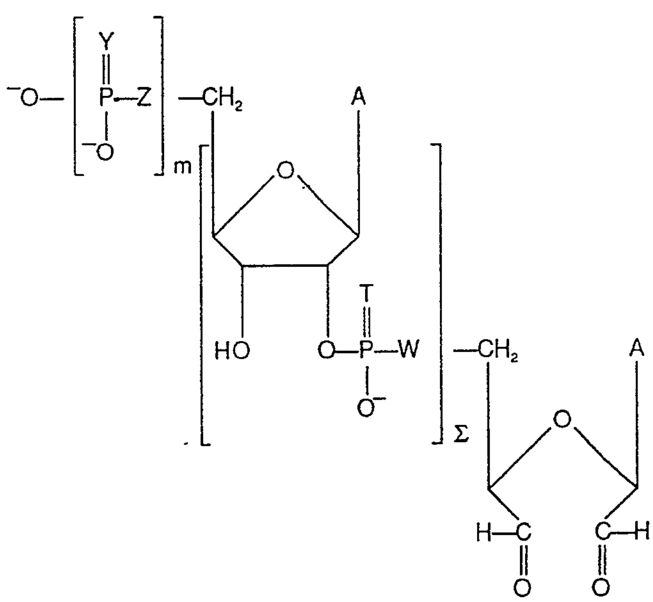

(I ter)

gegebenenfalls die Reduktion der beiden Aldehydgruppen in Alkoholgruppen, um die Verbindung der Formel (Iquater) zu erhalten,

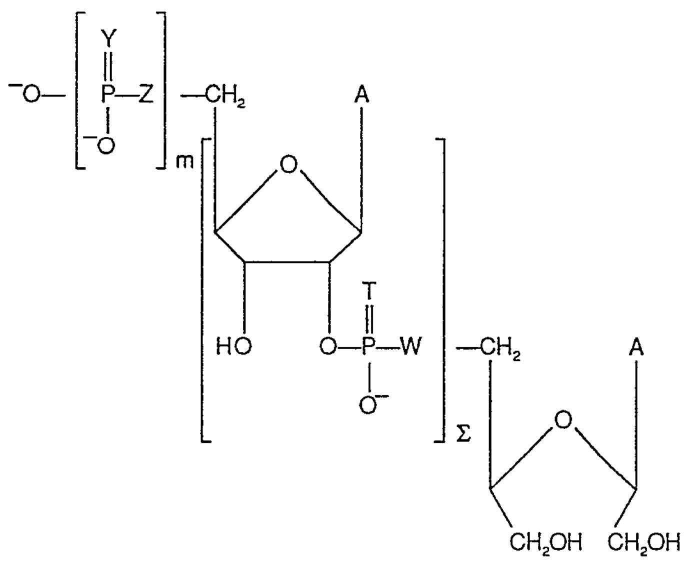

(I quater)

gegebenenfalls die Hydrolyse unter solchen Bedingungen, dass eine Beta-Elimination vermieden wird, um die Verbindung der folgenden Formel (Iquinquiès) zu erhalten

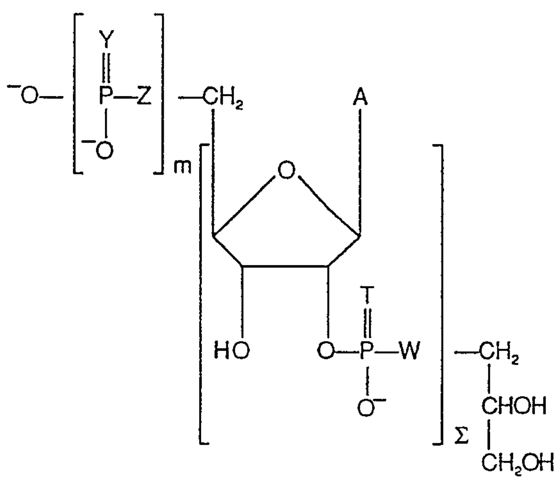

(I quinquies)

8. Verfahren nach Anspruch 7, zur Herstellung von Oligonukleotiden der Formel (XX),

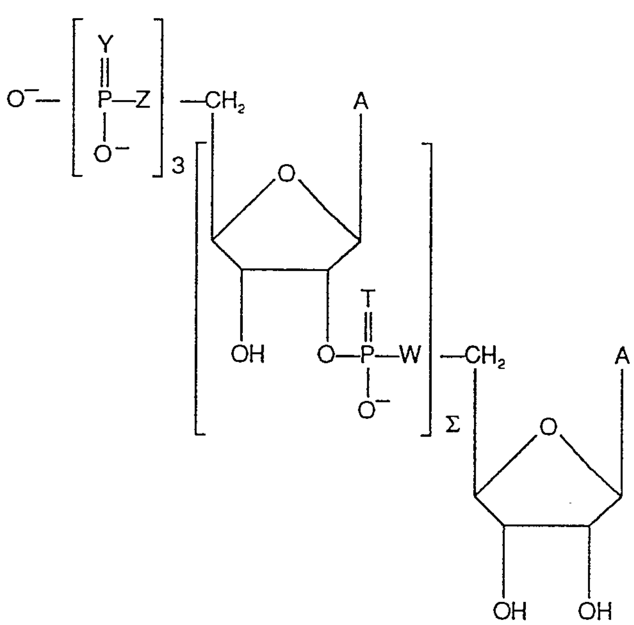

(XX)

wobei Y und T, gleich oder verschieden, O, S, Se, NH darstellen;
Z und W, gleich oder verschieden, O, S, NH darstellen;
die Elemente Y und Z von wenigstens einer der Phosphatgruppen nicht gleichzeitig Sauerstoff darstellt;
Σ eine ganze Zahl zwischen 1 und 9 ist;
A eine Base ausgewählt aus Adenin oder dessen Derivaten, im besonderen solcher der Formel

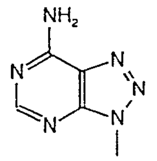
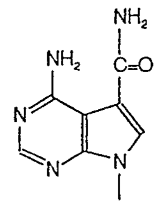
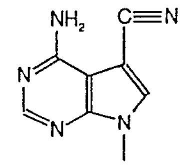
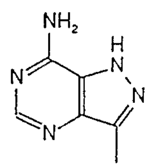
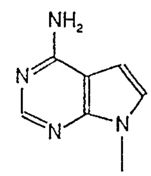
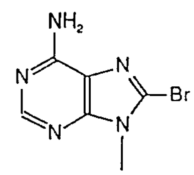

ist, dadurch gekennzeichnet, dass es
1) die Polymerisation einer Verbindung der folgenden Formel (XIXbis) umfasst,

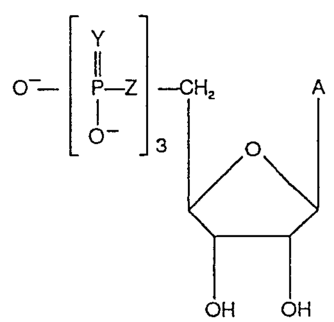
(XIX bis)

wobei Y O, S, Se oder NH darstellt;
Z O, S oder NH darstellt;
die Elemente Y und Z von wenigstens einer der Phosphatgruppen nicht gleichzeitig Sauerstoff darstellt;
A die in Anspruch 1 angegebenen Bedeutungen hat;
2) gegebenenfalls die Oxidation der Glykolendgruppe, im besonderen durch ein Perjodation, zur Umsetzung des Glykols in zwei Aldehydgruppen und zum Erhalt einer Verbindung der folgenden Formel (XXI) umfasst:

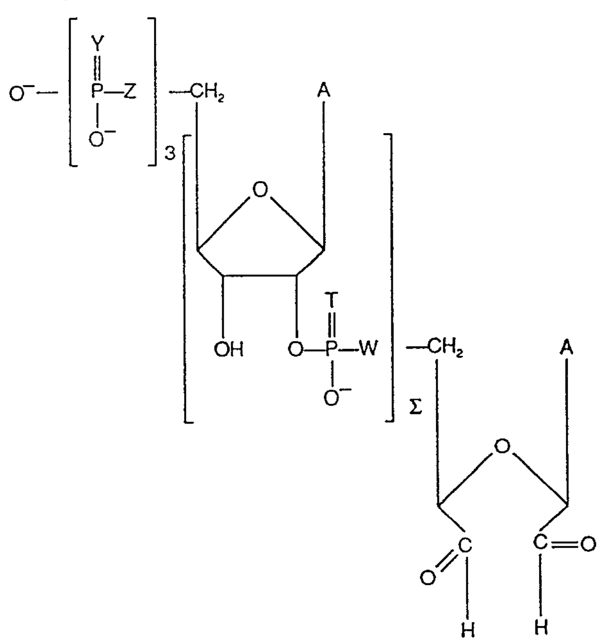
(XXI)

wobei Y, Z, T, W, Σ und A die oben genannten Bedeutungen haben;
3) gegebenenfalls die Reduktion der Aldehydgruppen, im besonderen durch das Natriumborhydrid, zur Umsetzung der beiden oben genannten Aldehydgruppen in Alkoholgruppen und zum Erhalt der Verbindung der folgenden Formel (XXII) umfasst:

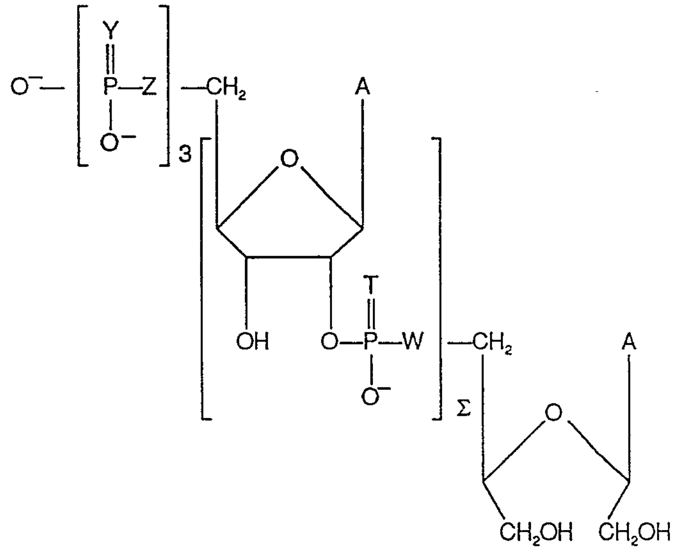

(XXII)

4) gegebenenfalls die Hydrolyse, im besonderen gesteuerte Hydrolyse, zur Freisetzung des Ribosekerns und zum Erhalt der Verbindung der folgenden Formel (XXIII):

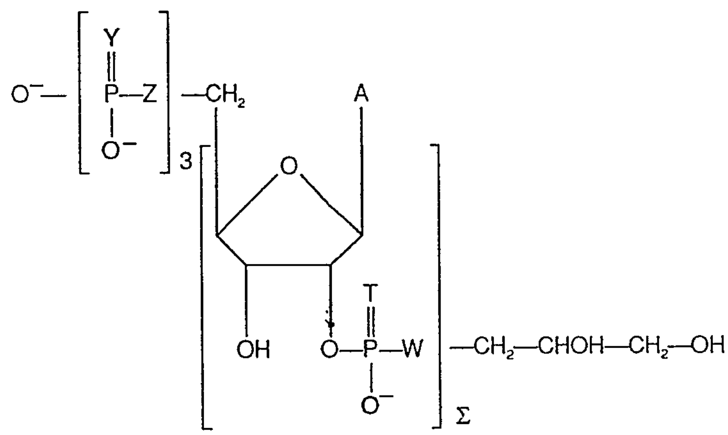

(XXIII)

9. Verfahren nach Anspruch 7, zur Herstellung von Oligonukleotiden der Formel (V)

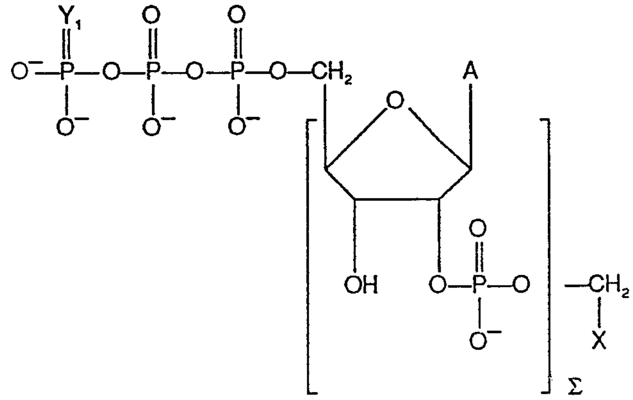

(V)

wobei $Y_1$ NH, Se, S darstellt;
X

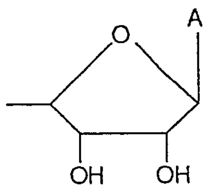
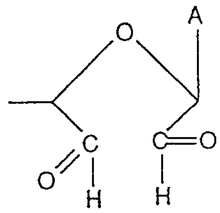
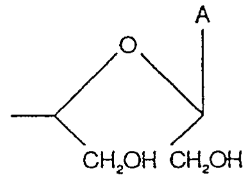
$—CHOHCH_2OH$

darstellt;
Σ eine ganze Zahl gleich n ist, wenn X $-CHOHCH_2OH$ darstellt, und n-1, wenn sich X von $-CHOHCH_2OH$ unterscheidet, wobei n zwischen 2 und 10 liegt,
dadurch gekennzeichnet, dass die Verbindung der folgenden Formel (XXIV) polymerisiert wird:

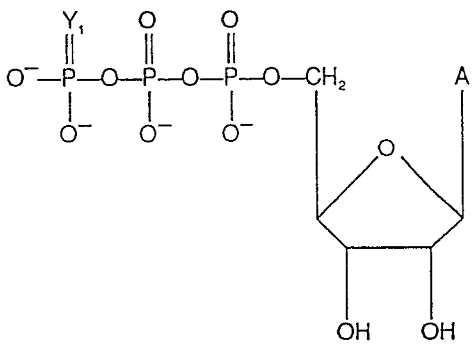

(XXIV)

und dass die Schritte 2), 3) und 4) gemäss Anspruch 7 durchgeführt werden.

10. Verfahren nach Anspruch 7, zur Herstellung von Oligonukleotiden der Formel (VI)

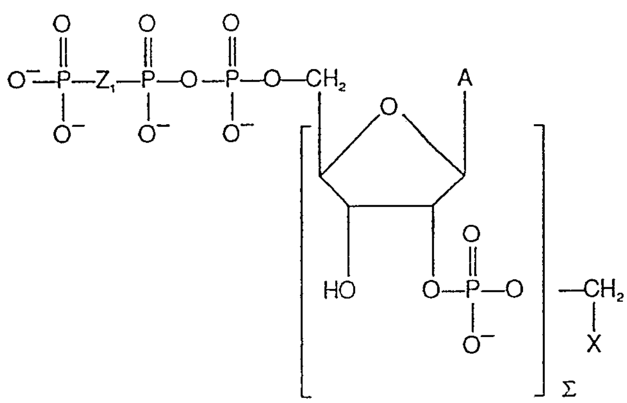

(VI)

wobei $Z_1$ S oder NH darstellt; X ausgewählt ist aus der Gruppe

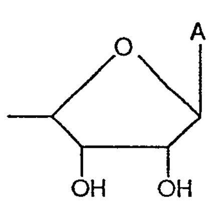
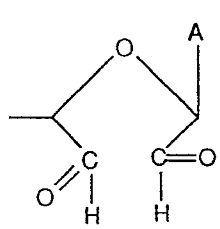
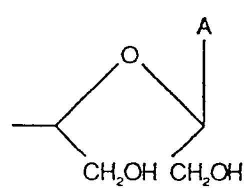
$—CHOHCH_2OH$

Σ eine ganze Zahl gleich 1 ist, wenn X $-CHOHCH_2OH$ darstellt, und gleich n-1 ist, wenn sich X von $-CHOHCH_2OH$ unterscheidet, wobei n zwischen 2 und 10 liegt,
dadurch gekennzeichnet, dass die Verbindung der folgenden Formel (XXV) polymerisiert wird,

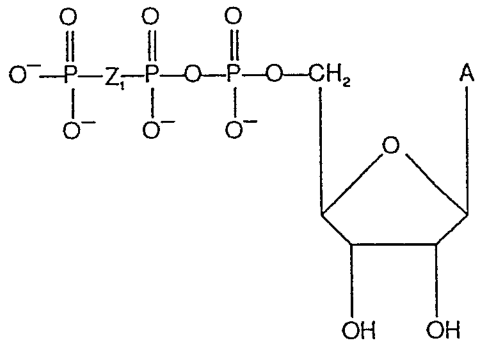

(XXV)

wobei $Z_1$ S oder NH darstellt und A die in Anspruch 1 angegebene Bedeutung hat; und dass die Schritte 2), 3) und 4) gemäss Anspruch 7 durchgeführt werden.

11. Verfahren nach Anspruch 7, zur Herstellung von Oligonukleotiden der Formel (IV)

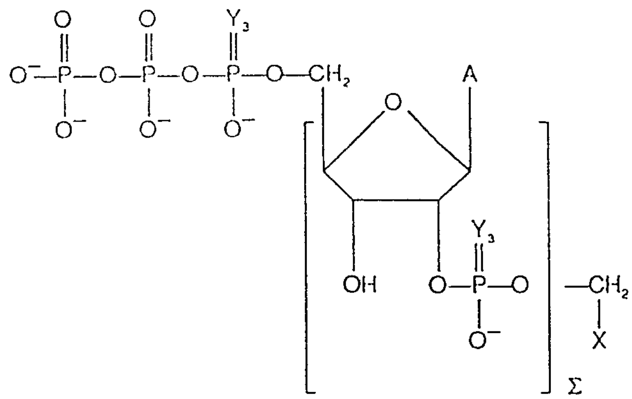

(IV)

wobei $Y_3$ NH, S oder Se darstellt;
Σ, X und A die oben genannten Bedeutungen haben, dadurch gekennzeichnet, dass die Verbindung der folgenden Formel (XXVI) polymerisiert wird,

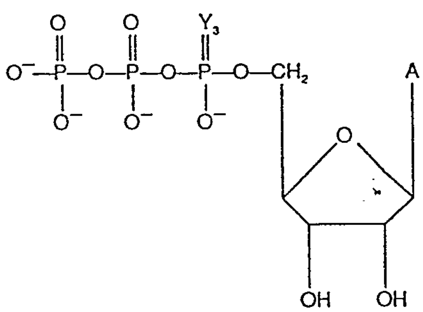

(XXVI)

wobei $Y_3$ S, Se, NH darstellt;
A die oben angegebenen Bedeutungen hat;
und dass die Schritte 2), 3) und 4) gemäss Anspruch 7 durchgeführt werden.

12. Verfahren nach Anspruch 7, zur Herstellung von Oligonukleotiden der folgenden Formel (Vbis)

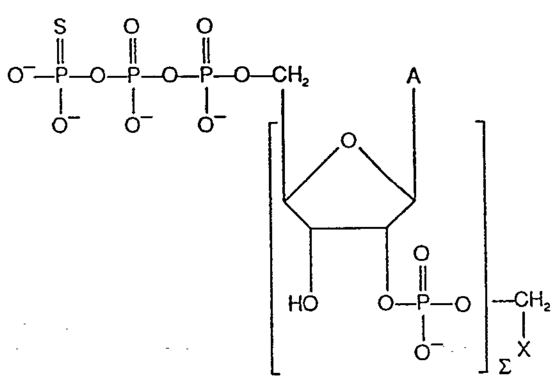

(V bis)

wobei X

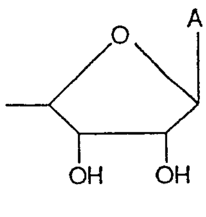 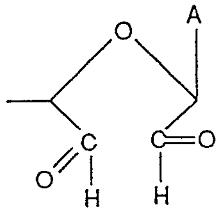 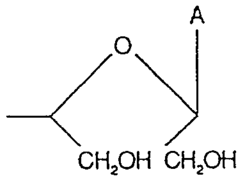 —$CHOHCH_2OH$

darstellt,

Σ eine ganze Zahl gleich n ist, wenn X $-CHOHCH_2OH$ darstellt, und n-1, wenn sich X von $-CHOHCH_2OH$ unterscheidet;

A die Bedeutungen wie in Anspruch 1 angegeben, vorzugsweise Adenin, hat, dadurch gekennzeichnet, dass die Verbindung der folgenden Formel (XXIVbis) polymerisiert wird,

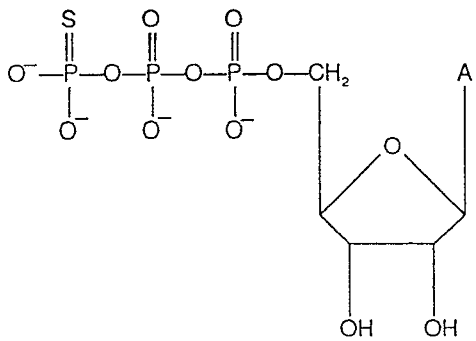

(XXIV bis)

und dass die Schritte 2), 3) und 4) gemäss Anspruch 7 durchgeführt werden.

**Claims for the Contracting State: AT**

1. Process for the preparation of oligonucleotides of formula (I):

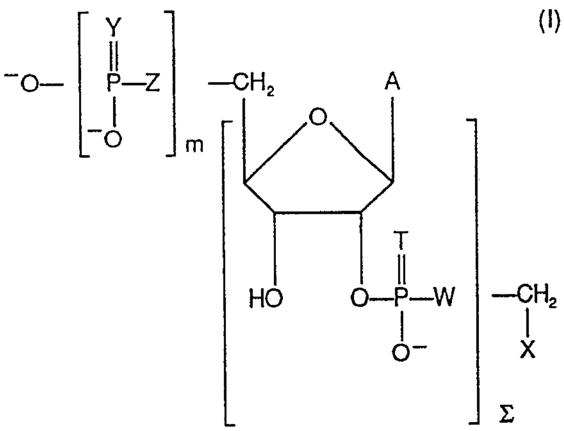

(I)

in which:

- Y and T are identical or different and represent O, S, Se or NH;
- Z and W are identical or different and represent O, S or NH;
- one at least of the Y and Z elements of at least one of the phosphate groups being different from oxygen;
- m is greater than or equal to 1;
- X is chosen in the group constituted by:

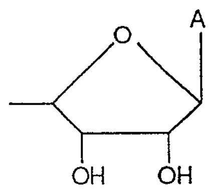 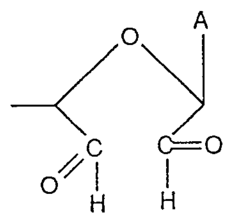

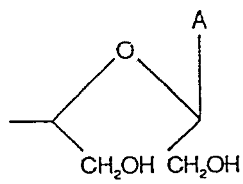 —$CHOHCH_2OH$

the alcohol functions of these radicals being possibly esterified by carboxylic acids $R_3COOH$, $R_3$ representing an alkyl radical from 1 to 5 carbon atoms or a phenyl group;

- Σ is an integer equal to:
  n when X represents $-CHOHCH_2OH$,
  n-1 when X represents:

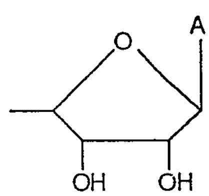 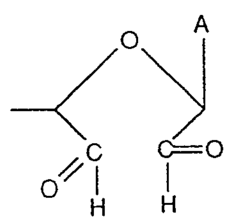

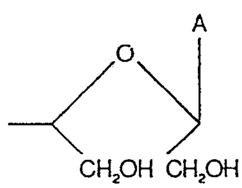

n being an integer greater than or equal to 2;
- m is an integer greater than or equal to 1;
- A is a base chosen among adenine or its derivatives, particularly those of formula:

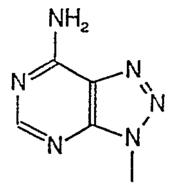
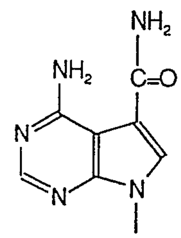
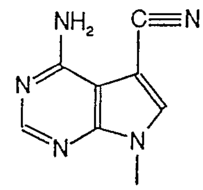
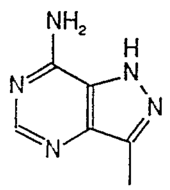
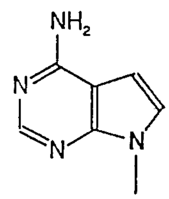
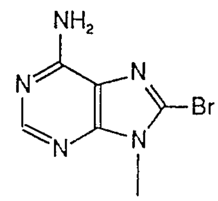

characterized in that it comprises:
1) the polymerization of compounds of the following formula (XIX):

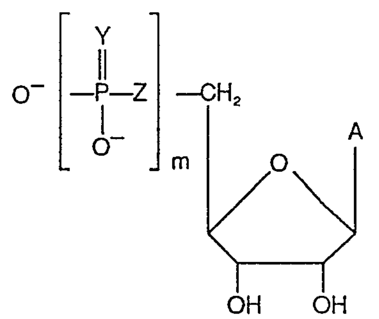
(XIX)

to obtain a compound of the following formula (Ibis):

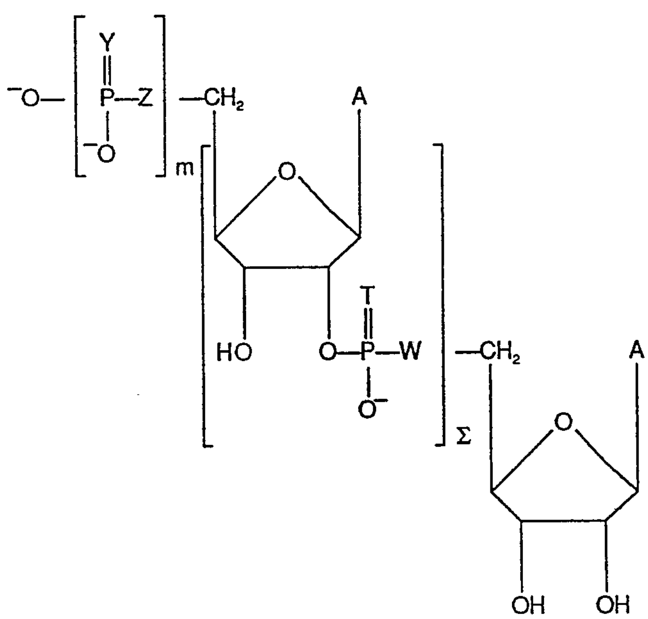
(I bis)

in which Y, T, Z, W, m and A have the above mentioned meanings;
- Σ is an integer equal to n-1, n'being greater than or equal to 2;
- and if necessary the following chemical steps i.e.:
2) the possible oxidation of the glycol group to introduce aldehyde functions on to the carbons at 2' and 3' positions of the last nucleosidic unit and to obtain the compound of formula (Iter):

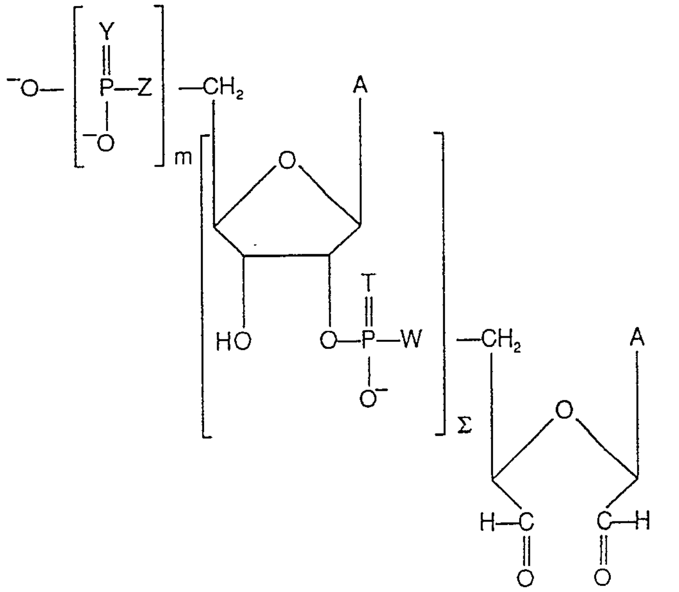

(I ter)

3) the possible reduction of the two aldehyde functions into alcohol functions to obtain the compound of formula (Iquater):

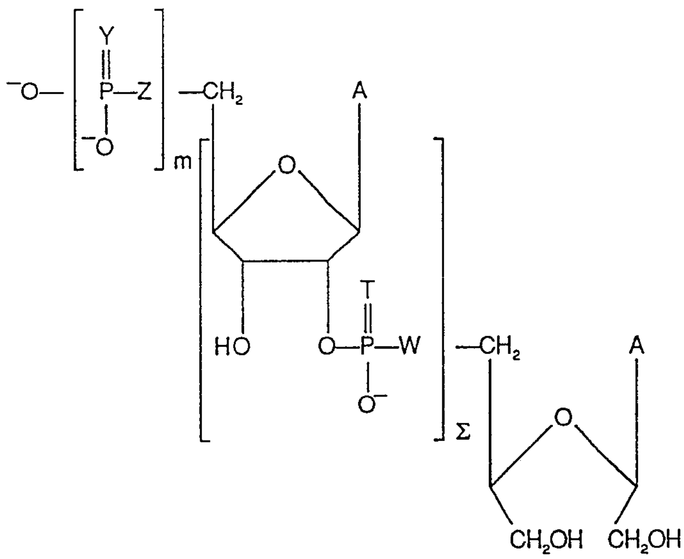

(I quater)

4) the possible hydrolysis under conditions avoiding beta-elimination, to obtain a compound of the following formula (Iquinquiès):

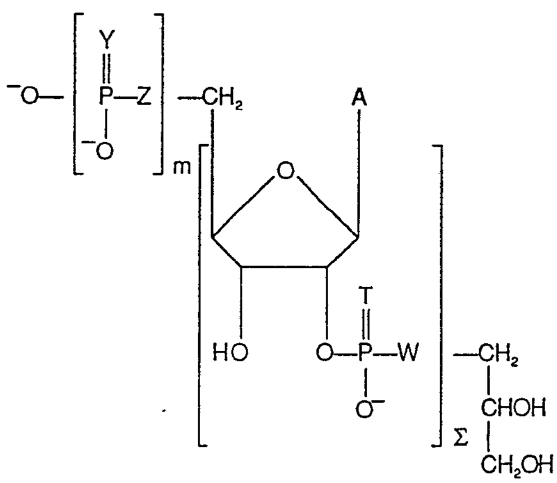

(I quinquies)

2. Process according to claim 1 for the preparation of oligonucleotides of formula (XX):

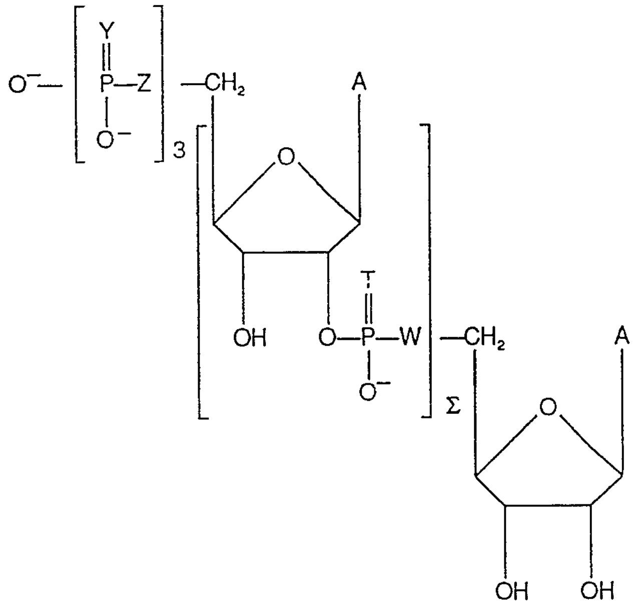

(XX)

in which:
- Y and T, identical or different, represent O, S, Se, NH;
- Z and W, identical or different, represent O, S, NH;
- the Y and Z elements of at least one of the phosphate groups representing not simultaneously oxygen;
- Σ is an integer varying from 1 to 9;
- A is a base chosen among adenine or its derivatives particularly those of formula:

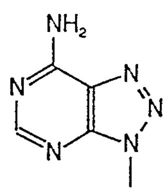

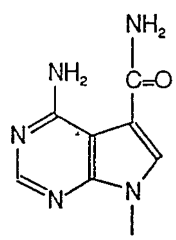

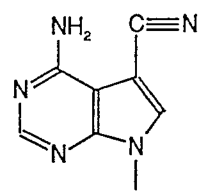

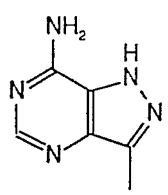

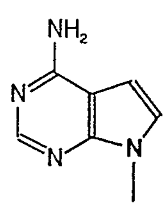

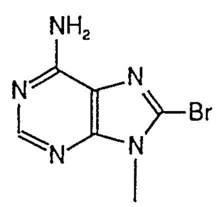

characterized in that it comprises:
1) the polymerization of a compound of the following formula (XIXbis):

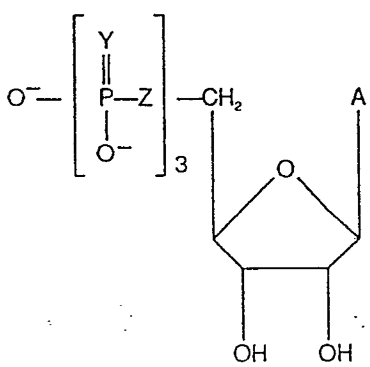

(XIX bis)

in which:
- Y represents O, S, Se or NH;
- Z represents O, S or NH;
- the Y and Z elements of at least one of the phosphate groups representing not simultaneously oxygen;
- A has the meaning mentioned in claim 1;

2) the possible oxidation of the terminal glycol group, particularly by periodate ion to transform the glycol in two aldehyde functions and obtain a compound for the following formula (XXI):

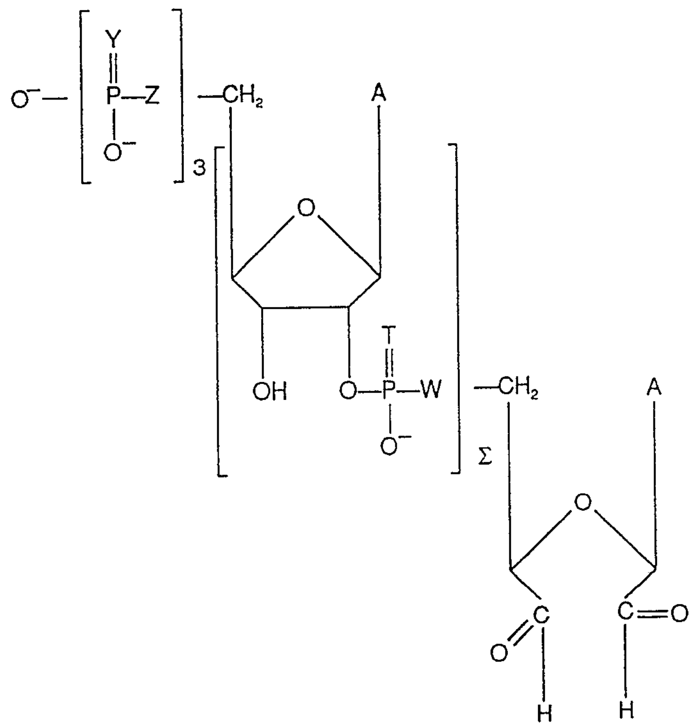

(XXI)

in which:
- Y, Z, T, W, Σ and A have the meanings above mentioned;

3) the possible reduction of the aldehyde functions, particularly by sodium borohydride to transform the two above mentioned aldehyde functions into alcohol functions and to obtain the compound of the following formula (XXII):

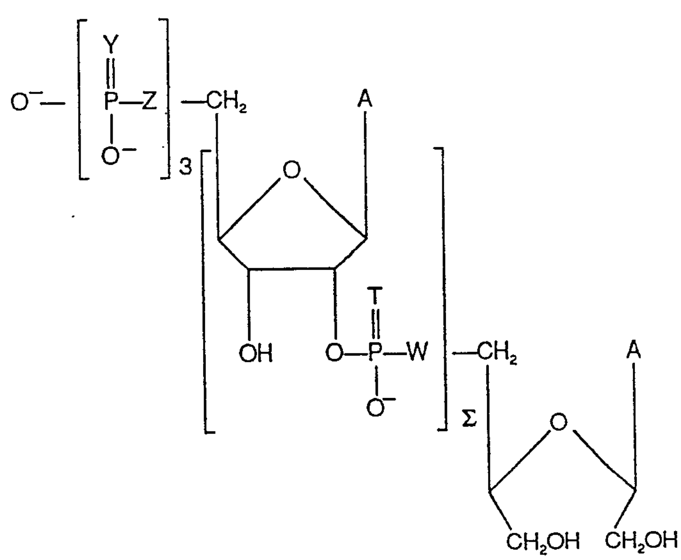

(XXII)

4) the possible hydrolysis, particularly controlled acid hydrolysis, to eliminate the ribose nucleus and obtain the compound of the following formula (XXIII):

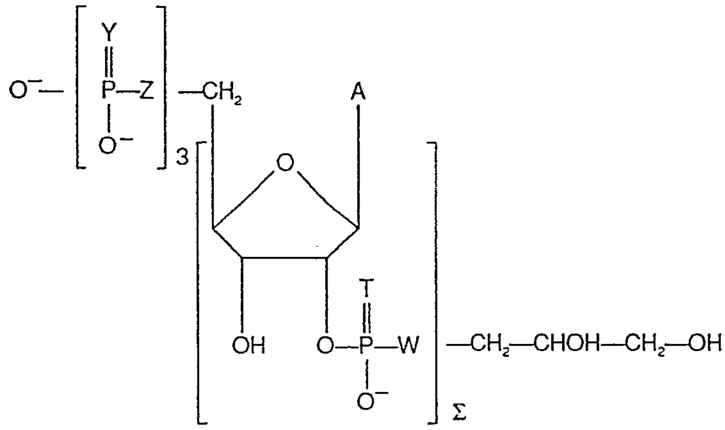

(XXIII)

3. Process according to claim 1, for the preparation of oligonucleotides according to claim 1 having the general formula (II):

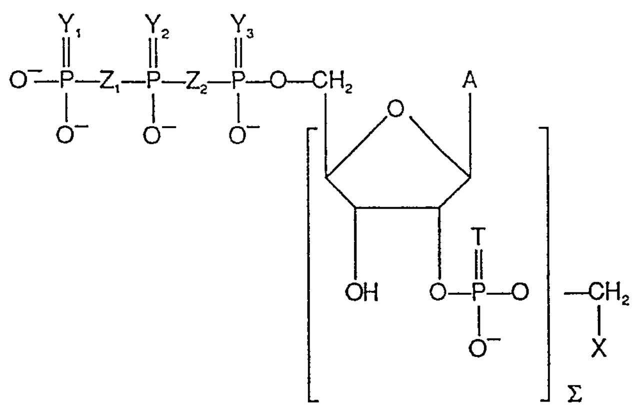

(II)

in which:
- $Y_1$, $Y_2$, $Y_3$, T are identical or different and represent O, S, Se, NH;
- $Z_1$ and $Z_2$ are identical or different and represent O, S, NH;
one at least of the $Y_1$, $Y_2$, $Y_3$, $Z_1$, $Z_2$ elements being different from oxygen;
- X is chosen among the group constituted by:

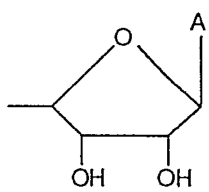

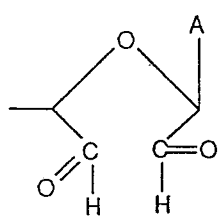

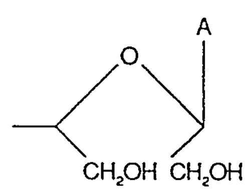

$—CHOHCH_2OH$

the alcohol functions of those radicals being possibly esterified by carboxylic acids $R_3COOH$, $R_3$ representing an alkyl radical from 1 to 5 carbon atoms or a phenyl group;
- Σ represents an integer equal to n when X represents $-CHOHCH_2OH$, and Σ represents an integer equal to n–1 when X is different from $-CHOHCH_2OH$, n being an integer greater than or equal to 2;
- A is a base chosen among adenine or its derivatives, particularly those of formula:

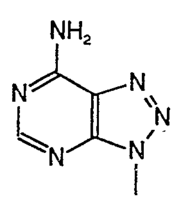

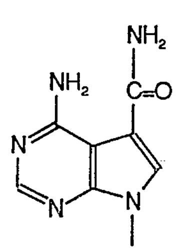

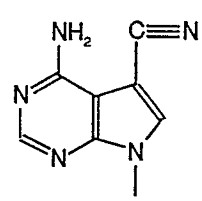

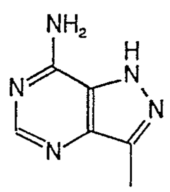

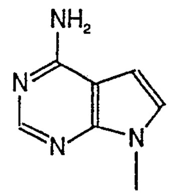

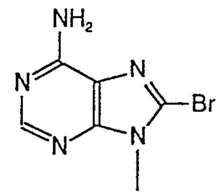

4. Process according to claim 1 for the preparation of oligonucleotides of formula (V):

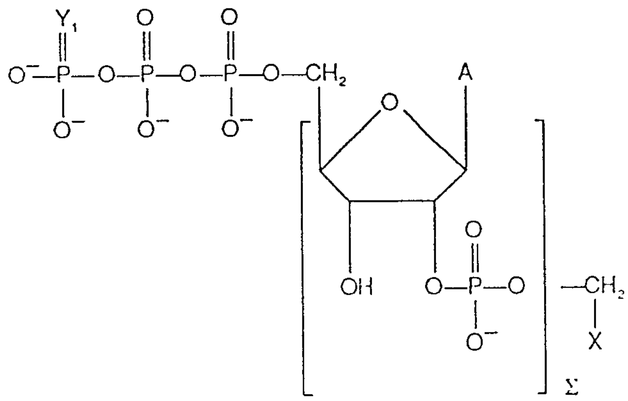

(V)

in which: - $Y_1$ represents NH, Se, S; - X represents:

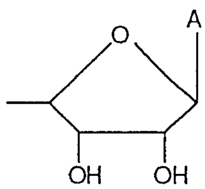

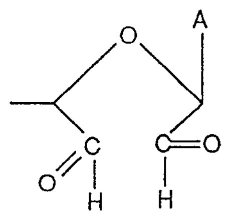

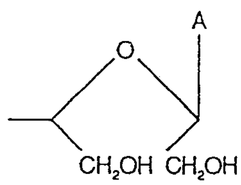

$-CHOHCH_2OH$

- Σ is an integer equal to n, when X represents $-CHOHCH_2OH$, n-1 when X is different from $-CHOHCH_2OH$, n varying from 2 to 10;

characterized in that the compound of the following formula (XXIV):

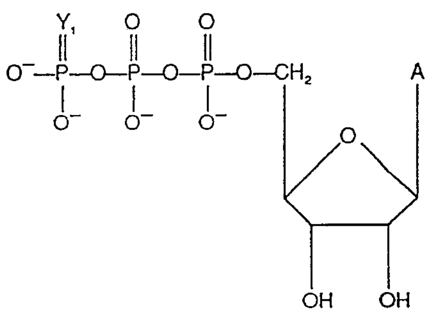

(XXIV)

is polymerized and the steps 2), 3) and 4) are carried out as mentioned in claim 1.

5. Process according to claim 1 for the preparation of oligonucleotides of formula (VI):

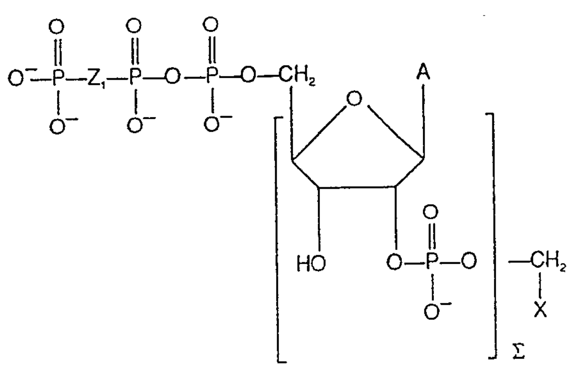

(VI)

in which:
- $Z_1$ represents S or NH; - X is chosen in the group constituted by:

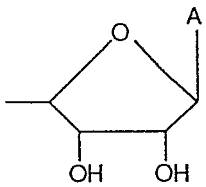
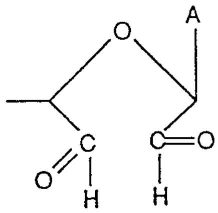
.
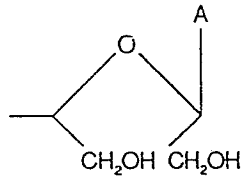
$—CHOHCH_2OH$

- Σ is an integer equal to 1, when X represents $-CHOHCH_2OH$, and equal to n–1 when X is different from $-CHOHCH_2OH$, n varying from 2 to 10;

characterized in that the compound of the following formula (XXV):

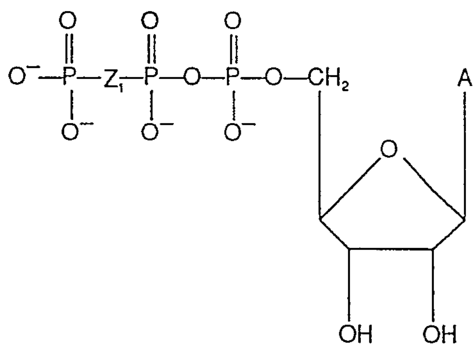
(XXV)

is polymerized
- $Z_1$ representing S or NH and A having the meaning mentioned in claim 1;
and that the steps 2), 3) and 4) are carried out as mentioned in claim 1.

6. Process according to claim 1 for the preparation of oligonucleotides of formula (IV):

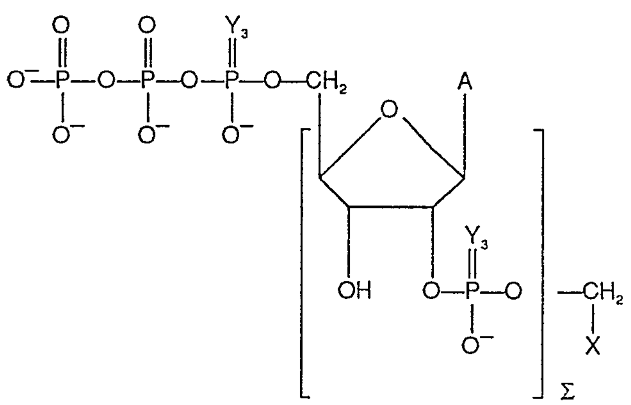
(IV)

in which:
- $Y_3$ represents NH, S or Se;
- Σ, X and A have the meanings above mentioned;
characterized in that the compound of the following formula (XXVI):

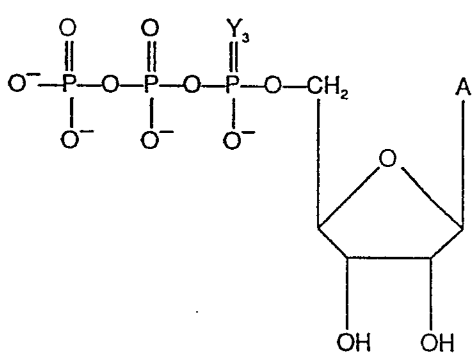
(XXVI)

is polymerized:
- $Y_3$ representing S, Se, NH;
- A has the meaning above mentioned;
and that the steps 2), 3) and 4) are carried out as mentioned in claim 1.

7. Process according to claim 1 for the preparation of oligonucleotides of the following formula (Vbis):

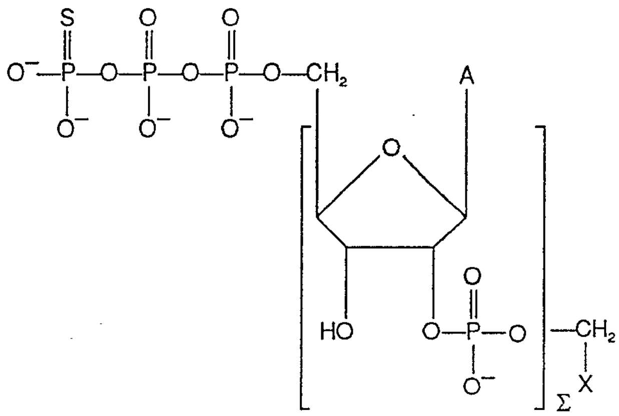

(V bis)

in which: - X represents:

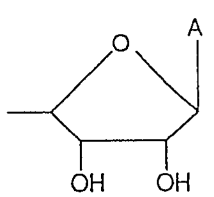

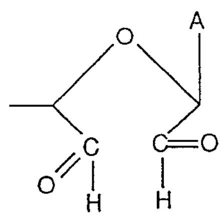

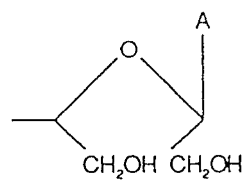

—$CHOHCH_2OH$

- Σ is an integer equal to n, when X represents -$CHOHCH_2OH$, n-1 when X is different from -$CHOHCH_2OH$,
- A has the meaning mentioned in claim 1, preferably adenine;

characterized in that the compound of the following formula (XXIVbis):

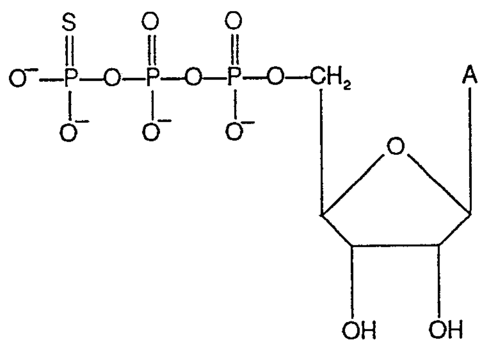

(XXIV bis)

is polymerized
and in that the steps 2), 3) and 4) are carried out as mentioned in claim 1.

8. Process for the preparation of oligonucleotides, according to claim 1, of formula (VII):

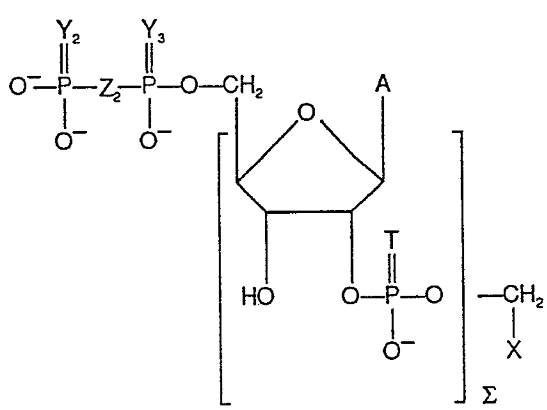

(VII)

in which:
- $Y_2$, $Y_3$, T are identical or different and represent O, S, Se, NH;
- $Z_2$ represents O, S, NH;

one at least of the $Y_2$, $Y_3$, $Z_2$ elements being different from oxygen;
- X is chosen in the group constituted by:

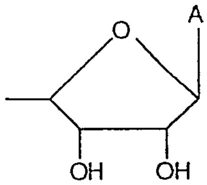
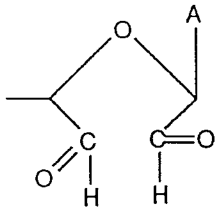
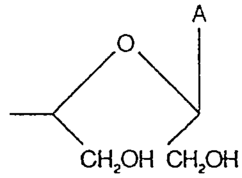
$—CHOHCH_2OH$

Σ is an integer equal to n when X represents $-CHOHCH_2OH$, and is an integer equal to n−1 when X is different from $-CHOHCH_2OH$, n being an integer varying from 2 to 10;
- A is a base chosen among adenine or its derivatives, particularly those of formula:

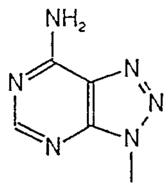
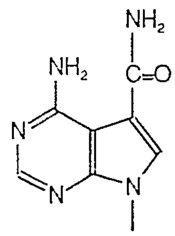
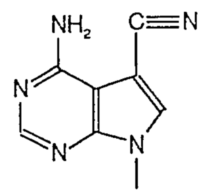
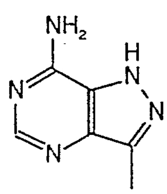
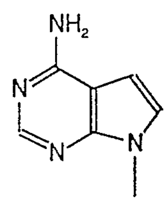
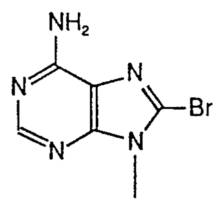

9. Process for the preparation of oligonucleotides, according to claim 1, of formula (VIII):

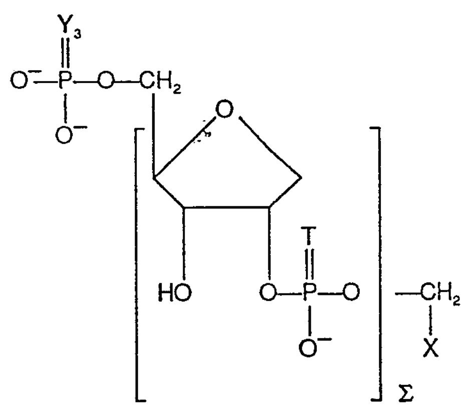
(VIII)

in which:
- $Y_3$ represents S, Se, NH;
- T represents O, S, Se, NH;
- X is chosen in the group constituted by:

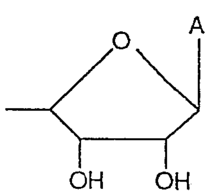
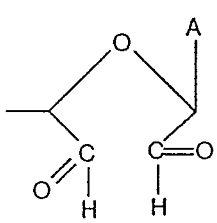
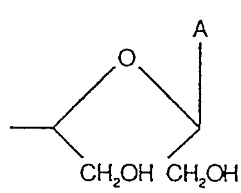
$—CHOHCH_2OH$

– Σ represents an integer equal to n when X represents $-CHOHCH_2OH$, and an integer equal to n–1 when X is different from $-CHOHCH_2OH$, n being an integer varying from 2 to 10;

– A is a base chosen among adenine or its derivatives, particularly those of formula:

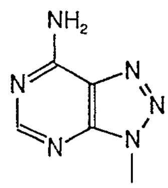

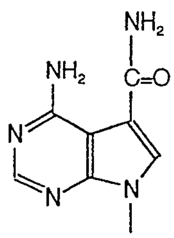

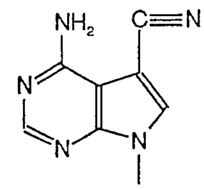

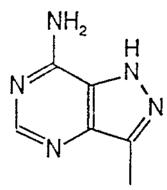

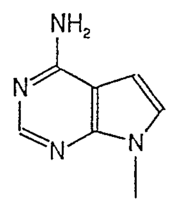

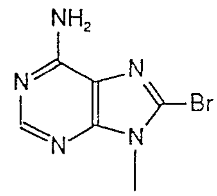

10. Process for the preparation of oligonucleotides according to anyone of the preceeding claims, in which T represents oxygen.

11. Process for the preparation of oligonucleotides, according to claim 1, of formulae:

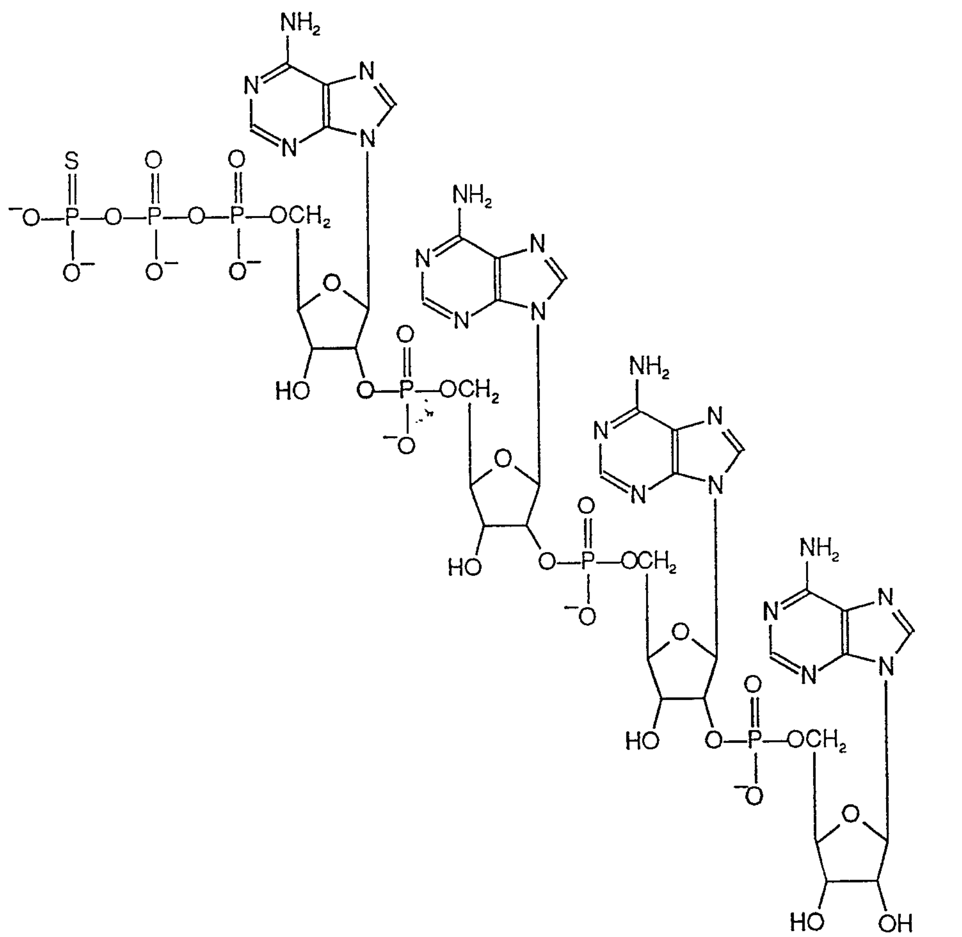

(XV)

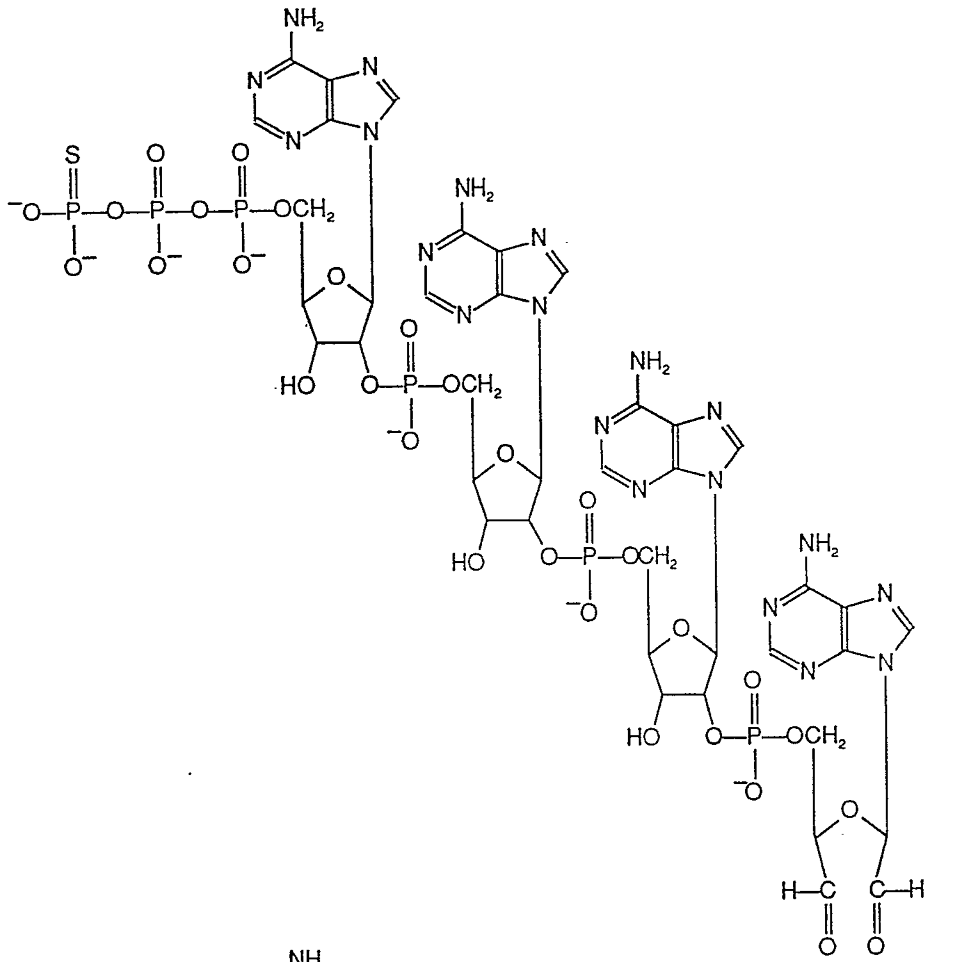

(XVI)

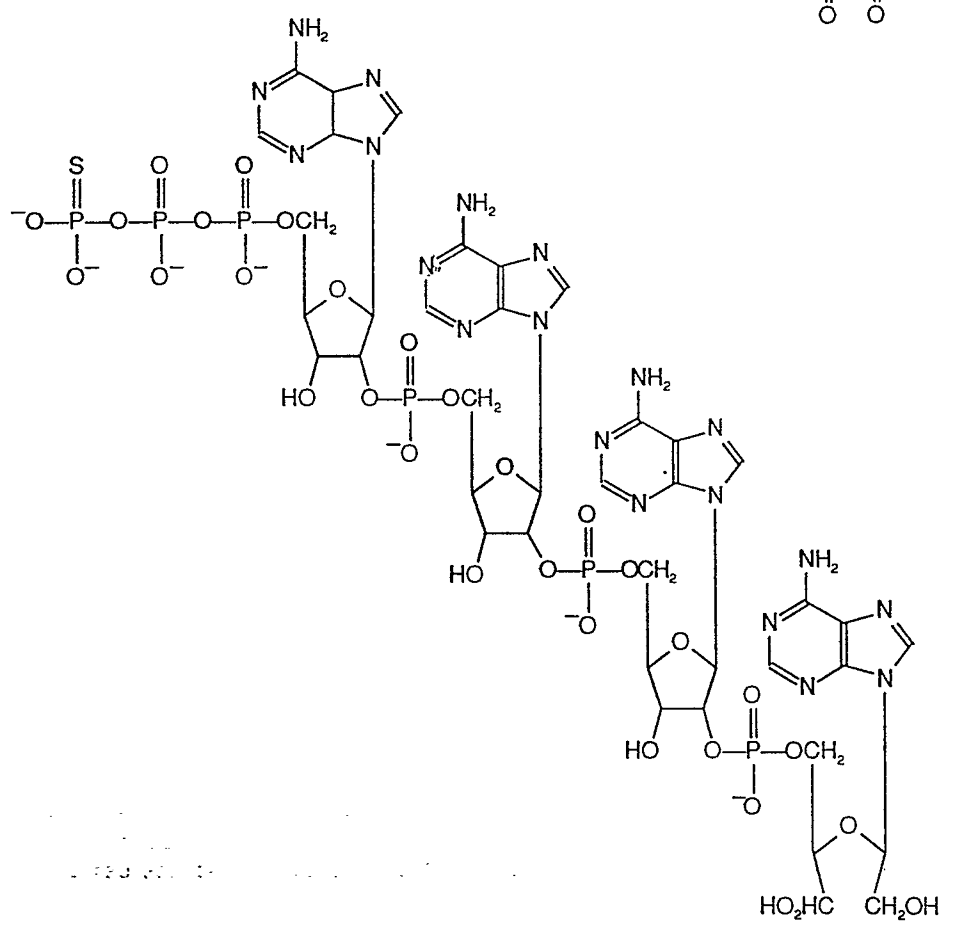

(XVII)

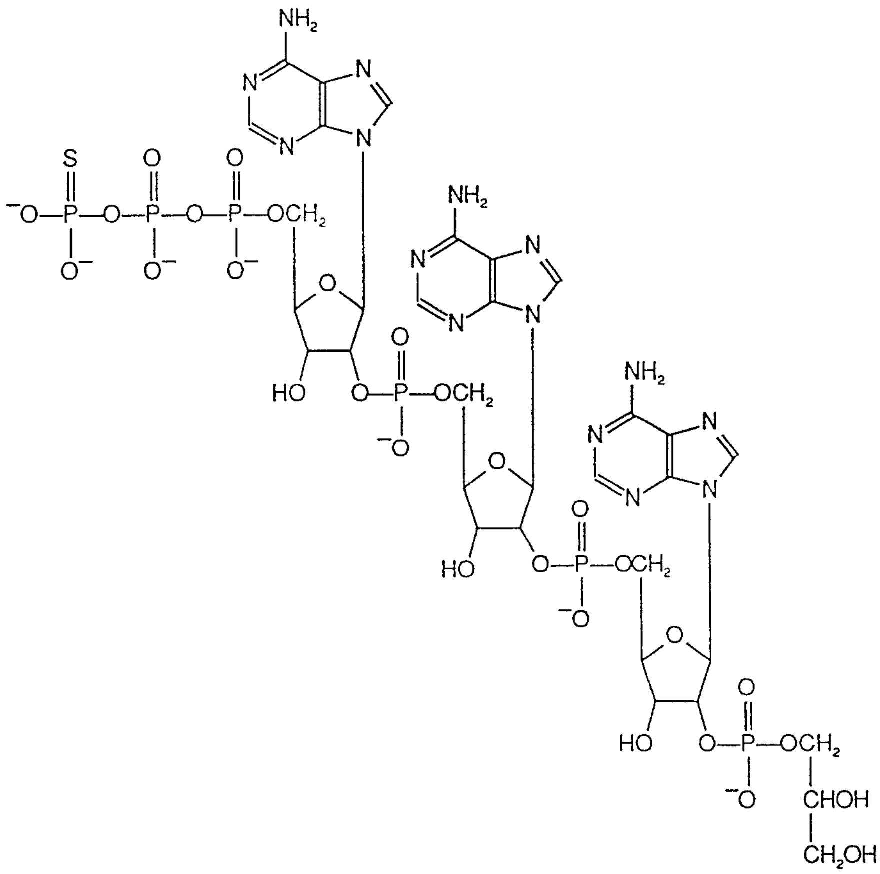

(XVIII)

**Claims for the Contracting States BE, CH, DE, GB, IT, LI, LU, NL, SE**

1. Oligonucleotides of general formula (I):

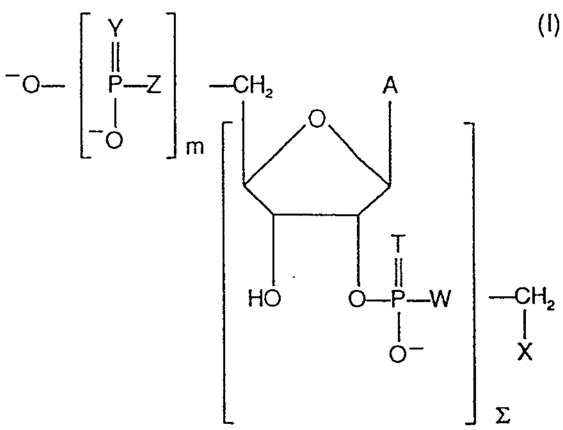

(I)

in which:
- Y and T are identical or different and represent O, S, Se, NH;
- Z and W are identical or different and represent O, S, NH;
- one at least of the Y and Z elements of at least one of the phosphate groups being different from oxygen;
- X is chosen in the group constituted by:

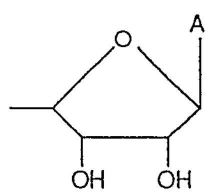

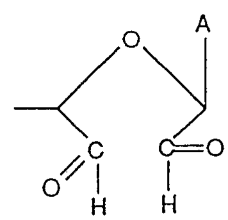

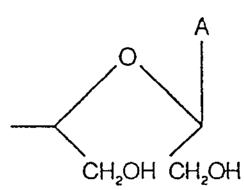

$-CHOHCH_2OH$

the alcohol functions of these radicals being possibly esterified by carboxylic aids $R_3COOH$, $R_3$ representing an alkyl radical from 1 to 5 carbon atoms or a phenyl group;
- Σ is an integer equal to:
n when X represents $-CHOHCH_2OH$,
n−1 when X represents:

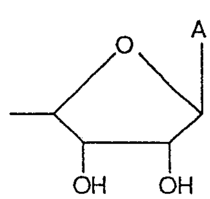

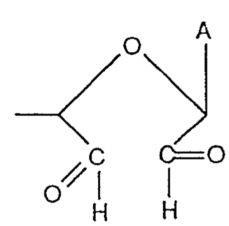

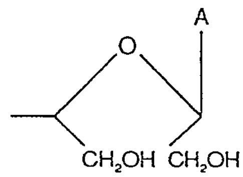

n being an integer greater than or equal to 2;
- m is an integer greater than or equal to 1;
- A is a base chosen among adenine or its derivatives, particularly those of formula:

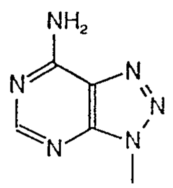
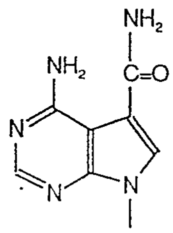
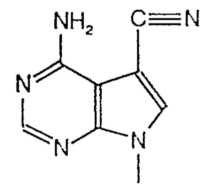
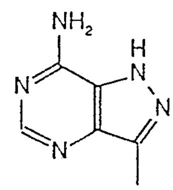
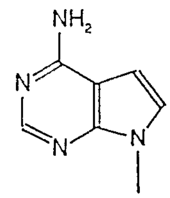
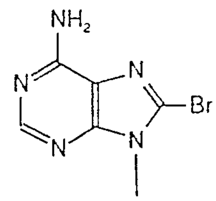

2. Oligonucleotides according to claim 1 having the general formula (II):

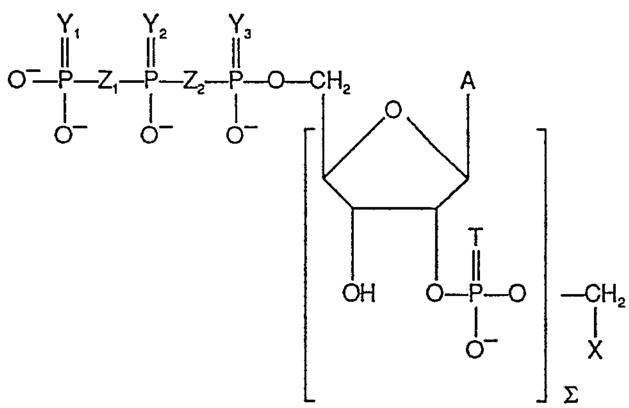

(II)

in which:
- $Y_1$, $Y_2$, $Y_3$, T are identical or different and represent O, S, Se, NH;
- $Z_1$ and $Z_2$ are identical or different and represent O, S, NH;
one at least of the $Y_1$, $Y_2$, $Y_3$, $Z_1$, $Z_2$ elements being different from oxygen;
- X is chosen among the group constituted by:

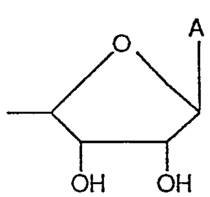
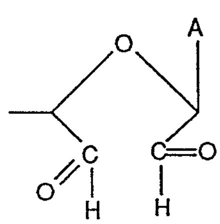
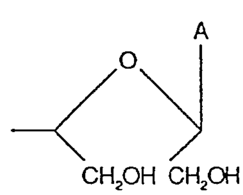
$-CHOHCH_2OH$

the alcohol functions of those radicals being possibly esterified by carboxylic acids $R_3COOH$, $R_3$ representing an alkyl radical from 1 to 5 carbon atoms or a phenyl group;
- Σ represents an integer equal to n when X represents $-CHOHCH_2OH$, and Σ represents an integer equal to n-1 when X is different from $-CHOHCH_2OH$, n being an integer greater than or equal to 2;
- A is a base chosen among adenine or its derivatives, particularly those of formula:

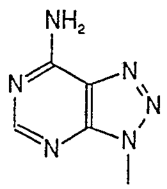
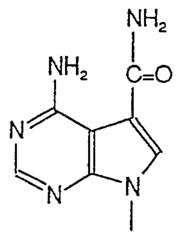
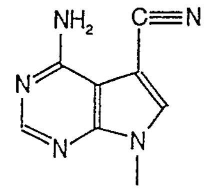
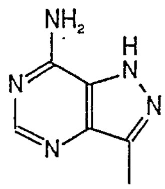
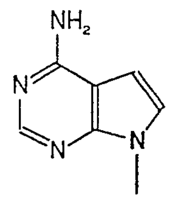
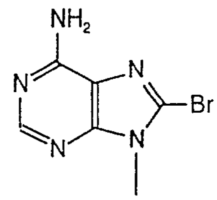

3. Oligonucleotides according to claim 1 of formula (VII):

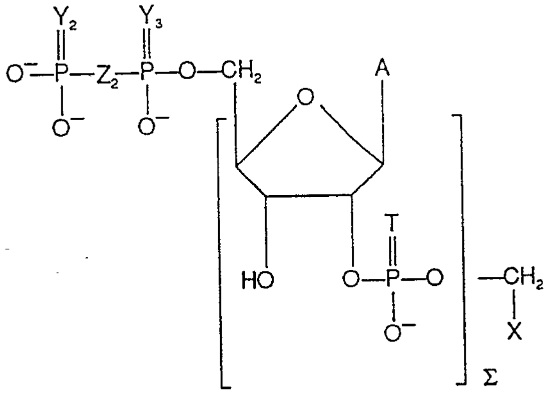

(VII)

in which:
- $Y_2$, $Y_3$, T are identical or different and represent O, S, Se, NH;
- $Z_2$ represents O, S, NH;
one at least of the $Y_2$, $Y_3$, $Z_2$ elements being different from oxygen;
- X is chosen in the group constituted by:

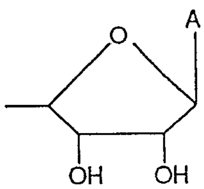
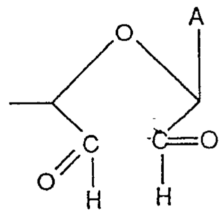
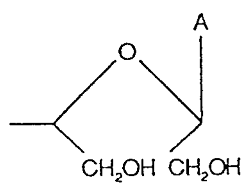
$—CHOHCH_2OH$

- Σ is an integer equal to n when X represents $-CHOHCH_2OH$, and is an integer equal to n-1 when X is different from $-CHOHCH_2OH$, n being an integer varying from 2 to 10;
- A is a base chosen among adenine or its derivatives, particularly those of formula:

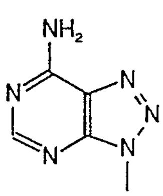
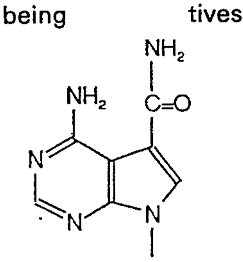
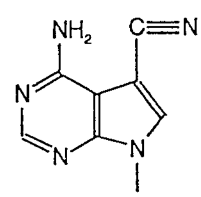
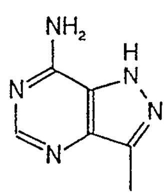
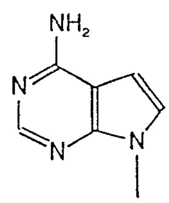
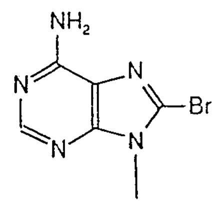

4. Oligonucleotides according to claim 1 of formula (VIII):

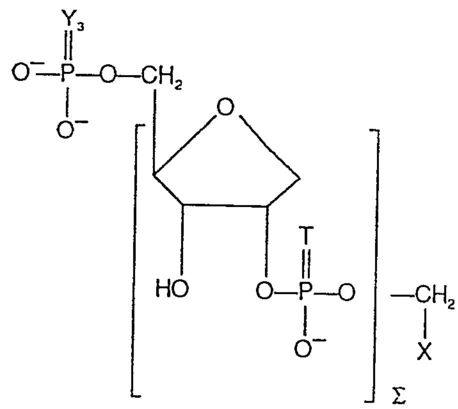

(VIII)

in which:
- $Y_3$ represents S, Se, NH;
- T represents O, S, Se, NH;
- X is chosen in the group constituted by:

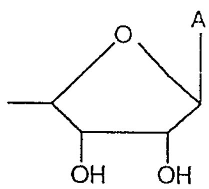

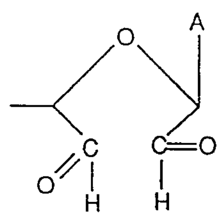

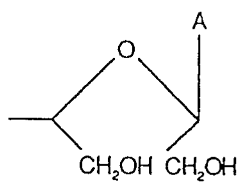

$-CHOHCH_2OH$

- Σ represents an integer equal to n when X represents $-CHOHCH_2OH$, and an integer equal to n−1 when X is different from $-CHOHCH_2OH$, n being an integer varying from 2 to 10;
- A is a base chosen among adenine or its derivatives, particularly those of formula:

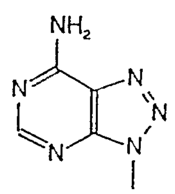

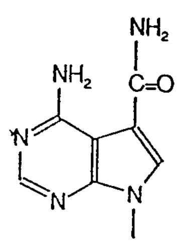

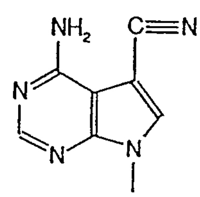

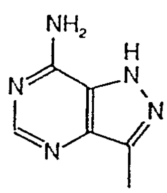

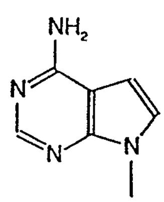

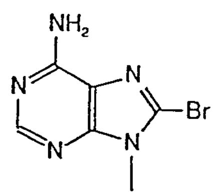

5. Oligonucleotides according to anyone of the preceeding claims, in which T represents oxygen.

6. Oligonucleotides according to claim 1 of formulae:

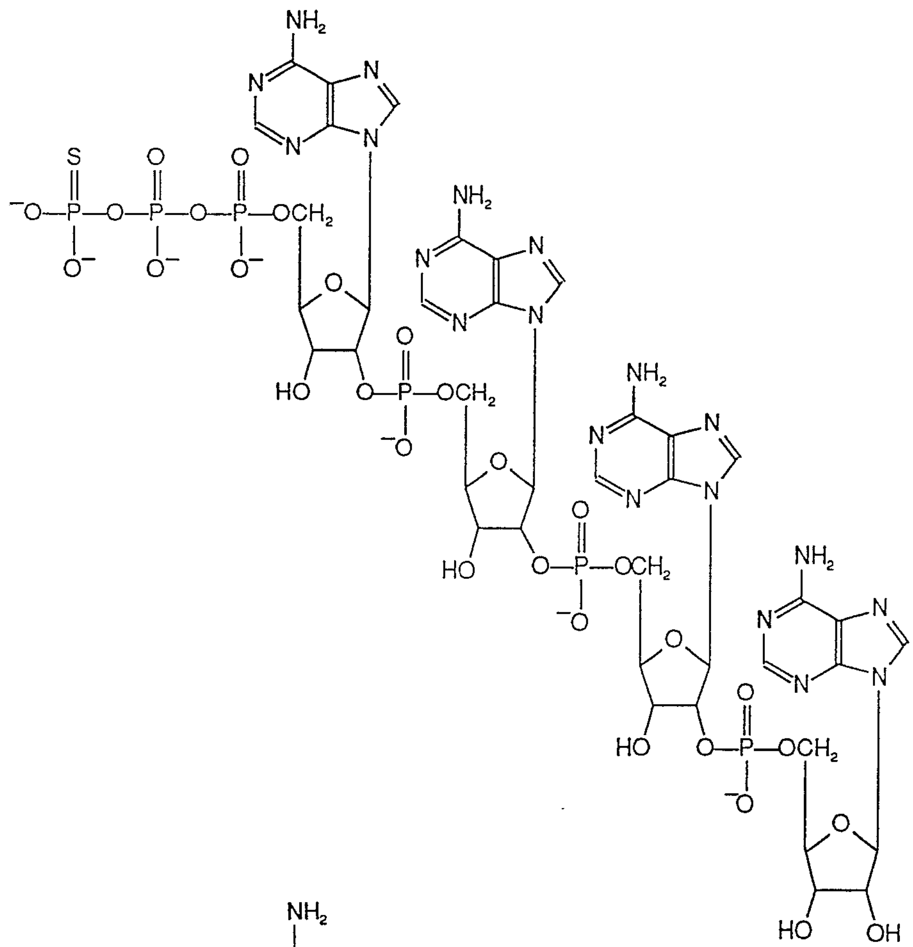

(XV)

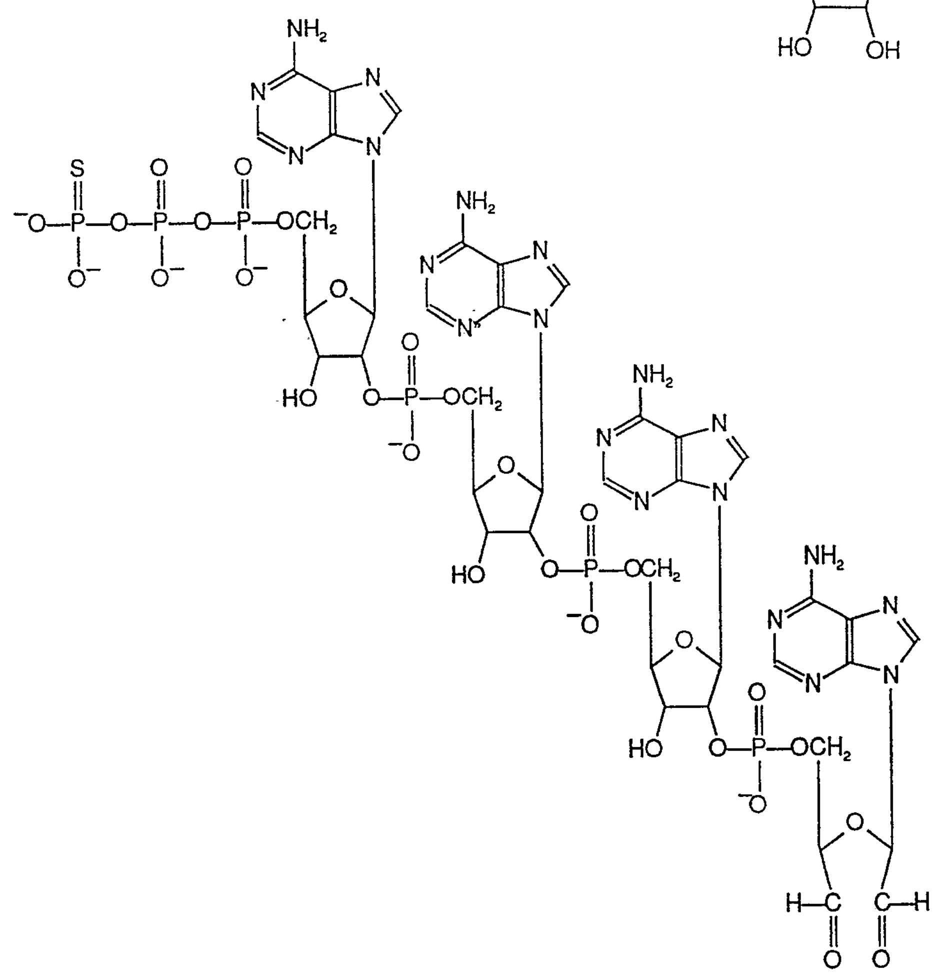

(XVI)

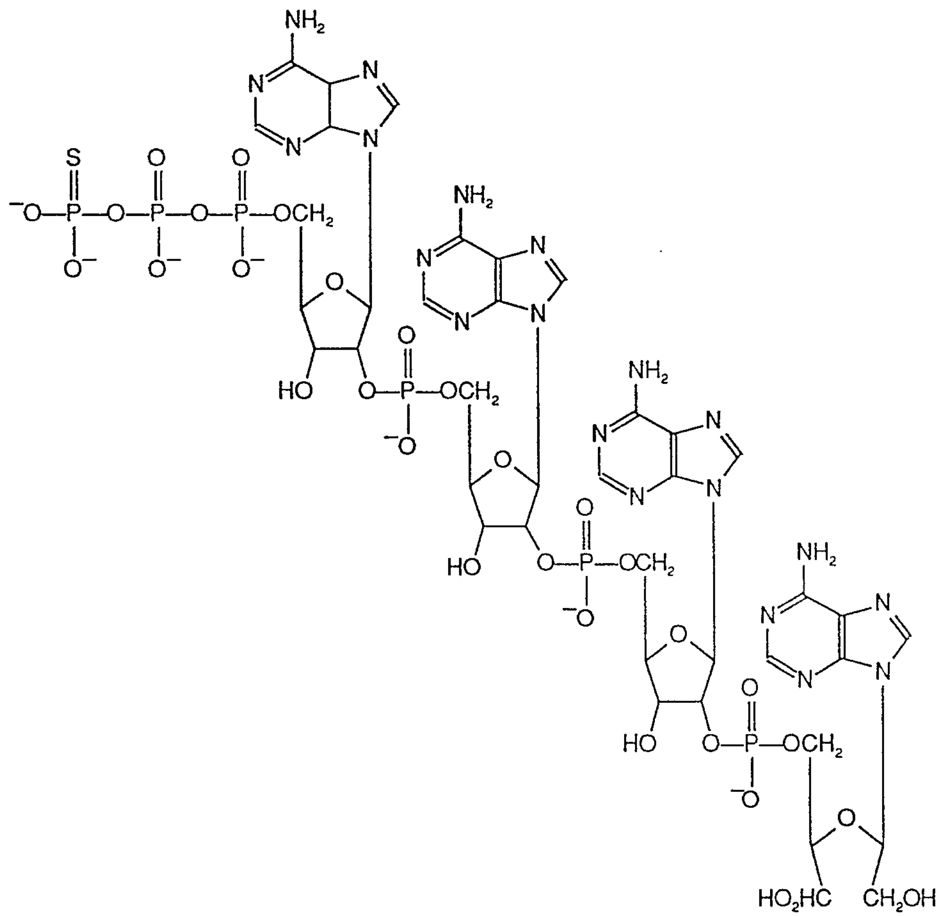

(XVII)

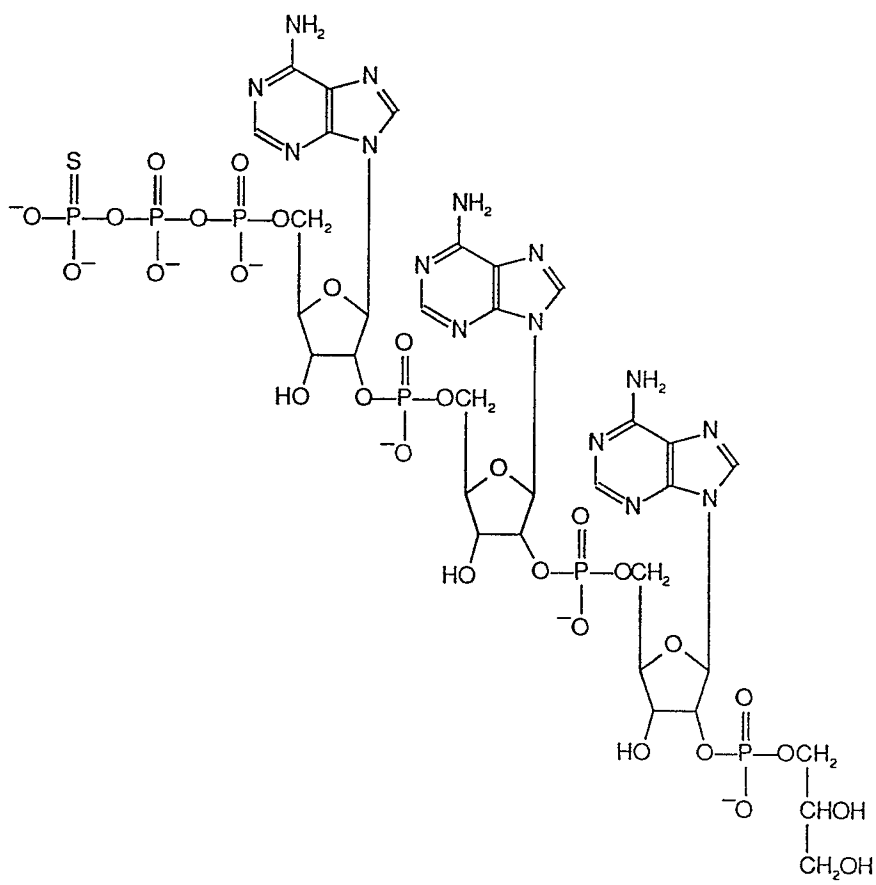

(XVIII)

7. Process for the preparation of oligonucleotides of formula (I) according to claim 1:

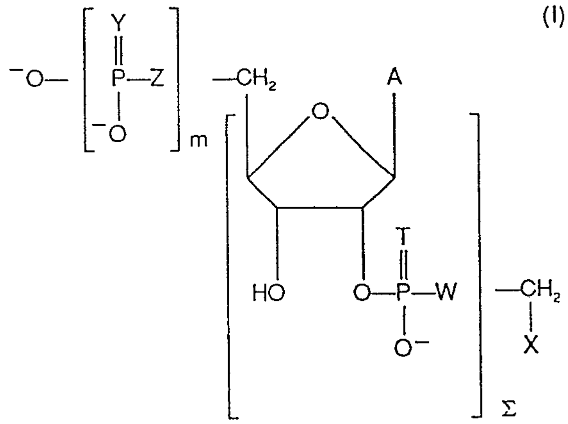

(I)

in which:
- Y and T are identical or different and represent O, S, Se or NH;
- Z and W are identical or different and represent O, S or NH;
- one at least of the Y and Z elements of at least one of the phosphate groups being different from oxygen;
- m is greater than or equal to 1;
- A and X have the meanings mentioned in claim 1, characterized in that it comprises:
- the polymerization of compounds of the following formula (XIX):

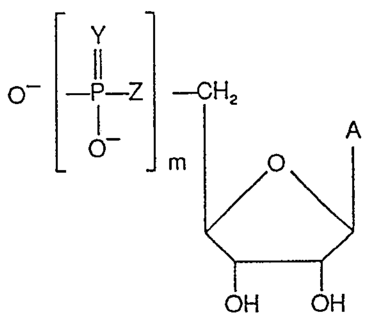

(XIX)

to obtain a compound of the following formula (Ibis):

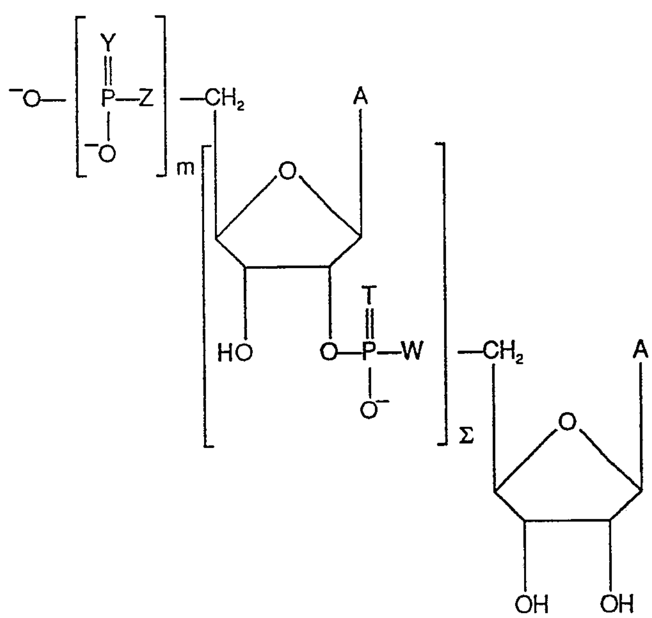

(I bis)

in which:
- Y and T are identical or different and represent O, S, Se or NH;
- Z and W are identical or different and represent O, S or NH;
- one at least of the Y and Z elements of at least

one of the phosphate groups being different from oxygen;
- $\Sigma$ is an integer equal to n-1, n being greater than or equal to 2;
- m is an integer greater than or equal to 1;
- A has the above mentioned meanings,

- and if necessary the following chemical steps i.e.:
the possible oxidation of the glycol group to introduce aldehyde functions on to the carbons at 2′ and 3′ positions of the last nucleosidic unit and to obtain the compound of formula (Iter):

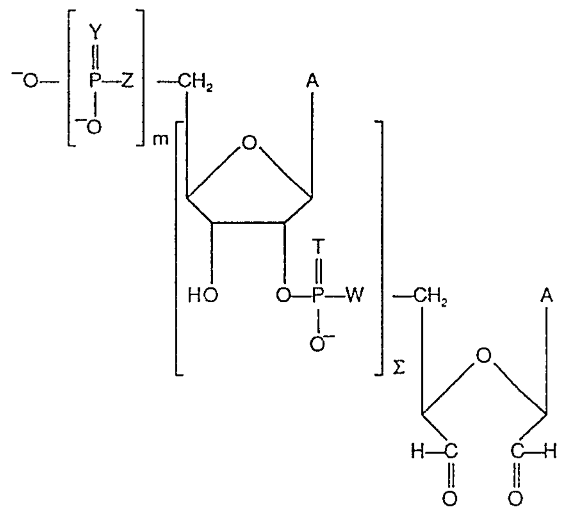

(I ter)

- the possible reduction of the two aldehyde functions into alcohol functions to obtain the compound of formula (Iquater):

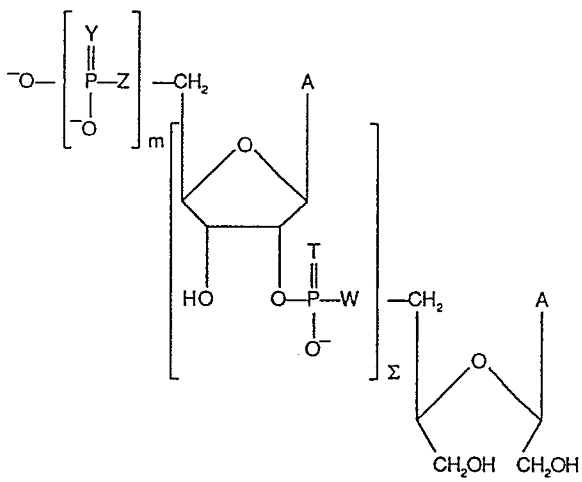

(I quater)

- the possible hydrolysis under conditions avoiding beta-eleminiation, to obtain a compound of the following formula (Iquinquiès):

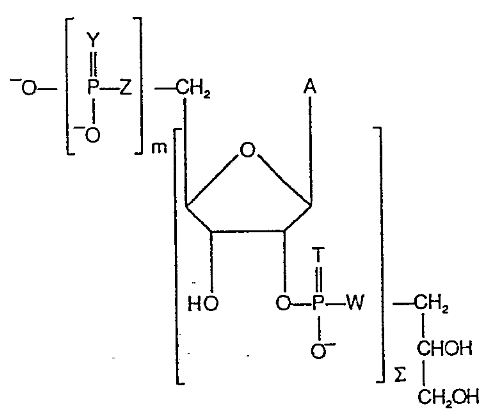

(I quinquies)

8. Process according to claim 7 for the preparation of oligonucleotides of formula (XX):

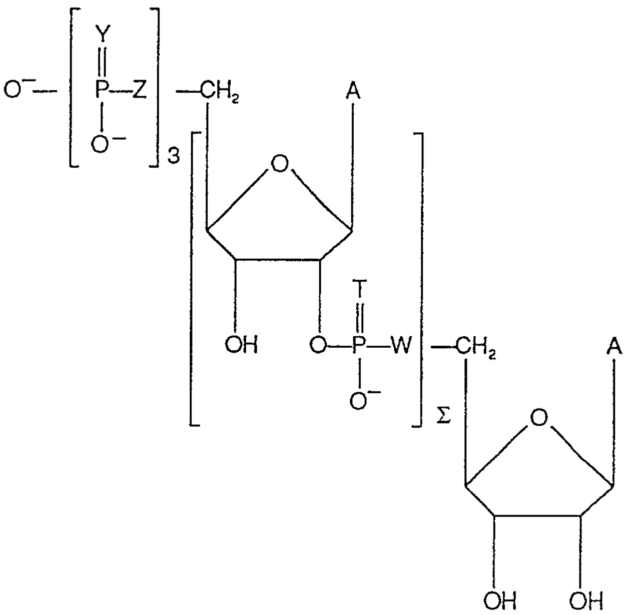

(XX)

in which:

- Y and T are identical or different and represent O, S, Se, NH;
- Z and W are identical or different and represent O, S, NH;
- the Y and Z elements of at least one of the phosphate groups representing not simultaneously oxygen;
- Σ is an integer varying from 1 to 9;
- A is a base chosen among adenine or its derivatives particularly those of formula:

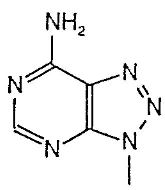

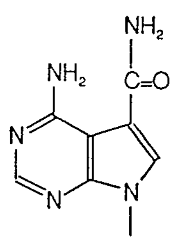

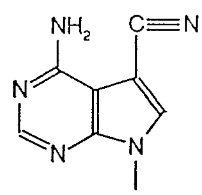

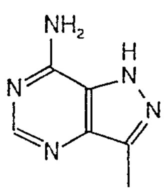

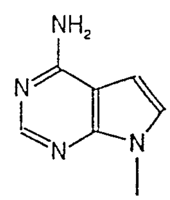

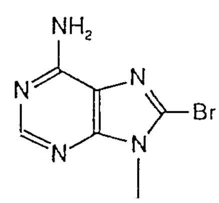

characterized in that it comprises:

1) the polymerization of a compound of the following formula (XIXbis):

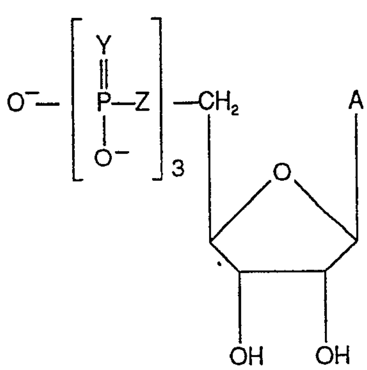

(XIX bis)

in which:
- Y represents O, S, Se or NH;
- Z represents O, S or NH;
- the Y and Z elements of at least one of the phosphate groups representing not simultaneously oxygen;
- A has the meaning mentioned in claim 1;

2) the possible oxidation of the terminal glycol group, particularly by periodate ion to transform the glycol in two aldehyde functions and obtain a compound for the following formula (XXI):

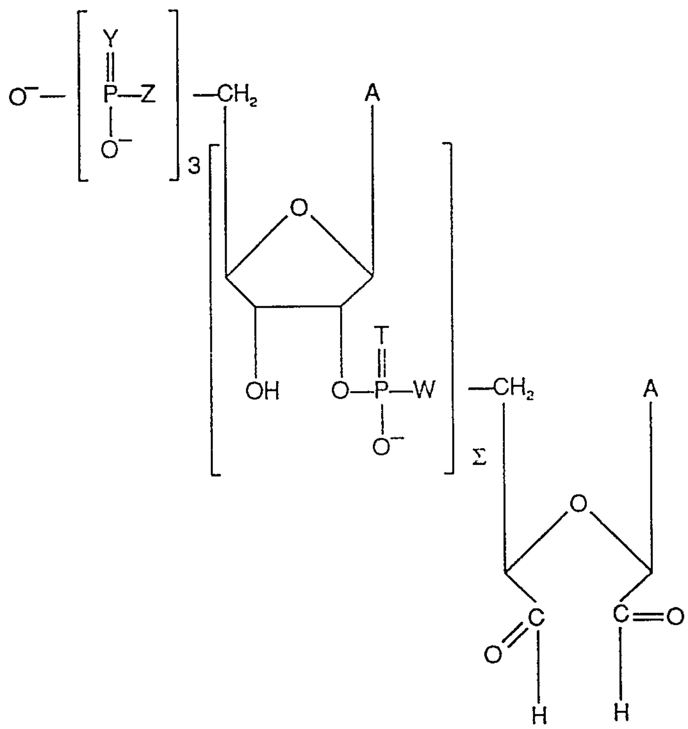

(XXI)

in which:
- Y, Z, T, W, Σ and A have the meanings above mentioned;

3) the possible reduction of the aldehyde functions, particularly by sodium borohydride to transform the two above mentioned aldehyde functions into alcohol functions and to obtain the compound of the following formula (XXII):

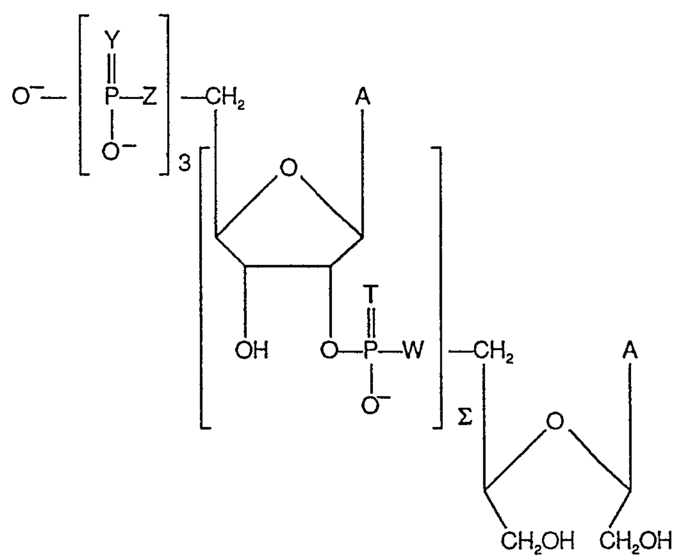

(XXII)

4) the possible hydrolysis, particularly controlled acid hydrolysis, to eliminate the ribose nucleus and obtain the compound of the following formula (XXIII):

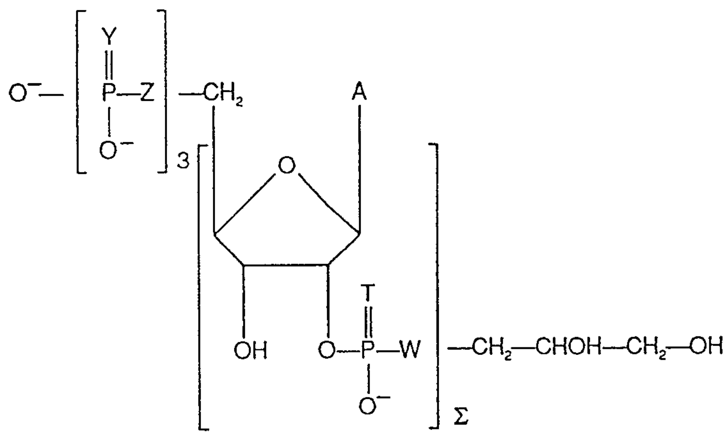

(XXIII)

9. Process according to claim 7 for the preparation of oligonucleotides of formula (V):

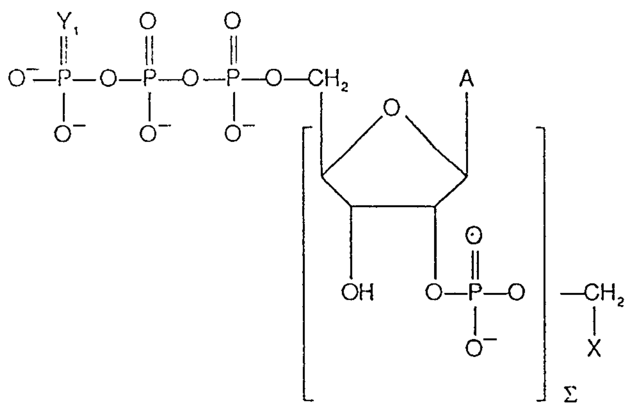

(V)

in which: - $Y_1$ represents NH, Se, S; - X represents:

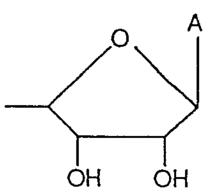

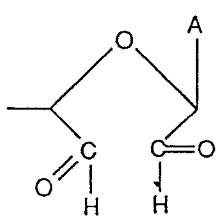

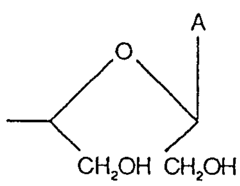

$-CHOHCH_2OH$

- $\Sigma$ is an integer equal to n, when X represents $-CHOHCH_2OH$, n-1 when X is different from $-CHOHCH_2OH$, n varying from 2 to 10;

is characterized in that the compound of the following formula (XXIV):

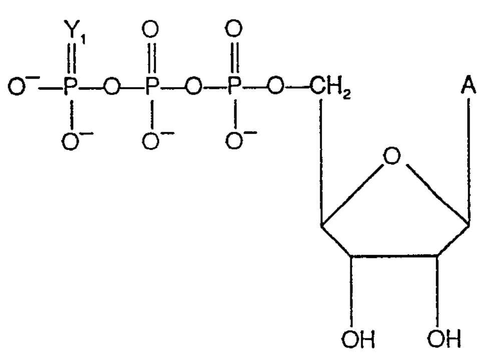

(XXIV)

is polymerized and the steps 2), 3) and 4) are carried out as mentioned in claim 7.

10. Process according to claim 7 for the preparation of oligonucleotides of formula (VI):

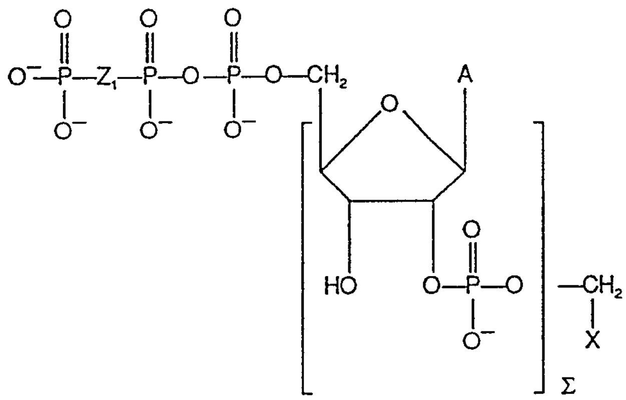

(VI)

in which:
- $Z_1$ represents S or NH; - X is chosen in the group constituted by:

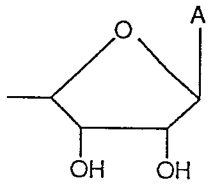

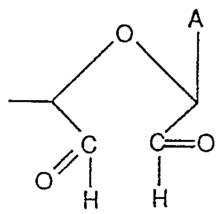

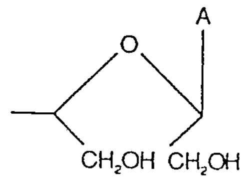

$—CHOHCH_2OH$

- Σ is an integer equal to 1, when X represents $-CHOHCH_2OH$, and equal to n–1 when X is different from $-CHOHCH_2OH$, n varying from 2 to 10;

characterized in that the compound of the following formula (XXV):

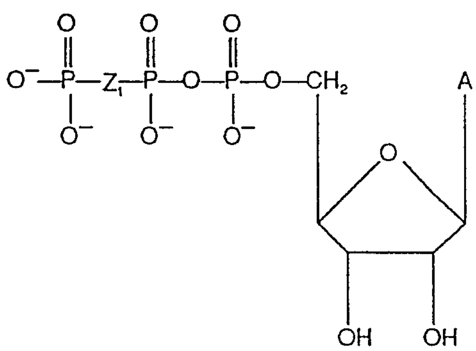

(XXV)

is polymerized
$Z_1$ representing S or NH and A having the meaning mentioned in claim 1;
and that the steps 2), 3) and 4) are carried out as mentioned in claim 7.

11. Process according to claim 7 for the preparation of oligonucleotides of formula (IV):

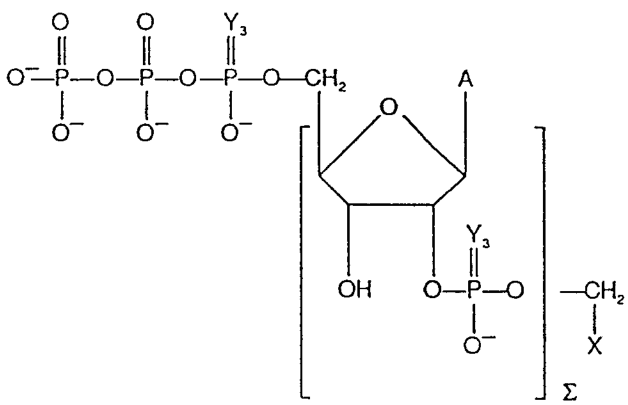

(IV)

in which:
- $Y_3$ represents NH, S or Se;
- Σ, X and A have the meanings above mentioned;

characterized in that the compound of the following formula (XXVI):

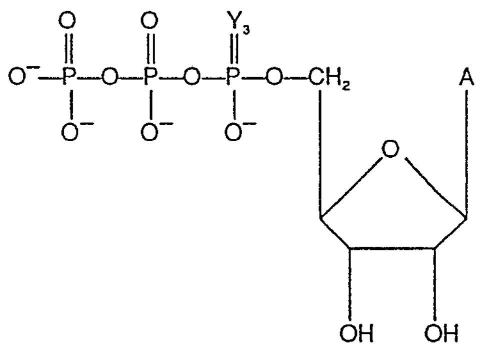

(XXVI)

is polymerized:

- $Y_3$ representing S, Se, NH;
- A has the meaning above mentioned;

and that the steps 2), 3) and 4) are carried out as mentioned in claim 7.

12. Process according to claim 7 for the preparation of oligonucleotides of the following formula (Vbis):

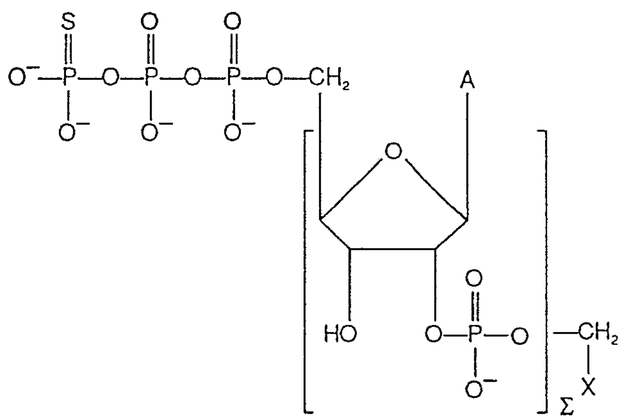

(V bis)

in which: - X represents:

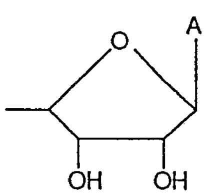 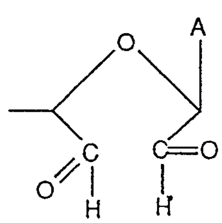 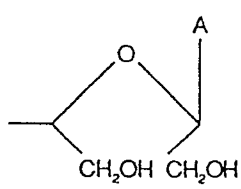 $—CHOHCH_2OH$

- Σ is an integer equal to n, when X represents $-CHOHCH_2OH$, n-1 when X is different from $-CHOHCH_2OH$,
- A has the meaning mentioned in claim 1, preferably adenine;

characterized in that the compound of the following formula (XXIVbis):

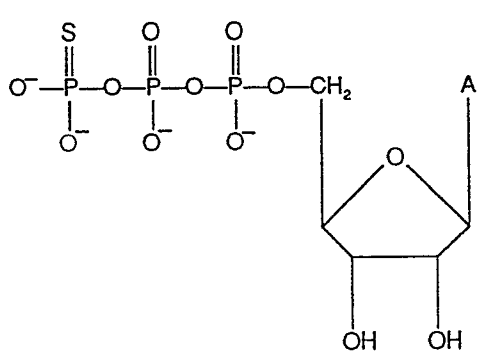

(XXIV bis)

is polymerized

and in that the steps 2), 3) and 4) are carried out as mentioned in claim 7.

FIG.1.

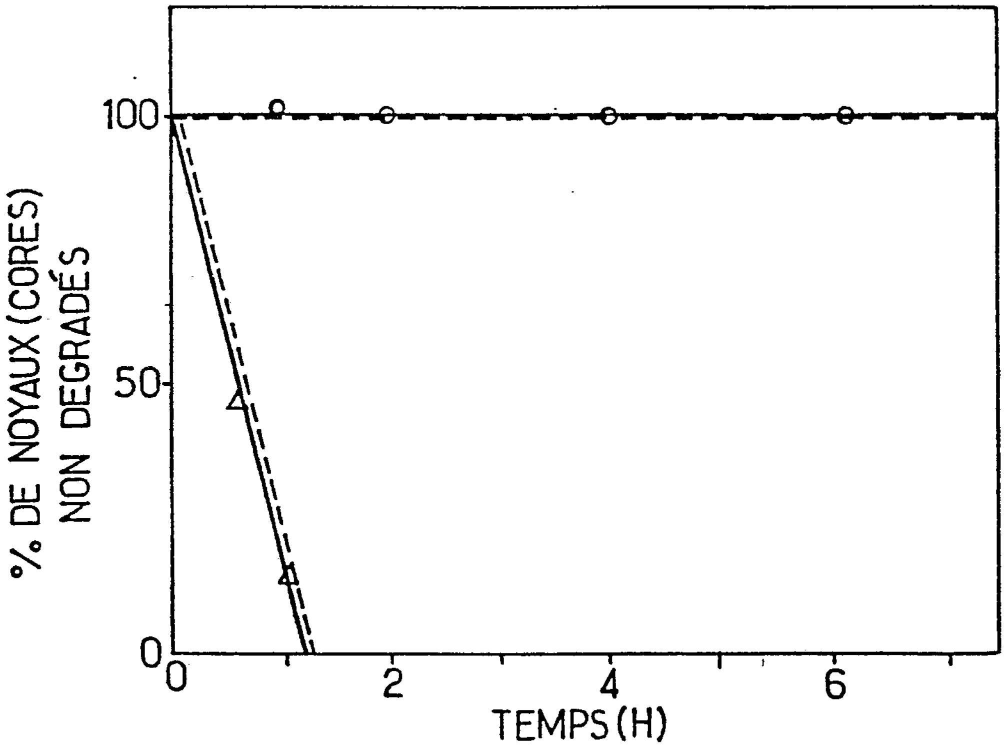

FIG.2.

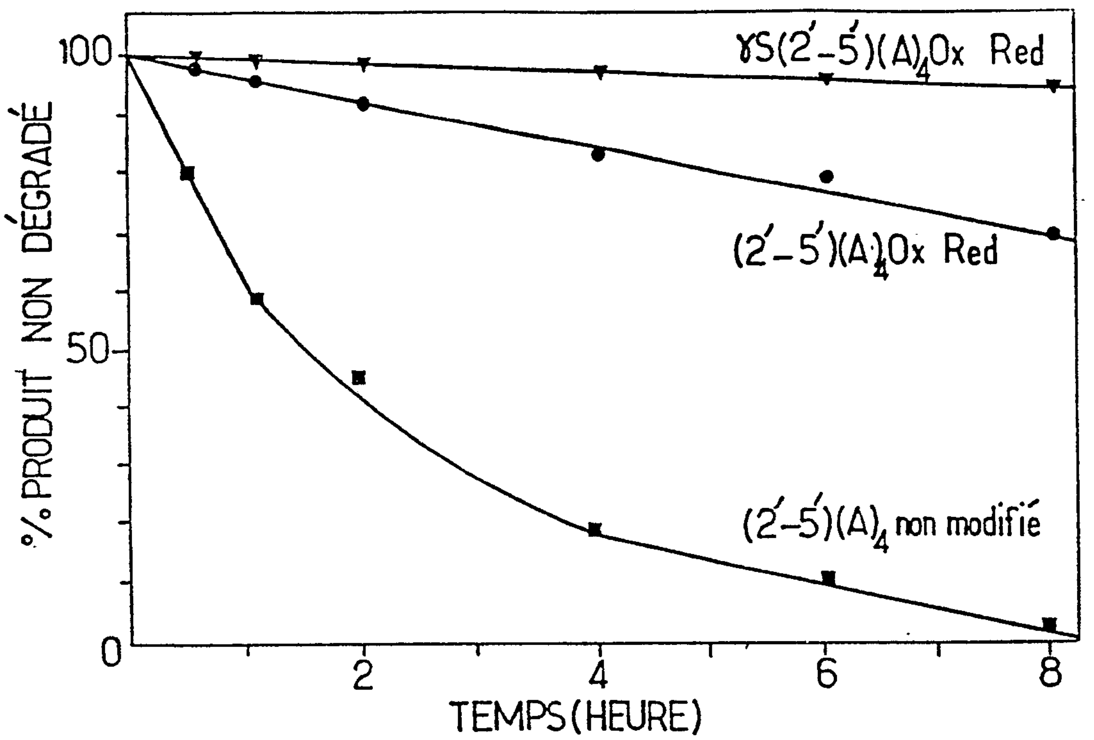
100
50
0
% PRODUIT NON DÉGRADÉ
2
4
6
8
TEMPS (HEURE)
γS(2'-5')(A)4Ox Red
(2'-5')(A)4Ox Red
(2'-5')(A)4 non modifié

FIG.3.

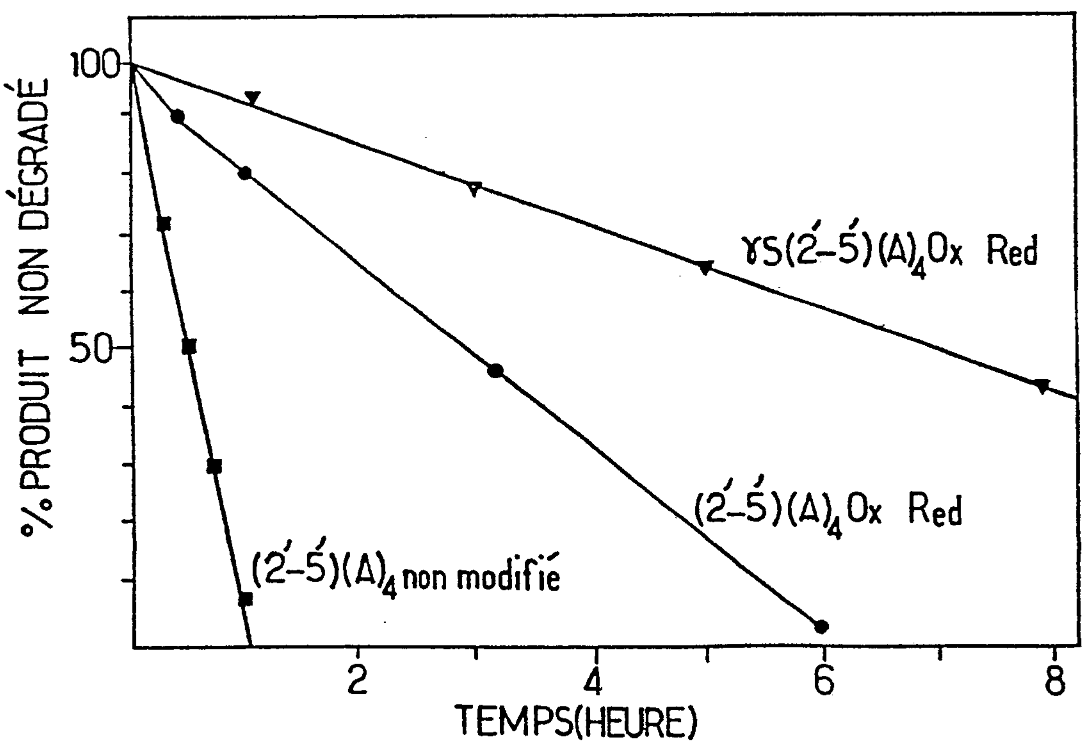
100
50
% PRODUIT NON DÉGRADÉ
2
4
6
8
TEMPS(HEURE)
γS(2'-5')(A)4Ox Red
(2'-5')(A)4Ox Red
(2'-5')(A)4 non modifié